# EUROPEAN PATENT SPECIFICATION

(11) **EP 2 068 907 B1**
(45) Date of publication and mention of the grant of the patent: **29.11.2017**
(21) Application number: 07868373.7
(22) Date of filing: 04.10.2007
(51) Int. Cl.: C07K 14/00, C07K 16/00

(54) **GLYCEROL LINKED PEGYLATED SUGARS AND GLYCOPEPTIDES**
MIT GLYCERIN VERBUNDENE PEGYLIERTE ZUCKER UND GLYCOPEPTIDE
GLYCOPEPTIDES ET SUCRES PEGYLES À LIAISON GLYCEROL

(30) Priority: 04.10.2006 US 828208 P
(43) Date of publication of application: 17.06.2009
(73) Proprietor: Novo Nordisk A/S, 2880 Bagsværd (DK)
(72) Inventor: DEFREES, Shawn, North Wales, PA 19454 (US); ZENG, Xiao, Warrington, PA 18976 (US)
(74) Representative: Engelhard, Markus
(86) International application number: PCT/US2007/080471
(87) International publication number: WO 2008/060780

(56) References cited:
- WO-A1-2006/082517
- WO-A2-2005/051327
- WO-A2-2005/072371
- WO-A2-2007/056191
- US-B2- 7 125 843
- DEFREES S ET AL: "GlycoPEGylation of recombinant therapeutic proteins produced in Escherichia coli", GLYCOBIOLOGY, OXFORD UNIVERSITY PRESS, US, vol. 16, no. 9, 1 September 2006 (2006-09-01), pages 833-843, XP002403450, ISSN: 0959-6658, DOI: 10.1093/GLYCOB/CWL004
- C. NEGRIER ET AL: "Enhanced pharmacokinetic properties of a glycoPEGylated recombinant factor IX: a first human dose trial in patients with hemophilia B", BLOOD, vol. 118, no. 10, 8 September 2011 (2011-09-08), pages 2695-2701, XP055265689, US ISSN: 0006-4971, DOI: 10.1182/blood-2011-02-335596
- P. W. COLLINS ET AL: "Recombinant long-acting glycoPEGylated factor IX in hemophilia B: a multinational randomized phase 3 trial", BLOOD, vol. 124, no. 26, 18 December 2014 (2014-12-18), pages 3880-3886, XP055173746, ISSN: 0006-4971, DOI: 10.1182/blood-2014-05-573055

## Description

The present application claims priority to U.S. Provisional Patent Application No. 60/828,208, filed on October 4, 2006.

WO 2005/072371, DeFrees et al., Glycobiology 16: 833-843 (2006) and US 7,125,843 disclose peptides modified by means of PEG moieties, which are attached via sugar-containing linkages.

WO 2006/082517 discloses the PEGylation of the specific peptide PPY₃₋₃₆, using linear or branched mPEG maleimides, wherein the linear or branched mPEG is conjugated directly to functional groups in the amino acid side chains of PPY_{3-36,} in particular thiol group of cysteines at residues 10 and 11.

WO 2005/051327 discloses glycoPEGylated erythropoietin (EPO).

US 2006/0040856 discloses glycoPEGylated factor IX variants.

### SUMMARY OF THE INVENTION

The present invention relates to peptide conjugates of Factor IX in accordance with the appended claims and to pharmaceutical formulations comprising such conjugates in accordance with the appended claims.

In an exemplary embodiment, "glycopegylated" molecules of the invention are produced by the enzyme mediated formation of a conjugate between a glycosylated or non-glycosylated peptide and an enzymatically transferable saccharyl moiety that includes a modifying group, such as a polymeric modifying group such as poly(ethylene glycol), within its structure. The peptide is Factor IX. The polymeric modifying group is attached to the saccharyl moiety (i.e., through a single group formed by the reaction of two reactive groups) or through a linker moiety, e.g., substituted or unsubstituted alkyl, substituted or unsubstituted heteroalkyl, etc.

Thus, in one aspect, the present invention provides a conjugate between a PEG moiety, *e.g.,* PEG and a peptide that has an *in vivo* activity similar or otherwise analogous to art-recognized therapeutic peptide. In the conjugate of the invention, the PEG moiety is covalently attached to the peptide via an intact glycosyl linking group. Exemplary intact glycosyl linking groups include sialic acid moieties that are derivatized with PEG.

The polymeric modifying group can be attached at any position of a glycosyl moiety on a peptide. Moreover, the polymeric modifying group can be bound to a glycosyl residue at any position in the amino acid sequence of a wild type or mutant peptide.

In an exemplary embodiment, the invention provides a peptide that is conjugated through a glycosyl linking group to a polymeric modifying group

In an exemplary embodiment, the polymeric modifying group is bound to the glycosyl linking group, generally through a heteroatom on the glycosyl core (e.g., N, O), through a linker, L, as shown below: 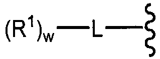 R¹ is the polymeric modifying group and L is selected from a bond and a linking group. The index w represents an integer selected from 1-6, preferably 1-3 and more preferably 1-2. Exemplary linking groups include substituted or unsubstituted alkyl, substituted or unsubstituted heteroalkyl moieties and sialic acid. An exemplary component of the linker is an acyl moiety. Another exemplary linking group is an amino acid residue (e.g., cysteine, serine, lysine, and short oligopeptides, e.g., Lys-Lys, Lys-Lys-Lys, Cys-Lys, Ser-Lys, etc.).

When L is a bond, it is formed by reaction of a reactive functional group on a precursor of R¹ and a reactive functional group of complementary reactivity on a precursor of the glycosyl linking group. When L is a non-zero order linking group, L can be in place on the glycosyl moiety prior to reaction with the R¹ precursor. Alternatively, the precursors of R¹ and L can be incorporated into a preformed cassette that is subsequently attached to the glycosyl moiety. As set forth herein, the selection and preparation of precursors with appropriate reactive functional groups is within the ability of those skilled in the art. Moreover, coupling of the precursors proceeds by chemistry that is well understood in the art.

In an exemplary embodiment L is a linking group that is formed from an amino acid, or small peptide (e.g., 1-4 amino acid residues) providing a modified sugar in which the polymeric modifying moiety is attached through a substituted alkyl linker. Exemplary linkers include glycine, lysine, serine and cysteine. Amino acid analogs, as defined herein, are also of use as linker components. The amino acid may be modified with an additional component of a linker, e.g., alkyl, heteroalkyl, covalently attached through an acyl linkage, for example, an amide or urethane formed through an amine moiety of the amino acid residue.

In an exemplary embodiment, the glycosyl linking group has a structure according to Formulae I or Ia and R⁵ includes the polymeric modifying group. In another exemplary embodiment, R⁵ includes both the polymeric modifying group and a linker, L, joining the polymeric modifying group to the glycosyl core. L can be a linear or branched structure. Similarly, the polymeric modifying group can be branched or linear.

The polymeric modifying group comprises two or more repeating units that can be water-soluble or essentially insoluble in water. Exemplary water-soluble polymers of use in the compounds of the invention include PEG, e.g., m-PEG, PPG, e.g., m-PPG, polysialic acid, polyglutamate, polyaspartate, polylysine, polyethyeleneimine, biodegradable polymers (e.g., polylactide, polyglyceride), and functionalized PEG, e.g., terminal-functionalized PEG.

The glycosyl core of the glycosyl linking groups of use in the peptide conjugates are selected from both natural and unnatural furanoses and pyranoses. The unnatural saccharides optionally include an alkylated or acylated hydroxyl and/or amine moiety, e.g., ethers, esters and amide substituents on the ring. Other unnatural saccharides include an H, hydroxyl, ether, ester or amide substituent at a position on the ring at which such a substituent is not present in the natural saccharide. Alternatively, the carbohydrate is missing a substituent that would be found in the carbohydrate from which its name is derived, e.g., deoxy sugars. Still further exemplary unnatural sugars include both oxidized (e.g., -onic and -uronic acids) and reduced (sugar alcohols) carbohydrates. The sugar moiety can be a mono-, oligo- or poly-saccharide.

Exemplary natural sugars of use as components of glycosyl linking groups in the present invention include glucose, glucosamine, galactose, galactosamine, fucose, mannose, mannosamine, xylanose, ribose, N-acetyl glucose, N-acetyl glucosamine, N-acetyl galactose, N-acetyl galactosamine, and sialic acid.

In another aspect, the invention provides a peptide conjugate comprising a glycosyl linking group, wherein the glycosyl linking group is attached to an amino acid residue of said peptide, and wherein said glycosyl linking group comprises a sialyl linking group having a formula which is a member selected from: 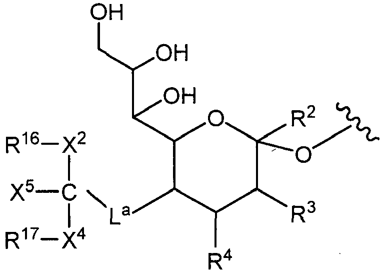 wherein 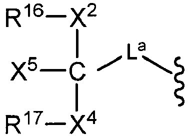 are modifying groups. R² is a member selected from H, CH₂OR⁷, COOR⁷, COO⁻ and OR⁷. R⁷ is a member selected from H, substituted or unsubstituted alkyl and substituted or unsubstituted heteroalkyl. R³ and R⁴ are members independently selected from H, substituted or unsubstituted alkyl, OR⁸, and NHC(O)R⁹. R⁸ and R⁹ are independently selected from H, substituted or unsubstituted alkyl, substituted or unsubstituted heteroalkyl and sialyl. L^{a} is a linker selected from a bond, substituted or unsubstituted alkyl and substituted or unsubstituted heteroalkyl. X⁵, R¹⁶ and R¹⁷ are independently selected from non-reactive group and polymeric moieties (e.g. poly(alkylene oxide), e.g., PEG). Non-reactive groups include groups that are considered to be essentially unreactive, neutral and/ or stable at physiological pH, e.g., H, substituted or unsubstituted alkyl, substituted or unsubstituted heteroalkyl and the like. An exemplary polymeric moiety includes the branched structures set forth in Formula IIIa and its exemplars, below. One of skill in the art will appreciate that the PEG moiety in these formulae can be replaced with other polymers. Exemplary polymers include those of the poly(alkylene oxide) family. X² and X⁴ are independently selected linkage fragments joining polymeric moieties R¹⁶ and R¹⁷ to C. The index j is an integer selected from 1 to 15.

In an exemplary embodiment, the polymeric modifying group has a structure including a moiety according to the following formulae:

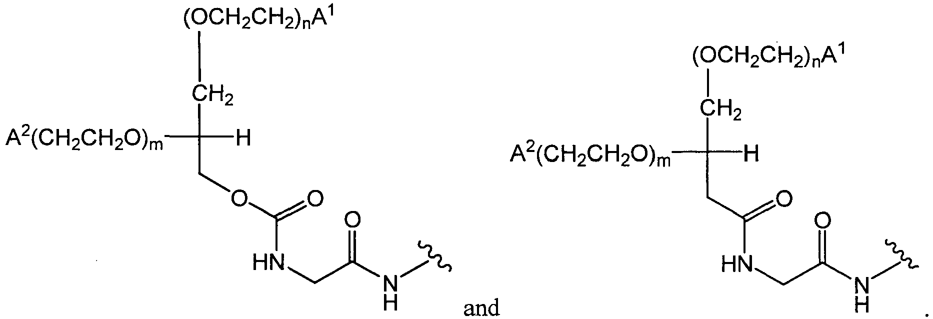

In another exemplary embodiment according to the formula above, the polymeric modifying group has a structure according to the following formula: 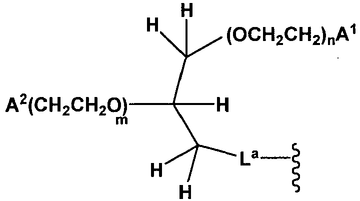 m and n are integers independently selected from about 100 to about 4000, preferably from about 200 to about 3000, even more preferably from about 300 to about 2000 and still more preferably from about 400 to about 1000. In an exemplary embodiment, m and n are integers independently selected from about 1 to about 500. In an exemplary embodiment, m and n are integers independently selected from about 1 to about 70, about 70 to about 150, about 150 to about 250, about 250 to about 375 and about 375 to about 500. In an exemplary embodiment, m and n are integers independently selected from about 10 to about 35, about 45 to about 65, about 95 to about 130, about 210 to about 240, about 310 to about 370 and about 420 to about 480. In an exemplary embodiment, m and n are integers selected from about 15 to about 30. In an exemplary embodiment, m and n are integers selected from about 50 to about 65. In an exemplary embodiment, m and n are integers selected from about 100 to about 130. In an exemplary embodiment, m and n are integers selected from about 210 to about 240. In an exemplary embodiment, m and n are integers selected from about 310 to about 370. In an exemplary embodiment, m and n are integers selected from about 430 to about 470. A¹ and A² are each-OCH₃.

Exemplary polymeric modifying groups according to this embodiment include the moiety:

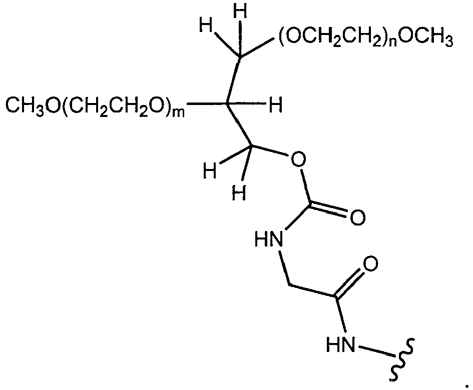

In an exemplary embodiment, the invention provides a modified sugar having the following formula: 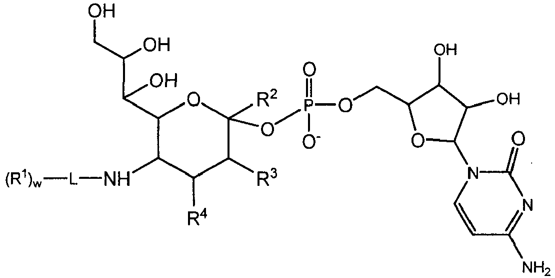 wherein R¹ is the polymeric moiety; L is selected from a bond and a linking group; R² is a member selected from H, CH₂OR⁷, COOR⁷ and OR⁷; R⁷ is a member selected from H, substituted or unsubstituted alkyl and substituted or unsubstituted heteroalkyl; R³ and R⁴ are members independently selected from H, substituted or unsubstituted alkyl, OR⁸ and NHC(O)R⁹; and R⁸ and R⁹ are independently selected from H, substituted or unsubstituted alkyl, substituted or unsubstituted heteroalkyl, sialic acid and polysialic acid. The index w represents an integer selected from 1-6, preferably 1-3 and more preferably 1-2. Exemplary linking groups include substituted or unsubstituted alkyl, substituted or unsubstituted heteroalkyl moieties and sialic acid. An exemplary component of the linker is an acyl moiety.

Also disclosed are methods of forming conjugates of peptides. The methods include contacting a peptide with a modified sugar donor that bears a modifying group covalently attached to a sugar. The modified sugar moiety is transferred from the donor onto an amino acid or glycosyl residue of the peptide by the action of an enzyme. Representative enzymes include, but are not limited to, glycosyltransferases, e.g., sialyltransferases. The method includes contacting the peptide with: a) a modified sugar donor; and b) an enzyme capable of transferring a modified sugar moiety from the modified sugar donor onto an amino acid or glycosyl residue of the peptide, under conditions appropriate to transfer a modified sugar moiety from the donor to an amino acid or glycosyl residue of the peptide, thereby synthesizing said peptide conjugate.

In a preferred aspect, prior to step a), the peptide is contacted with a sialidase, thereby removing at least a portion of the sialic acid on the peptide.

In another preferred aspect, the peptide is contacted with a sialidase, a glycosyltransferase and a modified sugar donor. In this embodiment, the peptide is in contact with the sialidase, glycosyltransferase and modified sugar donor essentially simultaneously, no matter the order of addition of the various components. The reaction is carried out under conditions appropriate for the sialidase to remove a sialic acid residue from the peptide; and the glycosyltransferase to transfer a modified sugar moiety from the modified sugar donor to an amino acid or glycosyl residue of the peptide.

In another preferred aspect, the desialylation and conjugation are performed in the same vessel, and the desialylated peptide is preferably not purified prior to the conjugation step. In another exemplary embodiment, the method further comprises a 'capping' step involving sialylation of the peptide conjugate. This step is performed in the same reaction vessel that contains the sialidase, sialyltransferase and modified sugar donor without prior purification.

In another preferred aspect, the desialylation of the peptide is performed, and the asialo peptide is purified. The purified asialo peptide is then subjected to conjugation reaction conditions. In another exemplary embodiment, the method further comprises a 'capping' step involving sialylation of the peptide conjugate. This step is performed in the same reaction vessel that contains the sialidase, sialyltransferase and modified sugar donor without prior purification.

In another exemplary aspect, the capping step, sialylation of the peptide conjugate, is performed in the same reaction vessel that contains the sialidase, sialyltransferase and modified sugar donor without prior purification.

In an exemplary aspect, the contacting is for a time less than 20 hours, preferably less than 16 hours, more preferably less than 12 hours, even more preferably less than 8 hours, and still more preferably less than 4 hours.

In a further aspect, there is also disclosed a peptide conjugate reaction mixture. The reaction mixture comprises: a) a sialidase; b) an enzyme which is a member selected from glycosyltransferase, exoglycosidase and endoglycosidase; c) a modified sugar; and d) a peptide.

In another exemplary aspect, the ratio of the sialidase to the peptide is selected from 0.1 U/L:2 mg/mL to 10 U/L:1 mg/mL, preferably 0.5 U/L:2 mg/mL, more preferably 1.0 U/L:2 mg/mL, even more preferably 10 U/L:2 mg/mL, still more preferably 0.1 U/L:1 mg/mL, more preferably 0.5 U/L:1 mg/mL, even more preferably 1.0 U/L:1 mg/mL, and still more preferably 10 U/L:1 mg/mL.

In an exemplary aspect, at least 10%, 20%, 30%, 40%, 50%, 60%, 70% or 80% of said peptide conjugate includes at most two PEG moieties. The PEG moieties can be added in a one-pot process, or they can be added after the asialo is purified.

In another exemplary aspect, at least 10%, 20%, 30%, 40%, 50%, 60%, 70% or 80% of the peptide conjugate include at most one PEG moiety. The PEG moiety can be added in a one-pot process, or it can be added after the asialo peptide is purified.

In a further exemplary aspect, the method further comprises "capping", or adding sialic acid to the peptide conjugate. In another exemplary embodiment, sialidase is added, followed by a delay of 30 min, 1 hour, 1.5 hours, or 2 hours, followed by the addition of the glycosyltransferase, exoglycosidase, or endoglycosidase.

In another exemplary aspect, sialidase is added, followed by a delay of 30 min, 1 hour, 1.5 hours, or 2 hours, followed by the addition of the glycosyltransfase, exoglycosidase, or endoglycosidase. Other objects and advantages of the invention will be apparent to those of skill in the art from the detailed description that follows.

In another exemplary aspect, the method includes: (a) contacting a peptide comprising a glycosyl group selected from: 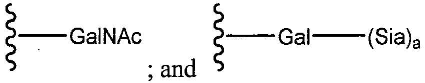 wherein a is an integer from 0 to 10, with a modified sugar having the formula: 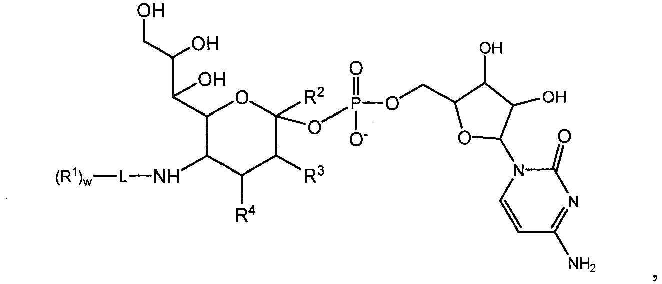 and an appropriate transferase which transfers the glysocyl linking group onto a member selected from the GalNAc, Gal and the Sia of said glycosyl group, under conditions appropriate for said transfer. An exemplary modified sugar is CMP-sialic acid modified, through a linker moiety, with a polymer, e.g., a straight chain or branched poly(ethylene glycol) moiety. The radicals in the formula above are substantially the same identity as those found in the identical formula hereinabove.

The peptide can be acquired from essentially any source, however, in one embodiment, prior to being modified as discussed above, the peptide is expressed in a suitable host. Mammalian (e.g., BHK, CHO), bacteria (e.g., E. coli) and insect cells (e.g., Sf-9) are exemplary expression systems providing a peptide of use in the compositions and methods set forth herein.

Other objects and advantages of the invention will be apparent to those of skill in the art from the detailed description that follows.

### DESCRIPTION OF THE DRAWINGS

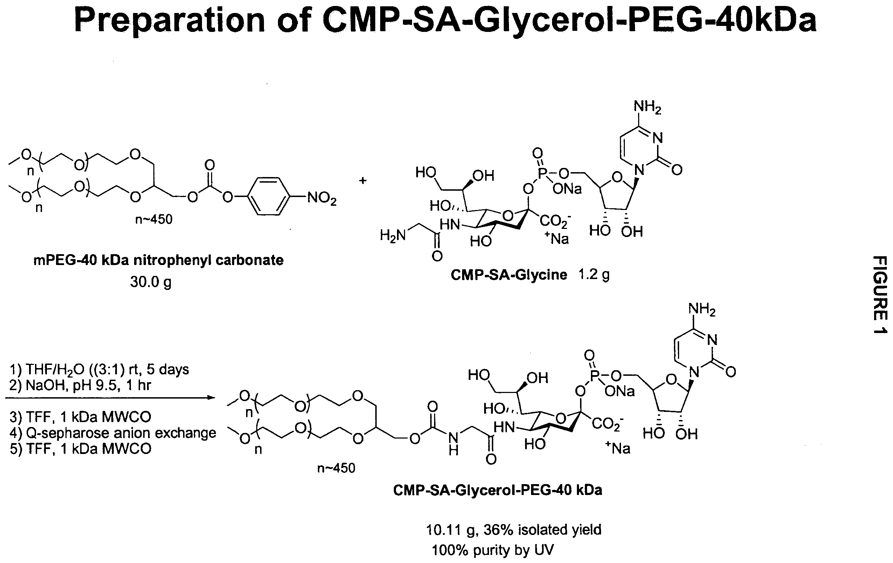

**FIG. 1** illustrates the preparation of CMP-sialic acid-Glycerol PEG 40 kD.

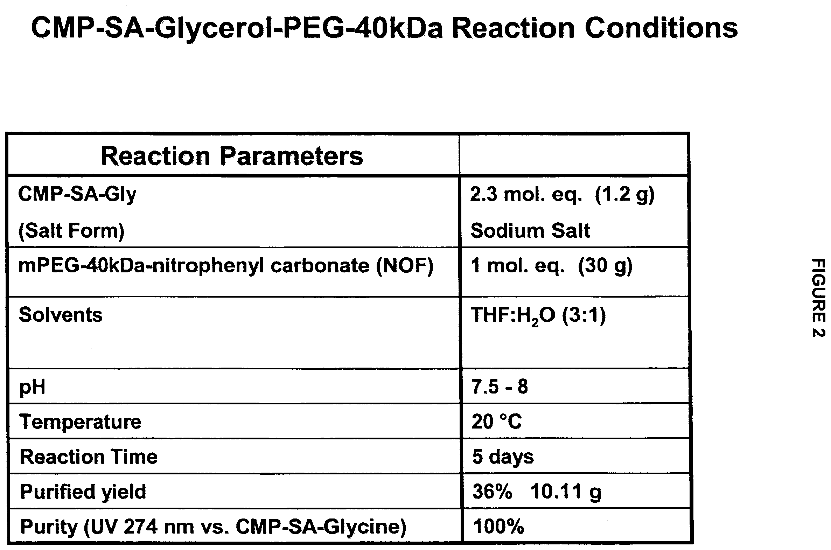

**FIG. 2** illustrates reaction conditions for the preparation of CMP-sialic acid-Glycerol PEG 40 kD.

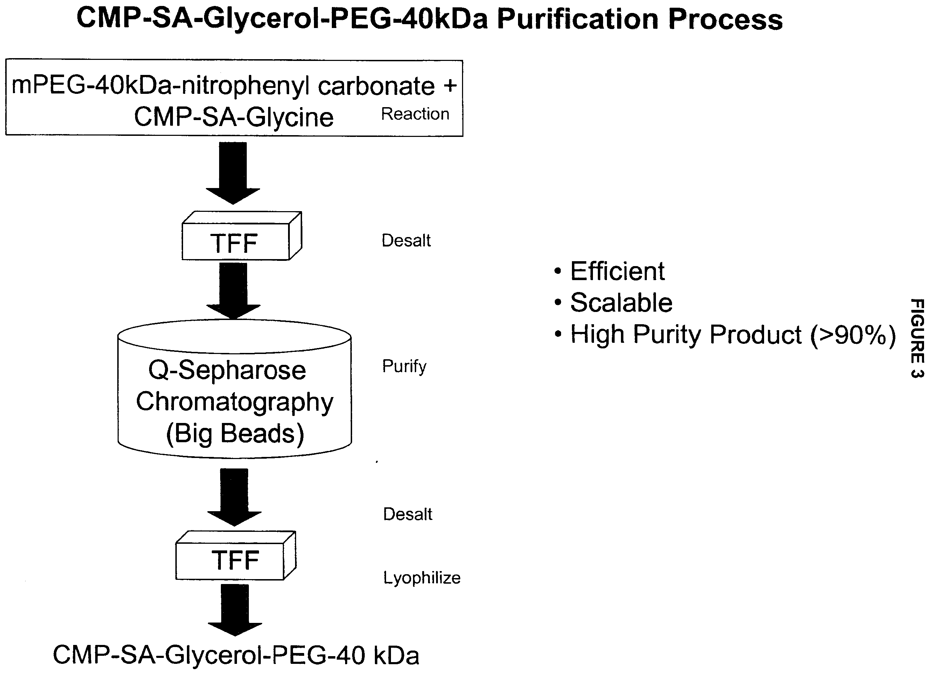

**FIG. 3** illustrates the purification process for CMP-sialic acid-Glycerol PEG 40 kD.

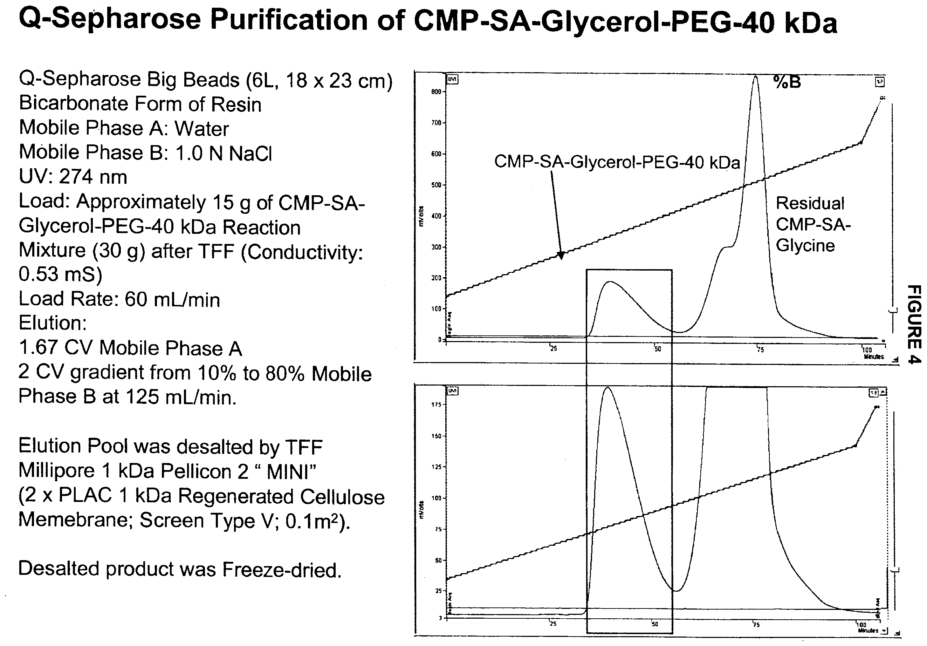

**FIG. 4** illustrates the purification process involving Q-Sepharose for CMP-sialic acid-Glycerol PEG 40 kD.

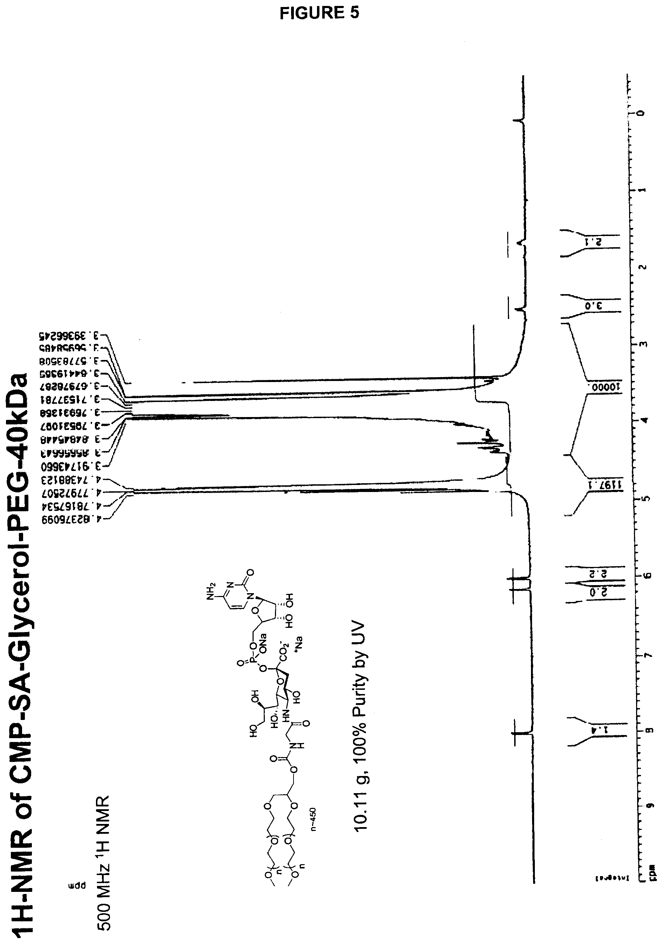

**FIG. 5** is an ¹H NMR spectra of CMP-sialic acid-Glycerol PEG 40 kD.
**FIG. 6** is a table providing exemplary sialyltransferases of use in forming the glycoconjugates of the invention, e.g., to glycoPEGylate peptides with a modified sialic acid.
**FIG. 7** is a table of the peptides to which one or more glycosyl linking groups can be attached. In the peptide conjugates of the invention, Factor IX is the peptide.

### DETAILED DESCRIPTION OF THE INVENTION AND THE PREFERRED EMBODIMENTS

### Abbreviations

PEG, poly(ethyleneglycol); PPG, poly(propyleneglycol); Ara, arabinosyl; Fru, fructosyl; Fuc, fucosyl; Gal, galactosyl; GalNAc, N-acetylgalactosaminyl; Glc, glucosyl; GlcNAc, N-acetylglucosaminyl; Man, mannosyl; ManAc, mannosaminyl acetate; Xyl, xylosyl; NeuAc, sialyl or N-acetylneuraminyl; Sia, sialyl or N-acetylneuraminyl; and derivatives and analogues thereof.

### Definitions

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization are those well known and commonly employed in the art. Standard techniques are used for nucleic acid and peptide synthesis. The techniques and procedures are generally performed according to conventional methods in the art and various general references (*see generally,* Sambrook et al. MOLECULAR CLONING: A LABORATORY MANUAL, 2d ed. (1989) Cold Spring Harbor Laboratory Press, Cold Spring Harbor, N.Y), which are provided throughout this document. The nomenclature used herein and the laboratory procedures in analytical chemistry, and organic synthetic described below are those well known and commonly employed in the art. Standard techniques, or modifications thereof, are used for chemical syntheses and chemical analyses.

All oligosaccharides described herein are described with the name or abbreviation for the non-reducing saccharide (*i.e.,* Gal), followed by the configuration of the glycosidic bond (α or β), the ring bond (1 or 2), the ring position of the reducing saccharide involved in the bond (2, 3, 4, 6 or 8), and then the name or abbreviation of the reducing saccharide (*i.e.,* GlcNAc). Each saccharide is preferably a pyranose. For a review of standard glycobiology nomenclature, *see,* Essentials of Glycobiology Varki et al. eds. CSHL Press (1999).

Oligosaccharides are considered to have a reducing end and a non-reducing end, whether or not the saccharide at the reducing end is in fact a reducing sugar. In accordance with accepted nomenclature, oligosaccharides are depicted herein with the non-reducing end on the left and the reducing end on the right.

The term "sialic acid" or "sialyl" refers to any member of a family of nine-carbon carboxylated sugars. The most common member of the sialic acid family is N-acetylneuraminic acid (2-keto-5-acetamido-3,5-dideoxy-D-glycero-D-galactononulopyranos-1-onic acid (often abbreviated as Neu5Ac, NeuAc, or NANA). A second member of the family is N-glycolyl-neuraminic acid (Neu5Gc or NeuGc), in which the N-acetyl group of NeuAc is hydroxylated. A third sialic acid family member is 2-keto-3-deoxy-nonulosonic acid (KDN) (Nadano et al. (1986) J. Biol. Chem. 261: 11550-11557; Kanamori et al., J. Biol. Chem. 265: 21811-21819 (1990)). Also included are 9-substituted sialic acids such as a 9-O-C₁-C₆ acyl-Neu5Ac like 9-O-lactyl-Neu5Ac or 9-O-acetyl-Neu5Ac, 9-deoxy-9-fluoro-Neu5Ac and 9-azido-9-deoxy-Neu5Ac. For review of the sialic acid family, *see, e.g.,* Varki, Glycobiology 2: 25-40 (1992); Sialic Acids: Chemistry, Metabolism and Function, R. Schauer, Ed. (Springer-Verlag, New York (1992)). The synthesis and use of sialic acid compounds in a sialylation procedure is disclosed in international application WO 92/16640, published October 1, 1992.

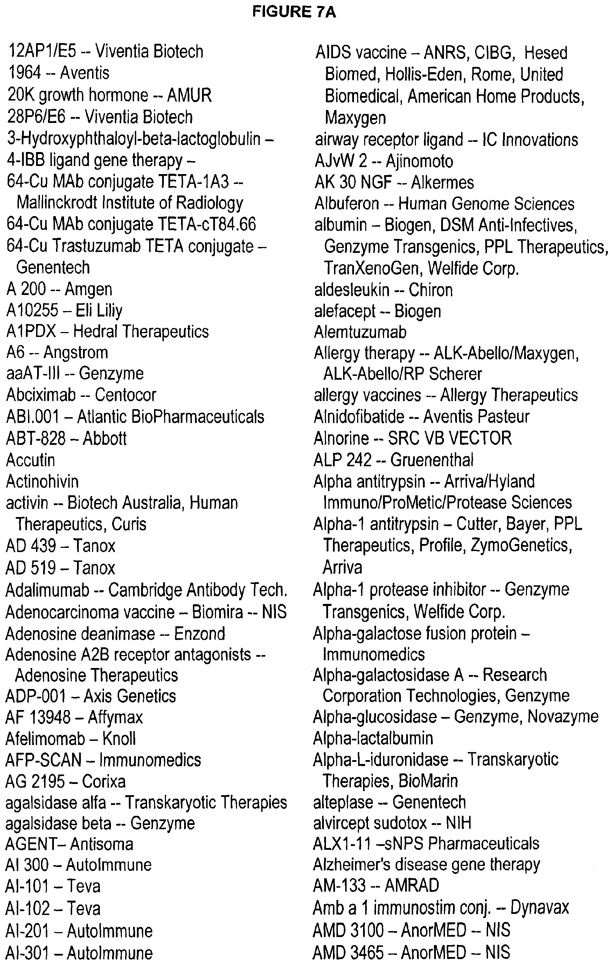

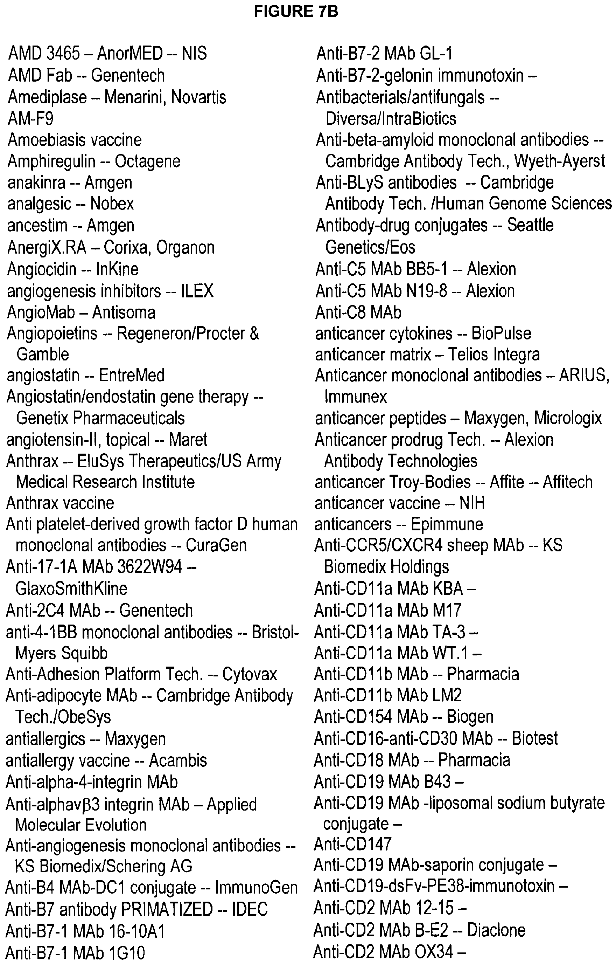

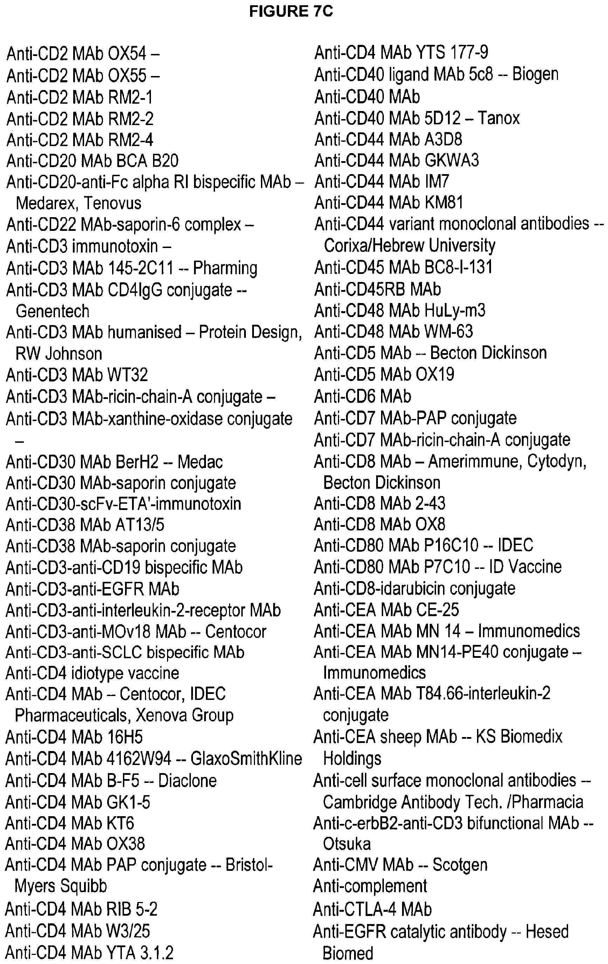

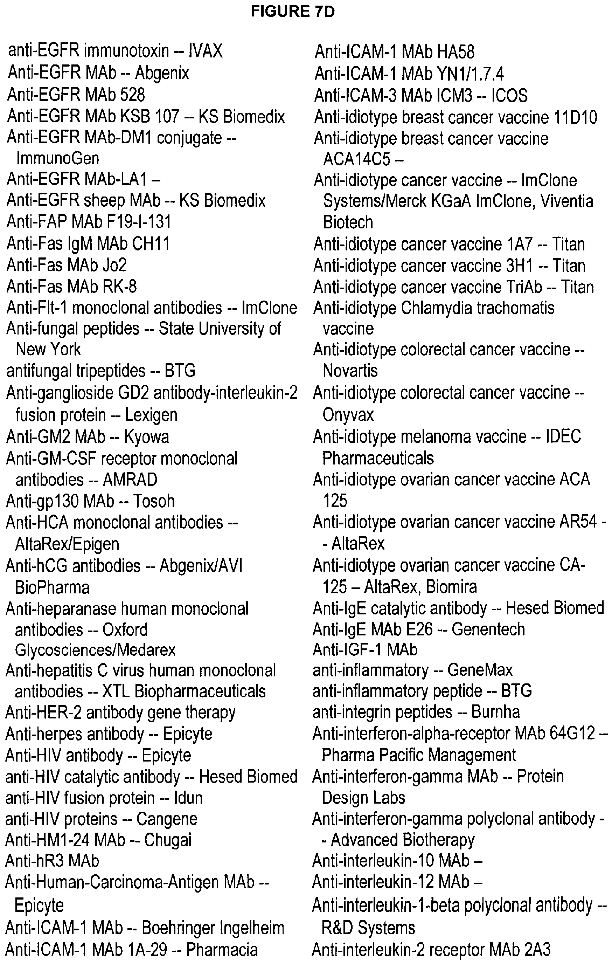

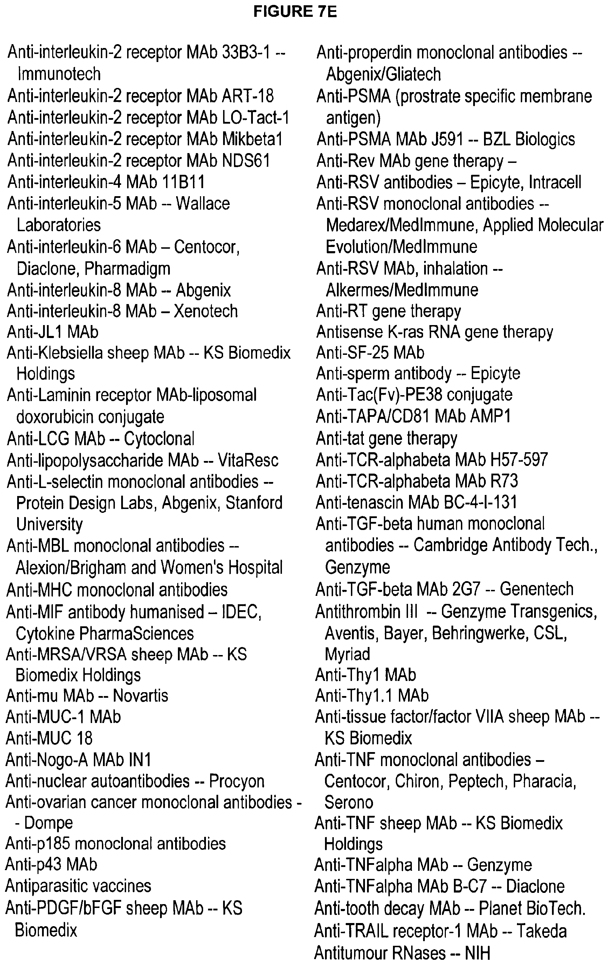

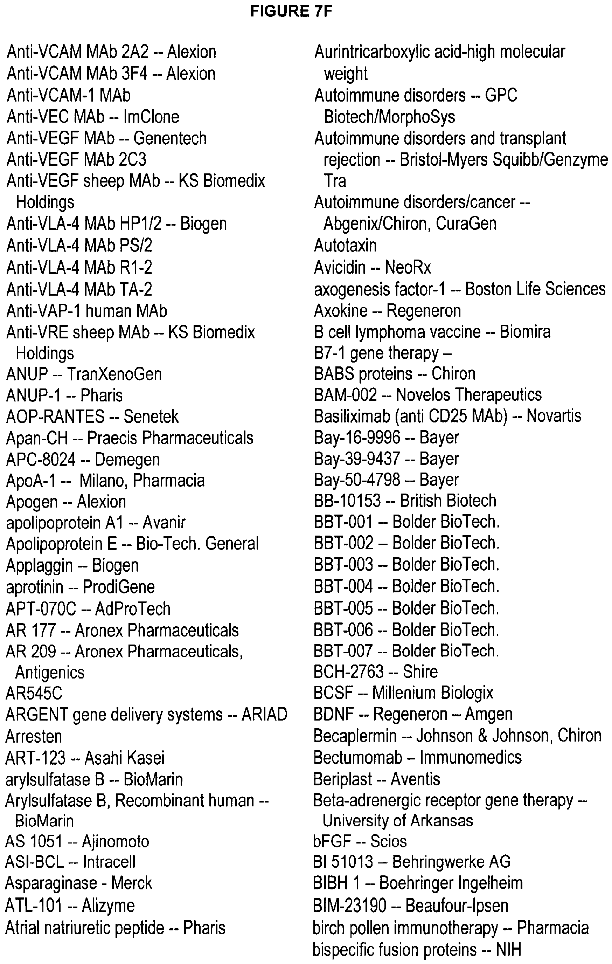

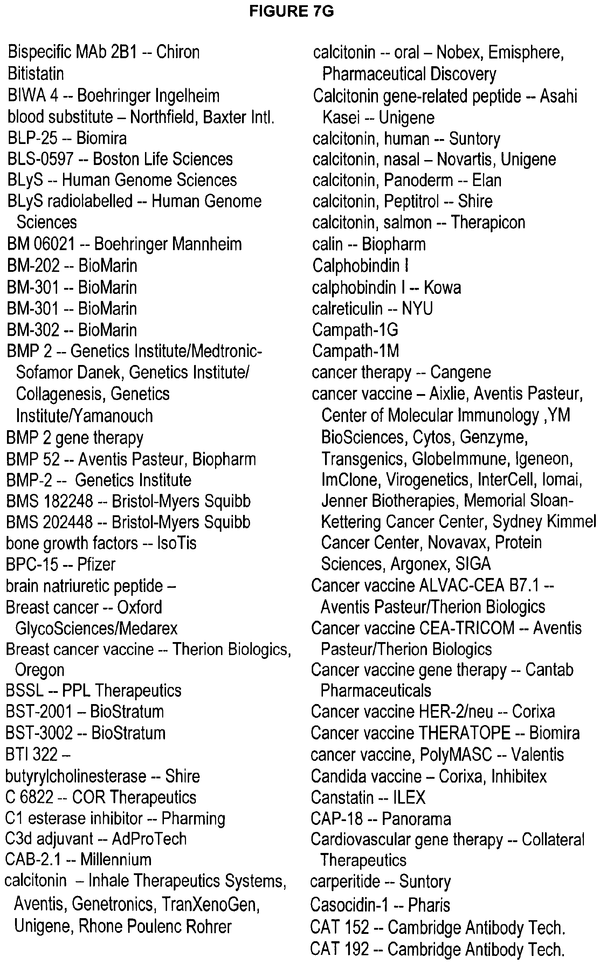

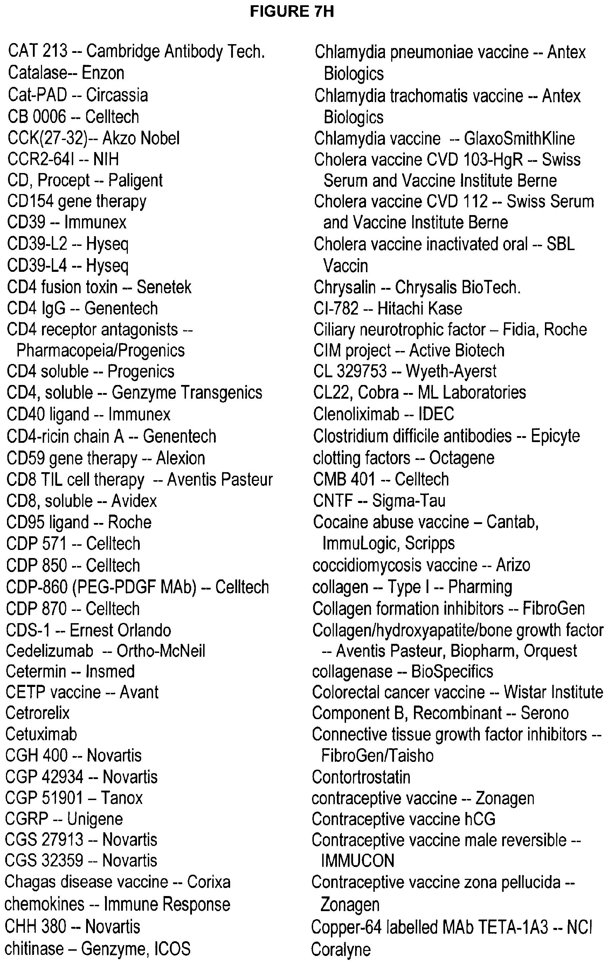

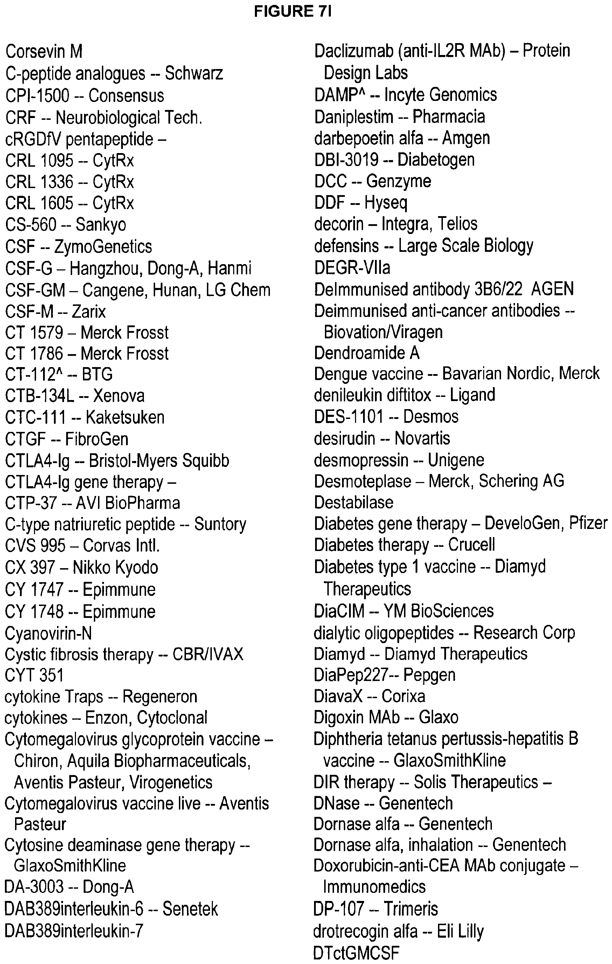

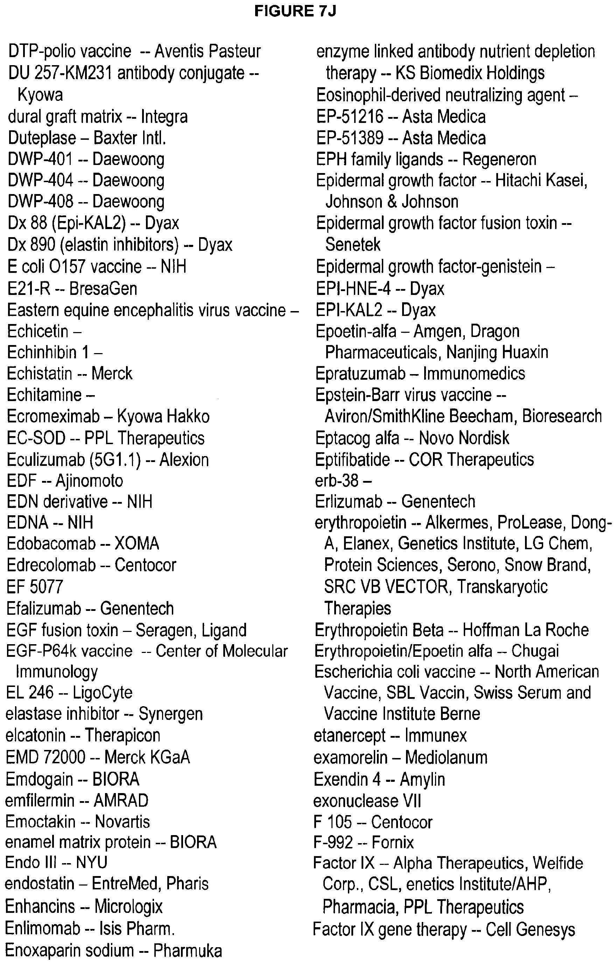

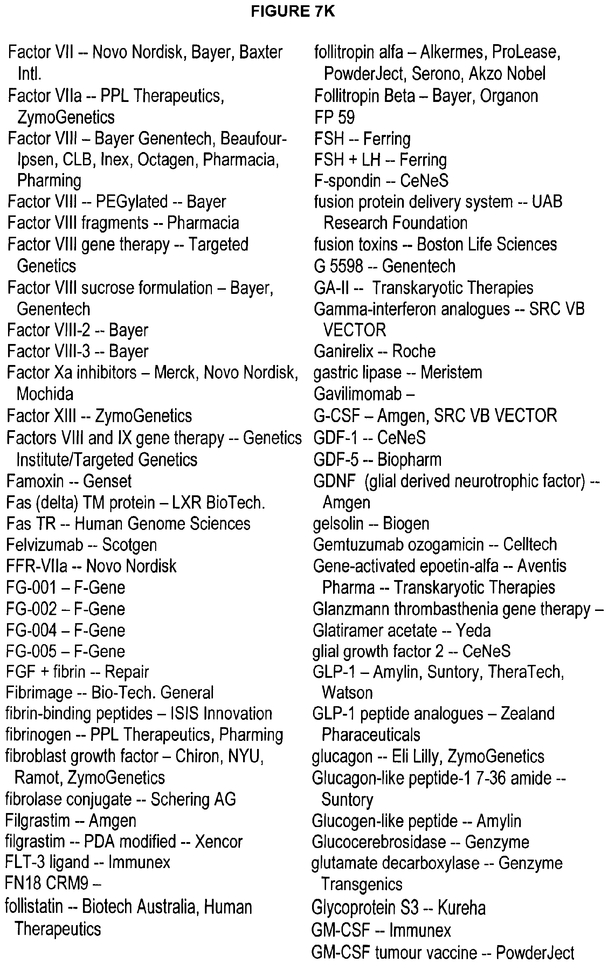

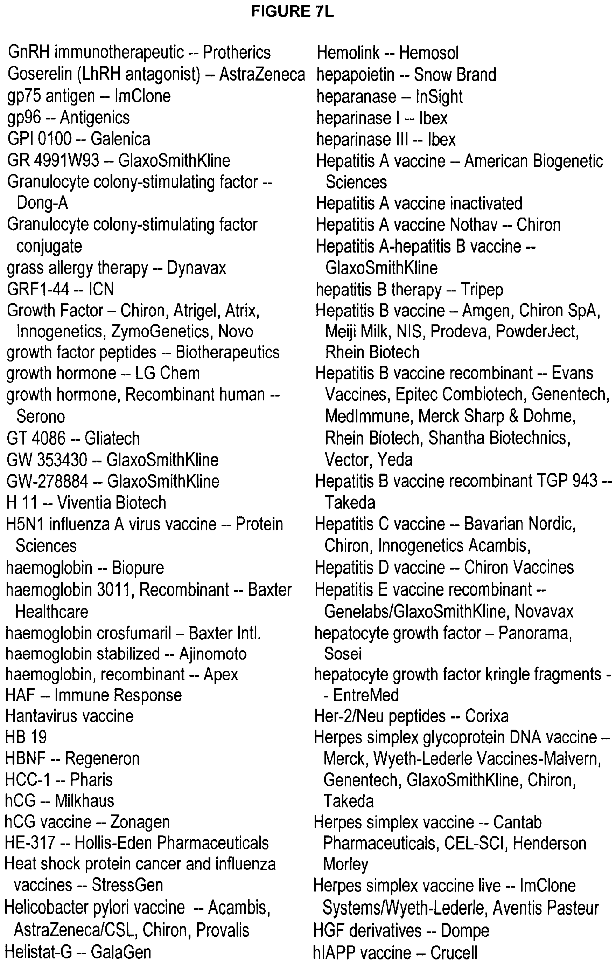

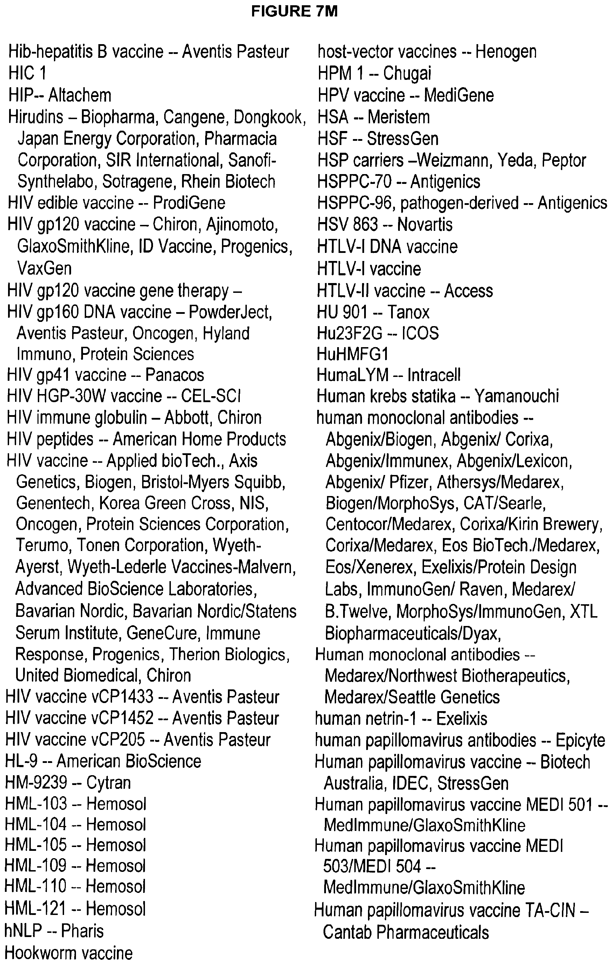

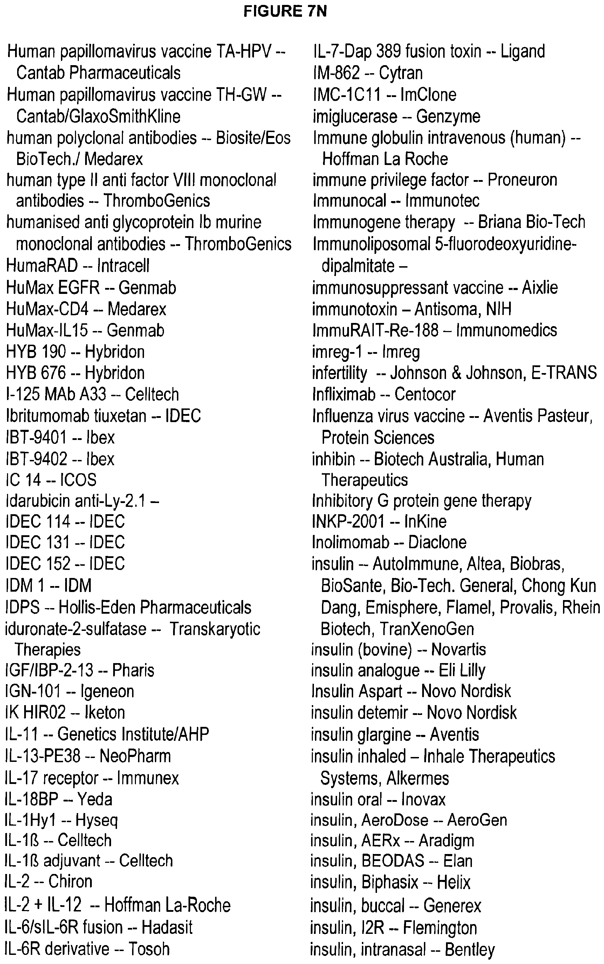

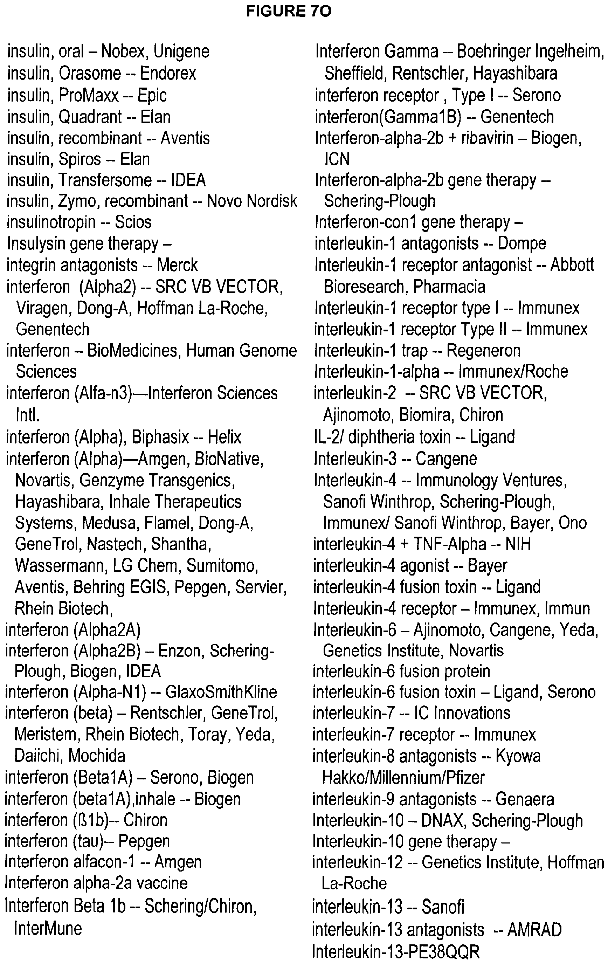

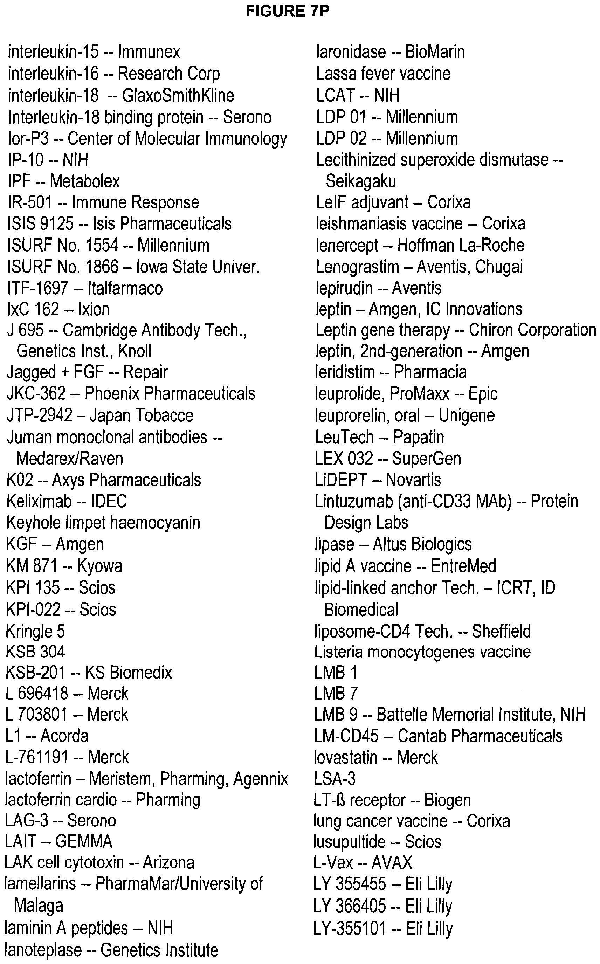

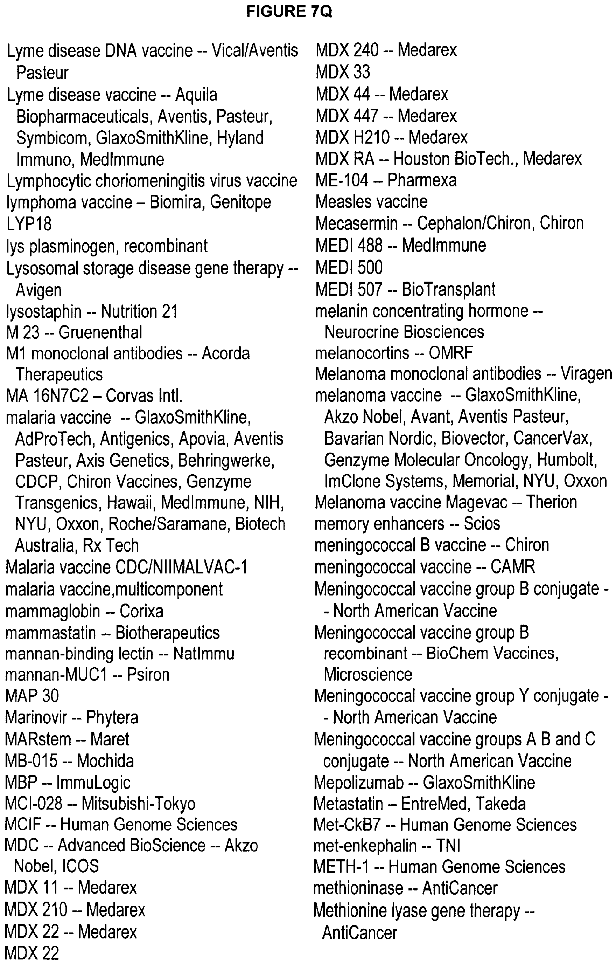

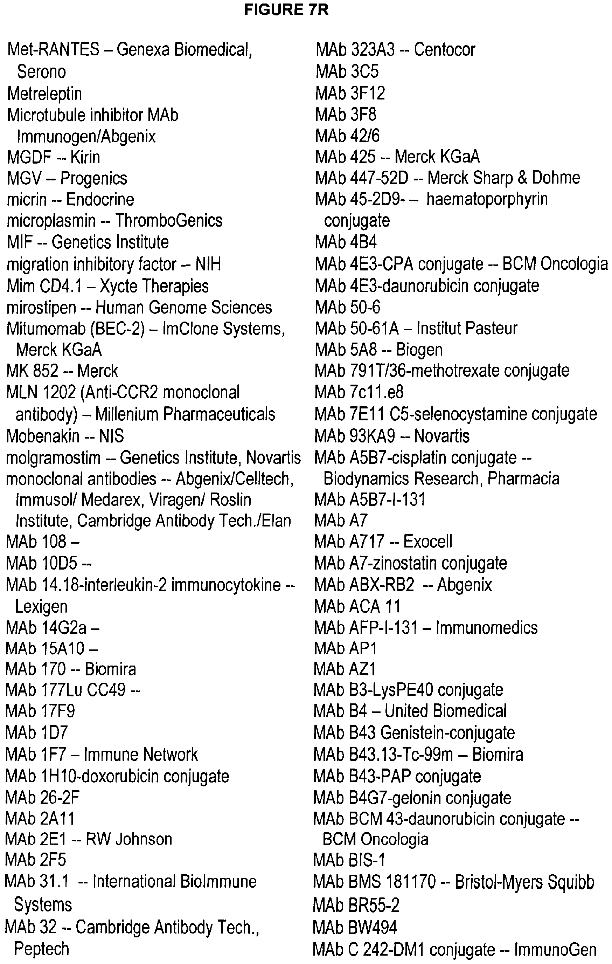

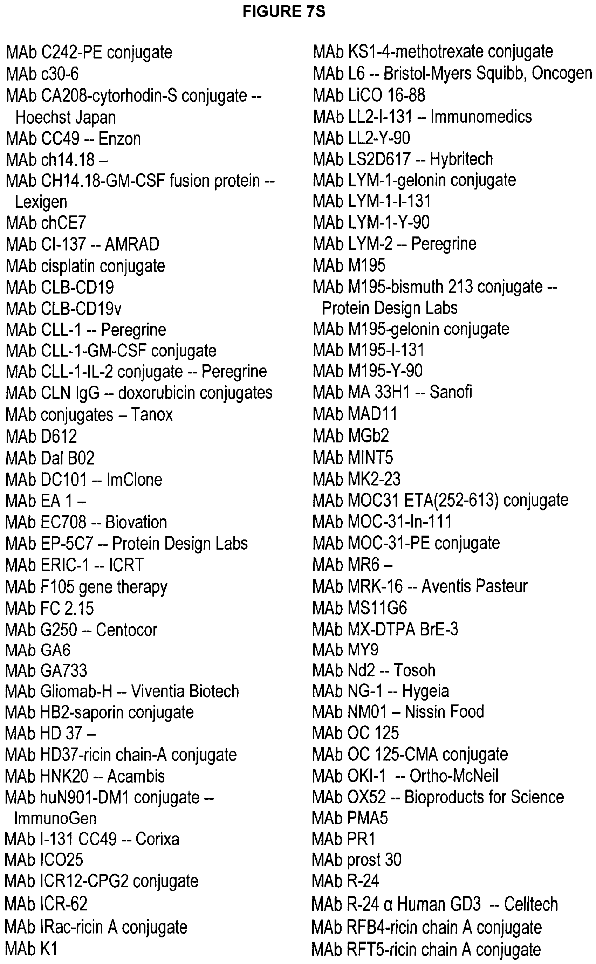

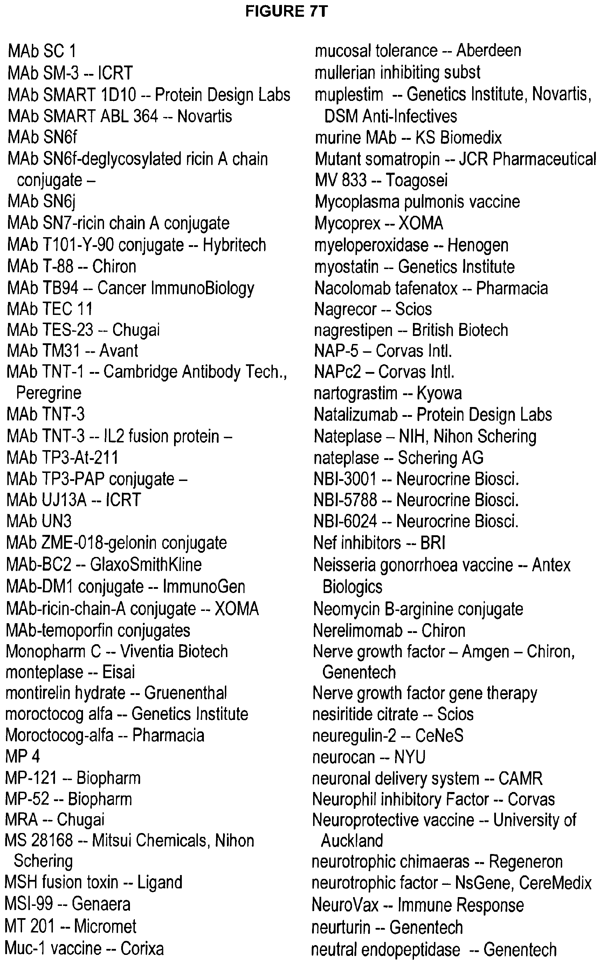

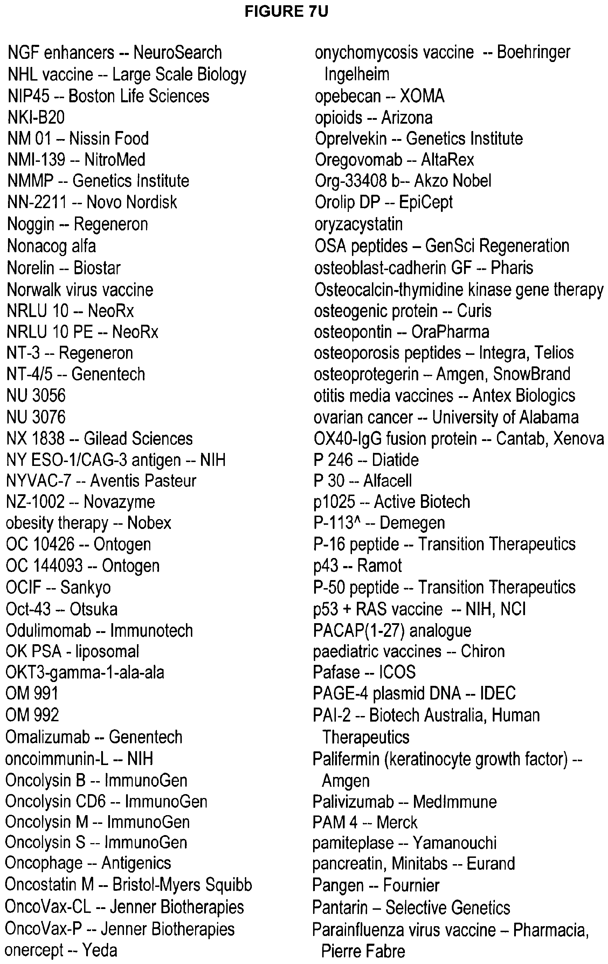

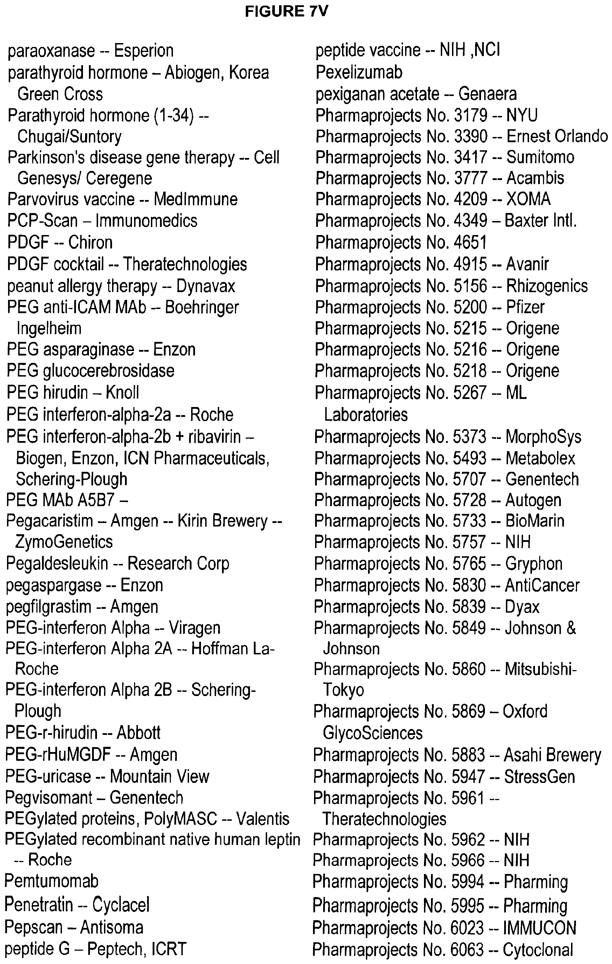

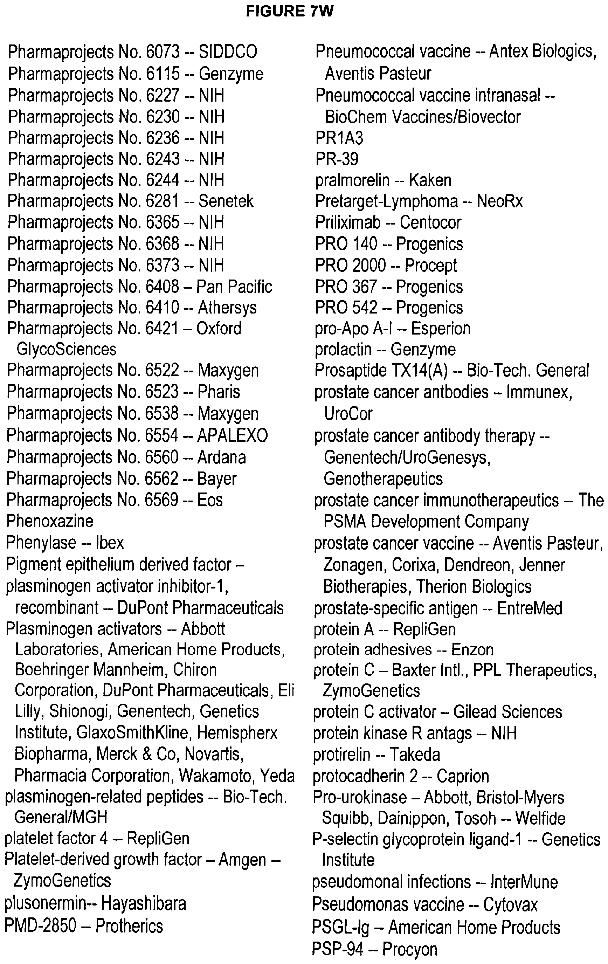

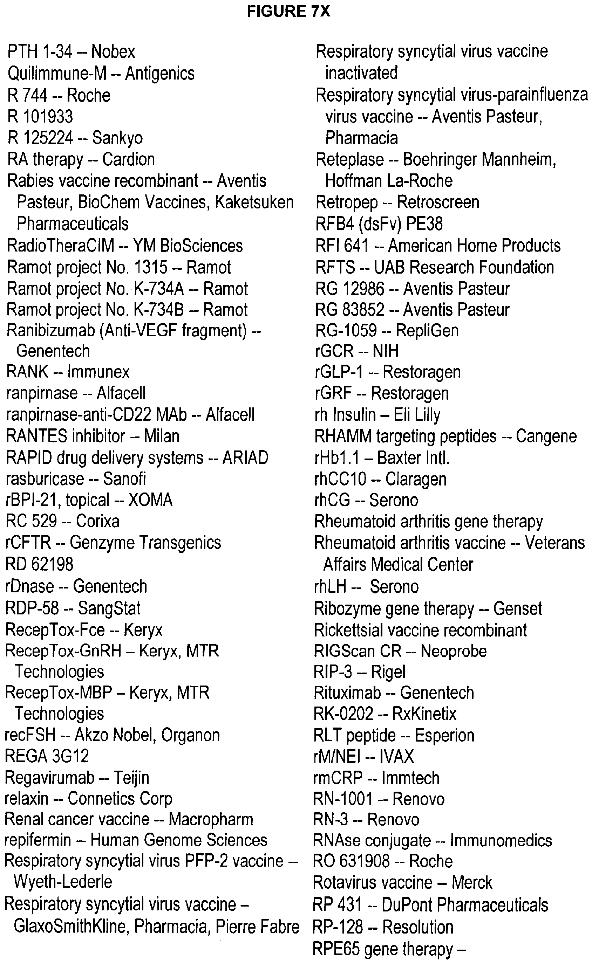

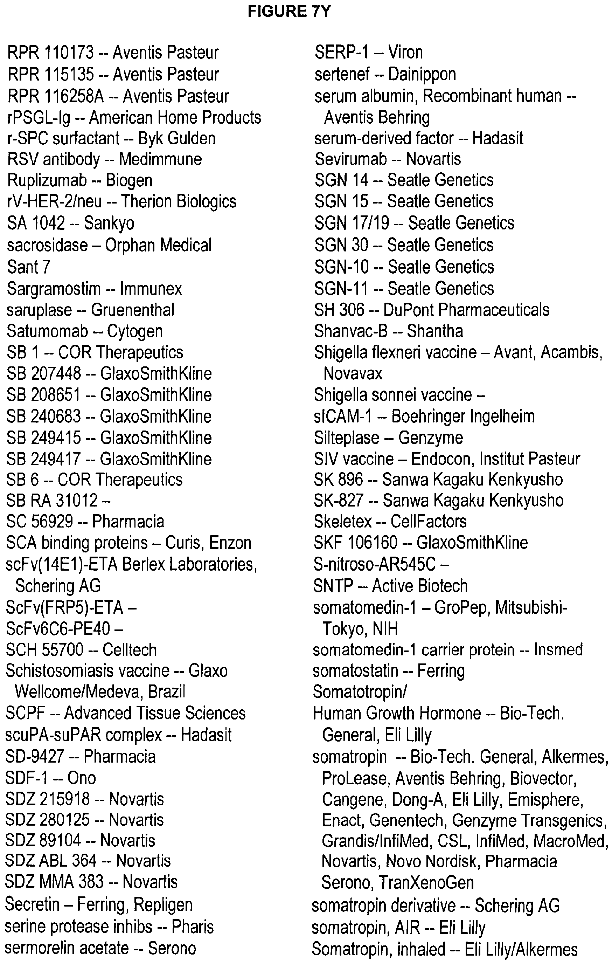

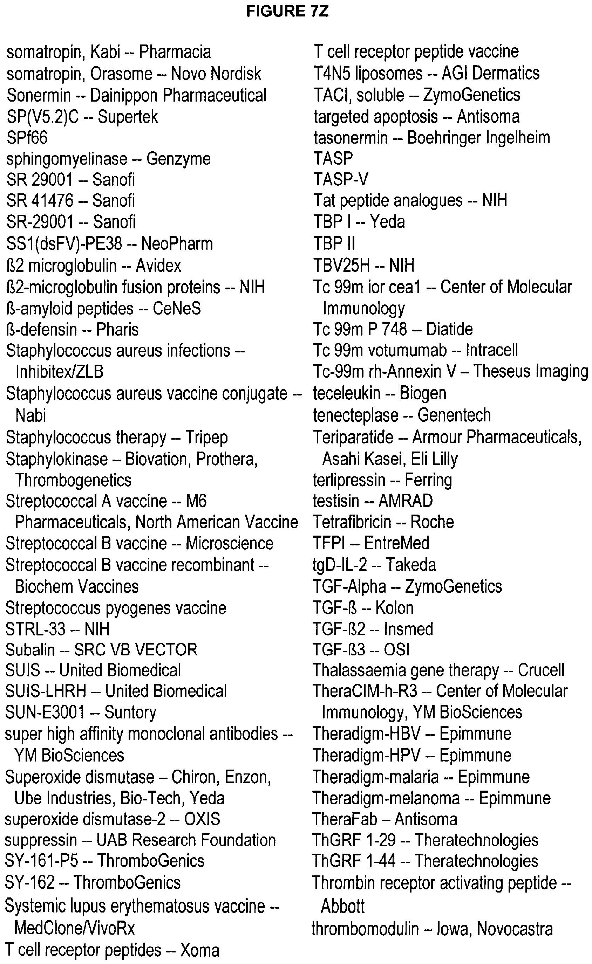

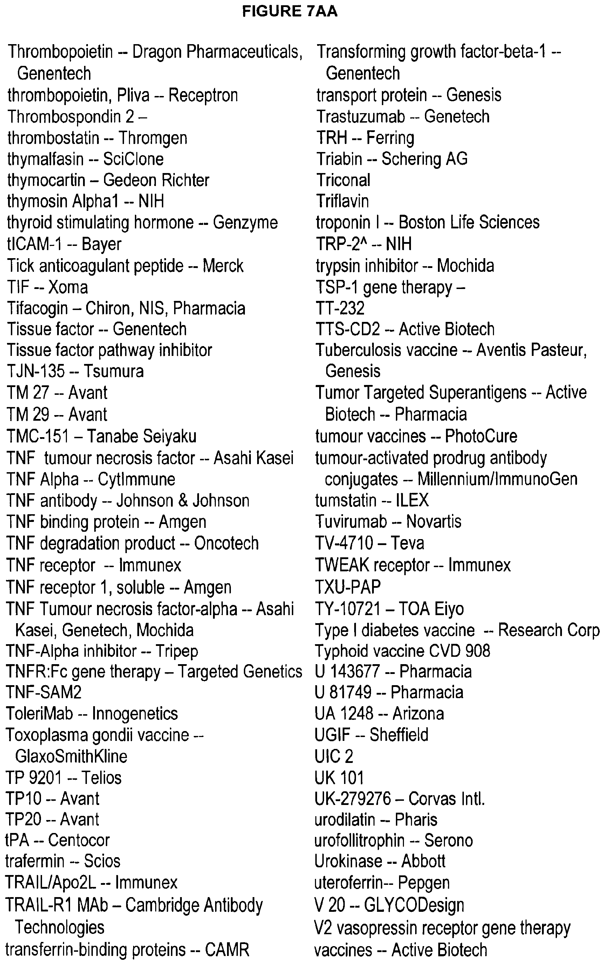

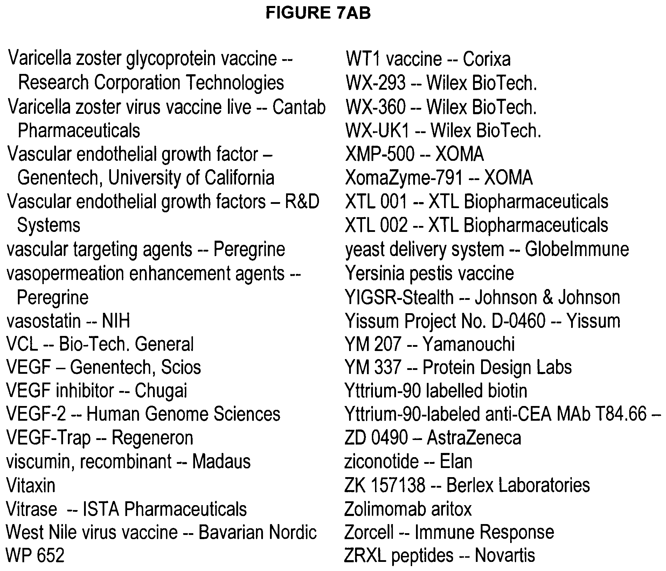

"Peptide" refers to a polymer in which the monomers are amino acids and are joined together through amide bonds, alternatively referred to as a polypeptide. Additionally, unnatural amino acids, for example, β-alanine, phenylglycine and homoarginine are also included. Amino acids that are not gene-encoded may also be used in the present invention. Furthermore, amino acids that have been modified to include reactive groups, glycosylation sites, polymers, therapeutic moieties, biomolecules and the like may also be used in the invention. All of the amino acids used in the present invention may be either the D - or L - isomer. The L -isomer is generally preferred. In addition, other peptidomimetics are also useful in the present invention. As used herein, "peptide" refers to both glycosylated and unglycosylated peptides. Also included are peptides that are incompletely glycosylated by a system that expresses the peptide. For a general review, *see,* Spatola, A. F., in CHEMISTRY AND BIOCHEMISTRY OF AMINO ACIDS, PEPTIDES AND PROTEINS, B. Weinstein, eds., Marcel Dekker, New York, p. 267 (1983). A listing of some of the peptides of the invention is provided in **FIG. 7****.**

The term "peptide conjugate," refers to species of the invention in which a peptide is conjugated with a modified sugar as set forth herein.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid.

As used herein, the term "modified sugar," or "modified sugar residue", refers to a naturally- or non-naturally-occurring carbohydrate that is enzymatically added onto an amino acid or a glycosyl residue of a peptide in a process of the invention. The modified sugar is selected from enzyme substrates including, but not limited to sugar nucleotides (mono-, di-, and tri-phosphates), activated sugars (*e.g*., glycosyl halides, glycosyl mesylates) and sugars that are neither activated nor nucleotides. The "modified sugar" is covalently functionalized with a "modifying group." Useful modifying groups include, but are not limited to, PEG moieties, therapeutic moieties, diagnostic moieties, biomolecules and the like. The modifying group is preferably not a naturally occurring, or an unmodified carbohydrate. The locus of functionalization with the modifying group is selected such that it does not prevent the "modified sugar" from being added enzymatically to a peptide.

As used herein, the term "polymeric moiety" refers to a water-soluble or water-insoluble polymer. The term "water-soluble" refers to moieties that have some detectable degree of solubility in water. Methods to detect and/or quantify water solubility are well known in the art. Exemplary water-soluble polymers include peptides, saccharides, poly(ethers), poly(amines), poly(carboxylic acids) and the like. Peptides can have mixed sequences of be composed of a single amino acid, *e.g*., poly(lysine). An exemplary polysaccharide is poly(sialic acid). An exemplary poly(ether) is poly(ethylene glycol). Poly(ethylene imine) is an exemplary polyamine, and poly(acrylic) acid is a representative poly(carboxylic acid). Preferred water-soluble polymers are essentially non-fluorescent, or emit such a minimal amount of fluorescence that they are inappropriate for use as a fluorescent marker in an assay. Polymers that are not naturally occurring sugars may be used. In addition, the use of an otherwise naturally occurring sugar that is modified by covalent attachment of another entity (*e.g*., poly(ethylene glycol), poly(propylene glycol), poly(aspartate), biomolecule, therapeutic moiety, diagnostic moiety, *etc*.) is also contemplated. The term water-soluble polymer also encompasses species such as saccharides (e.g., dextran, amylose, hyalouronic acid, poly(sialic acid), heparans, heparins, etc.); poly (amino acids), e.g., poly(glutamic acid); nucleic acids; synthetic polymers (e.g., poly(acrylic acid), poly(ethers), e.g., poly(ethylene glycol); peptides, proteins, and the like. Representative water-insoluble polymers include, but are not limited to, polyphosphazines, poly(vinyl alcohols), polyamides, polycarbonates, polyalkylenes, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate) polyethylene, polypropylene, poly(ethylene glycol), poly(ethylene oxide), poly (ethylene terephthalate), poly(vinyl acetate), polyvinyl chloride, polystyrene, polyvinyl pyrrolidone, pluronics and polyvinylphenol and copolymers thereof. In addition, the use of an otherwise naturally occurring sugar that is modified by covalent attachment of another entity (*e.g*., poly(ethylene glycol), poly(propylene glycol), poly(aspartate), biomolecule, therapeutic moiety, diagnostic moiety, *etc.*) is also contemplated. Additional examples of water-soluble and water-insoluble polymers are described in the application.

The polymer backbone of the water-soluble polymer is poly(ethylene glycol) (i.e. PEG). However, it should be understood that other related polymers are also suitable for use in the practice of this invention and that the use of the term PEG or poly(ethylene glycol) is intended to be inclusive and not exclusive in this respect. The term PEG includes poly(ethylene glycol) in any of its forms, including alkoxy PEG, difunctional PEG, multiarmed PEG, forked PEG, branched PEG, pendent PEG (i.e. PEG or related polymers having one or more functional groups pendent to the polymer backbone), or PEG with degradable linkages therein.

The polymer can be linear or branched. Branched polymers are generally known in the art. Typically, a branched polymer has a central branch core moiety and a plurality of linear or branched polymer chains linked to the central branch core. PEG is commonly used in branched forms that can be prepared by addition of ethylene oxide to various polyols, such as glycerol, pentaerythritol and sorbitol. The central branch moiety can also be derived from several amino acids, such as lysine. The branched poly(ethylene glycol) can be represented in general form as R(-PEG-OH)ₘ in which R represents the core moiety, namely glycerol, and m represents the number of arms. Multi-armed PEG molecules, such as those described in U.S. Pat. No. 5,932,462, can also be used as the polymer backbone. In an exemplary embodiment, the branched polymer is itself attached to a branching moiety (e.g., cysteine, serine, lysine, and oligomers of lysine).

The "area under the curve" or "AUC", as used herein in the context of administering a peptide drug to a patient, is defined as total area under the curve that describes the concentration of drug in systemic circulation in the patient as a function of time from zero to infinity.

The term "half-life" or "t½", as used herein in the context of administering a peptide drug to a patient, is defined as the time required for plasma concentration of a drug in a patient to be reduced by one half. There may be more than one half-life associated with the peptide drug depending on multiple clearance mechanisms, redistribution, and other mechanisms well known in the art. Usually, alpha and beta half-lives are defined such that the alpha phase is associated with redistribution, and the beta phase is associated with clearance. However, with protein drugs that are, for the most part, confined to the bloodstream, there can be at least two clearance half-lives. For some glycosylated peptides, rapid beta phase clearance may be mediated via receptors on macrophages, or endothelial cells that recognize terminal galactose, N-acetylgalactosamine, N-acetylglucosamine, mannose, or fucose. Slower beta phase clearance may occur via renal glomerular filtration for molecules with an effective radius < 2 nm (approximately 68 kD) and/or specific or nonspecific uptake and metabolism in tissues. GlycoPEGylation may cap terminal sugars (*e.g*., galactose or N-acetylgalactosamine) and thereby block rapid alpha phase clearance via receptors that recognize these sugars. It may also confer a larger effective radius and thereby decrease the volume of distribution and tissue uptake, thereby prolonging the late beta phase. Thus, the precise impact of glycoPEGylation on alpha phase and beta phase half-lives may vary depending upon the size, state of glycosylation, and other parameters, as is well known in the art. Further explanation of "half-life" is found in Pharmaceutical Biotechnology (1997, DFA Crommelin and RD Sindelar, eds., Harwood Publishers, Amsterdam, pp 101 - 120).

The term "glycoconjugation," as used herein, refers to the enzymatically mediated conjugation of a modified sugar species to an amino acid or glycosyl residue of a polypeptide, *e.g*., a G-CSF peptide of the present invention. A subgenus of "glycoconjugation" is "glyco-PEGylation," in which the modifying group of the modified sugar is poly(ethylene glycol), and alkyl derivative (*e.g.,* m-PEG) or reactive derivative (*e.g.,* H₂N-PEG, HOOC-PEG) thereof.

The terms "large-scale" and "industrial-scale" are used interchangeably and refer to a reaction cycle that produces at least about 250 mg, preferably at least about 500 mg, and more preferably at least about 1 gram of glycoconjugate at the completion of a single reaction cycle.

The term, "glycosyl linking group," as used herein refers to a glycosyl residue to which a modifying group (e.g., PEG moiety, therapeutic moiety, biomolecule) is covalently attached; the glycosyl linking group joins the modifying group to the remainder of the conjugate. In the methods of the invention, the "glycosyl linking group" becomes covalently attached to a glycosylated or unglycosylated peptide, thereby linking the agent to an amino acid and/or glycosyl residue on the peptide. A "glycosyl linking group" is generally derived from a "modified sugar" by the enzymatic attachment of the "modified sugar" to an amino acid and/or glycosyl residue of the peptide. The glycosyl linking group can be a saccharide-derived structure that is degraded during formation of modifying group-modified sugar cassette (e.g., oxidation→Schiff base formation→reduction), or the glycosyl linking group may be intact. An "intact glycosyl linking group" refers to a linking group that is derived from a glycosyl moiety in which the saccharide monomer that links the modifying group and to the remainder of the conjugate is not degraded, e.g., oxidized, e.g., by sodium metaperiodate. "Intact glycosyl linking groups" of the invention may be derived from a naturally occurring oligosaccharide by addition of glycosyl unit(s) or removal of one or more glycosyl unit from a parent saccharide structure.

The term, "non-glycosidic modifying group", as used herein, refers to modifying groups which do not include a naturally occurring sugar linked directly to the glycosyl linking group.

The term "targeting moiety," as used herein, refers to species that will selectively localize in a particular tissue or region of the body. The localization is mediated by specific recognition of molecular determinants, molecular size of the targeting agent or conjugate, ionic interactions, hydrophobic interactions and the like. Other mechanisms of targeting an agent to a particular tissue or region are known to those of skill in the art. Exemplary targeting moieties include antibodies, antibody fragments, transferrin, HS-glycoprotein, coagulation factors, serum proteins, β-glycoprotein, G-CSF, GM-CSF, M-CSF, EPO and the like.

As used herein, "therapeutic moiety" means any agent useful for therapy including, but not limited to, antibiotics, anti-inflammatory agents, anti-tumor drugs, cytotoxins, and radioactive agents. "Therapeutic moiety" includes prodrugs of bioactive agents, constructs in which more than one therapeutic moiety is bound to a carrier, e.g, multivalent agents. Therapeutic moiety also includes proteins and constructs that include proteins. Exemplary proteins include, but are not limited to, Granulocyte Colony Stimulating Factor (GCSF), Granulocyte Macrophage Colony Stimulating Factor (GMCSF), Interferon (e.g., Interferon-α, -β, -γ), Interleukin (e.g., Interleukin II), serum proteins (e.g., Factors VII, VIIa, VIII, IX, and X), Human Chorionic Gonadotropin (HCG), Follicle Stimulating Hormone (FSH) and Lutenizing Hormone (LH) and antibody fusion proteins (e.g. Tumor Necrosis Factor Receptor ((TNFR)/Fc domain fusion protein)).

As used herein, "pharmaceutically acceptable carrier" includes any material, which when combined with the conjugate retains the conjugates' activity and is non-reactive with the subject's immune systems. Examples include, but are not limited to, any of the standard pharmaceutical carriers such as a phosphate buffered saline solution, water, emulsions such as oil/water emulsion, and various types of wetting agents. Other carriers may also include sterile solutions, tablets including coated tablets and capsules. Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

As used herein, "administering," means oral administration, administration as a suppository, topical contact, intravenous, intraperitoneal, intramuscular, intralesional, intranasal or subcutaneous administration, or the implantation of a slow-release device *e.g*., a mini-osmotic pump, to the subject. Administration is by any route including parenteral, and transmucosal (e.g., oral, nasal, vaginal, rectal, or transdermal). Parenteral administration includes, e.g., intravenous, intramuscular, intra-arteriole, intradermal, subcutaneous, intraperitoneal, intraventricular, and intracranial. Moreover, where injection is to treat a tumor, e.g., induce apoptosis, administration may be directly to the tumor and/or into tissues surrounding the tumor. Other modes of delivery include, but are not limited to, the use of liposomal formulations, intravenous infusion, transdermal patches, etc.

The term "ameliorating" or "ameliorate" refers to any indicia of success in the treatment of a pathology or condition, including any objective or subjective parameter such as abatement, remission or diminishing of symptoms or an improvement in a patient's physical or mental well-being. Amelioration of symptoms can be based on objective or subjective parameters; including the results of a physical examination and/or a psychiatric evaluation.

The term "therapy" refers to "treating" or "treatment" of a disease or condition including preventing the disease or condition from occurring in an animal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease).

The term "effective amount" or "an amount effective to" or a "therapeutically effective amount" or any grammatically equivalent term means the amount that, when administered to an animal for treating a disease, is sufficient to effect treatment for that disease.

The term "isolated" refers to a material that is substantially or essentially free from components, which are used to produce the material. For peptide conjugates of the invention, the term "isolated" refers to material that is substantially or essentially free from components which normally accompany the material in the mixture used to prepare the peptide conjugate. "Isolated" and "pure" are used interchangeably. Typically, isolated peptide conjugates of the invention have a level of purity preferably expressed as a range. The lower end of the range of purity for the peptide conjugates is about 60%, about 70% or about 80% and the upper end of the range of purity is about 70%, about 80%, about 90% or more than about 90%.

When the peptide conjugates are more than about 90% pure, their purities are also preferably expressed as a range. The lower end of the range of purity is about 90%, about 92%, about 94%, about 96% or about 98%. The upper end of the range of purity is about 92%, about 94%, about 96%, about 98% or about 100% purity.

Purity is determined by any art-recognized method of analysis (*e.g*., band intensity on a silver stained gel, polyacrylamide gel electrophoresis, HPLC, or a similar means).

"Essentially each member of the population," as used herein, describes a characteristic of a population of peptide conjugates of the invention in which a selected percentage of the modified sugars added to a peptide are added to multiple, identical acceptor sites on the peptide. "Essentially each member of the population" speaks to the "homogeneity" of the sites on the peptide conjugated to a modified sugar and refers to conjugates of the invention, which are at least about 80%, preferably at least about 90% and more preferably at least about 95% homogenous.

"Homogeneity," refers to the structural consistency across a population of acceptor moieties to which the modified sugars are conjugated. Thus, in a peptide conjugate of the invention in which each modified sugar moiety is conjugated to an acceptor site having the same structure as the acceptor site to which every other modified sugar is conjugated, the peptide conjugate is said to be about 100% homogeneous. Homogeneity is typically expressed as a range. The lower end of the range of homogeneity for the peptide conjugates is about 60%, about 70% or about 80% and the upper end of the range of purity is about 70%, about 80%, about 90% or more than about 90%.

When the peptide conjugates are more than or equal to about 90% homogeneous, their homogeneity is also preferably expressed as a range. The lower end of the range of homogeneity is about 90%, about 92%, about 94%, about 96% or about 98%. The upper end of the range of purity is about 92%, about 94%, about 96%, about 98% or about 100% homogeneity. The purity of the peptide conjugates is typically determined by one or more methods known to those of skill in the art, e.g., liquid chromatography-mass spectrometry (LC-MS), matrix assisted laser desorption mass time of flight spectrometry (MALDITOF), capillary electrophoresis, and the like.

"Substantially uniform glycoform" or a "substantially uniform glycosylation pattern," when referring to a glycopeptide species, refers to the percentage of acceptor moieties that are glycosylated by the glycosyltransferase of interest (*e.g*., fucosyltransferase). For example, in the case of a α1,2 fucosyltransferase, a substantially uniform fucosylation pattern exists if substantially all (as defined below) of the Galβ1,4-GlcNAc-R and sialylated analogues thereof are fucosylated in a peptide conjugate of the invention. In the fucosylated structures set forth herein, the Fuc-GlcNAc linkage is generally α1,6 or α1,3, with α1,6 generally preferred. It will be understood by one of skill in the art, that the starting material may contain glycosylated acceptor moieties (*e.g.,* fucosylated Galβ1,4-GlcNAc-R moieties). Thus, the calculated percent glycosylation will include acceptor moieties that are glycosylated by the methods of the invention, as well as those acceptor moieties already glycosylated in the starting material.

The term "substantially" in the above definitions of "substantially uniform" generally means at least about 40%, at least about 70%, at least about 80%, or more preferably at least about 90%, and still more preferably at least about 95% of the acceptor moieties for a particular glycosyltransferase are glycosylated.

Where substituent groups are specified by their conventional chemical formulae, written from left to right, they equally encompass the chemically identical substituents, which would result from writing the structure from right to left, e.g., -CH₂O- is intended to also recite -OCH₂-.

The term "alkyl," by itself or as part of another substituent means, unless otherwise stated, a straight or branched chain, or cyclic hydrocarbon radical, or combination thereof, which may be fully saturated, mono- or polyunsaturated and can include di- and multivalent radicals, having the number of carbon atoms designated (*i.e.* C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below, such as "heteroalkyl." Alkyl groups that are limited to hydrocarbon groups are termed "homoalkyl".

The term "alkylene" by itself or as part of another substituent means a divalent radical derived from an alkane, as exemplified, but not limited, by -CH₂CH₂CH₂CH₂-, and further includes those groups described below as "heteroalkylene." Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (*e.g*., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1 -(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1 -piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like.

The term "aryl" means, unless otherwise stated, a polyunsaturated, aromatic, substituent that can be a single ring or multiple rings (preferably from 1 to 3 rings), which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, tetrazolyl, benzo[b]furanyl, benzo[b]thienyl, 2,3-dihydrobenzo[1,4]dioxin-6-yl, benzo[1,3]dioxol-5-yl and 6-quinolyl. Substituents for each of the above noted aryl and heteroaryl ring systems are selected from the group of acceptable substituents described below.

For brevity, the term "aryl" when used in combination with other terms (e.g., aryloxy, arylthioxy, arylalkyl) includes both aryl and heteroaryl rings as defined above. Thus, the term "arylalkyl" is meant to include those radicals in which an aryl group is attached to an alkyl group (*e.g*., benzyl, phenethyl, pyridylmethyl and the like) including those alkyl groups in which a carbon atom (*e.g.,* a methylene group) has been replaced by, for example, an oxygen atom (*e.g.,* phenoxymethyl, 2-pyridyloxymethyl, 3-(1-naphthyloxy)propyl, and the like).

Each of the above terms (*e.g*., "alkyl," "heteroalkyl," "aryl" and "heteroaryl") is meant to include both substituted and unsubstituted forms of the indicated radical. Preferred substituents for *each* type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents," and they can be one or more of a variety of groups selected from, but not limited to: -OR', =O, =NR', =N-OR', -NR'R", -SR', -halogen, -SiR'R"R"', -OC(O)R',-C(O)R', -CO₂R', -CONR'R", -OC(O)NR'R", -NR"C(O)R', -NR'-C(O)NR"R"',-NR"C(O)₂R', -NR-C(NR'R"R"')=NR"", -NR-C(NR'R")=NR"', -S(O)R', -S(O)₂R',-S(O)₂NR'R", -NRSO₂R', -CN and -NO₂ in a number ranging from zero to (2m'+1), where m' is the total number of carbon atoms in such radical. R', R", R"' and R"" each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (*e.g*., -CF₃ and -CH₂CF₃) and acyl (*e.g.,* -C(O)CH₃, -C(O)CF₃, -C(O)CH₂OCH₃, and the like).

Similar to the substituents described for the alkyl radical, substituents for the aryl and heteroaryl groups are generically referred to as "aryl group substituents." The substituents are selected from, for example: halogen, -OR', =O, =NR', =N-OR', -NR'R",-SR', -halogen, -SiR'R"R"', -OC(O)R', -C(O)R', -CO₂R', -CONR'R", -OC(O)NR'R",-NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -CN and -NO₂, -R',-N₃, -CH(Ph)₂, fluoro(C₁-C₄)alkoxy, and fluoro(C₁-C₄)alkyl, in a number ranging from zero to the total number of open valences on the aromatic ring system; and where R', R", R"' and R"" are preferably independently selected from hydrogen, substituted or unsubstituted alkyl, substituted or unsubstituted heteroalkyl, substituted or unsubstituted aryl and substituted or unsubstituted heteroaryl. When a compound of the invention includes more than one R group, for example, each of the R groups is independently selected as are each R', R", R"' and R"" groups when more than one of these groups is present. In the schemes that follow, the symbol X represents "R" as described above.

Two of the substituents on adjacent atoms of the aryl or heteroaryl ring may optionally be replaced with a substituent of the formula -T-C(O)-(CRR')ᵤ-U-, wherein T and U are independently -NR-, -O-, -CRR'- or a single bond, and u is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula-(CRR')_{z}-X-(CR"R'")_{d}-, where z and d are independently integers of from 0 to 3, and X is -O-, -NR'-, -S-, -S(O)-, -S(O)₂-, or -S(O)₂NR'-. The substituents R, R', R" and R"' are preferably independently selected from hydrogen or substituted or unsubstituted (C₁-C₆)alkyl.

As used herein, the term "heteroatom" is meant to include oxygen (O), nitrogen (N), sulfur (S) and silicon (Si).

As used herein, Factor VII peptide refers to both Factor VII and Factor VIIa peptides. The terms generally refer to variants and mutants of these peptides, including addition, deletion, substitution and fusion protein mutants. Where both Factor VII and Factor VIIa are used, the use is intended to be illustrative of two species of the genus "Factor VII peptide".

The invention is meant to include salts of the compounds of the invention which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of base addition salts include sodium, potassium, lithium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science 66: 1-19 (1977)). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compounds in the conventional manner. The parent form of the compound differs from the various salt forms in certain physical properties, such as solubility in polar solvents.

"Salt counterion", as used herein, refers to positively charged ions that associate with a compound of the invention when one of its moieties is negatively charged (e.g. COO-). Examples of salt counterions include H⁺, H₃O⁺, ammonium, potassium, calcium, lithium, magnesium and sodium.

As used herein, the term "CMP-SA-PEG" is a cytidine monophosphate molecule which is conjugated to a sialic acid which comprises a polyethylene glycol moiety. If a length of the polyethylene glycol chain is not specified, then any PEG chain length is possible (e.g. 1kD, 2 kD, 5 kD, 10 kD, 20 kD, 30 kD, 40 kD). An exemplary CMP-SA-PEG is compound 5 in Scheme 1.

### I. Introduction

To improve the effectiveness of recombinant peptides used for therapeutic purposes, the present invention provides conjugates of glycosylated and unglycosylated peptides with a modifying group. The modifying groups can be selected from polymeric modifying groups such as, e.g., PEG (m-PEG), PPG (m-PPG), etc., therapeutic moieties, diagnostic moieties, targeting moieties and the like. Modification of the peptides, e.g., with a water-soluble polymeric modifying group can improve the stability and retention time of the recombinant peptides in a patient's circulation, and/or reduce the antigenicity of recombinant peptides.

The peptide conjugates of the invention can be formed by the enzymatic attachment of a modified sugar to the glycosylated or unglycosylated peptide. A glycosylation site and/or a modified glycosyl group provides a locus for conjugating a modified sugar bearing a modifying group to the peptide, e.g., by glycoconjugation.

The methods of the invention also make it possible to assemble peptide conjugates and glycopeptide conjugates that have a substantially homogeneous derivatization pattern. The enzymes used in the invention are generally selective for a particular amino acid residue, combination of amino acid residues, particular glycosyl residues, or combination of glycosyl residues of the peptide. The methods are also practical for large-scale production of peptide conjugates. Thus, the methods of the invention provide a practical means for large-scale preparation of peptide conjugates having preselected uniform derivatization patterns. The methods are particularly well suited for modification of therapeutic peptides, including but not limited to, glycopeptides that are incompletely glycosylated during production in cell culture cells (*e.g*., mammalian cells, insect cells, plant cells, fungal cells, yeast cells, or prokaryotic cells) or transgenic plants or animals.

The present invention also provides conjugates of glycosylated and unglycosylated peptides with increased therapeutic half-life due to, for example, reduced clearance rate, or reduced rate of uptake by the immune or reticuloendothelial system (RES). Moreover, the methods of the invention provide a means for masking antigenic determinants on peptides, thus reducing or eliminating a host immune response against the peptide. Selective attachment of targeting agents can also be used to target a peptide to a particular tissue or cell surface receptor that is specific for the particular targeting agent.

Determining optimal conditions for the preparation of peptide conjugates with water-soluble polymers, e.g., involves the optimization of numerous parameters, which are dependent on the identity of the peptide and of the water-soluble polymer. For example, when the polymer is poly(ethylene glycol), e.g., a branched poly(ethylene glycol), a balance is preferably established between the amount of polymer utilized in the reaction and the viscosity of the reaction mixture attributable to the presence of the polymer: if the polymer is too highly concentrated, the reaction mixture becomes viscous, slowing the rate of mass transfer and reaction.

Furthermore, though it is intuitively apparent to add an excess of enzyme, the present inventors have recognized that, when the enzyme is present in too great of an excess, the excess enzyme becomes a contaminant whose removal requires extra purification steps and material and unnecessarily increases the cost of the final product.

Moreover, it is generally desired to produce a peptide with a controlled level of modification. In some instances, it is desireable to add one modified sugar preferentially. In other instances, it is desireable to add two modified sugars preferentially. Thus, the reaction conditions are preferably controlled to influence the degree of conjugation of the modifying groups to the peptide.

The present invention provides conditions under which the yield of a peptide, having the desired level of conjugation, is maximized. The conditions in the exemplary embodiments of the inventions also recognize the expense of the various reagents and the materials and time necessary to purify the product: the reaction conditions set forth herein are optimized to provide excellent yields of the desired product, while minimizing waste of costly reagents.

### II. The Compositions of Matter/Peptide Conjugates

In a first aspect, the present invention provides a conjugate between a modified sugar and a peptide. The present invention also provides a conjugate between a modifying group and a peptide. A peptide conjugate can have one of several forms. In an exemplary embodiment, a peptide conjugate can comprise a peptide and a modifying group linked to an amino acid of the peptide through a glycosyl linking group. In another exemplary embodiment, a peptide conjugate can comprise a peptide and a modifying group linked to a glycosyl reside of the peptide through a glycosyl linking group. In another exemplary embodiment, the peptide conjugate can comprise a peptide and a glycosyl linking group which is bound to both a glycopeptide carbohydrate and directly to an amino acid residue of the peptide backbone. In yet another exemplary embodiment, a peptide conjugate can comprise a peptide and a modifying group linked directly to an amino acid residue of the peptide. In this embodiment, the peptide conjugate may not comprise a glycosyl group. In any of these embodiments, the peptide may or not be glycosylated.

The conjugates of the invention will typically correspond to the general structure: 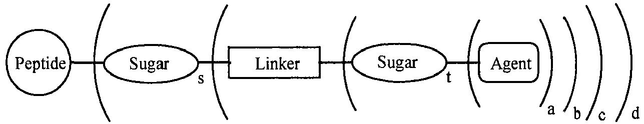 in which the symbols a, b, c, d and s represent a positive, non-zero integer; and t is either 0 or a positive integer. The "agent", or modifying group, can be a therapeutic agent, a bioactive agent, a detectable label, a polymeric modifying group such as a water-soluble polymer (*e.g*., PEG, m-PEG, PPG, and m-PPG) or the like. The "agent", or modifying group, can be a peptide, *e.g*., enzyme, antibody, antigen, etc. The linker can be any of a wide array of linking groups, *infra.* Alternatively, the linker may be a single bond or a "zero order linker."

### II. A. Peptide

The peptide in the peptide conjugate is Factor IX.

In an exemplary embodiment, the polymeric modifying group has a structure including a moiety according to the following formulae:

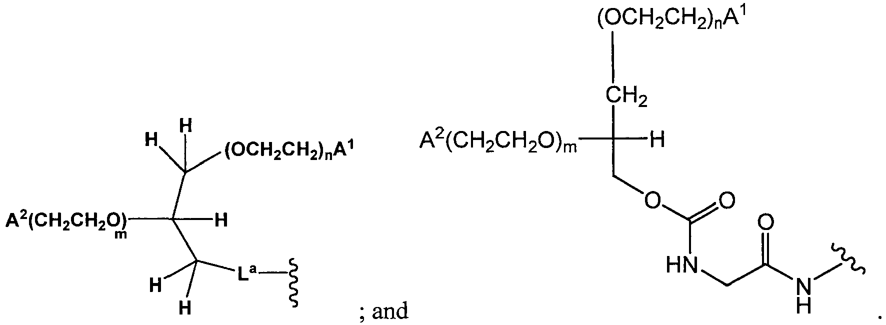

In an exemplary embodiment, m and n are integers independently selected from about 1 to about 5000, preferably from about 100 to about 4000, more preferably from about 200 to about 3000, even more preferably from about 300 to about 2000 and still more preferably from about 400 to about 1000. In an exemplary embodiment, m and n are integers independently selected from about 1 to about 500. In an exemplary embodiment, m and n are integers independently selected from about 1 to about 70, about 70 to about 150, about 150 to about 250, about 250 to about 375 and about 375 to about 500. In an exemplary embodiment, m and n are integers independently selected from about 10 to about 35, about 45 to about 65, about 95 to about 130, about 210 to about 240, about 310 to about 370 and about 420 to about 480. In an exemplary embodiment, m and n are integers selected from about 15 to about 30. In an exemplary embodiment, m and n are integers selected from about 50 to about 65. In an exemplary embodiment, m and n are integers selected from about 100 to about 130. In an exemplary embodiment, m and n are integers selected from about 210 to about 240. In an exemplary embodiment, m and n are integers selected from about 310 to about 370. In an exemplary embodiment, m and n are integers selected from about 430 to about 470. In an exemplary embodiment, A¹ and A² are each members selected from -OH and -OCH₃.

Exemplary polymeric modifying groups according to this embodiment include the moiety:

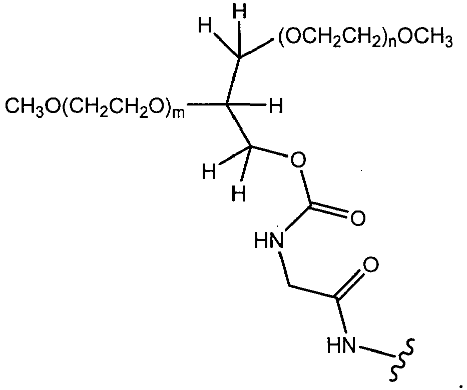

In an exemplary embodiment, in which the modifying group is a branched water-soluble polymer, such as those shown above, it is generally preferred that the concentration of sialidase is about 1.5 to about 2.5 U/L of reaction mixture. More preferably the amount of sialidase is about 2 U/L.

In another exemplary embodiment, about 5 to about 9 grams of peptide substrate is contacted with the amounts of sialidase set forth above.

The modified sugar is present in the reaction mixture in an amount from about 1 gram to about 6 grams, preferably from about 3 grams to about 4 grams. It is generally preferred to maintain the concentration of a modified sugar having a branched water-soluble polymer modifying moiety, e.g., the moiety shown above, at less than about 0.5 mM.

In certain embodiments, the modifying group is a branched poly(alkylene oxide), e.g., poly(ethylene glycol), having a molecular weight from about 20 kD to about 60 kD, more preferably, from about 30 kD to about 50 kD, and even more preferably about 40 kD. In other embodiments, the modifying group is a branched poly(alkylene oxide), e.g., poly(ethylene glycol), having a molecular weight of at least about 80 kD, preferably at least about 100 kD, more preferably at least about 120 kD, at least about 140 kDor at least about 160 kD. In yet another embodiment, the branched poly(alkylene oxide), e.g., poly(ethylene glycol) is at least about 200 kD, such as from at least about 80 kD to at least about 200 kD, including at least about 160 kD and at least about 180 kD. As those of skill will appreciate, the molecular weight of polymers is often polydisperse, thus, the phrase "about" in the context of molecular weight preferably encompasses a range of values around the stated number. For example, a preferred modifying group having a molecular weight of about 40 kD is one that has a molecular weight from about 35 kD to about 45 kD. Those of skill will appreciate that the reliance on branched PEG structures set forth above is simply for clarity of illustration, the PEG can be replaced by substantially any polymeric moiety, including, without limitation those species set forth in the definition of "polymeric moiety" found herein.

Regarding the glycosyltransferase concentration, in a presently preferred embodiment, using the modifying group set forth above, the ratio of glycosyltransferase to peptide is about 40 µg/mL transferase to about 200 µM peptide.

### II. B. Modified Sugar

In an exemplary embodiment, the peptides of the invention, namely Factor IX listed in FIG. 7, are reacted with a modified sugar, thus forming a peptide conjugate. A modified sugar comprises a "sugar donor moiety" as well as a "sugar transfer moiety". The sugar donor moiety is any portion of the modified sugar that will be attached to the peptide, either through a glycosyl moiety or amino acid moiety, as a conjugate of the invention. The sugar donor moiety includes those atoms that are chemically altered during their conversion from the modified sugar to the glycosyl linking group of the peptide conjugate. The sugar transfer moiety is any portion of the modified sugar that will be not be attached to the peptide as a conjugate of the invention. For example, a modified sugar of the invention is the PEGylated sugar nucleotide, PEG-sialic acid CMP. For PEG-sialic acid CMP, the sugar donor moiety, or PEG-sialyl donor moiety, comprises PEG-sialic acid while the sugar transfer moiety, or sialyl transfer moiety, comprises CMP.

In modified sugars of use in the invention, the saccharyl moiety is preferably a saccharide, a deoxy-saccharide, an amino-saccharide, or an N-acyl saccharide. The term "saccharide" and its equivalents, "saccharyl," "sugar," and "glycosyl" refer to monomers, dimers, oligomers and polymers. The sugar moiety is also functionalized with a modifying group. The modifying group is conjugated to the saccharyl moiety, typically, through conjugation with an amine, sulfhydryl or hydroxyl, e.g., primary hydroxyl, moiety on the sugar. In an exemplary embodiment, the modifying group is attached through an amine moiety on the sugar, e.g., through an amide, a urethane or a urea that is formed through the reaction of the amine with a reactive derivative of the modifying group.

Any saccharyl moiety can be utilized as the sugar donor moiety of the modified sugar. The saccharyl moiety can be a known sugar, such as mannose, galactose or glucose, or a species having the stereochemistry of a known sugar. The general formulae of these modified sugars are: 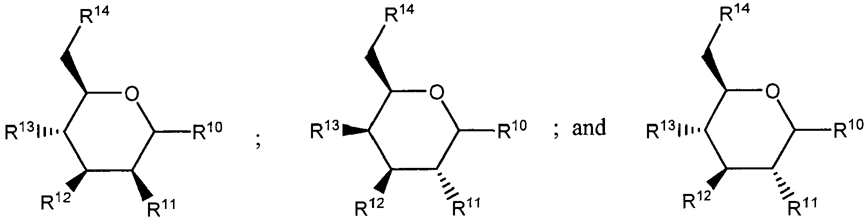 Other saccharyl moieties that are useful in forming the compositions of the invention include, but are not limited to fucose and sialic acid, as well as amino sugars such as glucosamine, galactosamine, mannosamine, the 5-amine analogue of sialic acid and the like. The saccharyl moiety can be a structure found in nature or it can be modified to provide a site for conjugating the modifying group. For example, in one embodiment, the modified sugar provides a sialic acid derivative in which the 9-hydroxy moiety is replaced with an amine. The amine is readily derivatized with an activated analogue of a selected modifying group.

Examples of modified sugars of use in the invention are described in PCT Patent Application No. PCT/US05/002522.

In a further exemplary embodiment, the invention utilizes modified sugars in which the 6-hydroxyl position is converted to the corresponding amine moiety, which bears a linker-modifying group cassette such as those set forth above. Exemplary glycosyl groups that can be used as the core of these modified sugars include Glu, Gal, GalNAc, Glc, GlcNAc, Fuc, Xyl, Man, and the like. A representative modified sugar according to this embodiment has the formula: 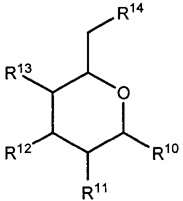 in which R¹¹-R¹⁴ are members independently selected from H, OH, C(O)CH₃, NH, and NH C(O)CH₃. R¹⁰ is a link to another glycosyl residue (-O-glycosyl) or to an amino acid of the Factor VII/Factor VIIa peptide (-NH-(Factor VII/Factor VIIa)). R¹⁴ is OR¹, NHR¹ or NH-L-R¹. R¹ and NH-L-R¹ are as described above.

### II. C. Glycosyl Linking Groups

In an exemplary embodiment, the invention provides a peptide conjugate formed between a modified sugar of the invention and a peptide. The peptide in the peptide conjugate is Factor IX.

In an exemplary embodiment, the polymeric modifying group includes a moiety having a structure according to the following formulae:

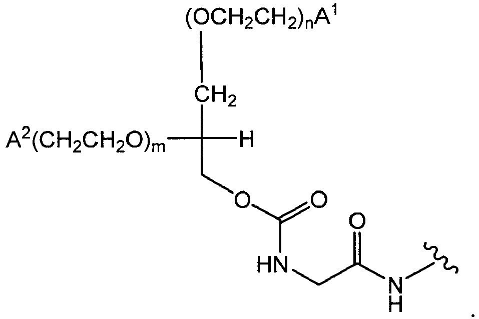

In an exemplary embodiment, modifying group on the modified sugar includes the moiety: 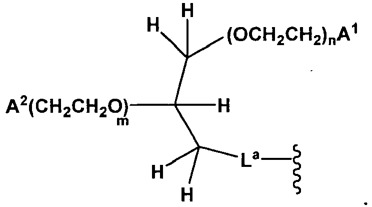 A¹ and A² are each-OCH₃.

Exemplary polymeric modifying groups according to this embodiment include the moiety:

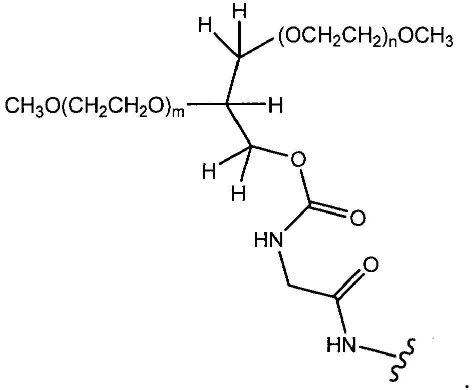

Due to the versatility of the methods available for adding and/or modifying glycosyl residues on a peptide, the glycosyl linking groups can have substantially any structure. In the discussion that follows, the invention is illustrated by reference to the use of selected derivatives of furanose and pyranose. Those of skill in the art will recognize that the focus of the discussion is for clarity of illustration and that the structures and compositions set forth are generally applicable across the genus of glycosyl linking groups and modified sugars. The glycosyl linking group can comprise virtually any mono- or oligo-saccharide. The glycosyl linking groups can be attached to an amino acid either through the side chain or through the peptide backbone. Alternatively the glycosyl linking groups can be attached to the peptide through a saccharyl moiety. This saccharyl moiety can be a portion of an O-linked or N-linked glycan structure on the peptide.

Also disclosed is a peptide conjugate comprising an intact glycosyl linking group having a formula that is selected from: 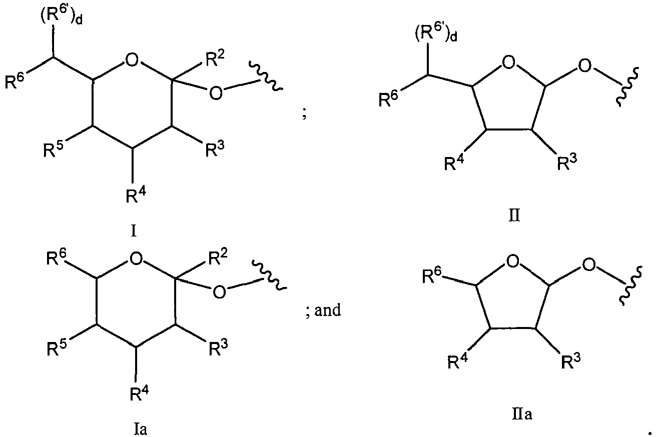 In Formulae I and Ia R² is H, CH₂OR⁷, COOR⁷ or OR⁷, in which R⁷ represents H, substituted or unsubstituted alkyl or substituted or unsubstituted heteroalkyl. When COOR⁷ is a carboxylic acid or carboxylate, both forms are represented by the designation of the single structure COO⁻ or COOH. In Formulae I, Ia, II or IIa, the symbols R³, R⁴, R⁵, R⁶ and R^{6'} independently represent H, substituted or unsubstituted alkyl, OR⁸, NHC(O)R⁹. The index d is 0 or 1. R⁸ and R⁹ are independently selected from H, substituted or unsubstituted alkyl, substituted or unsubstituted heteroalkyl, sialic acid or polysialic acid. At least one of R³, R⁴, R⁵, R⁶ or R^{6'} includes a modifying group. This modifying group can be a polymeric modifying moiety *e.g*., PEG, linked through a bond or a linking group. In an exemplary embodiment, R⁶ and R^{6'}, together with the carbon to which they are attached are components of the pyruvyl side chain of sialic acid. In a further exemplary embodiment, the pyruvyl side chain is functionalized with the polymeric modifying group. In another exemplary embodiment, R⁶ and R^{6'}, together with the carbon to which they are attached are components of the side chain of sialic acid and the polymeric modifying group is a component of R⁵.

In an exemplary embodiment, the invention utilizes a glycosyl linking group that has the formula: 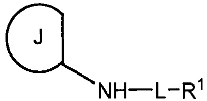 in which J is a glycosyl moiety, L is a bond or a linker and R¹ is a modifying group, e.g., a polymeric modifying group. Exemplary bonds are those that are formed between an NH₂ moiety on the glycosyl moiety and a group of complementary reactivity on the modifying group. For example, when R¹ includes a carboxylic acid moiety, this moiety may be activated and coupled with the NH₂ moiety on the glycosyl residue affording a bond having the structure NHC(O)R¹. J is preferably a glycosyl moiety that is "intact", not having been degraded by exposure to conditions that cleave the pyranose or furanose structure, e.g. oxidative conditions, e.g., sodium periodate.

Exemplary linkers include alkyl and heteroalkyl moieties. The linkers include linking groups, for example acyl-based linking groups, e.g., -C(O)NH-, -OC(O)NH-, and the like. The linking groups are bonds formed between components of the species of the invention, e.g., between the glycosyl moiety and the linker (L), or between the linker and the modifying group (R¹). Other exemplary linking groups are ethers, thioethers and amines. For example, in one embodiment, the linker is an amino acid residue, such as a glycine residue. The carboxylic acid moiety of the glycine is converted to the corresponding amide by reaction with an amine on the glycosyl residue, and the amine of the glycine is converted to the corresponding amide or urethane by reaction with an activated carboxylic acid or carbonate of the modifying group.

An exemplary species of NH-L-R¹ has the formula: -NH{C(O)(CH₂)ₐNH}ₛ{C(O)(CH₂)_{b}(OCH₂CH₂)_{c}O(CH₂)_{d}NH}ₜR¹, in which the indices s and t are independently 0 or 1. The indices a, b and d are independently integers from 0 to 20, and c is an integer from 1 to 2500. Other similar linkers are based on species in which an -NH moiety is replaced by another group, for example, -S, -O or -CH₂. As those of skill will appreciate one or more of the bracketed moieties corresponding to indices s and t can be replaced with a substituted or unsubstituted alkyl or heteroalkyl moiety.

More particularly, the invention utilizes compounds in which NH-L-R¹ is: NHC(O)(CH₂)ₐNHC(O)(CH₂)_{b}(OCH₂CH₂)_{c}O(CH₂)_{d}NHR¹, NHC(O)(CH₂)_{b}(OCH₂CH₂)_{c}O(CH₂)_{d}NHR¹, NHC(O)O(CH₂)_{b}(OCH₂CH₂)_{c}O(CH₂)_{d}NHR¹, NH(CH₂)ₐNHC(O)(CH₂)_{b}(OCH₂CH₂)_{c}O(CH₂)_{d}NHR¹, NHC(O)(CH₂)ₐNHR¹, NH(CH₂)ₐNHR¹, and NHR¹. In these formulae, the indices a, b and d are independently selected from the integers from 0 to 20, preferably from 1 to 5. The index c is an integer from 1 to about 2500.

In an exemplary embodiment, c is selected such that the PEG moiety is approximately 1 kD, 5 kD, 10, kD, 15 kD, 20 kD, 25 kD, 30 kD, 35 kD, 40 kD or 45 kD.

For the purposes of convenience, the glycosyl linking groups in the remainder of this section will be based on a sialyl moiety. However, one of skill in the art will recognize that another glycosyl moiety, such as mannosyl, galactosyl, glucosyl, or fucosyl, could be used in place of the sialyl moiety.

In an exemplary embodiment, the glycosyl linking group is an intact glycosyl linking group, in which the glycosyl moiety or moieties forming the linking group are not degraded by chemical (e.g., sodium metaperiodate) or enzymatic (e.g., oxidase) processes. Selected conjugates of the invention include a modifying group that is attached to the amine moiety of an amino-saccharide, e.g., mannosamine, glucosamine, galactosamine, sialic acid etc. Exemplary modifying group-intact glycosyl linking group cassettes according to this motif are based on a sialic acid structure, such as those having the formulae:

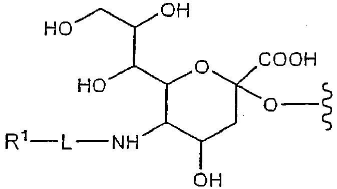

In the formulae above, R¹ and L are as described above. Further detail about the structure of exemplary R¹ groups is provided below.

In still a further exemplary embodiment, the conjugate is formed between a peptide and a modified sugar in which the modifying group is attached through a linker at the 6-carbon position of the modified sugar. Thus, illustrative glycosyl linking groups according to this embodiment have the formula: 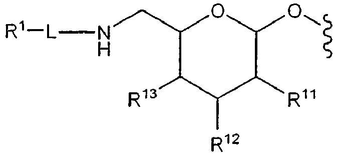 in which the radicals are as discussed above. Glycosyl linking groups include, without limitation, glucose, glucosamine, N-acetyl-glucosamine, galactose, galactosamine, N-acetylgalactosamine, mannose, mannosamine, N-acetyl-mannosamine, and the like.

In one embodiment, the present invention provides a peptide conjugate comprising the following glycosyl linking group: 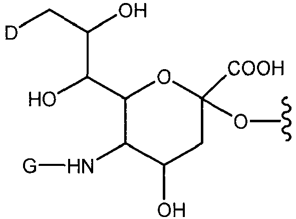 wherein D is -OH; G is a member selected from R¹-L- and -C(O)(C₁-C₆)alkyl; R¹ is a moiety comprising a straight-chain or branched poly(ethylene glycol) residue; and L is a linker, e.g., a bond ("zero order"), substituted or unsubstituted alkyl and substituted or unsubstituted heteroalkyl. In exemplary embodiments, when D is OH, G is R¹-L-, and when G is-C(O)(C₁-C₆)alkyl, D is R¹-L-NH-.

In one embodiment, the present invention provides a peptide conjugate comprising the following glycosyl linking group: 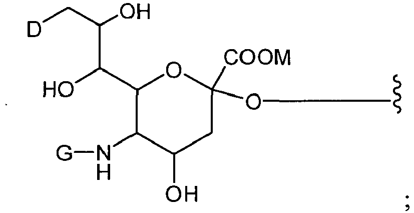
D is -OH;G is a member selected from R¹-L- and -C(O)(C₁-C₆)alkyl-R¹; R¹ is a moiety comprising a member selected from a straight-chain poly(ethylene glycol) residue and branched poly(ethylene glycol) residue; and
M is a member selected from H, a salt counterion and a single negative charge; L is a linker which is a member selected from a bond, substituted or unsubstituted alkyl and substituted or unsubstituted heteroalkyl. In an exemplary embodiment, when D is OH, G is R¹-L-. In another exemplary embodiment, when G is -C(O)(C₁-C₆)alkyl, D is R¹-L-NH-.

In any the compounds of the invention, a COOH group can alternatively be COOM, wherein M is a member selected from H, a negative charge, and a salt counterion.

The invention provides a peptide conjugate that includes a glycosyl linking group having the formula: 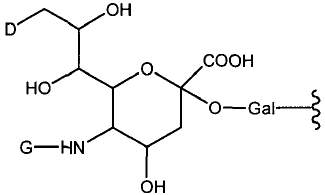 wherein D and G are as described above.

In other embodiments, the glycosyl linking group has the formula: 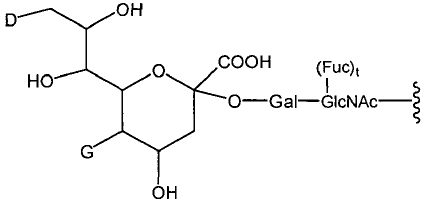 wherein D and G are as described above and the index t is 0 or 1.

In a still further exemplary embodiment, the glycosyl linking group has the formula: 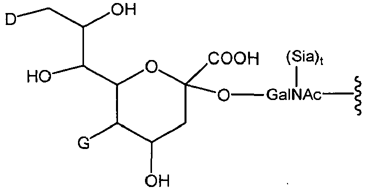 wherein D and G are as described above and the index t is 0 or 1.

In yet another embodiment, the glycosyl linking group has the formula: 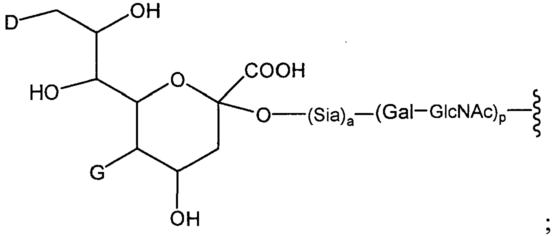 wherein D and G are as described above and the index p represents and integer from 1 to 10; and a is either 0 or 1.

In another exemplary embodiment, the peptide conjugate comprises a glycosyl moiety selected from the formulae: 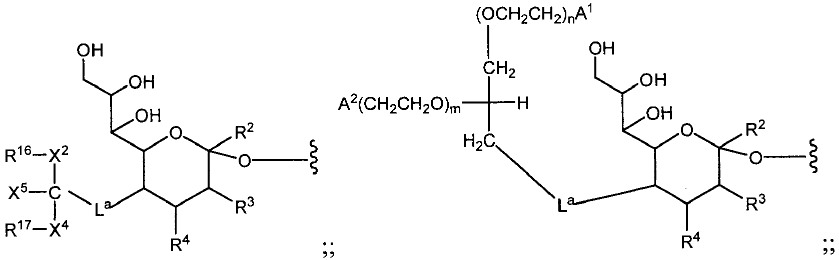 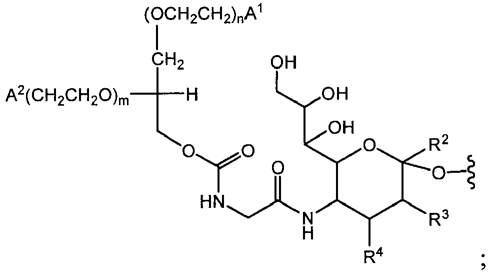 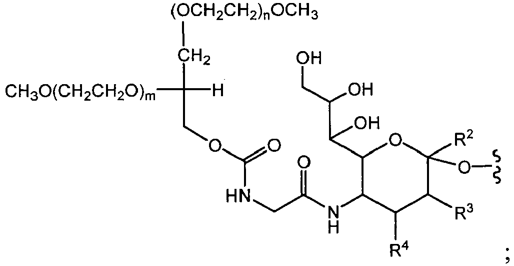 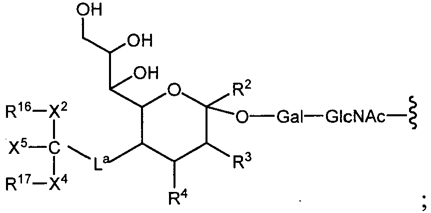 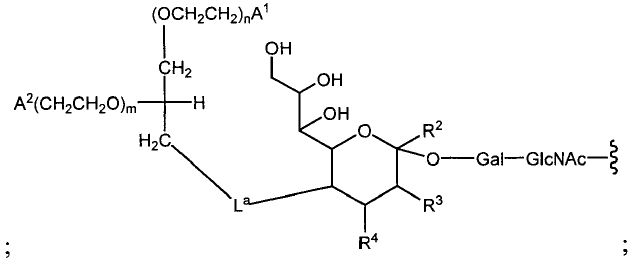 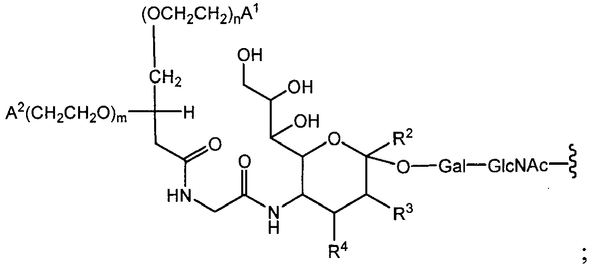 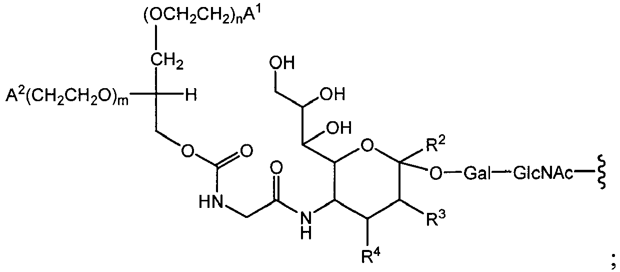 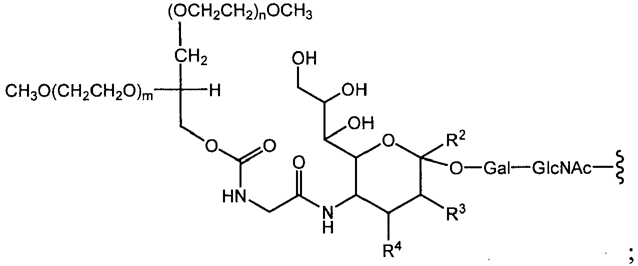 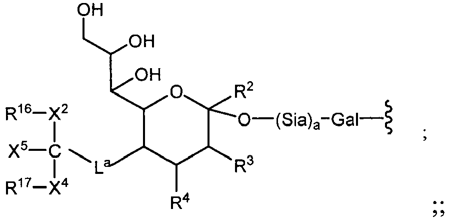 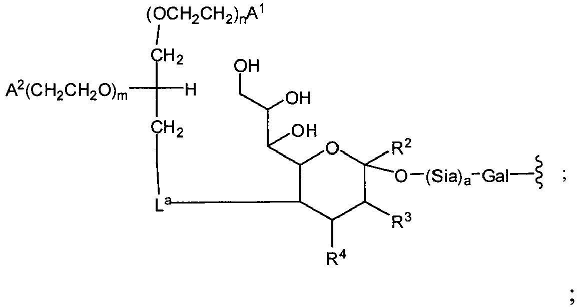 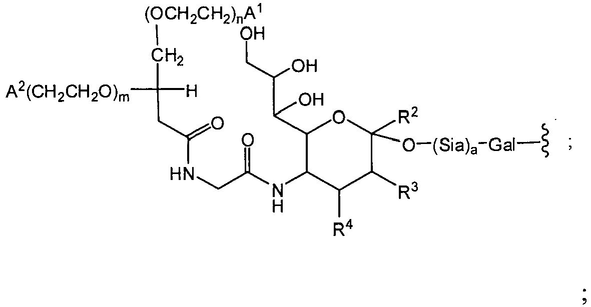 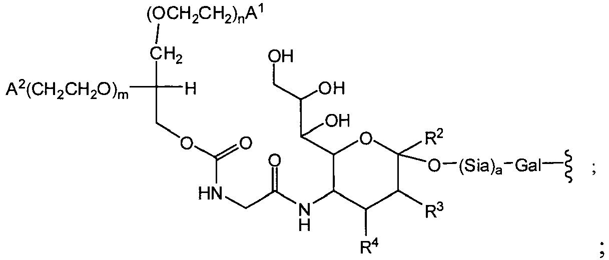 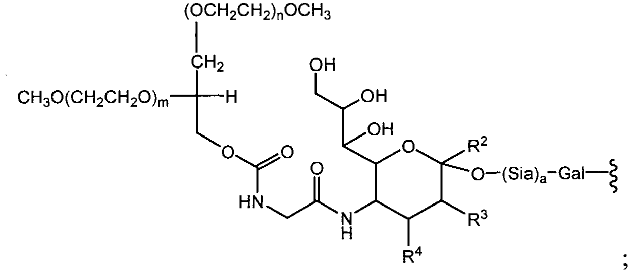 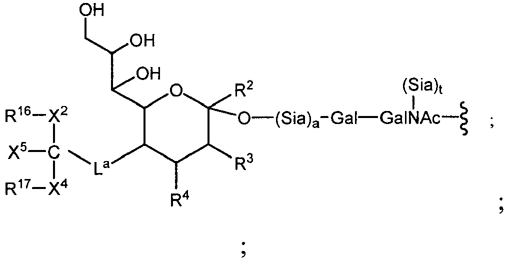 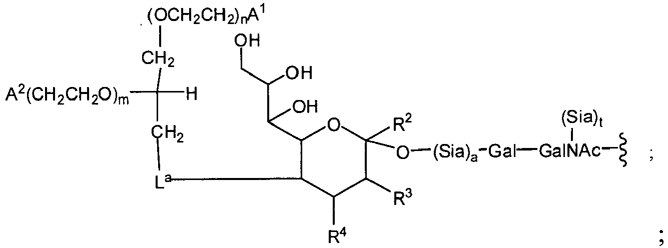 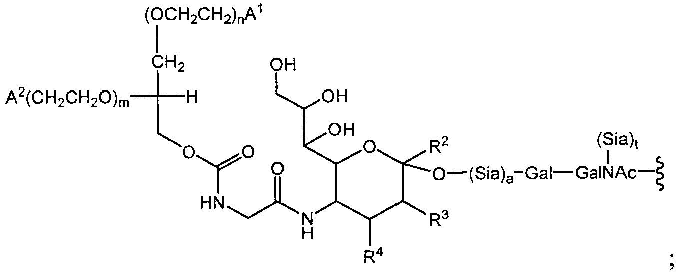 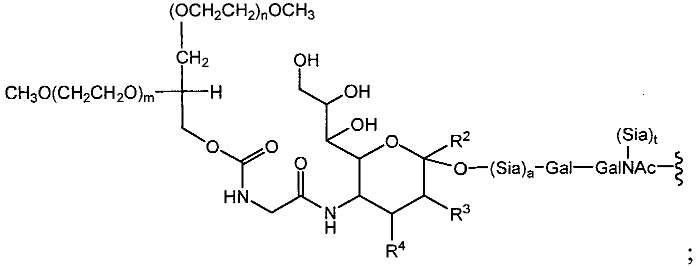 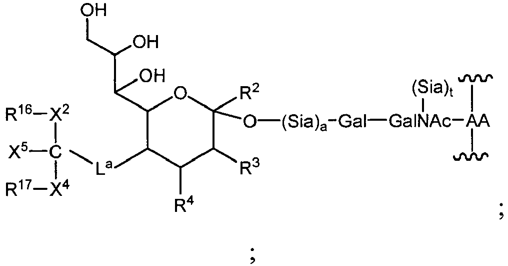 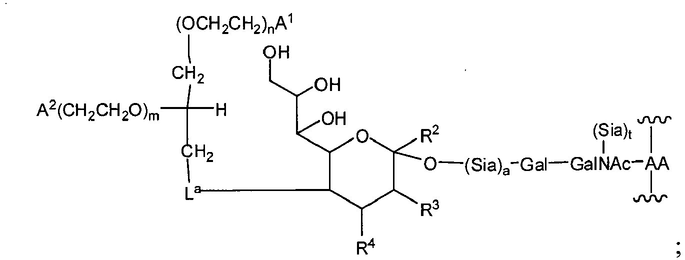 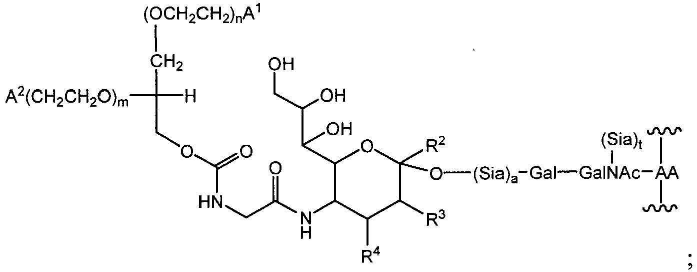 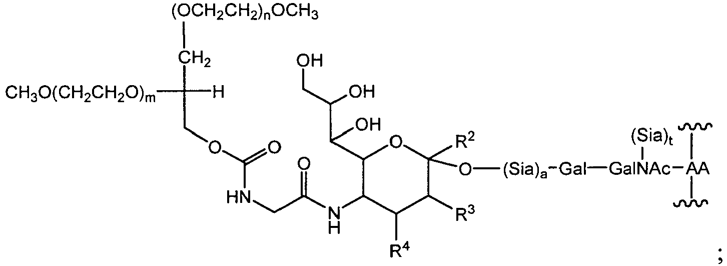 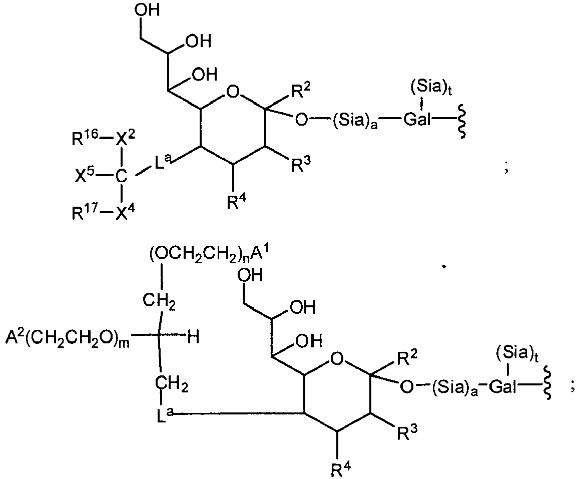 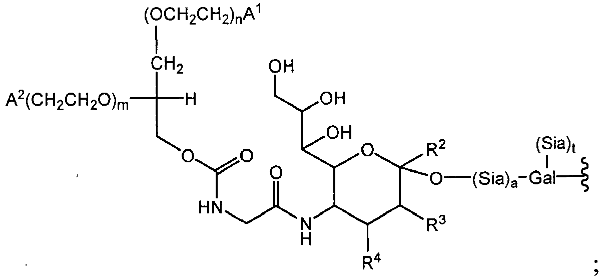 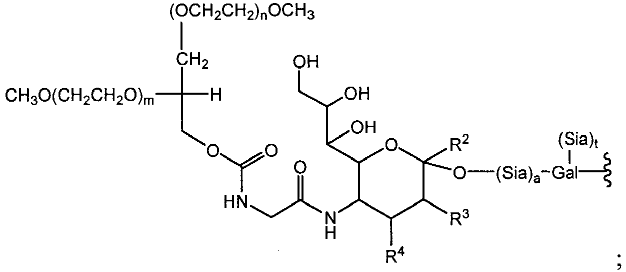 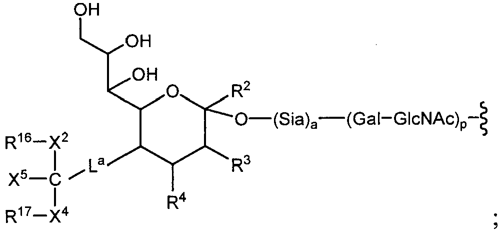 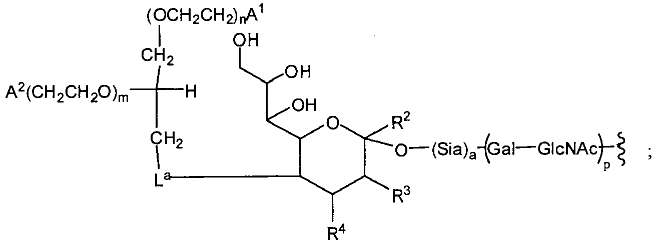 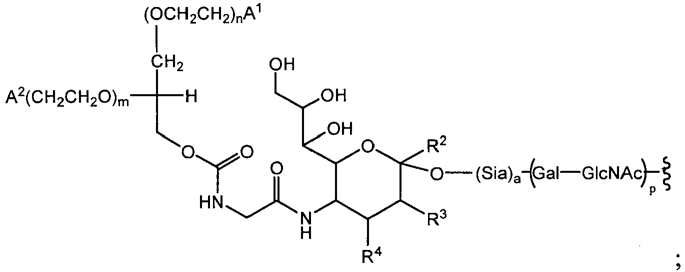 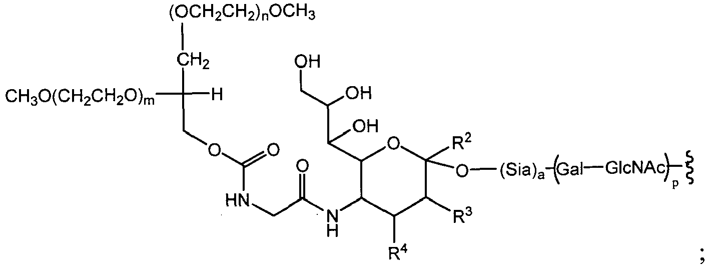 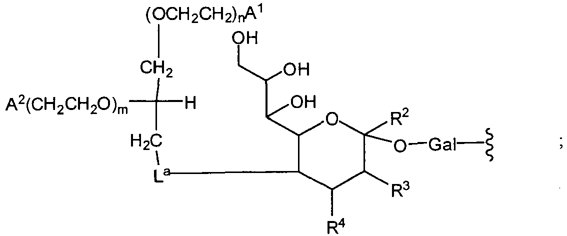 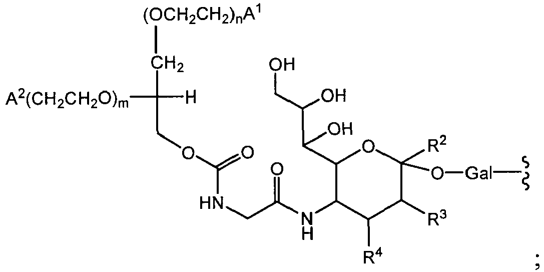 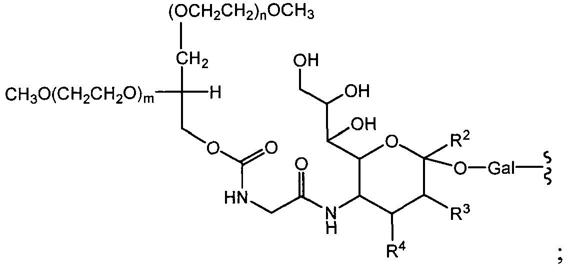 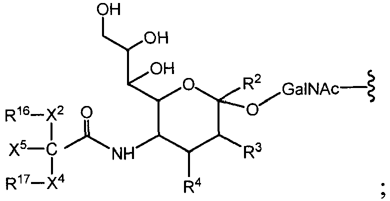 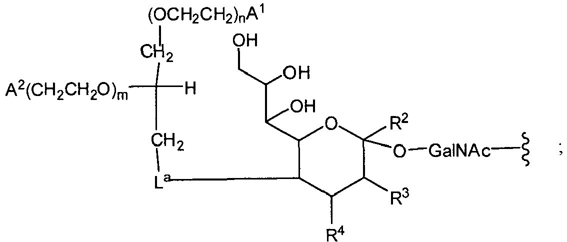 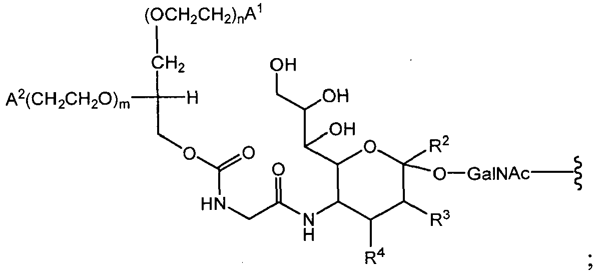 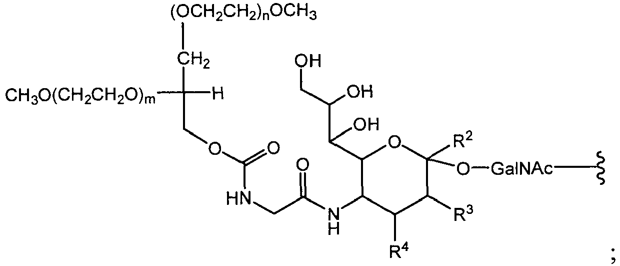 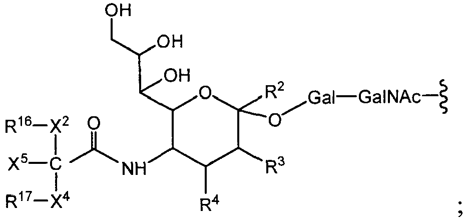 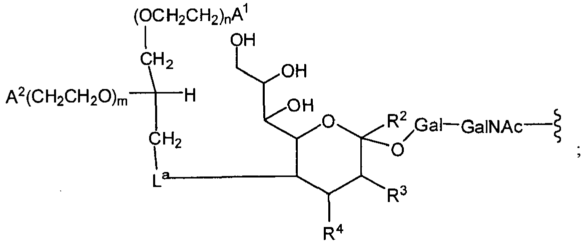 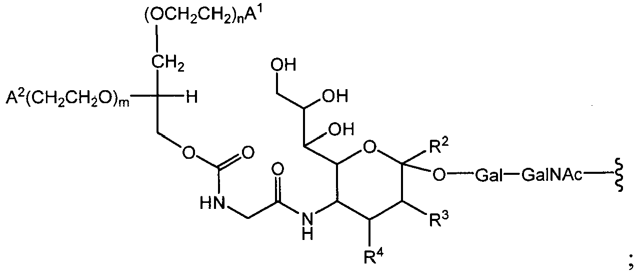 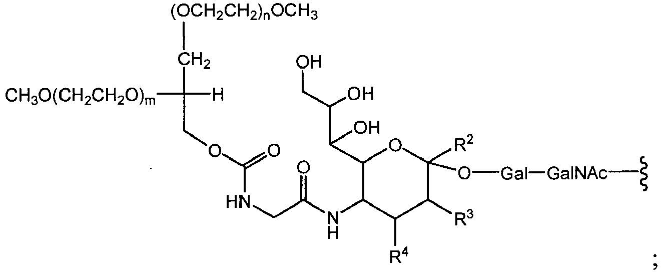 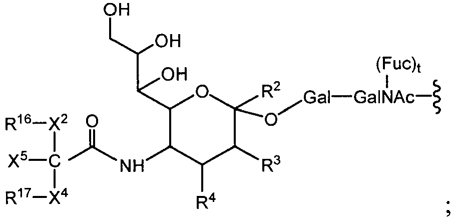 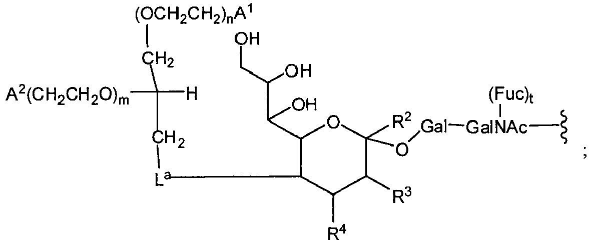 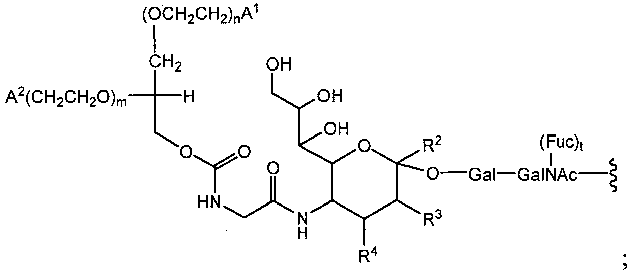 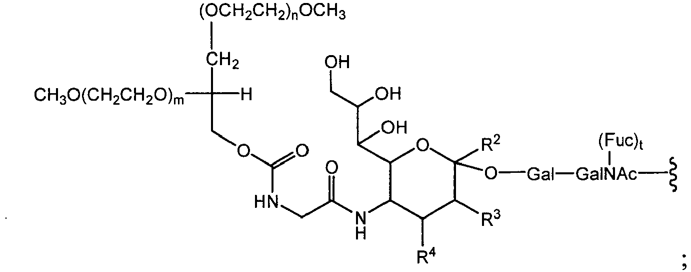 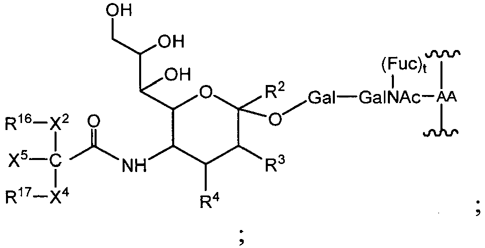 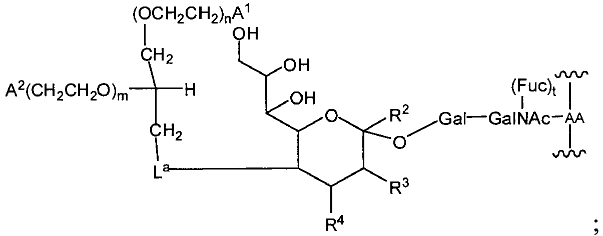 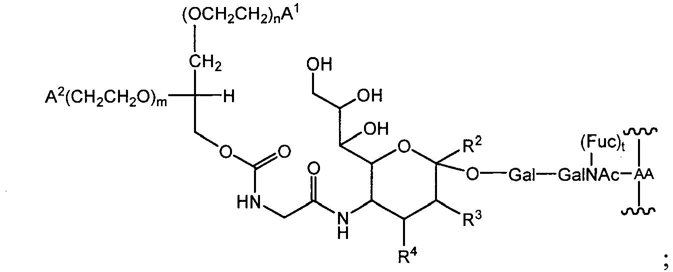 and 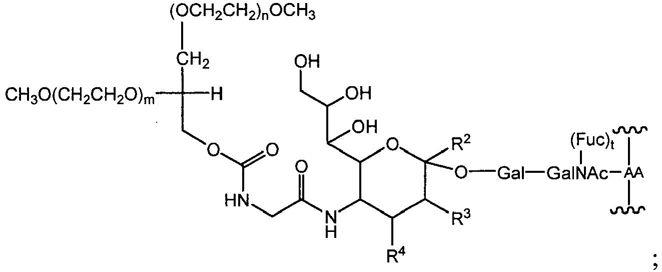 in which the index a and the linker L^{a} are as discussed above. The index p is an integer from 1 to 10. The indices t and a are independently selected from 0 or 1. Each of these groups can be included as components of the mono-, bi-, tri- and tetra-antennary saccharide structures set forth above. AA is an amino acid residue of the peptide. One of skill in the art will appreciate that the PEG moiety in these formulae can be replaced with other non-reactive group and polymeric moieties. Exemplary polymers include those of the poly(alkylene oxide) family. Non-reactive groups include groups that are considered to be essentially unreactive, neutral and/ or stable at physiological pH, e.g., H, substituted or unsubstituted alkyl, substituted or unsubstituted heteroalkyl and the like. An exemplary polymeric moiety includes the branched structures set forth in Formula IIIa and its exemplars.

In an exemplary embodiment, the PEG moiety has a molecular weight of about 20 kD. In another exemplary embodiment, the PEG moiety has a molecular weight of about 5 kD. In another exemplary embodiment, the PEG moiety has a molecular weight of about 10 kD. In another exemplary embodiment, the PEG moiety has a molecular weight of about 40 kD. In other embodiments, the modifying group is a branched poly(alkylene oxide), e.g., poly(ethylene glycol), having a molecular weight of at least about 80 kD, preferably at least about 100 kD, more preferably at least about 120 kD, at least about 140 kDor at least about 160 kD. In yet another embodiment, the branched poly(alkylene oxide), e.g., poly(ethylene glycol) is at least about 200 kD, such as from at least about 80 kD to at least about 200 kD, including at least about 160 kD and at least about 180 kD. In an exemplary embodiment, the branched polymer is itself attached to a branching moiety (e.g., cysteine, serine, lysine, and oligomers of lysine).

In an exemplary embodiment, the glycosyl linking group is a branched SA-PEG-10 kD moiety based on a cysteine residue, and one or two of these glycosyl linking groups are covalently attached to the peptide. In another exemplary embodiment, the glycosyl linking group is a branched SA-PEG-10 kD moiety based on a lysine residue, and one or two of these glycosyl linking groups are covalently attached to the peptide. In an exemplary embodiment, the glycosyl linking group is a branched SA-PEG-10 kD moiety based on a cysteine residue, and one or two of these glycosyl linking groups are covalently attached to the peptide. In an exemplary embodiment, the glycosyl linking group is a branched SA-PEG-10 kD moiety based on a lysine residue, and one or two of these glycosyl linking groups are covalently attached to the peptide. In an exemplary embodiment, the glycosyl linking group is a branched SA-PEG-5 kD moiety based on a cysteine residue, and one, two or three of these glycosyl linking groups are covalently attached to the peptide. In an exemplary embodiment, the glycosyl linking group is a branched SA-PEG-5 kD moiety based on a lysine residue, and one, two or three of these glycosyl linking groups are covalently attached to the peptide. In an exemplary embodiment, the glycosyl linking group is a branched SA-PEG-40 kD moiety based on a cysteine residue, and one or two of these glycosyl linking groups are covalently attached to the peptide. In an exemplary embodiment, the glycosyl linking group is a branched SA-PEG-40 kD moiety based on a lysine residue, and one or two of these glycosyl linking groups are covalently attached to the peptide.

In another exemplary embodiment, the peptide conjugate comprises a glycosyl moiety selected from the formulae: 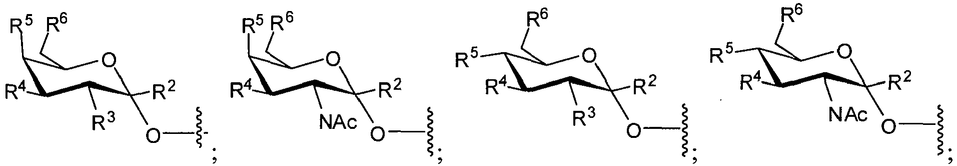 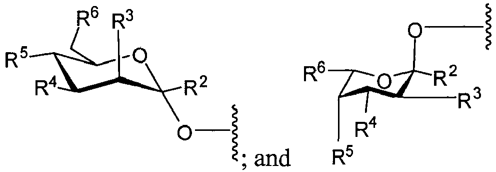 wherein at least one of R², R³, R⁴, R⁵ or R⁶ has a structure which is a member selected from 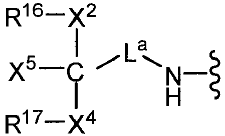 in which the variables are as described above. Those of skill will appreciate that the reliance on branched PEG structures set forth above is simply for clarity of illustration, the PEG can be replaced by substantially any polymeric moiety, including, without limitation those species set forth in the definition of "polymeric moiety" found herein.

In an exemplary embodiment, at least one of R², R³, R⁴, R⁵ or R⁶ has a structure according to the following formula: 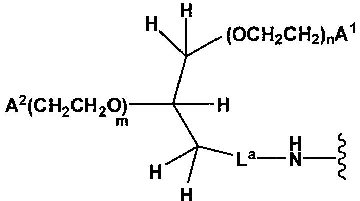 A¹ and A² are each-OCH₃.

Exemplary polymeric modifying groups according to this embodiment include:

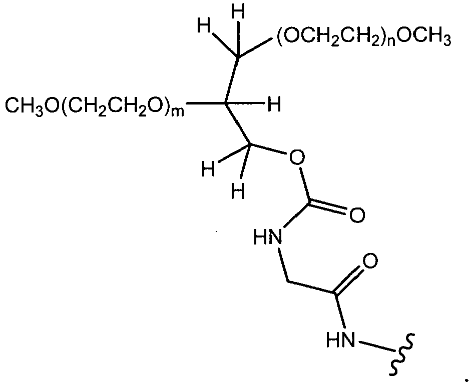

In an exemplary embodiment, only one of R², R³, R⁴, R⁵ or R⁶ has a structure which includes the modifying groups described above.

In another exemplary embodiment, the peptide conjugate comprises a glycosyl moiety selected from the formulae: 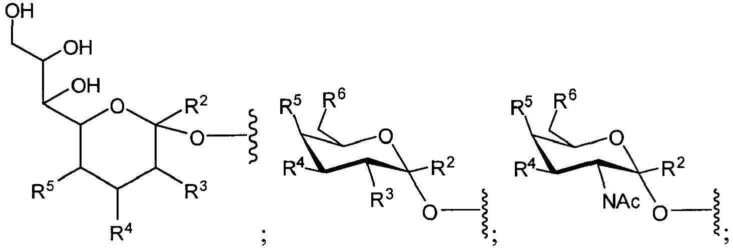 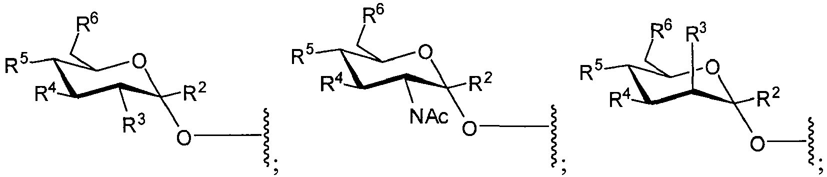 and 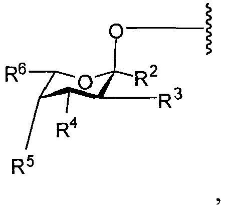 wherein R², R³, R⁴, R⁵ or R⁶ are as described above.

In another exemplary embodiment, the peptide conjugate comprises a glycosyl moiety selected from the formulae: 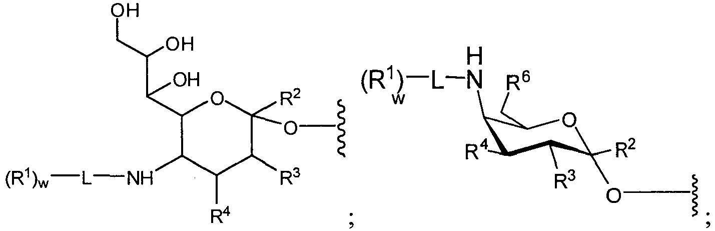 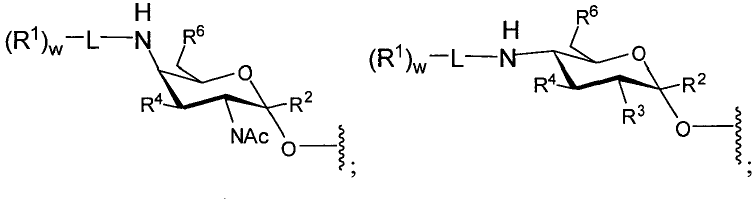 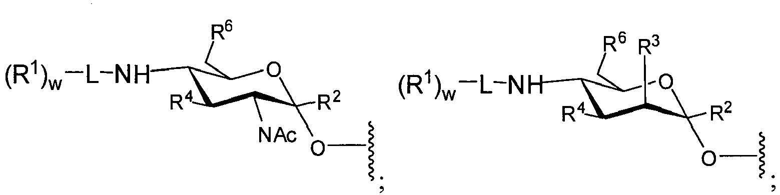 and 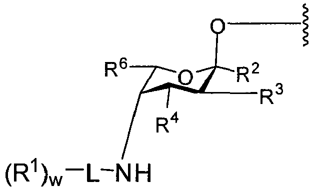 in which L-(R¹)_{w} is a member selected from 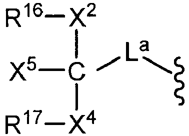 in which the variables are as described above.

In an exemplary embodiment, L-(R¹)_{w} has a structure according to the following formula: 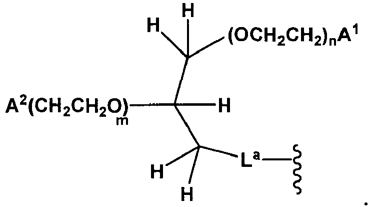 In an exemplary embodiment, A¹ and A² are each-OCH₃.

Exemplary polymeric modifying groups according to this embodiment include: 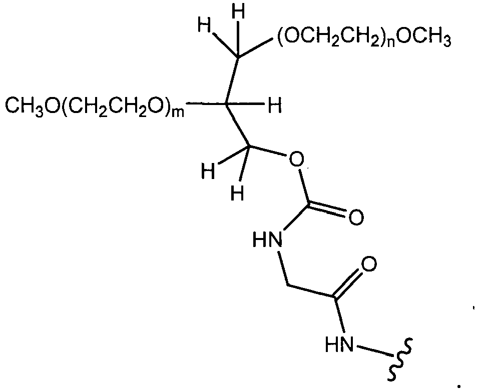 In an exemplary embodiment, m and n are integers independently selected from about 1 to about 1000. In an exemplary embodiment, m and n are integers independently selected from about 1 to about 500. In an exemplary embodiment, m and n are integers independently selected from about 1 to about 70, about 70 to about 150, about 150 to about 250, about 250 to about 375 and about 375 to about 500. In an exemplary embodiment, m and n are integers independently selected from about 10 to about 35, about 45 to about 65, about 95 to about 130, about 210 to about 240, about 310 to about 370 and about 420 to about 480. In an exemplary embodiment, m and n are integers selected from about 15 to about 30. In an exemplary embodiment, m and n are integers selected from about 50 to about 65. In an exemplary embodiment, m and n are integers selected from about 100 to about 130. In an exemplary embodiment, m and n are integers selected from about 210 to about 240. In an exemplary embodiment, m and n are integers selected from about 310 to about 370. In an exemplary embodiment, m and n are integers selected from about 430 to about 470.

In another exemplary embodiment, the peptide conjugate comprises a glycosyl moiety selected from the formulae: 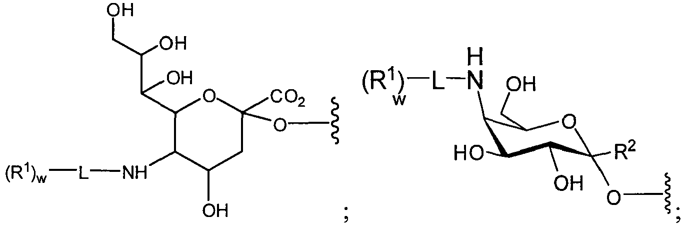 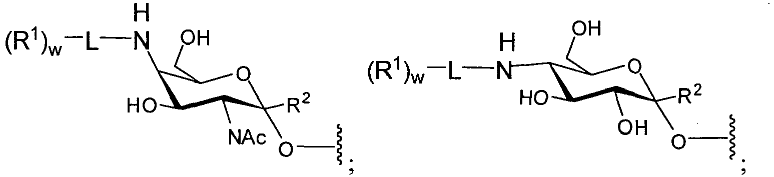 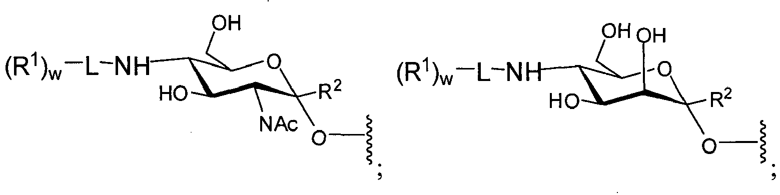 and 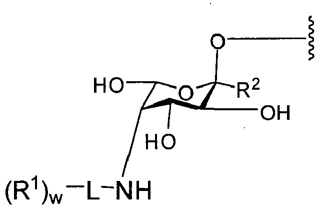 wherein the variables are as described above.

Species according to this embodiment include: 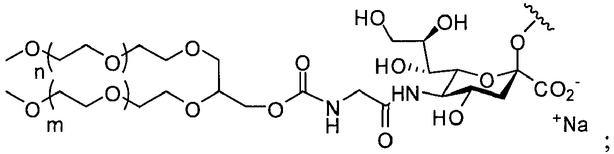 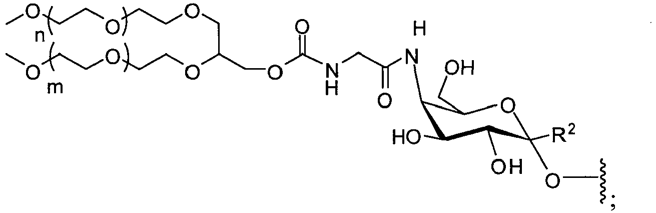 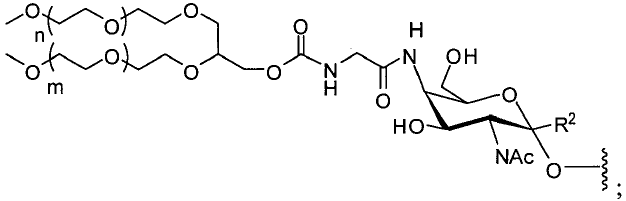 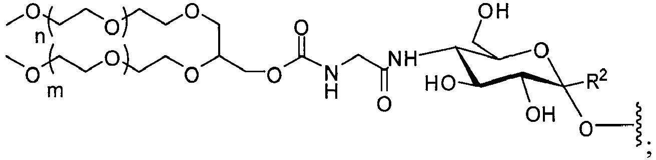 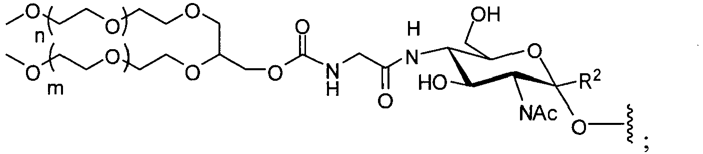 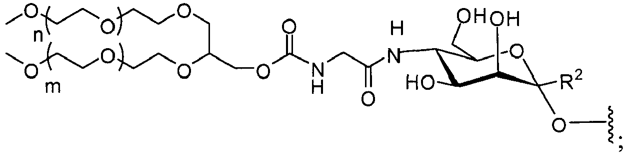 and 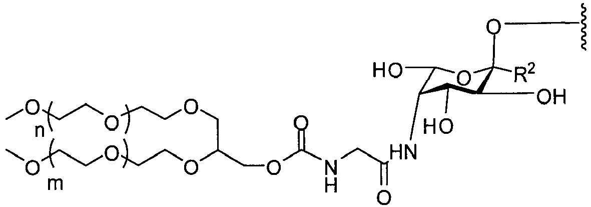 wherein the variables are as discussed above.

In an exemplary embodiment, a glycoPEGylated peptide conjugate of the invention is selected from the formulae set forth below: 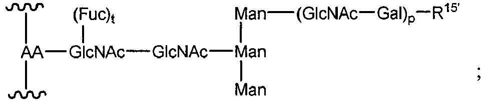 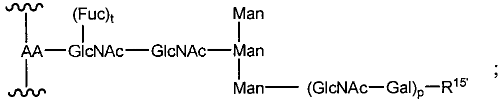 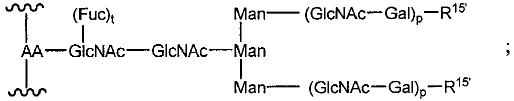 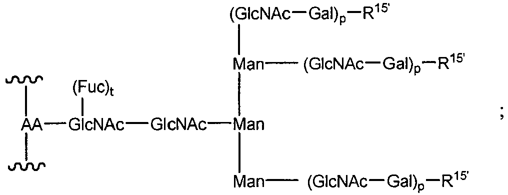 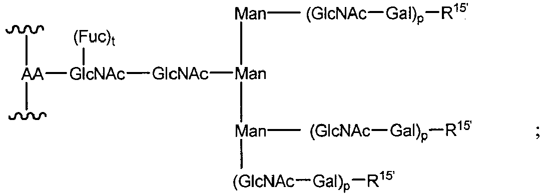 and 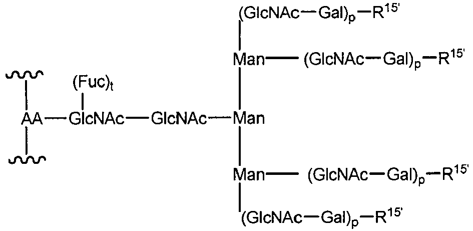 wherein the variables are as described above.

In the formulae above, the index t is an integer from 0 to 1 and the index p is an integer from 1 to 10. The symbol R^{15'} represents H, OH (e.g., Gal-OH), a sialyl moiety, a sialyl linking group (i.e., sialyl linking group-polymeric modifying group (Sia-L-R¹), or a sialyl moiety to which is bound a polymer modified sialyl moiety (e.g., Sia-Sia-L-R¹) ("Sia-Sia^{p}")), a galactosyl moiety, a galactosyl linking group (i.e., galactosyl linking group-polymeric modifying group (Gal-L-R¹), or a sialyl moiety to which is bound a polymer modified galactosyl moiety (e.g., Sia-Gal-L-R¹) ("Sia-Gal^{p}")), a galactosaminyl moiety, a galactosaminyl linking group (i.e., galactosaminyl linking group-polymeric modifying group (GalNAc-L-R¹), or a sialyl moiety to which is bound a polymer modified galactosaminyl moiety (e.g., Sia-GalNAc-L-R¹) ("Sia-GalNAc^{p}")), a glucosyl moiety, a glucosyl linking group (i.e., glucosyl linking group-polymeric modifying group (Glc-L-R¹), or a sialyl moiety to which is bound a polymer modified glucosyl moiety (e.g., Sia-Glc-L-R¹) ("Sia-Glc^{p}")), a glucosaminyl moiety, a glucosaminyl linking group (i.e., glucosaminyl linking group-polymeric modifying group (GlcNAc-L-R¹), or a sialyl moiety to which is bound a polymer modified glucosaminyl moiety (e.g., Sia-GlcNAc-L-R¹) ("Sia-GlcNAc^{p}")), a mannosyl moiety, a mannosyl linking group (i.e., mannosyl linking group-polymeric modifying group (Man-L-R¹), or a sialyl moiety to which is bound a polymer modified mannosyl moiety (e.g., Sia-Man-L-R¹) ("Sia-Man^{p}")), a fucosyl moiety, a fucosyl linking group (i.e., fucosyl linking group-polymeric modifying group (Fuc-L-R¹), or a sialyl moiety to which is bound a polymer modified fucosyl moiety (e.g., Sia-Fuc-L-R¹) ("Sia-Fuc^{p}")). Exemplary polymer modified saccharyl moieties have a structure according to Formulae I, Ia, II or IIa. An exemplary peptide conjugate of the invention will include at least one glycan having a R^{15'} that includes a structure according to Formulae I, Ia, II and IIa. The oxygen, with the open valence, of Formulae I, Ia, II or IIa is preferably attached through a glycosidic linkage to a carbon of a Gal or GalNAc moiety. In a further exemplary embodiment, the oxygen is attached to the carbon at position 3 of a galactose residue. In an exemplary embodiment, the modified sialic acid is linked α2,3-to the galactose residue. In another exemplary embodiment, the sialic acid is linked α2,6-to the galactose residue.

In an exemplary embodiment, the sialyl linking group is a sialyl moiety to which is bound a polymer modified sialyl moiety (e.g., Sia-Sia-L-R¹) ("Sia-Sia^{p}"). Here, the glycosyl linking group is linked to a galactosyl moiety through a sialyl moiety: 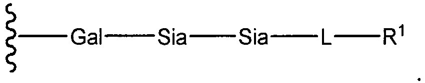 An exemplary species according to this motif is prepared by conjugating Sia-L-R¹ to a terminal sialic acid of a glycan using an enzyme that forms Sia-Sia bonds, e.g., CST-II, ST8Sia-II, ST8Sia-III and ST8Sia-IV.

In another exemplary embodiment, the glycans on the peptide conjugates have a formula that is selected from the group: 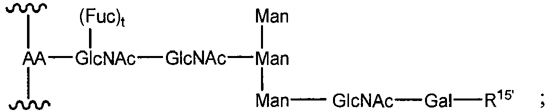 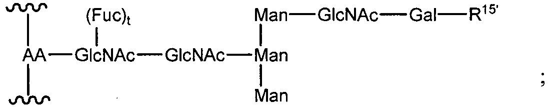 and 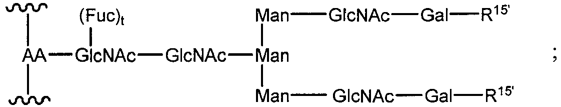 and combinations thereof.

In each of the formulae above, R^{15'} is as discussed above. Moreover, an exemplary peptide conjugate of the invention will include at least one glycan with an R¹⁵ moiety having a structure according to Formulae I, Ia, II or IIa.

In another exemplary embodiment, the glycosyl linking group comprises at least one glycosyl linking group having the formula: 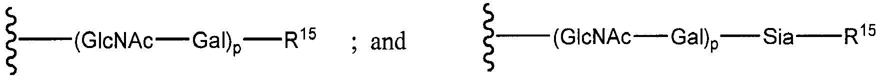 wherein R¹⁵ is said sialyl linking group; and the index p is an integer selected from 1 to 10.

In an exemplary embodiment, the glycosyl linking moiety has the formula: 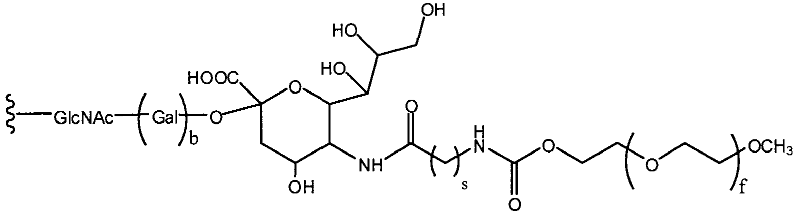 in which b is an integer from 0 to 1. The index s represents an integer from 1 to 10; and the index f represents an integer from 1 to 2500.

In an exemplary embodiment, the polymeric modifying group is PEG. In another exemplary embodiment, the PEG moiety has a molecular weight of about 20 kD. In another exemplary embodiment, the PEG moiety has a molecular weight of about 5 kD. In another exemplary embodiment, the PEG moiety has a molecular weight of about 10 kD. In another exemplary embodiment, the PEG moiety has a molecular weight of about 40 kD. In other embodiments, the modifying group is a branched poly(alkylene oxide), e.g., poly(ethylene glycol), having a molecular weight of at least about 80 kD, preferably at least about 100 kD, more preferably at least about 120 kD, at least about 140 kD or at least about 160 kD. In yet another embodiment, the branched poly(alkylene oxide), e.g., poly(ethylene glycol) is at least about 200 kD, such as from at least about 80 kD to at least about 200 kD, including at least about 160 kD and at least about 180 kD.

In an exemplary embodiment, the glycosyl linking group is a linear SA-PEG-10 kD moiety, and one or two of these glycosyl linking groups are covalently attached to the peptide. In another exemplary embodiment, the glycosyl linking group is a linear SA-PEG-20 kD moiety, and one or two of these glycosyl linking groups are covalently attached to the peptide. In an exemplary embodiment, the glycosyl linking group is a linear SA-PEG-5 kD moiety, and one, two or three of these glycosyl linking groups are covalently attached to the peptide. In an exemplary embodiment, the glycosyl linking group is a linear SA-PEG-40 kD moiety, and one or two of these glycosyl linking groups are covalently attached to the peptide.

In another exemplary embodiment, the glycosyl linking group is a sialyl linking group having the formula: 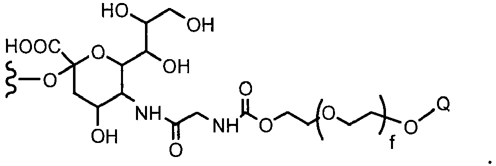 In another exemplary embodiment, Q is a member selected from H and CH₃. In another exemplary embodiment, wherein said glycosyl linking group has the formula: 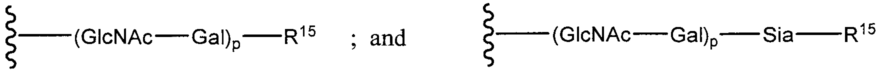 wherein R¹⁵ is said sialyl linking group; and the index p is an integer selected from 1 to 10. In an exemplary embodiment, the glycosyl linking group comprises the formula: 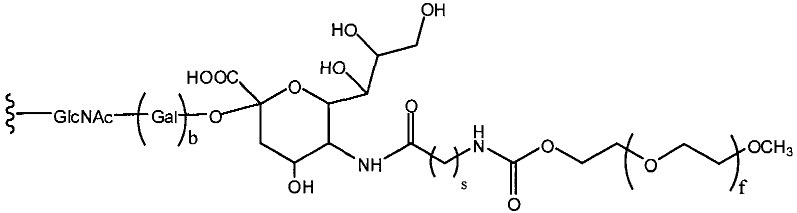 wherein the index b is an integer selected from 0 and 1. In an exemplary embodiment, the index s is 1; and the index f is an integer selceted from about 200 to about 300.

### II. D. Modifying Groups

The peptide conjugates of the invention comprise a modifying group. This group can be covalently attached to a peptide through an amino acid or a glycosyl linking group. The peptide in the peptide conjugate is Factor IX. "Modifying groups" can encompass a variety of structures including targeting moieties, therapeutic moieties, biomolecules. Additionally, "modifying groups" include polymeric modifying groups, which are polymers which can alter a property of the peptide such as its bioavailability or its half-life in the body.

In an exemplary embodiment, the polymeric modifying group has a structure including a moiety according to the following formulae:

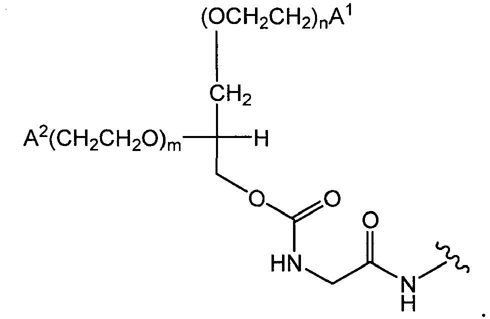

In another exemplary embodiment according to the formula above, the polymeric modifying group includes a moiety according to the following formula: 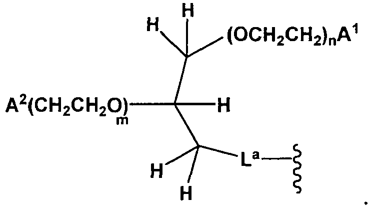 In an exemplary embodiment, A¹ and A² are each members selected from -OH and -OCH₃.

Exemplary polymeric modifying groups according to this embodiment include the moiety:

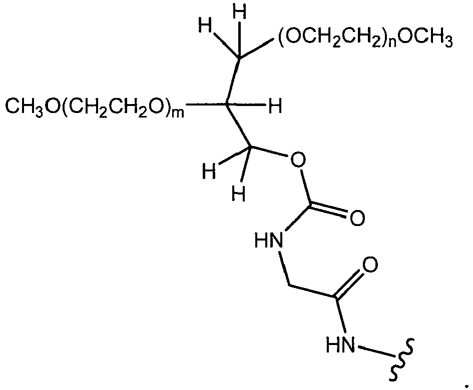

For the purposes of convenience, the modifying groups in the remainder of this section will be largely based on polymeric modifying groups such as water soluble and water insoluble polymers. However, one of skill in the art will recognize that other modifying groups, such as targeting moieties, therapeutic moieties and biomolecules, could be used in place of the polymeric modifying groups. In addition, those of skill will appreciate that the reliance on branched PEG structures set forth above is simply for clarity of illustration, the PEG can be replaced by substantially any polymeric moiety, including, without limitation those species set forth in the definition of "polymeric moiety" found herein.

### II. D. i. Linkers of the Modifying Groups

The linkers of the modifying group serve to attach the modifying group (ie polymeric modifying groups, targeting moieties, therapeutic moieties and biomolecules) to the peptide. In an exemplary embodiment, the polymeric modifying group is bound to a glycosyl linking group, generally through a heteroatom, e.g, nitrogen, on the core through a linker, L, as shown below: 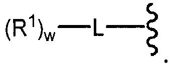 R¹ is the polymeric moiety and L is selected from a bond and a linking group. The index w represents an integer selected from 1-6, preferably 1-3 and more preferably 1-2. Exemplary linking groups include substituted or unsubstituted alkyl, substituted or unsubstituted heteroalkyl moieties and sialic acid. An exemplary component of the linker is an acyl moiety.

An exemplary compound according to the invention has a structure according to Formulae I, Ia, II or IIa above, in which at least one of R², R³, R⁴, R⁵, R⁶ or R^{6'} has the formula:

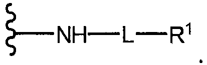

In another example according to this embodiment at least one of R², R³, R⁴, R⁵, R⁶ or R^{6'} has the formula: 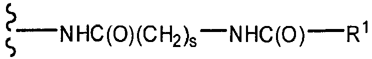 in which s is an integer from 0 to 20 and R¹ is a linear polymeric modifying moiety.

In an exemplary embodiment, the polymeric modifying group -linker construct is a branched structure that includes two or more polymeric chains attached to central moiety. In this embodiment, the construct has the formula: 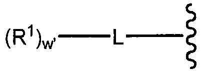 in which R¹ and L are as discussed above and w' is an integer from 2 to 6, preferably from 2 to 4 and more preferably from 2 to 3.

When L is a bond it is formed between a reactive functional group on a precursor of R¹ and a reactive functional group of complementary reactivity on the saccharyl core. When L is a non-zero order linker, a precursor of L can be in place on the glycosyl moiety prior to reaction with the R¹ precursor. Alternatively, the precursors of R¹ and L can be incorporated into a preformed cassette that is subsequently attached to the glycosyl moiety. As set forth herein, the selection and preparation of precursors with appropriate reactive functional groups is within the ability of those skilled in the art. Moreover, coupling the precursors proceeds by chemistry that is well understood in the art.

In an exemplary embodiment, L is a linking group that is formed from an amino acid, or small peptide (e.g., 1-4 amino acid residues) providing a modified sugar in which the polymeric modifying group is attached through a substituted alkyl linker. Exemplary linkers include glycine, lysine, serine and cysteine. The PEG moiety can be attached to the amine moiety of the linker through an amide or urethane bond. The PEG is linked to the sulfur or oxygen atoms of cysteine and serine through thioether or ether bonds, respectively.

In an exemplary embodiment, R⁵ includes the polymeric modifying group. In another exemplary embodiment, R⁵ includes both the polymeric modifying group and a linker, L, joining the modifying group to the remainder of the molecule. As discussed above, L can be a linear or branched structure. Similarly, the polymeric modifying group can be branched or linear.

### II. D. ii. Water-Soluble Polymers

The water-soluble polymer in the conjugates of the present invention is poly(ethylene glycol). Many water-soluble polymers are known to those of skill in the art and are useful. The term water-soluble polymer encompasses species such as saccharides (e.g., dextran, amylose, hyalouronic acid, poly(sialic acid), heparans, heparins, etc.); poly (amino acids), e.g., poly(aspartic acid) and poly(glutamic acid); nucleic acids; synthetic polymers (e.g., poly(acrylic acid), poly(ethers), e.g., poly(ethylene glycol); peptides, proteins, and the like. The present invention is practiced with poly(ethylene glycol)with the sole limitation that the polymer must include a point at which the remainder of the conjugate can be attached.

Methods for activation of polymers can also be found in WO 94/17039, U.S. Pat. No. 5,324,844, WO 94/18247, WO 94/04193, U.S. Pat. No. 5,219,564, U.S. Pat. No. 5,122,614, WO 90/13540, U.S. Pat. No. 5,281,698, and more WO 93/15189, and for conjugation between activated polymers and peptides, e.g. Coagulation Factor VIII (WO 94/15625), hemoglobin (WO 94/09027), oxygen carrying molecule (U.S. Pat. No. 4,412,989), ribonuclease and superoxide dismutase (Veronese at al., App. Biochem. Biotech. 11: 141-45 (1985)).

Exemplary water-soluble polymers are those in which a substantial proportion of the polymer molecules in a sample of the polymer are of approximately the same molecular weight; such polymers are "homodisperse."

The present invention is further illustrated by reference to a poly(ethylene glycol) conjugate. Several reviews and monographs on the functionalization and conjugation of PEG are available. *See,* for example, Harris, Macronol. Chem. Phys. C25: 325-373 (1985); Scouten, Methods in Enzymology 135: 30-65 (1987); Wong et al., Enzyme Microb. Technol. 14: 866-874 (1992); Delgado et al., Critical Reviews in Therapeutic Drug Carrier Systems 9: 249-304 (1992); Zalipsky, Bioconjugate Chem. 6: 150-165 (1995); and Bhadra, et al., Pharmazie, 57:5-29 (2002). Routes for preparing reactive PEG molecules and forming conjugates using the reactive molecules are known in the art. For example, U.S. Patent No. 5,672,662 discloses a water soluble and isolatable conjugate of an active ester of a polymer acid selected from linear or branched poly(alkylene oxides), poly(oxyethylated polyols), poly(olefinic alcohols), and poly(acrylomorpholine).

U.S. Patent No. 6,376,604 sets forth a method for preparing a water-soluble 1-benzotriazolylcarbonate ester of a water-soluble and non-peptidic polymer by reacting a terminal hydroxyl of the polymer with di(1-benzotriazoyl)carbonate in an organic solvent. The active ester is used to form conjugates with a biologically active agent such as a protein or peptide.

WO 99/45964 describes a conjugate comprising a biologically active agent and an activated water soluble polymer comprising a polymer backbone having at least one terminus linked to the polymer backbone through a stable linkage, wherein at least one terminus comprises a branching moiety having proximal reactive groups linked to the branching moiety, in which the biologically active agent is linked to at least one of the proximal reactive groups. Other branched poly(ethylene glycols) are described in WO 96/21469, U.S. Patent No. 5,932,462 describes a conjugate formed with a branched PEG molecule that includes a branched terminus that includes reactive functional groups. The free reactive groups are available to react with a biologically active species, such as a protein or peptide, forming conjugates between the poly(ethylene glycol) and the biologically active species. U.S. Patent No. 5,446,090 describes a bifunctional PEG linker and its use in forming conjugates having a peptide at each of the PEG linker termini.

Conjugates that include degradable PEG linkages are described in WO 99/34833; and WO 99/14259, as well as in U.S. Patent No. 6,348,558. Such degradable linkages are applicable in the present invention.

The art-recognized methods of polymer activation set forth above are of use in the context of the present invention in the formation of the branched polymers set forth herein and also for the conjugation of these branched polymers to other species, e.g., sugars, sugar nucleotides and the like.

An exemplary water-soluble polymer is poly(ethylene glycol), e.g., methoxy-poly(ethylene glycol). The poly(ethylene glycol) used in the present invention is not restricted to any particular form or molecular weight range. For unbranched poly(ethylene glycol) molecules the molecular weight is preferably between 500 and 100,000. A molecular weight of 2000-60,000 is preferably used and preferably of from about 5,000 to about 40,000.

### II. D. iii. Branched Water Soluble Polymers

In another embodiment the polymeric modifying moiety is a branched PEG structure having more than one linear or branched PEG moieties attached. Examples of branched PEGs are described in U.S. Pat. No. 5,932,462; U.S. Pat. No. 5,342,940; U.S. Pat. No. 5,643,575; U.S. Pat. No. 5,919,455; U.S. Pat. No. 6,113,906; U.S. Pat. No. 5,183,660; WO 02/09766; Kodera Y., Bioconjugate Chemistry 5: 283-288 (1994); and Yamasaki et al., Agric. Biol. Chem., 52: 2125-2127, 1998.

Representative polymeric modifying moieties include structures that are based on side chain-containing amino acids, e.g., serine, cysteine, lysine, and small peptides, e.g., lys-lys. In some embodiments, the polymeric modifying moiety is a branched PEG moiety that is based upon an oligo-peptide, such as a tri-lysine peptide. Exemplary amino acids suitable for use include lysine, cysteine, and serine. In such embodiments, each polymeric subunit attached to the peptide structure may be either a linear PEG moiety or a branched PEG moiety. For example, the tri-lysine can be mono-, di-, tri-, or tetra-PEG-ylated with linear PEG moieties, branched PEG moieties, or a combination of linear and branched PEG moieties. Exemplary branched structures include the following moieties: 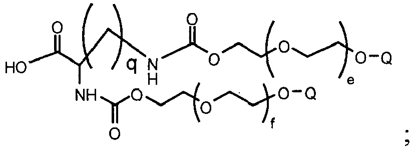 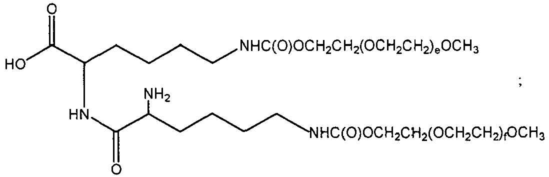 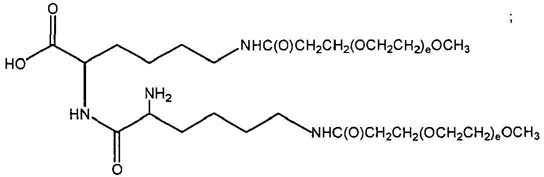 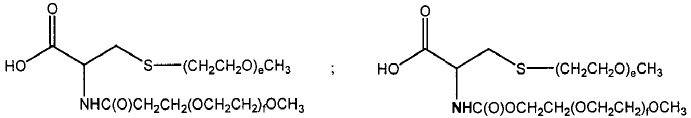 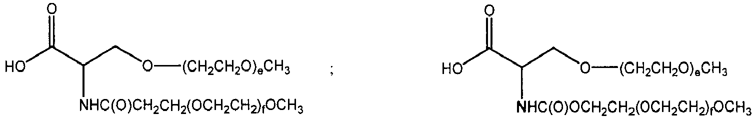 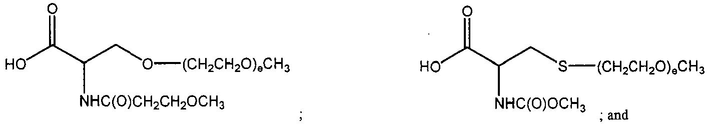 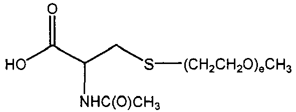 Those of skill will appreciate that the free amine in the di-lysine structures can also be pegylated through an amide or urethane bond with a either a linear PEG moiety or a branched PEG moiety.

It will be appreciated by one of skill in the art that in addition to the linear PEG structures shown above, the branched polymers exemplified in the previous sections can also be attached to a branching moiety (e.g., cysteine, serine, lysine, and oligomers of lysine) in place of one or more of the linear PEG structures. In addition, those of skill will appreciate that the reliance on PEG structures set forth above is simply for clarity of illustration, the PEG can be replaced by substantially any polymeric moiety, including, without limitation those species set forth in the definition of "polymeric moiety" found herein.

PEG of any molecular weight, e.g., 1 kD, 2 kD, 5 kD, 10 kD, 15 kD, 20 kD, 25 kD, 30 kD, 35 kD, 40 kD and 45 kD is of use in the present invention. PEG of a larger molecular weight can also be used in the present invention, including up to about 200 kD, such as at least about 180 kD, about 160 kD, about 140 kD, about 120 kD, about 100 kD, about 90 kD, about 80 kD, and about 70 kD. In certain embodiments the molecular weight of PEG is about 80 kD. In other embodiments, the molecular weight of PEG is at least about 200 kD, at least about 180 kD, at least about 160 kD, or at least about 140 kD.

Each PEG moiety of the branched polymeric modifying moiety may have a molecular weight as defined above or the total molecular weight of all PEG moieties of the polymeric modifying moiety may be as defined above. For example, in certain embodiments each PEG moiety of the branched polymeric modifying moiety may be about 80 kD or the total molecular weight of all PEG moieties of the polymeric modifying moiety may be about 80 kD. Likewise, in certain embodiments each PEG moiety of the branched polymeric modifying moiety may be about 200 kD or the total molecular weight of all PEG moieties of the polymeric modifying moiety may be about 200 kD.

Exemplary species according to this embodiment have the formulae: 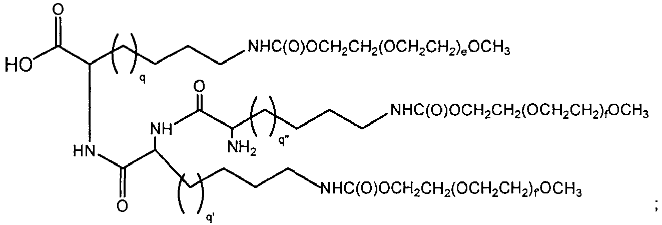 and 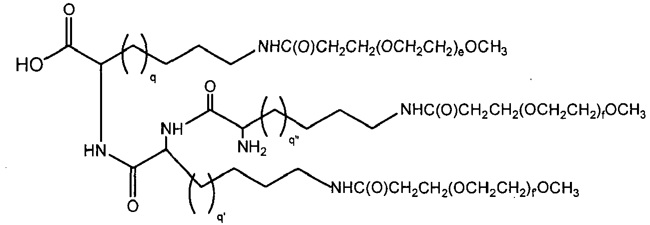 in which the indices e, f and f are independently selected integers from 1 to 2500; and the indices q, q' and q" are independently selected integers from 1 to 20.

As will be apparent to those of skill, the branched polymers of use in the invention include variations on the themes set forth above. For example the di-lysine-PEG conjugate shown above can include three polymeric subunits, the third bonded to the α-amine shown as unmodified in the structure above. Similarly, the use of a tri-lysine functionalized with three or four polymeric subunits labeled with the polymeric modifying moiety in a desired manner is within the scope of the invention.

As discussed herein, the PEG of use in the conjugates of the invention can be linear or branched. An exemplary precursor of use to form the branched PEG containing peptide conjugates according to this embodiment of the invention has the formula:

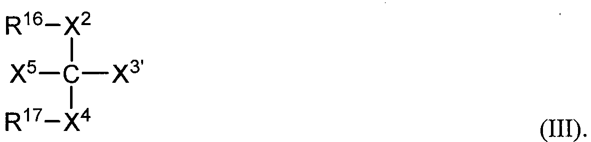

The branched polymer species according to this formula are essentially pure water-soluble polymers. X^{3'} is a moiety that includes an ionizable (e.g., OH, COOH, H₂PO₄, HSO₃, HPO₃, and salts thereof, etc.) or other reactive functional group, e.g., *infra.* C is carbon. X⁵, R¹⁶ and R¹⁷ are independently selected from non-reactive groups and polymeric moieties (e.g. poly(alkylene oxide), e.g., PEG). Non-reactive groups include groups that are considered to be essentially unreactive, neutral and/ or stable at physiological pH, e.g., H, substituted or unsubstituted alkyl, substituted or unsubstituted heteroalkyl and the like. An exemplary polymeric moiety includes the branched structures set forth in Formula IIIa and its exemplars. One of skill in the art will appreciate that the PEG moiety in these formulae can be replaced with other polymers. Exemplary polymers include those of the poly(alkylene oxide) family. (e.g., H, unsubstituted alkyl, unsubstituted heteroalkyl) and polymeric arms (e.g., PEG). X² and X⁴ are linkage fragments that are preferably essentially non-reactive under physiological conditions, which may be the same or different. An exemplary linker includes neither aromatic nor ester moieties. Alternatively, these linkages can include one or more moiety that is designed to degrade under physiologically relevant conditions, e.g., esters, disulfides, etc. X² and X⁴ join polymeric arms R¹⁶ and R¹⁷ to C. When X^{3'} is reacted with a reactive functional group of complementary reactivity on a linker, sugar or linker-sugar cassette, X^{3'} is converted to a component of linkage fragment X³.

Exemplary linkage fragments for X², X³ and X⁴ are independently selected and include S, SC(O)NH, HNC(O)S, SC(O)O, O, NH, NHC(O), (O)CNH and NHC(O)O, and OC(O)NH, CH₂S, CH₂O, CH₂CH₂O, CH₂CH₂S, (CH₂)ₒO, (CH₂)ₒS or (CH₂)ₒY'-PEG wherein, Y' is S, NH, NHC(O), C(O)NH, NHC(O)O, OC(O)NH, or O and o is an integer from 1 to 50. In an exemplary embodiment, the linkage fragments X² and X⁴ are different linkage fragments.

In an exemplary embodiment, the precursor (Formula III), or an activated derivative thereof, is reacted with, and thereby bound to a sugar, an activated sugar or a sugar nucleotide through a reaction between X^{3'} and a group of complementary reactivity on the sugar moiety, e.g., an amine. Alternatively, X^{3'} reacts with a reactive functional group on a precursor to linker, L. One or more of R², R³, R⁴, R⁵, R⁶ or R^{6'} of Formulae I, Ia, II or IIa can include the branched polymeric modifying moiety, or this moiety bound through L.

In an exemplary embodiment, the polymeric modifying group has a structure including a moiety according to the following formulae:

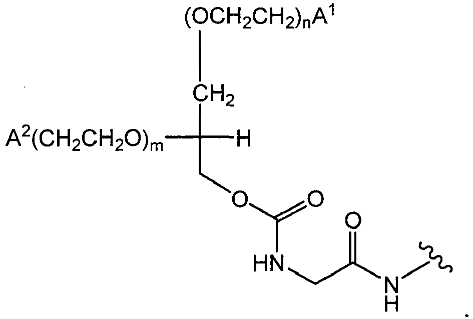

In another exemplary embodiment according to the formula above, the branched polymer has a structure according to the following formula: 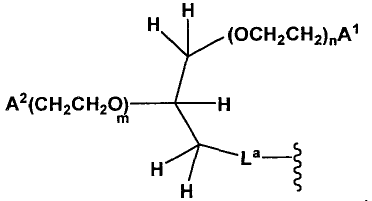 In an exemplary embodiment, A¹ and A² are each selected from -OH and -OCH₃.

Exemplary polymeric modifying groups according to this embodiment include the moiety:

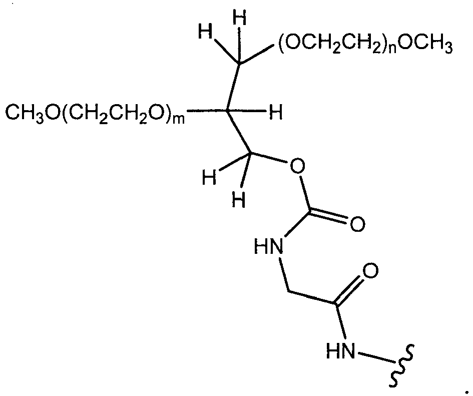

In an exemplary embodiment, the moiety: 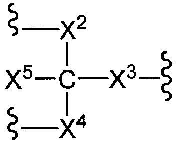 is the linker arm, L. In this embodiment, an exemplary linker is derived from a natural or unnatural amino acid, amino acid analogue or amino acid mimetic, or a small peptide formed from one or more such species. For example, certain branched polymers found in the compounds of the invention have the formula:

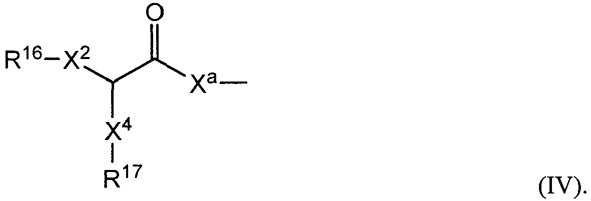

X^{a} is a linkage fragment that is formed by the reaction of a reactive functional group, e.g., X^{3'}, on a precursor of the branched polymeric modifying moiety and a reactive functional group on the sugar moiety, or a precursor to a linker. For example, when X^{3'} is a carboxylic acid, it can be activated and bound directly to an amine group pendent from an amino-saccharide (e.g., Sia, GalNH₂, GlcNH₂, ManNH₂, etc.), forming a X^{a} that is an amide. Additional exemplary reactive functional groups and activated precursors are described hereinbelow. The index c represents an integer from 1 to 10. The other symbols have the same identity as those discussed above.

In another exemplary embodiment, X^{a} is a linking moiety formed with another linker: 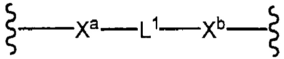 in which X^{b} is a second linkage fragment and is independently selected from those groups set forth for X^{a}, and, similar to L, L¹ is a bond, substituted or unsubstituted alkyl or substituted or unsubstituted heteroalkyl.

Exemplary species for X^{a} and X^{b} include S, SC(O)NH, HNC(O)S, SC(O)O, O, NH, NHC(O), C(O)NH and NHC(O)O, and OC(O)NH.

In another exemplary embodiment, X⁴ is a peptide bond to R¹⁷, which is an amino acid, di-peptide (e.g.,, Lys-Lys) or tri-peptide (e.g., Lys-Lys-Lys) in which the alpha-amine moiety(ies) and/or side chain heteroatom(s) are modified with a polymeric modifying moiety.

In a further exemplary embodiment, the peptide conjugates of the invention include a moiety, e.g., an R¹⁵ moiety that has a formula that is selected from: 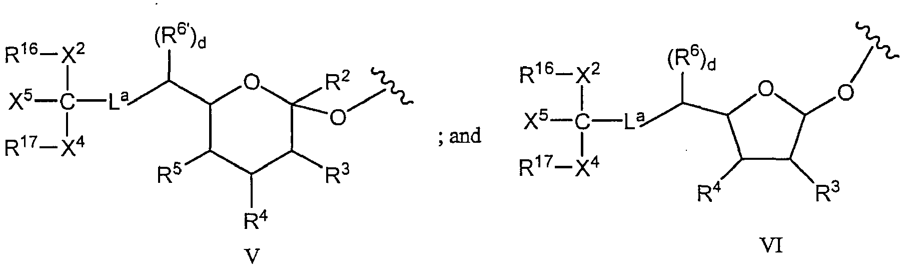 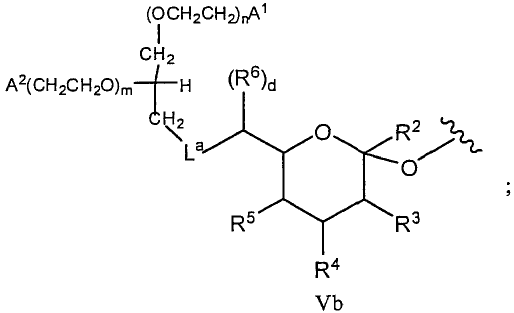 and 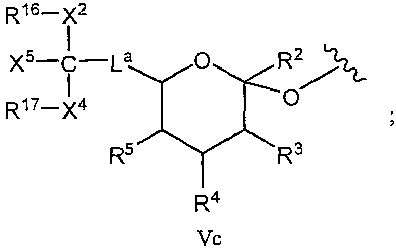 and 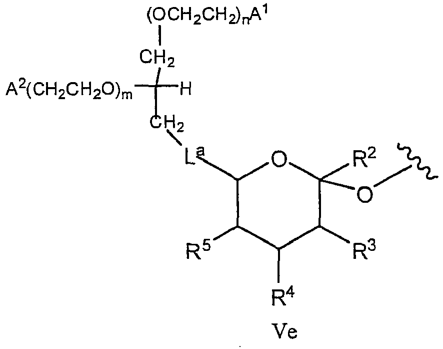 in which the identity of the radicals represented by the various symbols is the same as that discussed hereinabove. L^{a} is a bond or a linker as discussed above for L and L¹, e.g., substituted or unsubstituted alkyl or substituted or unsubstituted heteroalkyl moiety. In an exemplary embodiment, L^{a} is a moiety of the side chain of sialic acid that is functionalized with the polymeric modifying moiety as shown. Exemplary L^{a} moieties include substituted or unsubstituted alkyl chains that include one or more OH or NH₂.

In yet another exemplary embodiment, the invention provides peptide conjugates having a moiety, e.g., an R¹⁵ moiety with formula: 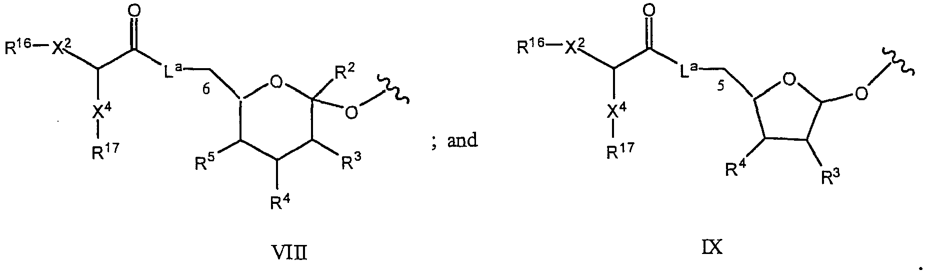 The identity of the radicals represented by the various symbols is the same as that discussed hereinabove. As those of skill will appreciate, the linker arm in Formulae VIII is equally applicable to other modified sugars set forth herein. In exemplary embodiment, the species of Formulae VIII are the R¹⁵ moieties attached to the glycan structures set forth herein.

In yet another exemplary embodiment, the peptide conjugate includes a R¹⁵ moiety with a formula which is a member selected from: 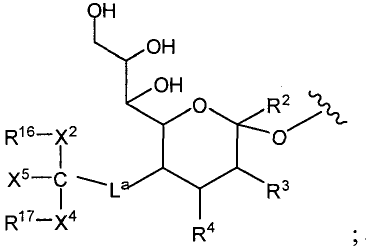 and 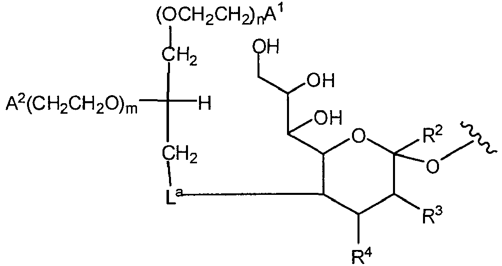 in which the identities of the radicals are as discussed above. An exemplary species for L^{a} is -(CH₂)ⱼC(O)NH(CH₂)ₕC(O)NH-, in which the indices h and j are independently selected integers from 0 to 10. A further exemplary species is -C(O)NH-. The indices m and n are integers independently selected from 0 to 5000. A¹, A², A³, A⁴, A⁵, A⁶, A⁷, A⁸, A⁹, A¹⁰ and A¹¹ are members independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, -NA¹²A¹³, -OA¹² and -SiA¹²A¹³. A¹² and A¹³ are members independently selected from substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl.

The embodiments of the invention set forth above are further exemplified by reference to species in which the polymer is a water-soluble polymer, particularly poly(ethylene glycol) ("PEG"), e.g., methoxy-poly(ethylene glycol ;

Formula IIIa is a subset of Formula III. The structures described by Formula IIIa are also encompassed by Formula III.

In an exemplary embodiment, the polymeric modifying group has a structure including a moiety according to the following formulae:

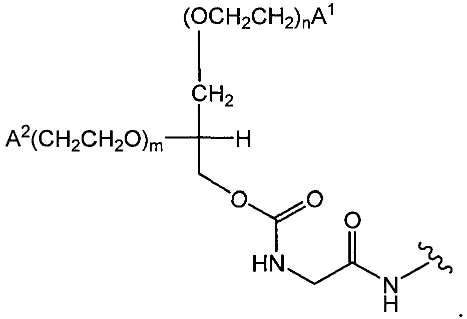

In another exemplary embodiment according to the formula above, the branched polymer has a structure including a moiety according to the following formula: 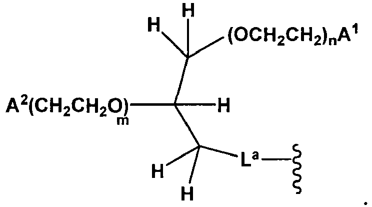 In an exemplary embodiment, A¹ and A² are-OCH₃.

Exemplary polymeric modifying groups according to this embodiment include the moiety: 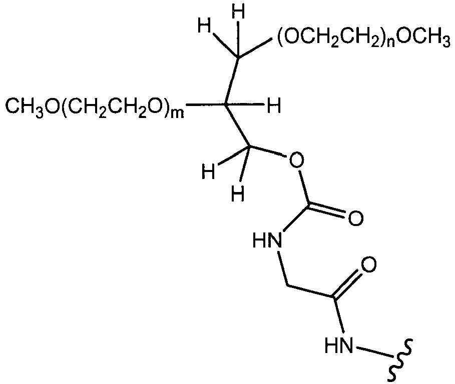 wherein the variables are as described above.

In an illustrative embodiment, the modified sugar is sialic acid and selected modified sugar compounds of use in the invention have the formulae: 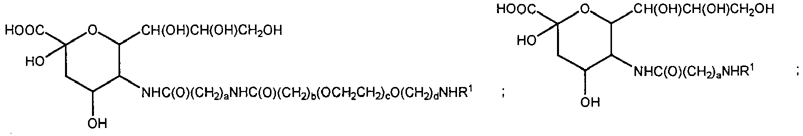 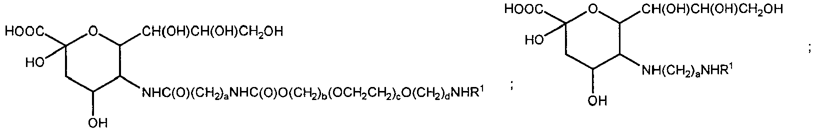 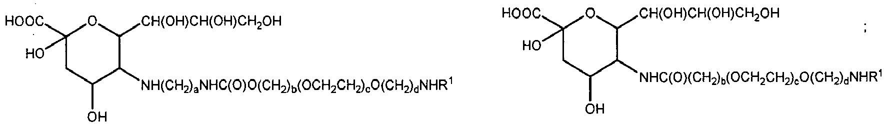 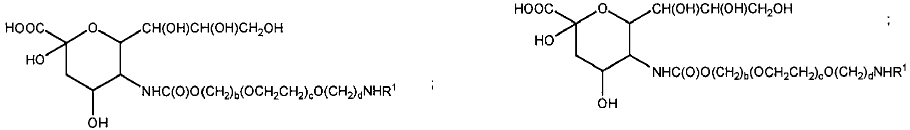 and 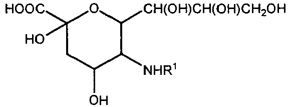 The indices a, b and d are integers from 0 to 20. The index c is an integer from 1 to 2500. The structures set forth above can be components of R¹⁵.

In another illustrative embodiment, a primary hydroxyl moiety of the sugar is functionalized with the modifying group. For example, the 9-hydroxyl of sialic acid can be converted to the corresponding amine and functionalized to provide a compound according to the invention. Formulae according to this embodiment include: 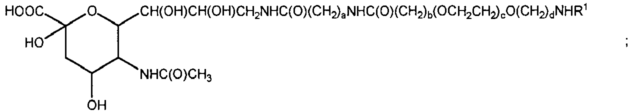 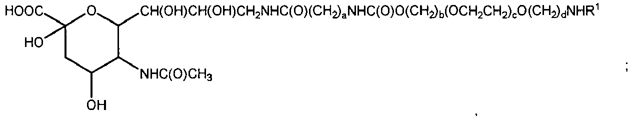 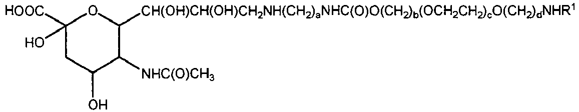 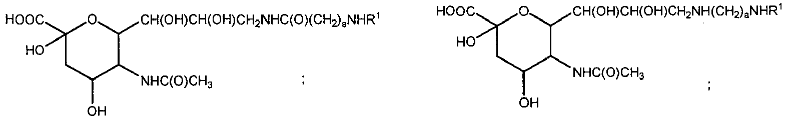 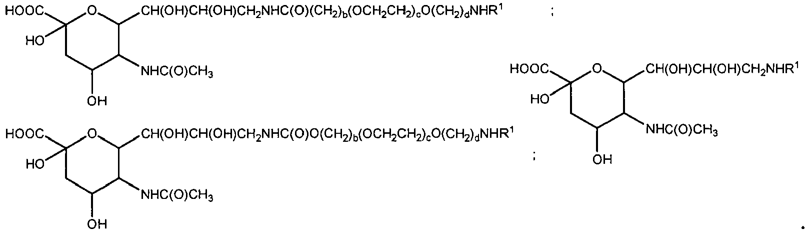 The structures set forth above can be components of R¹⁵.

In selected embodiments, R¹ or L-R¹ is a branched PEG, for example, one of the species set forth above

As discussed herein, the polymer-modified sialic acids of use in the invention may also be linear structures. Thus, the invention provides for conjugates that include a sialic acid moiety derived from a structure such as: 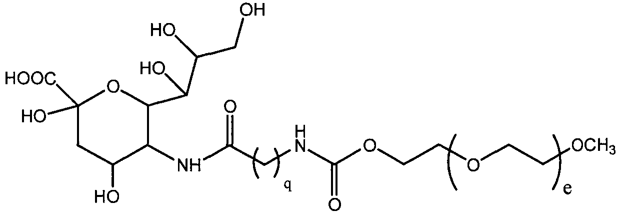 in which the indices q and e are as discussed above.

Exemplary modified sugars are modified with water-soluble or polymers. Examples of useful polymer are further exemplified below.

In one embodiment, the present invention provides a peptide conjugate comprising the following glycosyl linking group: 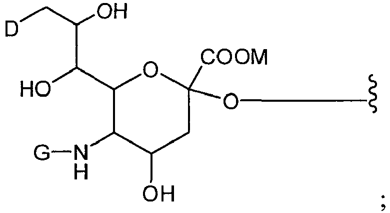
D is -OH;G is a member selected from R¹-L- and -C(O)(C₁-C₆)alkyl-R¹; R¹ is a moiety comprising a member selected from a straight-chain poly(ethylene glycol) residue and branched poly(ethylene glycol) residue; and
M is a member selected from H, a salt counterion and a single negative charge; L is a linker which is a member selected from a bond, substituted or unsubstituted alkyl and substituted or unsubstituted heteroalkyl. In an exemplary embodiment, when D is OH, G is R¹-L-. In another exemplary embodiment, when G is -C(O)(C₁-C₆)alkyl, D is R¹-L-NH-.

In an exemplary embodiment, L-R¹ has the formula: 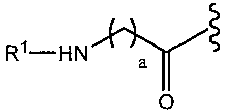 wherein a is an integer selected from 0 to 20.

In an exemplary embodiment, R¹ has a structure that includes a moiety selected from: 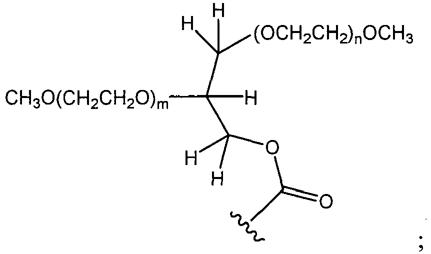 ; wherein e, f, m and n are integers independently selected from 1 to 2500.

In another exemplary embodiment, R¹ has a structure that is a member selected from: 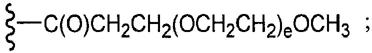 and 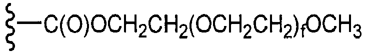 wherein e and f are integers independently selected from 1 to 2500.

In another exemplary embodiment, the glycosyl linker has the formula: 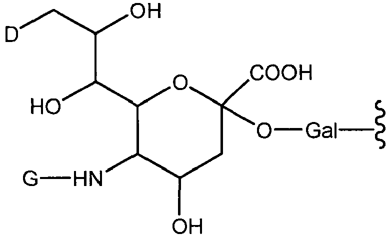 wherein the variables are as described above.

In another exemplary embodiment, the peptide conjugate comprises at least one of said glycosyl linker according to a formula selected from: 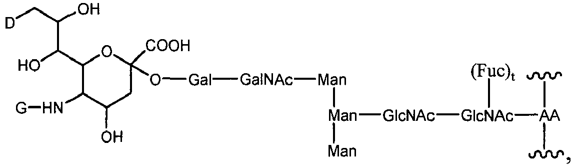 and 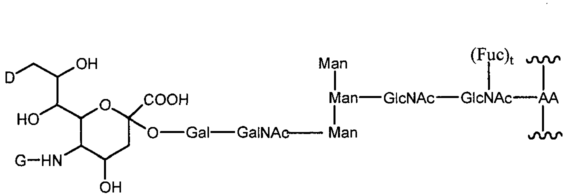 wherein D and G are as described above, AA is an amino acid residue of said peptide conjugate and t is an integer selected from 0 and 1.

In another exemplary embodiment, the peptide conjugate comprises at least one of said glycosyl linker wherein each of said glycosyl linker has a structure which is a member independently selected from the following formulae: 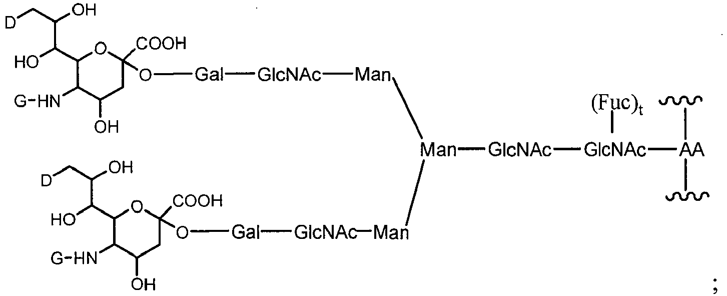 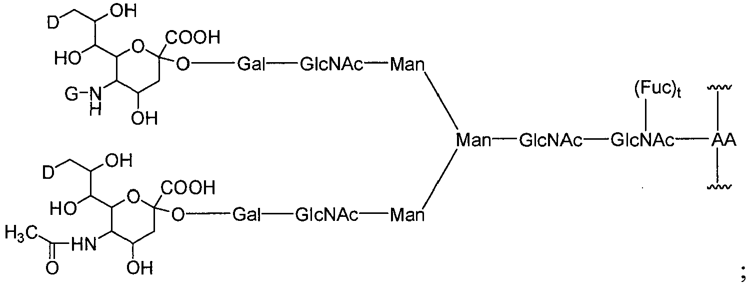 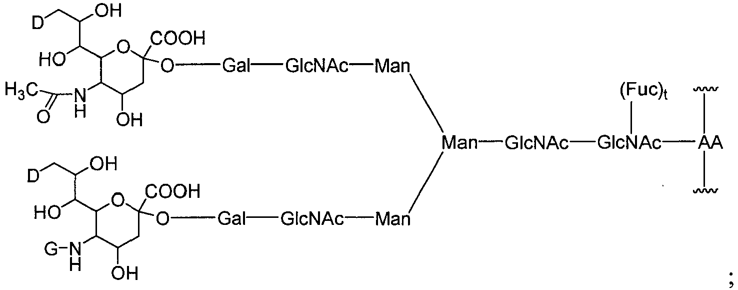 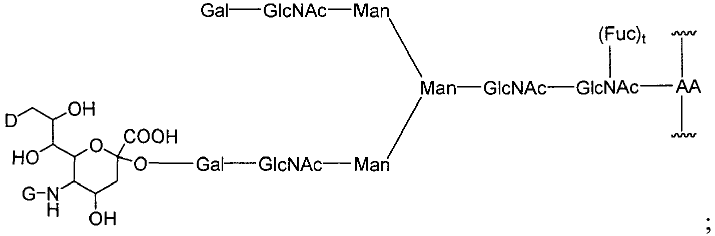 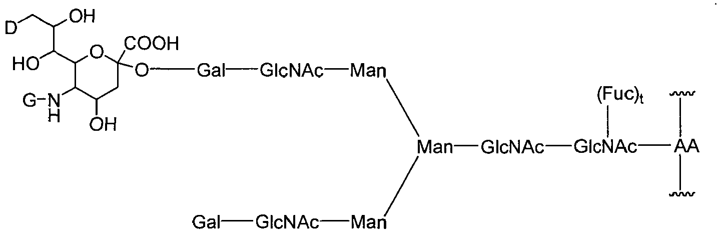 wherein D and G are as described above, AA is an amino acid residue of said peptide conjugate and t is an integer selected from 0 and 1.

In another exemplary embodiment, the peptide conjugate comprises at least one of said glycosyl linker according to a formula selected from: 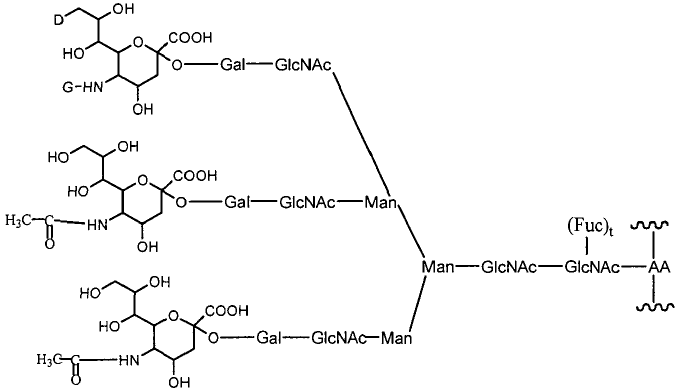 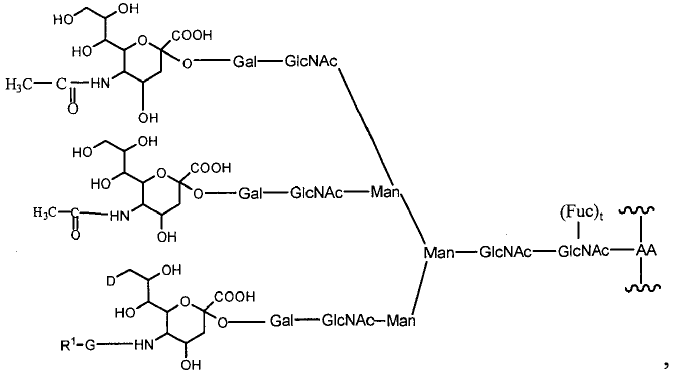 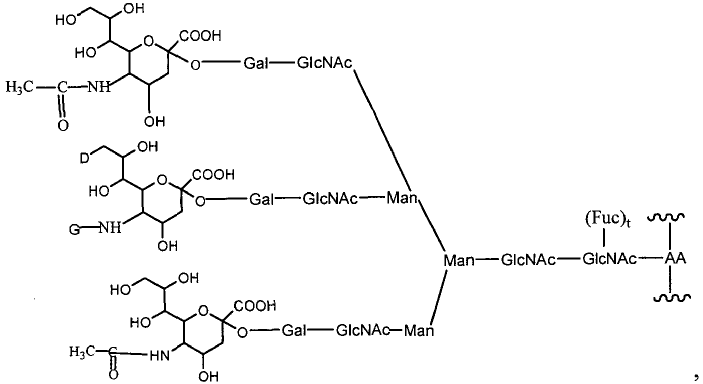 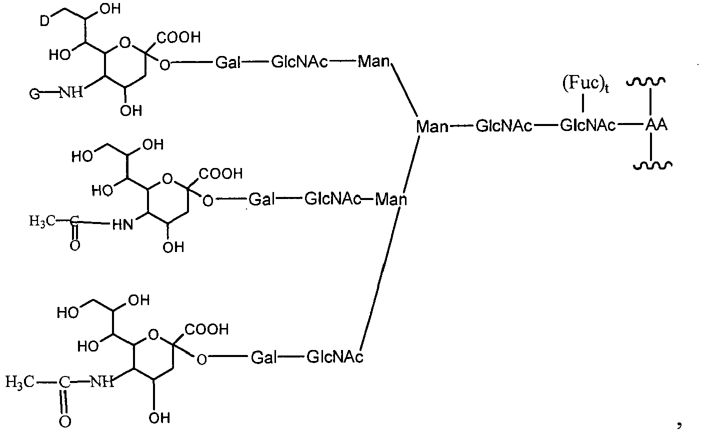 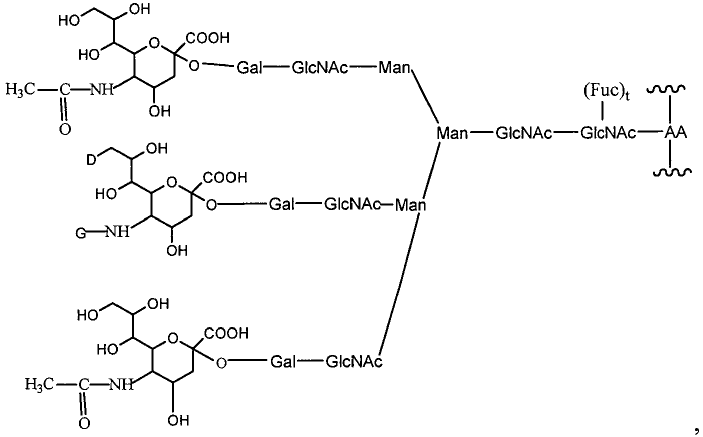 and 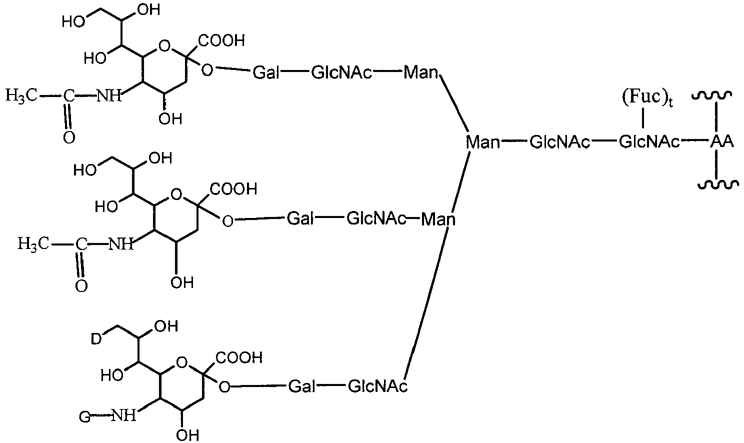 wherein D and G are as described above, AA is an amino acid residue of said peptide conjugate and t is an integer selected from 0 and 1. In an exemplary embodiment, a member selected from 0 and 2 of the sialyl moieties which do not comprise G are absent. In an exemplary embodiment, a member selected from 1 and 2 of the sialyl moieties which do not comprise G are absent.

In another exemplary embodiment, the peptide conjugate comprises at least one of said glycosyl linker according to a formula selected from: 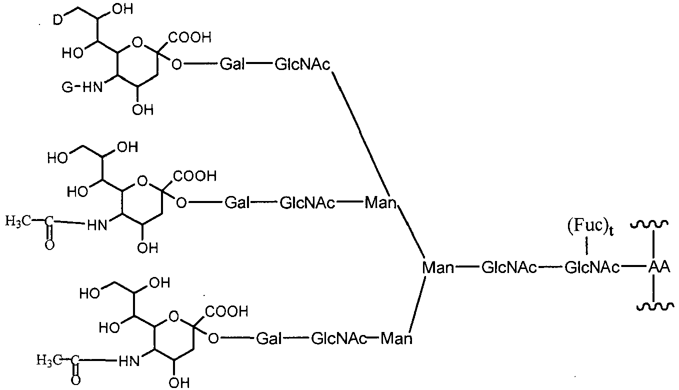 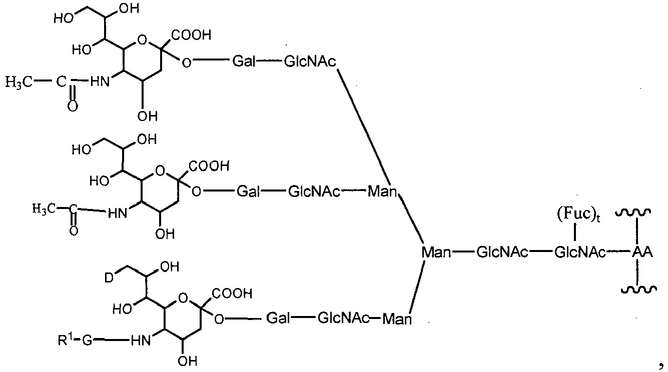 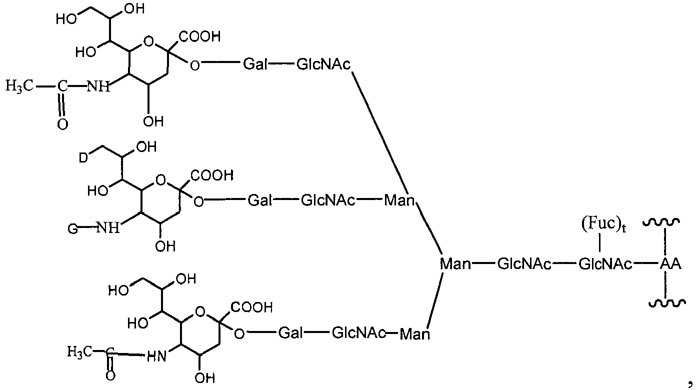 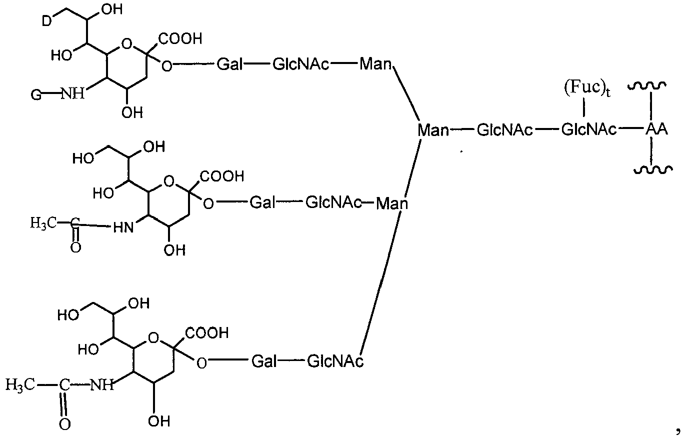 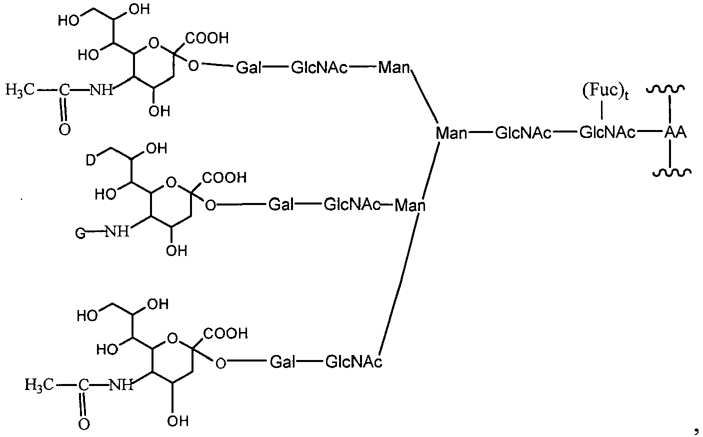 and 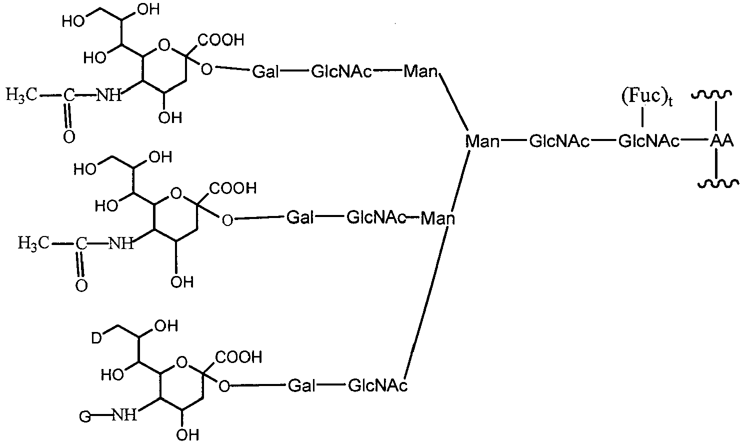 wherein D and G are as described above, AA is an amino acid residue of said peptide conjugate and t is an integer selected from 0 and 1. In an exemplary embodiment, a member selected from 0 and 2 of the sialyl moieties which do not comprise G are absent. In an exemplary embodiment, a member selected from 1 and 2 of the sialyl moieties which do not comprise G are absent.

In another exemplary embodiment, the peptide conjugate comprises at least one said glycosyl linker according to a formula selected from: 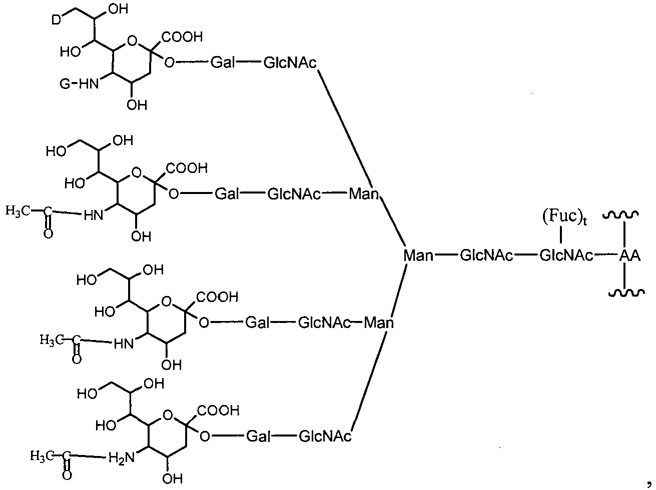 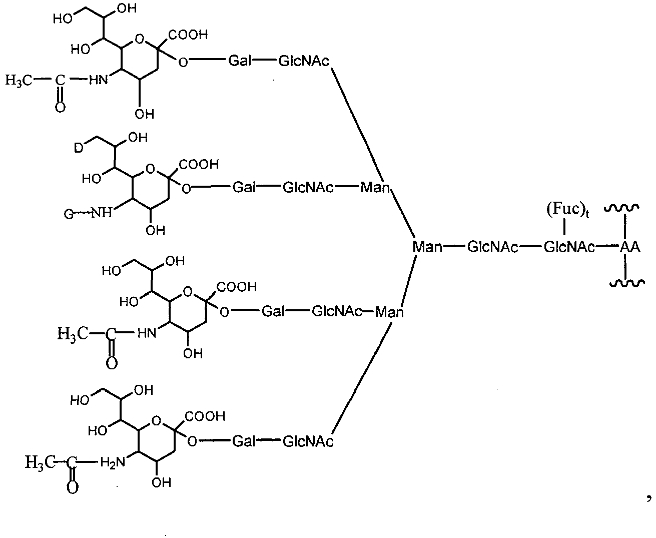 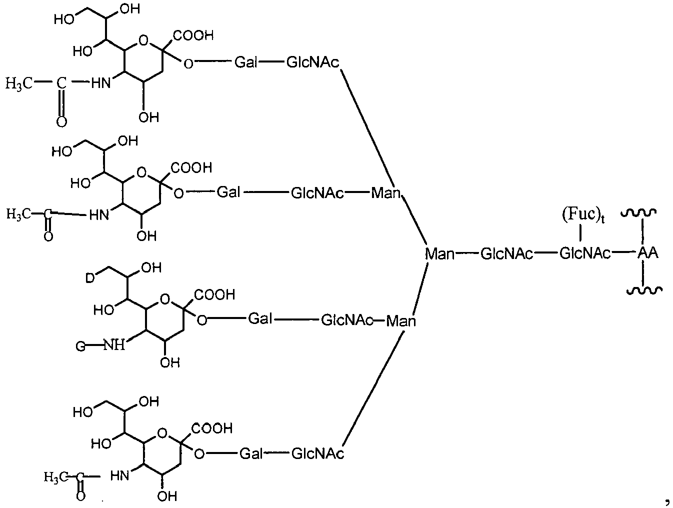 and 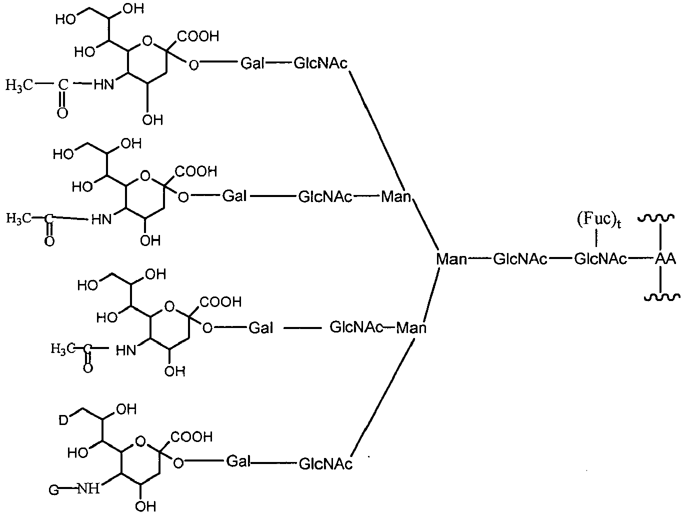 wherein D and G are as described above, AA is an amino acid residue of said peptide conjugate and t is an integer selected from 0 and 1. In an exemplary embodiment, a member selected from 0 and 2 of the sialyl moieties which do not comprise G are absent. In an exemplary embodiment, a member selected from 1 and 2 of the sialyl moieties which do not comprise G are absent.

In another exemplary embodiment, the invention provides a peptide which is produced in a suitable host. Also disclosed are methods of expressing this peptide. In another exemplary aspect, the host is a mammalian expression system.

Also disclosed is a method of treating a condition in a subject in need thereof, said condition characterized by compromised clotting potency in said subject, said method comprising the step of administering to the subject an amount of the peptide conjugate of invention, effective to ameliorate said condition in said subject. In another exemplary aspect, the method comprises administering to said mammal an amount of the peptide conjugate produced according to the methods described herein.

In another aspect, also disclosed isa method of making a peptide conjugate comprising a glycosyl linker described herein. The method comprises (a) contacting a peptide comprising the glycosyl moiety: 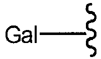 with a PEGylated nucleotide sugar described herein and an enzyme that transfers the PEGylated sugar onto the Gal of said glycosyl moiety, under conditions appropriate for said transfer.

The moiety: 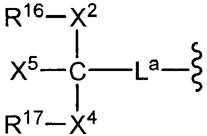 has a formula: 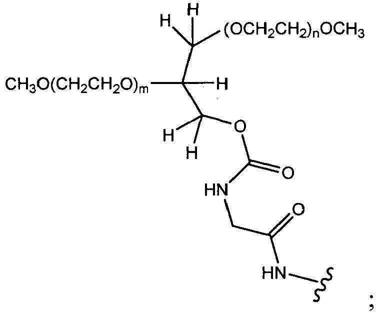 wherein m and n are integers independently selected from 1 to 2500; and q is an integer selected from 0 to 20.

In another exemplary embodiment, the glycosyl linker comprises the formula:

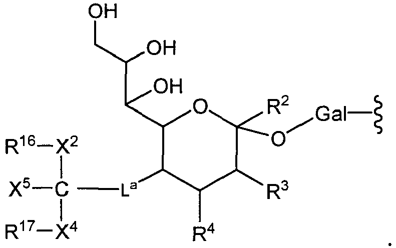

In another exemplary embodiment, the peptide conjugate comprises at least one glycosyl linker having the formula: 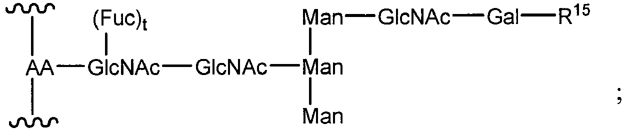 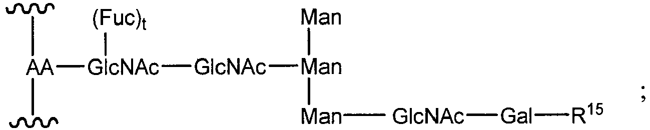 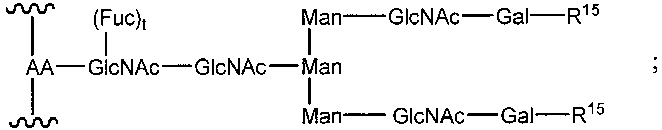 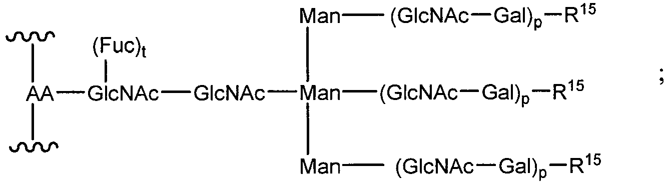 and 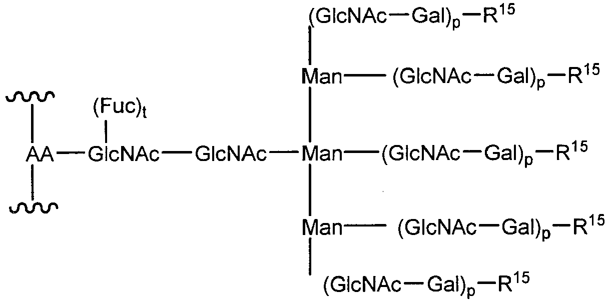 wherein AA is an amino acid residue of said peptide; t is an integer selected from 0 and 1; and R¹⁵ is the modified sialyl moiety.

In another exemplary embodiment, the method comprises, prior to step (a): (b) expressing the peptide in a suitable host.

### II. D. iv. Water-Insoluble Polymers

In another embodiment, analogous to those discussed above, the modified sugars include a water-insoluble polymer, rather than a water-soluble polymer. The conjugates of the invention may also include one or more water-insoluble polymers. This embodiment of the invention is illustrated by the use of the conjugate as a vehicle with which to deliver a therapeutic peptide in a controlled manner. Polymeric drug delivery systems are known in the art. *See,* for example, Dunn et al., Eds. POLYMERIC DRUGS AND DRUG DELIVERY SYSTEMS, ACS Symposium Series Vol. 469, American Chemical Society, Washington, D.C. 1991. Those of skill in the art will appreciate that substantially any known drug delivery system is applicable to the conjugates of the present invention.

The motifs forth above for R¹, L-R¹, R¹⁵, R^{15'} and other radicals are equally applicable to water-insoluble polymers, which may be incorporated into the linear and branched structures without limitation utilizing chemistry readily accessible to those of skill in the art.

Representative water-insoluble polymers include, but are not limited to, polyphosphazines, poly(vinyl alcohols), polyamides, polycarbonates, polyalkylenes, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate) polyethylene, polypropylene, poly(ethylene glycol), poly(ethylene oxide), poly (ethylene terephthalate), poly(vinyl acetate), polyvinyl chloride, polystyrene, polyvinyl pyrrolidone, pluronics and polyvinylphenol and copolymers thereof.

Synthetically modified natural polymers of use in conjugates of the invention include, but are not limited to, alkyl celluloses, hydroxyalkyl celluloses, cellulose ethers, cellulose esters, and nitrocelluloses. Particularly preferred members of the broad classes of synthetically modified natural polymers include, but are not limited to, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, and polymers of acrylic and methacrylic esters and alginic acid.

These and the other polymers discussed herein can be readily obtained from commercial sources such as Sigma Chemical Co. (St. Louis, MO.), Polysciences (Warrenton, PA.), Aldrich (Milwaukee, WI.), Fluka (Ronkonkoma, NY), and BioRad (Richmond, CA), or else synthesized from monomers obtained from these suppliers using standard techniques.

Representative biodegradable polymers of use in the conjugates of the invention include, but are not limited to, polylactides, polyglycolides and copolymers thereof, poly(ethylene terephthalate), poly(butyric acid), poly(valeric acid), poly(lactide-co-caprolactone), poly(lactide-co-glycolide), polyanhydrides, polyorthoesters, blends and copolymers thereof. Of particular use are compositions that form gels, such as those including collagen, pluronics and the like.

The polymers of use in the invention include "hybrid' polymers that include water-insoluble materials having within at least a portion of their structure, a bioresorbable molecule. An example of such a polymer is one that includes a water-insoluble copolymer, which has a bioresorbable region, a hydrophilic region and a plurality of crosslinkable functional groups per polymer chain.

For purposes of the present invention, "water-insoluble materials" includes materials that are substantially insoluble in water or water-containing environments. Thus, although certain regions or segments of the copolymer may be hydrophilic or even water-soluble, the polymer molecule, as a whole, does not to any substantial measure dissolve in water.

For purposes of the present invention, the term "bioresorbable molecule" includes a region that is capable of being metabolized or broken down and resorbed and/or eliminated through normal excretory routes by the body. Such metabolites or break down products are preferably substantially non-toxic to the body.

The bioresorbable region may be either hydrophobic or hydrophilic, so long as the copolymer composition as a whole is not rendered water-soluble. Thus, the bioresorbable region is selected based on the preference that the polymer, as a whole, remains water-insoluble. Accordingly, the relative properties, *i.e.,* the kinds of functional groups contained by, and the relative proportions of the bioresorbable region, and the hydrophilic region are selected to ensure that useful bioresorbable compositions remain water-insoluble.

Exemplary resorbable polymers include, for example, synthetically produced resorbable block copolymers of poly(α-hydroxy-carboxylic acid)/poly(oxyalkylene, (*see,* Cohn et al., U.S. Patent No. 4,826,945). These copolymers are not crosslinked and are water-soluble so that the body can excrete the degraded block copolymer compositions. *See,* Younes et al., J Biomed. Mater. Res. 21: 1301-1316 (1987); and Cohn et al., J Biomed. Mater. Res. 22: 993-1009 (1988).

Presently preferred bioresorbable polymers include one or more components selected from poly(esters), poly(hydroxy acids), poly(lactones), poly(amides), poly(ester-amides), poly (amino acids), poly(anhydrides), poly(orthoesters), poly(carbonates), poly(phosphazines), poly(phosphoesters), poly(thioesters), polysaccharides and mixtures thereof. More preferably still, the biosresorbable polymer includes a poly(hydroxy) acid component. Of the poly(hydroxy) acids, polylactic acid, polyglycolic acid, polycaproic acid, polybutyric acid, polyvaleric acid and copolymers and mixtures thereof are preferred.

In addition to forming fragments that are absorbed *in vivo* ("bioresorbed"), preferred polymeric coatings for use in the methods of the invention can also form an excretable and/or metabolizable fragment.

Higher order copolymers can also be used in the present invention. For example, Casey et al., U.S. Patent No. 4,438,253, which issued on March 20, 1984, discloses tri-block copolymers produced from the transesterification of poly(glycolic acid) and an hydroxyl-ended poly(alkylene glycol). Such compositions are disclosed for use as resorbable monofilament sutures. The flexibility of such compositions is controlled by the incorporation of an aromatic orthocarbonate, such as tetra-p-tolyl orthocarbonate into the copolymer structure.

Other polymers based on lactic and/or glycolic acids can also be utilized. For example, Spinu, U.S. Patent No. 5,202,413, which issued on April 13, 1993, discloses biodegradable multi-block copolymers having sequentially ordered blocks of polylactide and/or polyglycolide produced by ring-opening polymerization of lactide and/or glycolide onto either an oligomeric diol or a diamine residue followed by chain extension with a difunctional compound, such as, a diisocyanate, diacylchloride or dichlorosilane.

Bioresorbable regions of coatings useful in the present invention can be designed to be hydrolytically and/or enzymatically cleavable. For purposes of the present invention, "hydrolytically cleavable" refers to the susceptibility of the copolymer, especially the bioresorbable region, to hydrolysis in water or a water-containing environment. Similarly, "enzymatically cleavable" as used herein refers to the susceptibility of the copolymer, especially the bioresorbable region, to cleavage by endogenous or exogenous enzymes.

When placed within the body, the hydrophilic region can be processed into excretable and/or metabolizable fragments. Thus, the hydrophilic region can include, for example, polyethers, polyalkylene oxides, polyols, poly(vinyl pyrrolidine), poly(vinyl alcohol), poly(alkyl oxazolines), polysaccharides, carbohydrates, peptides, proteins and copolymers and mixtures thereof. Furthermore, the hydrophilic region can also be, for example, a poly(alkylene) oxide. Such poly(alkylene) oxides can include, for example, poly(ethylene) oxide, poly(propylene) oxide and mixtures and copolymers thereof.

Polymers that are components of hydrogels are also useful in the present invention. Hydrogels are polymeric materials that are capable of absorbing relatively large quantities of water. Examples of hydrogel forming compounds include, but are not limited to, polyacrylic acids, sodium carboxymethylcellulose, polyvinyl alcohol, polyvinyl pyrrolidine, gelatin, carrageenan and other polysaccharides, hydroxyethylenemethacrylic acid (HEMA), as well as derivatives thereof, and the like. Hydrogels can be produced that are stable, biodegradable and bioresorbable. Moreover, hydrogel compositions can include subunits that exhibit one or more of these properties.

Bio-compatible hydrogel compositions whose integrity can be controlled through crosslinking are known and are presently preferred for use in the methods of the invention. For example, Hubbell et al., U.S. Patent Nos. 5,410,016, which issued on April 25, 1995 and 5,529,914, which issued on June 25, 1996, disclose water-soluble systems, which are crosslinked block copolymers having a water-soluble central block segment sandwiched between two hydrolytically labile extensions. Such copolymers are further end-capped with photopolymerizable acrylate functionalities. When crosslinked, these systems become hydrogels. The water soluble central block of such copolymers can include poly(ethylene glycol); whereas, the hydrolytically labile extensions can be a poly(α-hydroxy acid), such as polyglycolic acid or polylactic acid. *See,* Sawhney et al., Macromolecules 26: 581-587 (1993).

In another preferred embodiment, the gel is a thermoreversible gel. Thermoreversible gels including components, such as pluronics, collagen, gelatin, hyalouronic acid, polysaccharides, polyurethane hydrogel, polyurethane-urea hydrogel and combinations thereof are presently preferred.

In yet another exemplary embodiment, the conjugate of the invention includes a component of a liposome. Liposomes can be prepared according to methods known to those skilled in the art, for example, as described in Eppstein et al., U.S. Patent No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its pharmaceutically acceptable salt is then introduced into the container. The container is then swirled by hand to free lipid material from the sides of the container and to disperse lipid aggregates, thereby forming the liposomal suspension.

The above-recited microparticles and methods of preparing the microparticles are offered by way of example and they are not intended to define the scope of microparticles of use in the present invention. It will be apparent to those of skill in the art that an array of microparticles, fabricated by different methods, is of use in the present invention.

The structural formats discussed above in the context of the water-soluble polymers, both straight-chain and branched are generally applicable with respect to the water-insoluble polymers as well. Thus, for example, the cysteine, serine, dilysine, and trilysine branching cores can be functionalized with two water-insoluble polymer moieties. The methods used to produce these species are generally closely analogous to those used to produce the water-soluble polymers.

### II. D. v. Methods of Producing the Polymeric Modifying Groups

The polymeric modifying groups can be activated for reaction with a glycosyl or saccharyl moiety or an amino acid moiety. Exemplary structures of activated species (e.g., carbonates and active esters) include: 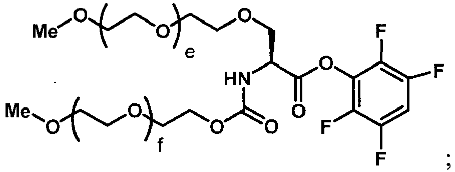 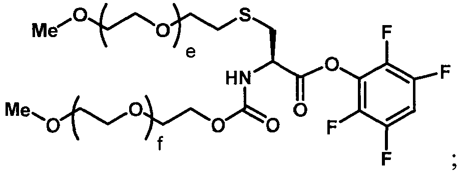 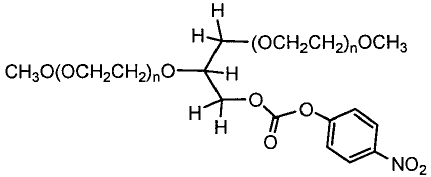 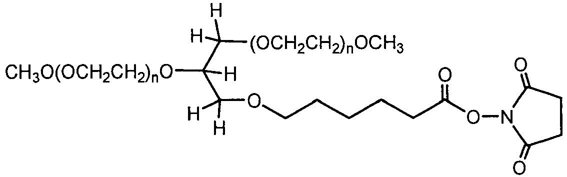 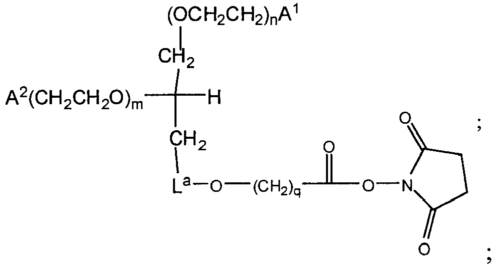 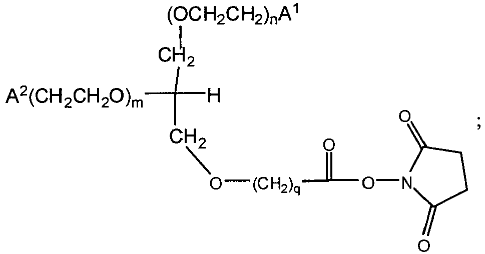 and

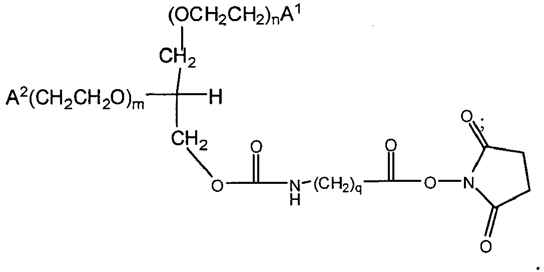

In the figure above, q is a member selected from 1-40. Other activating, or leaving groups, appropriate for activating linear and branched PEGs of use in preparing the compounds set forth herein include, but are not limited to the species: 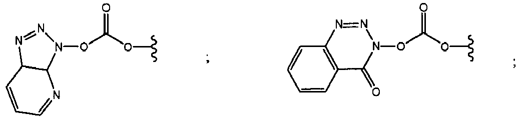 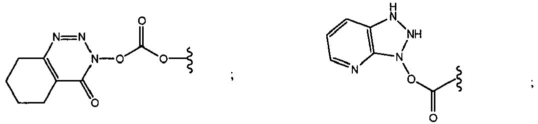 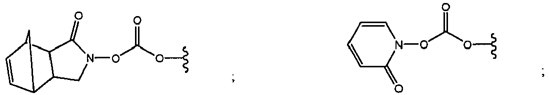 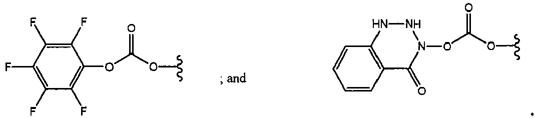 PEG molecules that are activated with these and other species and methods of making the activated PEGs are set forth in WO 04/083259.

Those of skill in the art will appreciate that one or more of the m-PEG arms of the branched polymers shown above can be replaced by a PEG moiety with a different terminus, e.g., OH, COOH, NH₂, C₂-C₁₀-alkyl, etc. Moreover, the structures above are readily modified by inserting alkyl linkers (or removing carbon atoms) between the α-carbon atom and the functional group of the amino acid side chain. Thus, "homo" derivatives and higher homologues, as well as lower homologues are within the scope of cores for branched PEGs of use in the present invention.

The branched PEG species set forth herein are readily prepared by methods such as that set forth in the scheme below: 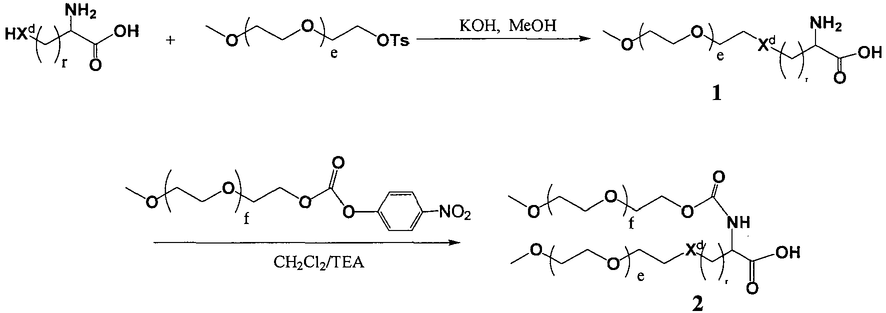 in which X^{d} is O or S and r is an integer from 1 to 5. The indices e and f are independently selected integers from 1 to 2500. In an exemplary embodiment, one or both of these indices are selected such that the polymer is about 5 kD, 10 kD, 15 kD, 20 kD, 25 kD, 30 kD, 35 kD, or 40 kD in molecular weight. PEG of a larger molecular weight can also be used in the present invention, including up to about 200 kD, such as at least about 180 kD, about 160 kD, about 140 kD, about 120 kD, about 100 kD, about 90 kD, about 80 kD, and about 70 kD. In certain embodiments the molecular weight of PEG is about 80 kD. In other embodiments, the molecular weight of PEG is at least about 200 kD, at least about 180 kD, at least about 160 kD, or at least about 140 kD.

Thus, according to this scheme, a natural or unnatural amino acid is contacted with an activated m-PEG derivative, in this case the tosylate, forming **1** by alkylating the side-chain heteroatom X^{d}. The mono-functionalize m-PEG amino acid is submitted to N-acylation conditions with a reactive m-PEG derivative, thereby assembling branched m-PEG **2.** As one of skill will appreciate, the tosylate leaving group can be replaced with any suitable leaving group, e.g., halogen, mesylate, triflate, etc. Similarly, the reactive carbonate utilized to acylate the amine can be replaced with an active ester, e.g., N-hydroxysuccinimide, etc., or the acid can be activated *in situ* using a dehydrating agent such as dicyclohexylcarbodiimide, carbonyldiimidazole, etc.

In other exemplary embodiments, the urea moiety is replaced by a group such as a amide.

### II. E. Homodisperse Peptide Conjugate Compositions of Matter

In addition to providing peptide conjugates that are formed through a chemically or enzymatically added glycosyl linking group, the present invention provides compositions of matter comprising peptide conjugates that are highly homogenous in their substitution patterns. Using the methods of the invention, it is possible to form peptide conjugates in which substantial proportion of the glycosyl linking groups and glycosyl moieties across a population of peptide conjugates are attached to a structurally identical amino acid or glycosyl residue. Thus, in a second aspect, the invention provides a peptide conjugate having a population of water-soluble polymer moieties, which are covalently bound to the peptide through a glycosyl linking group, e.g., an intact glycosyl linking group. In a an exemplary peptide conjugate of the invention, essentially each member of the water soluble polymer population is bound via the glycosyl linking group to a glycosyl residue of the peptide, and each glycosyl residue of the peptide to which the glycosyl linking group is attached has the same structure.

The present invention also provides conjugates analogous to those described above in which the peptide is conjugated to a modifying group, e.g. therapeutic moiety, diagnostic moiety, targeting moiety, toxin moiety or the like via a glycosyl linking group. Each of the above-recited modifying groups can be a small molecule, natural polymer (e.g., polypeptide) or synthetic polymer. When the modifying group is attached to a sialic acid, it is generally preferred that the modifying group is substantially non-fluorescent.

In an exemplary embodiment, the peptides of the invention include at least one O-linked or N-linked glycosylation site, which is glycosylated with a modified sugar that includes a polymeric modifying group, e.g., a PEG moiety. In an exemplary embodiment, the PEG is covalently attached to the peptide via an intact glycosyl linking group, or via a non-glycosyl linker, e.g., substituted or unsubstituted alkyl, substituted or unsubstituted heteroalkyl. The glycosyl linking group is covalently attached to either an amino acid residue or a glycosyl residue of the peptide. Alternatively, the glycosyl linking group is attached to one or more glycosyl units of a glycopeptide. The invention also provides conjugates in which a glycosyl linking group is attached to both an amino acid residue and a glycosyl residue.

The glycans on the peptides of the invention generally correspond to those found on a peptide that is produced by mammalian (BHK, CHO) cells or insect (e.g., Sf-9) cells, following remodeling according to the methods set forth herein. For example insect-derived peptide that is expressed with a tri-mannosyl core is subsequently contacted with a GlcNAc donor and a GlcNAc transferase and a Gal donor and a Gal transferase. Appending GlcNAc and Gal to the tri-mannosyl core is accomplished in either two steps or a single step. A modified sialic acid is added to at least one branch of the glycosyl moiety as discussed herein. Those Gal moieties that are not functionalized with the modified sialic acid are optionally "capped" by reaction with a sialic acid donor in the presence of a sialyl transferase.

In an exemplary embodiment, at least 60% of terminal Gal moieties in a population of peptides is capped with sialic acid, preferably at least 70%, more preferably, at least 80%, still more preferably at least 90% and even more preferably at least 95%, 96%, 97%, 98% or 99% are capped with sialic acid.

### II. F. Nucleotide Sugars

In another aspect of the invention, the invention also provides sugar nucleotides. Exemplary species according to this embodiment include: 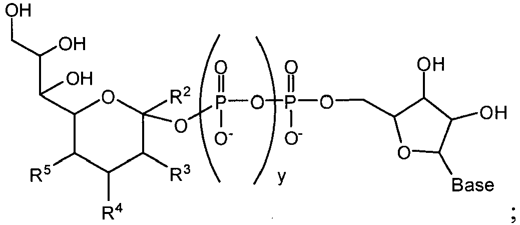 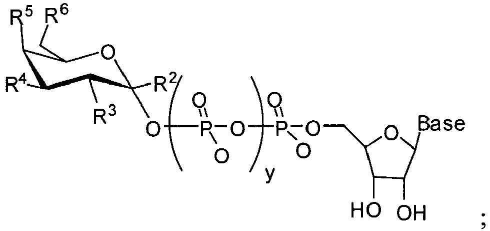 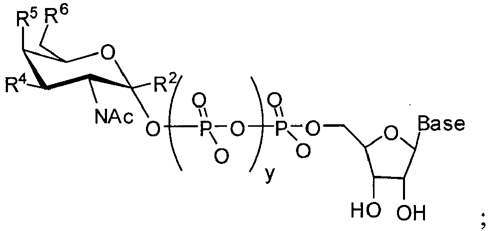 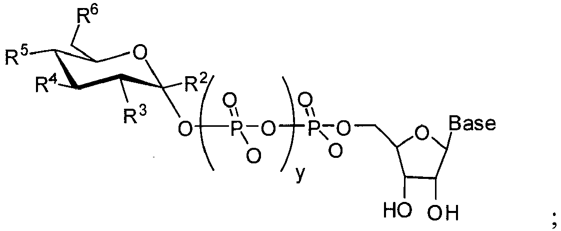 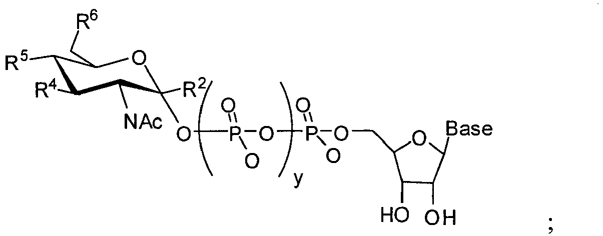 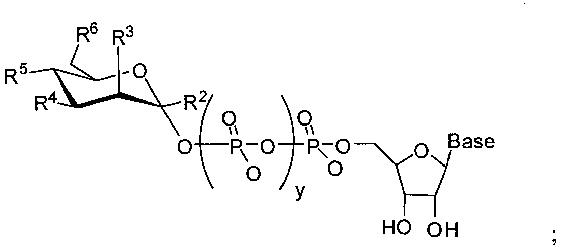 and 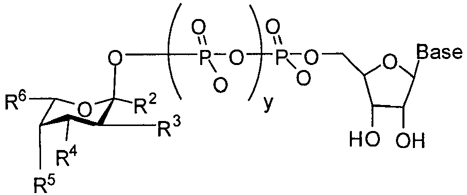 wherein y is an integer selected from 0 to 2 and at least one of R², R³, R⁴, R⁵ or R⁶ has a structure which is a member selected from 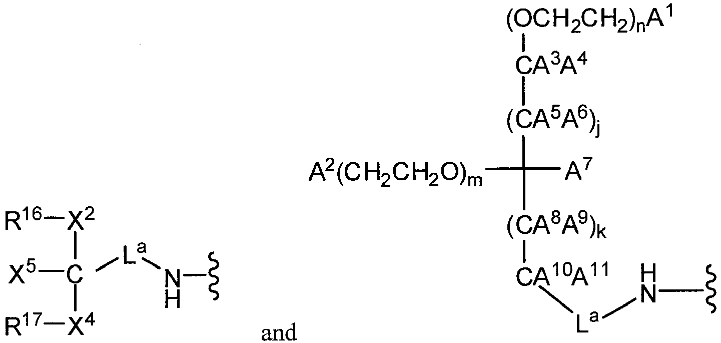 in which the variables are as described above.

In an exemplary embodiment, at least one of R², R³, R⁴, R⁵ or R⁶ has a structure according to the following formula: 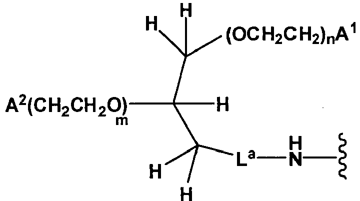 In an exemplary embodiment, A¹ and A² are each selected from -OH and -OCH₃.

Exemplary polymeric modifying groups according to this embodiment include the moiety:

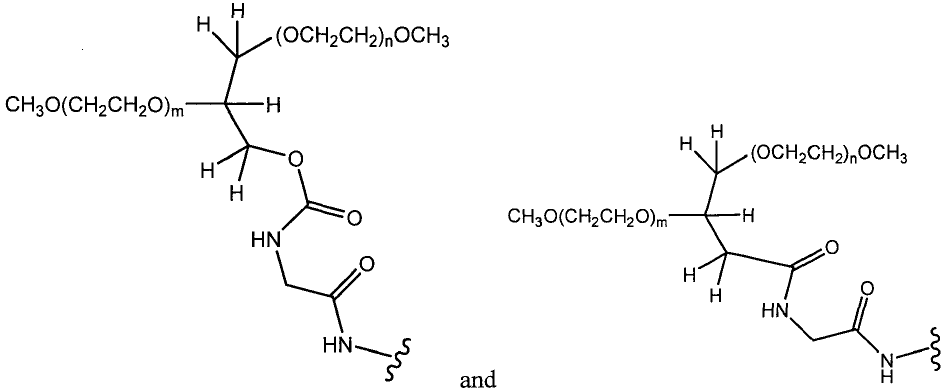

In an exemplary embodiment, only one of R², R³, R⁴, R⁵ or R⁶ has a structure which includes the modifying groups described above.

In another exemplary embodiment, species according to this embodiment include: 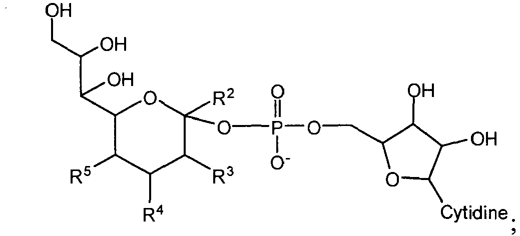 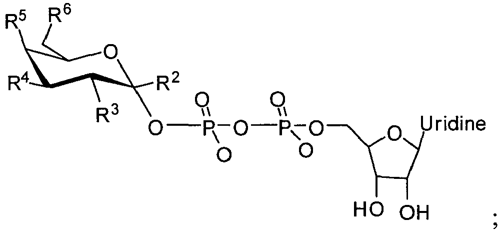 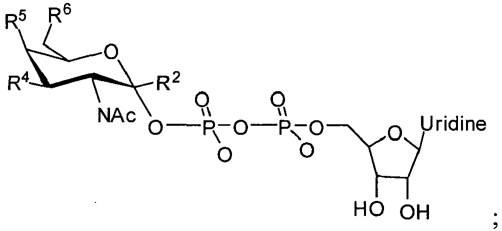 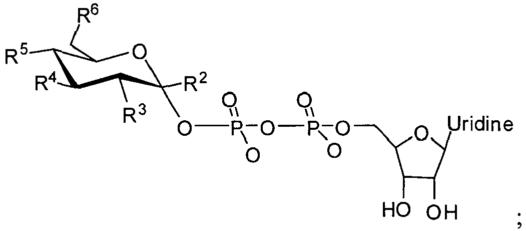 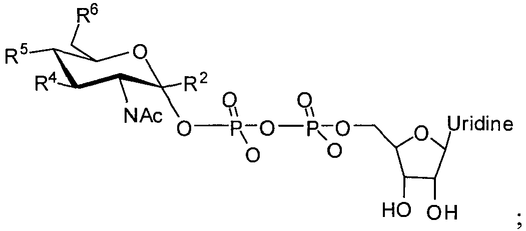 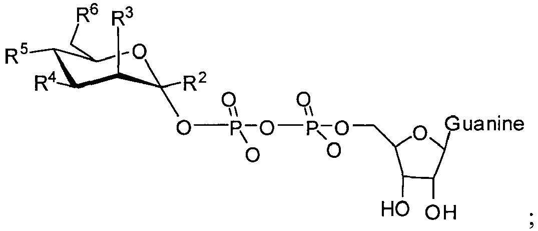 and 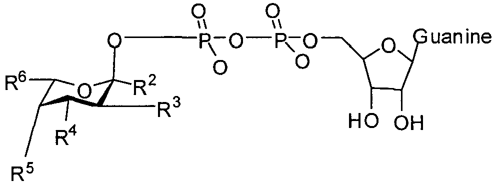 wherein the variables are as described above.

In another exemplary embodiment, species according to this embodiment include: 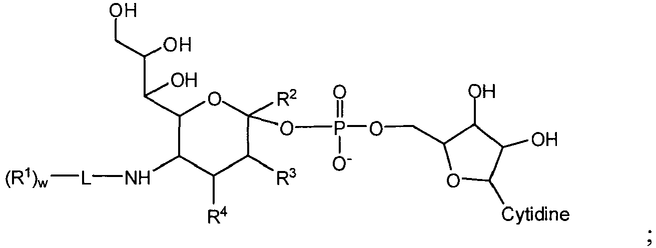 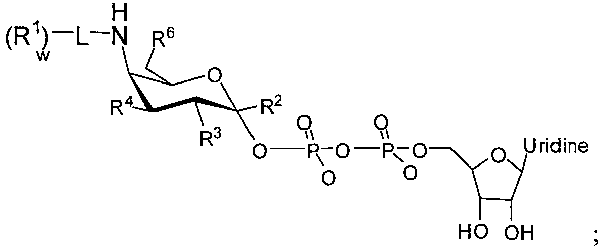 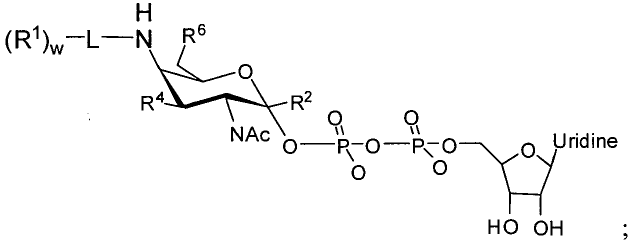 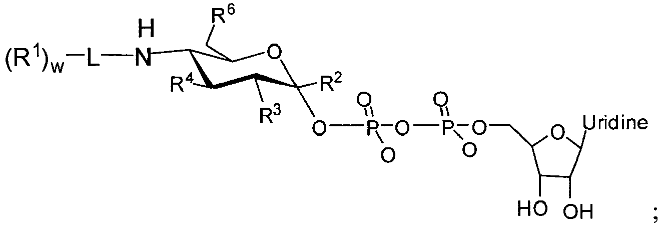 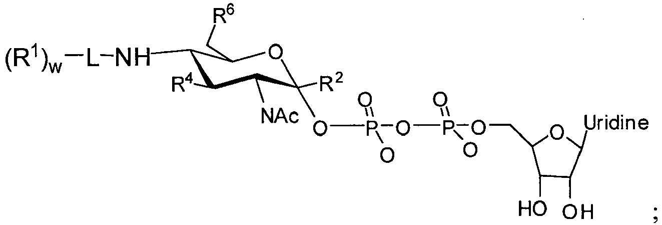 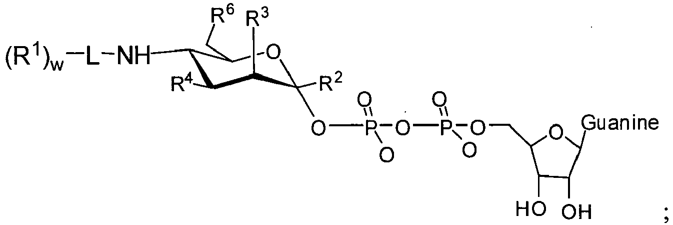 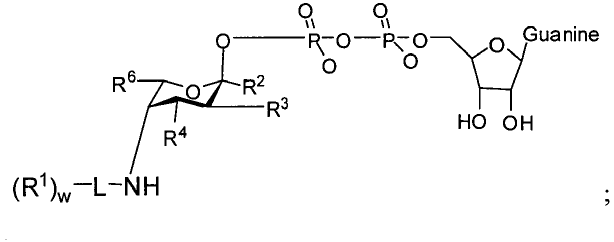 in which L-(R¹)_{w} is a member selected from 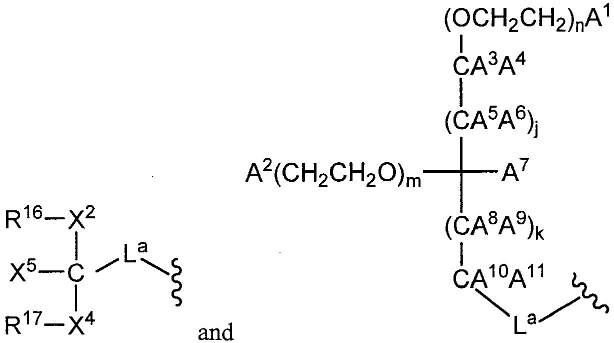 in which the variables are as described above.

In an exemplary embodiment, L-(R¹)_{w} has a structure according to the following formula: 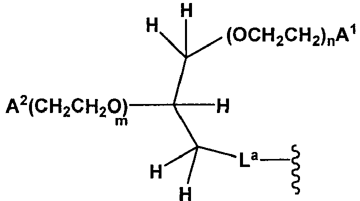 In an exemplary embodiment, A¹ and A² are each selected from -OH and -OCH₃.

Exemplary polymeric modifying groups according to this embodiment include the moiety: 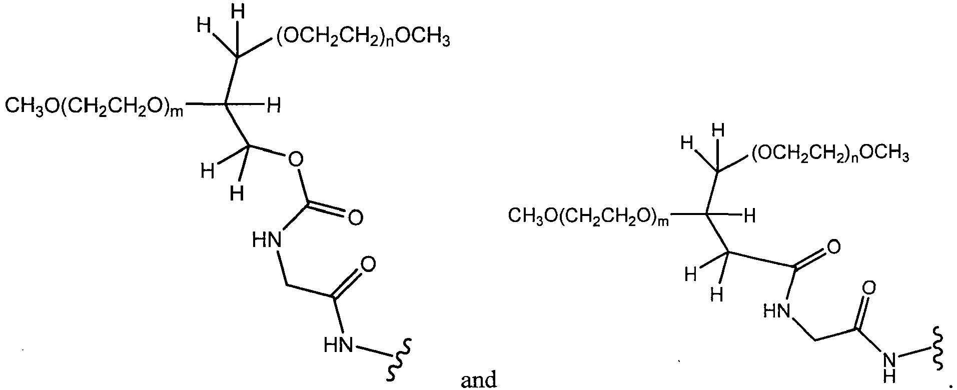 In an exemplary embodiment, m and n are integers independently selected from about 1 to about 1000. In an exemplary embodiment, m and n are integers independently selected from about 1 to about 500. In an exemplary embodiment, m and n are integers independently selected from about 1 to about 70, about 70 to about 150, about 150 to about 250, about 250 to about 375 and about 375 to about 500. In an exemplary embodiment, m and n are integers independently selected from about 10 to about 35, about 45 to about 65, about 95 to about 130, about 210 to about 240, about 310 to about 370 and about 420 to about 480. In an exemplary embodiment, m and n are integers selected from about 15 to about 30. In an exemplary embodiment, m and n are integers selected from about 50 to about 65. In an exemplary embodiment, m and n are integers selected from about 100 to about 130. In an exemplary embodiment, m and n are integers selected from about 210 to about 240. In an exemplary embodiment, m and n are integers selected from about 310 to about 370. In an exemplary embodiment, m and n are integers selected from about 430 to about 470.

In another exemplary embodiment, species according to this embodiment include: 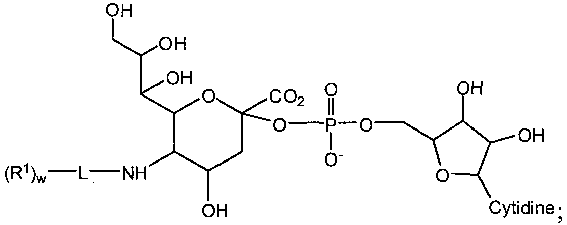 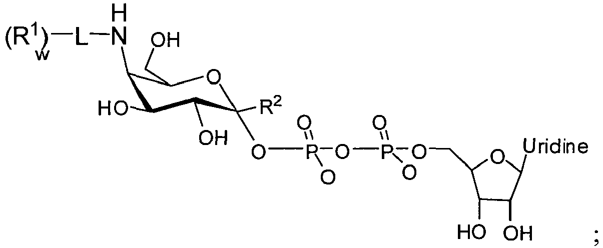 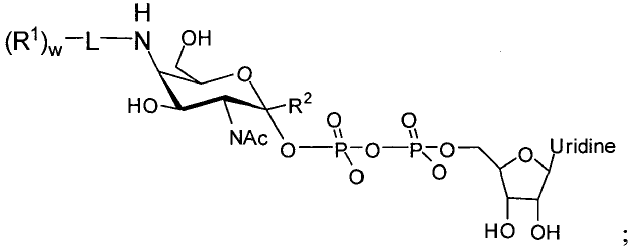 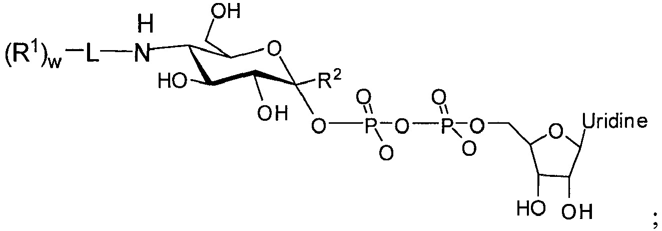 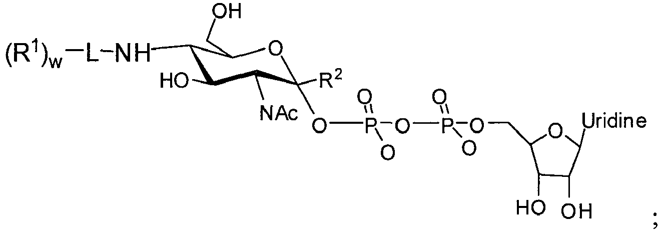 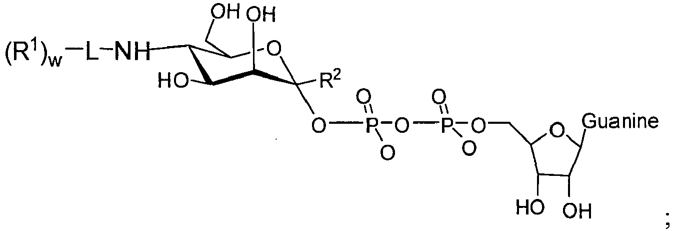 and 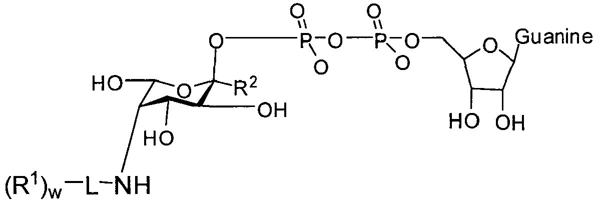 wherein the variables are as described above.

In another exemplary embodiment, species according to this embodiment include: 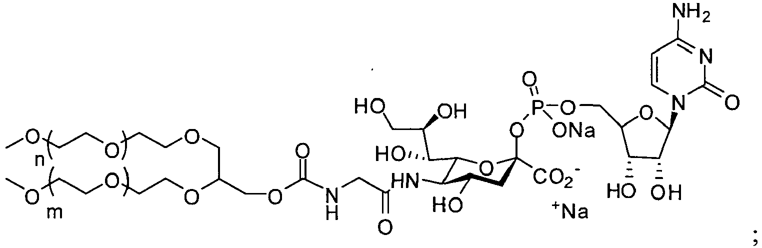 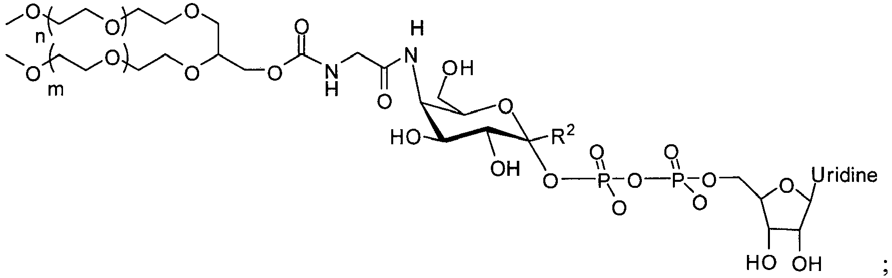 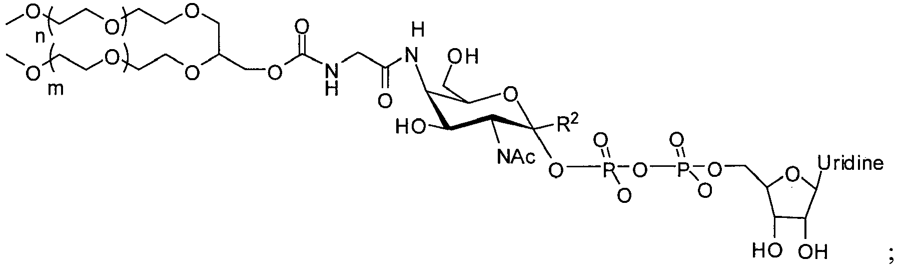 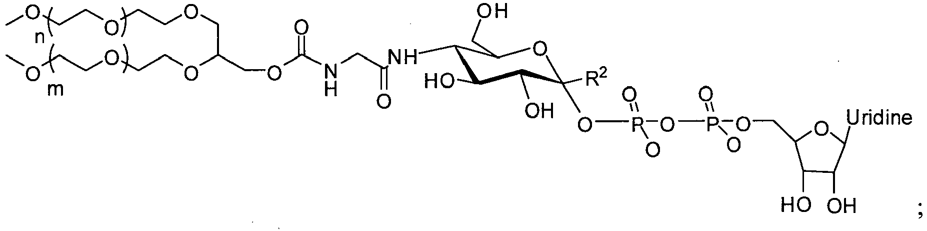 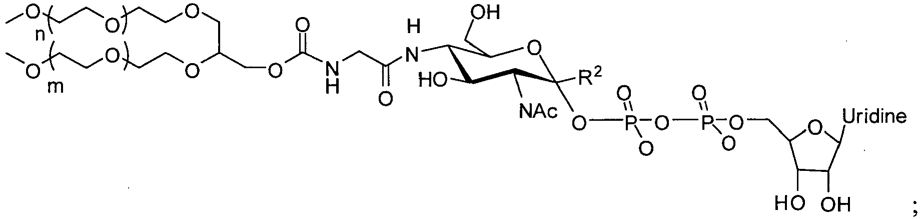 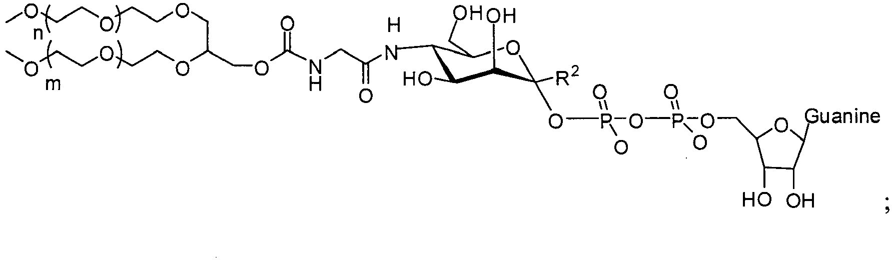 and 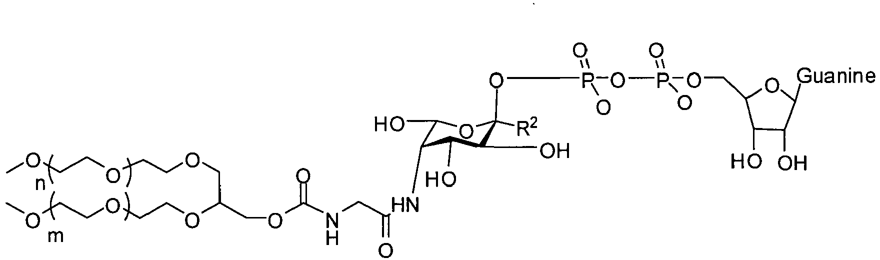 wherein the variables are as described above.

In another exemplary embodiment, the nucleotide sugars have a formula which is a member selected from: 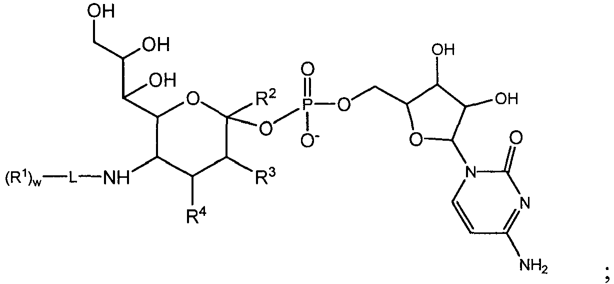 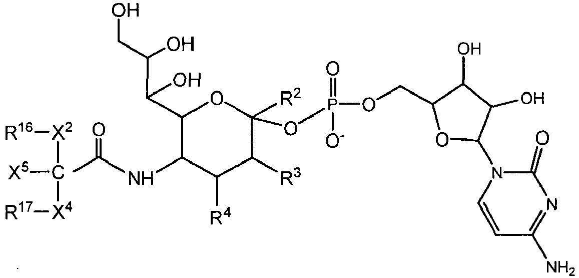 and 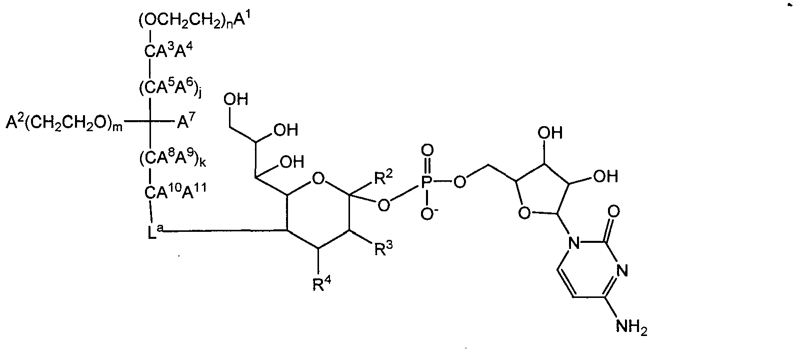 wherein the variables are as described above.

An exemplary nucleotide sugar according to this embodiment has the structure: 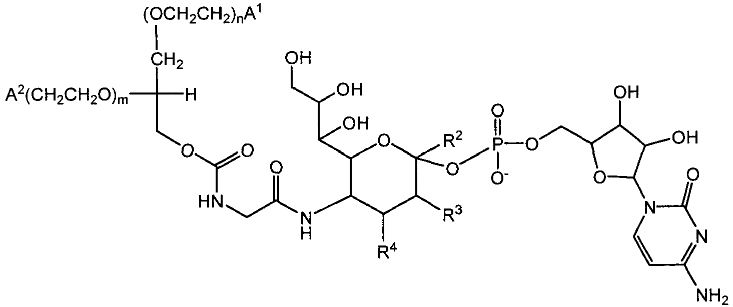 wherein the variables are as described above.

An exemplary nucleotide sugar according to this embodiment has the structure: 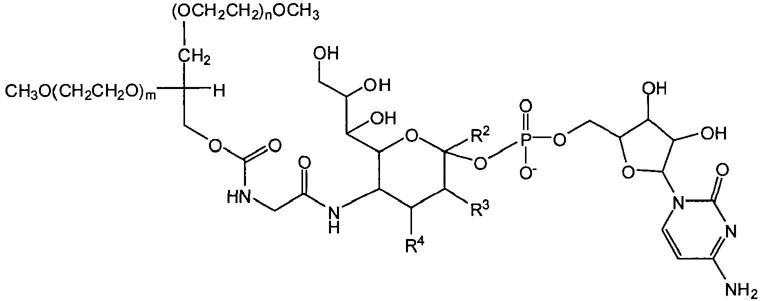 wherein the variables are as described above.

In another exemplary embodiment, the nucleotide sugar is based upon the following formula: 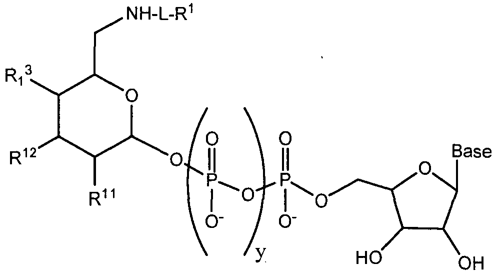 in which the R groups, and L, represent moieties as discussed above. The index "y" is 0, 1 or 2. In an exemplary embodiment, L is a bond between NH and R¹. The base is a nucleic acid base.

In an exemplary embodiment, L-R¹ is a member selected from 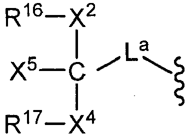 in which the variables are as described above.

In an exemplary embodiment, L-R¹ has a structure according to the following formula: 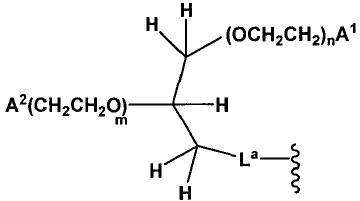 A¹ and A² are each-OCH₃.

### III. The Methods

In addition to the conjugates discussed above, the present invention provides methods for preparing these and other conjugates. Moreover, the invention provides methods of preventing, curing or ameliorating a disease state by administering a conjugate of the invention to a subject at risk of developing the disease or a subject that has the disease.

In exemplary embodiments, the conjugate is formed between a polymeric modifying moiety and a glycosylated or non-glycosylated peptide. The polymer is conjugated to the peptide via a glycosyl linking group, which is interposed between, and covalently linked to both the peptide (or glycosyl residue) and the modifying group (e.g., water-soluble polymer). The method includes contacting the peptide with a mixture containing a modified sugar and an enzyme, e.g., a glycosyltransferase that conjugates the modified sugar to the substrate. The reaction is conducted under conditions appropriate to form a covalent bond between the modified sugar and the peptide. The sugar moiety of the modified sugar is preferably selected from nucleotide sugars. The method of synthesizing a peptide conjugate, comprising combining a) sialidase; b) an enzyme capable of catalyzing the transfer of a glycosyl linking group such as a glycosyltransferase, exoglycosidase or endoglycosidase; c) modified sugar; d) peptide, thus synthesizing the peptide conjugate. The reaction is conducted under conditions appropriate to form a covalent bond between the modified sugar and the peptide. The sugar moiety of the modified sugar is preferably selected from nucleotide sugars.

In an exemplary embodiment, the modified sugar, such as those set forth above, is activated as the corresponding nucleotide sugars. Exemplary sugar nucleotides that are used in the present invention in their modified form include nucleotide mono-, di- or triphosphates or analogs thereof. In a preferred embodiment, the modified sugar nucleotide is selected from a UDP-glycoside, CMP-glycoside, or a GDP-glycoside. Even more preferably, the sugar nucleotide portion of the modified sugar nucleotide is selected from UDP-galactose, UDP-galactosamine, UDP-glucose, UDP-glucosamine, GDP-mannose, GDP-fucose, CMP-sialic acid, or CMP-NeuAc. In an exemplary embodiment, the nucleotide phosphate is attached to C-1.

Also disclosed is the use of sugar nucleotides modified with L-R¹ at the 6-carbon position. Exemplary species according to this embodiment include: 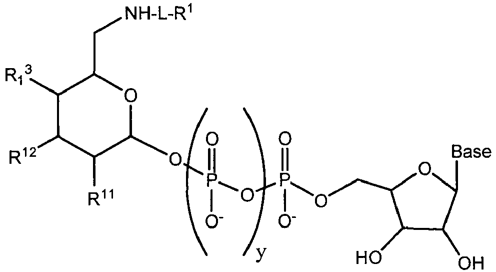 in which the R groups, and L, represent moieties as discussed above. The index "y" is 0, 1 or 2. In an exemplary embodiment, L is a bond between NH and R¹. The base is a nucleic acid base.

Exemplary nucleotide sugars of use in the invention are described herein. In another exemplary embodiment, nucleotide sugars of use in the invention are those in which the carbon at the 6-position is modified include species having the stereochemistry of GDP mannose, e.g.: 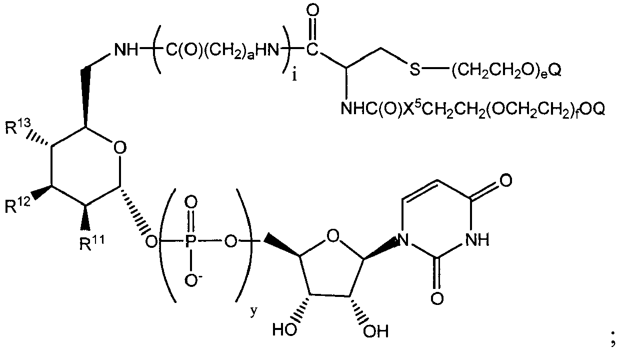 and 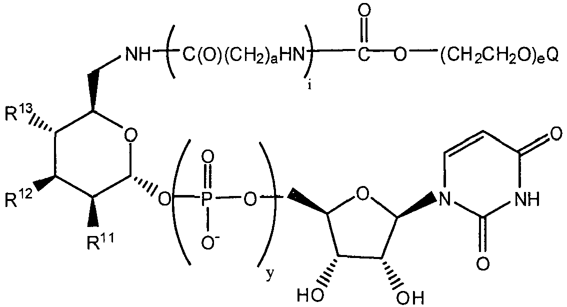 in which X⁵ is a bond or O and the remaining variables are as described above. The index i represents 0 or 1. The index a represents an integer from 1 to 20. The indices e and f independently represent integers from 1 to 2500. Q, as discussed above, is H or substituted or unsubstituted C₁-C₆ alkyl. As those of skill will appreciate, the serine derivative, in which S is replaced with O also falls within this general motif.

In a still further exemplary embodiment, the invention provides a conjugate in which the modified sugar is based on the stereochemistry of UDP galactose. An exemplary nucleotide sugar of use in this invention has the structure: 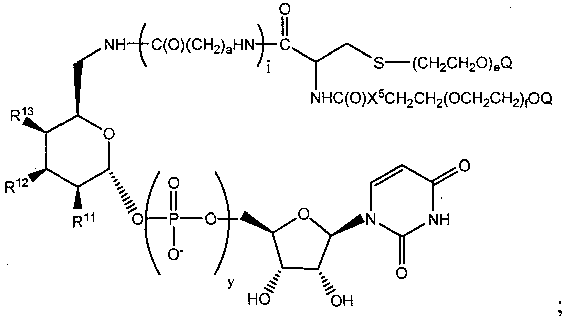 and 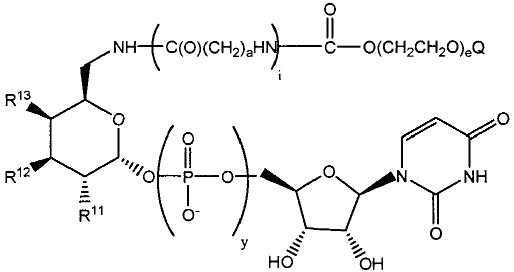 wherein the variables are as described above.

In another exemplary embodiment, the nucleotide sugar is based on the stereochemistry of glucose. Exemplary species according to this embodiment have the formulae: 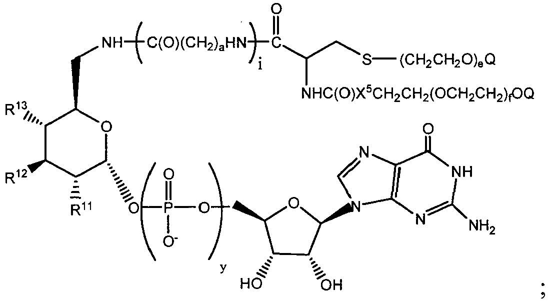 and 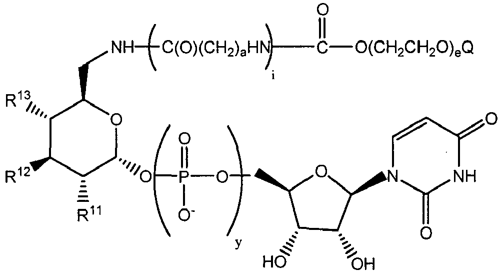 wherein the variables are as described above.

Thus, in an illustrative embodiment in which the glycosyl moiety is sialic acid, the method of the invention utilizes compounds having the formulae: 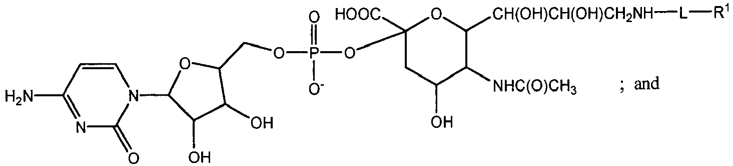 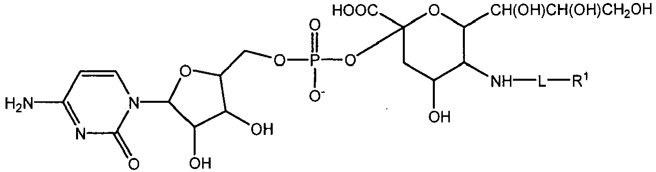 in which L-R¹ is as discussed above, and L¹-R¹ represents a linker bound to the modifying group. As with L, exemplary linker species according to L¹ include a bond, alkyl or heteroalkyl moieties.

Moreover, as discussed above, also disclosed is the use of nucleotide sugars that are modified with a water-soluble polymer, which is either straight-chain or branched. For example, compounds having the formula shown below are of use to prepare conjugates within the scope of the present invention: 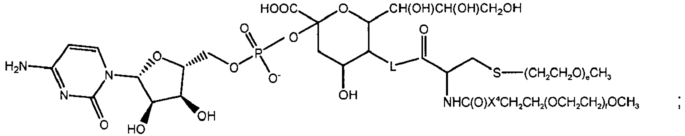 and 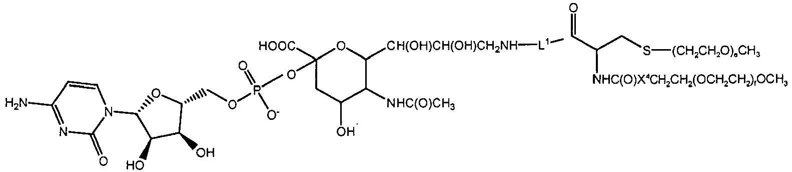 in which X⁴ is O or a bond.

In general, the sugar moiety or sugar moiety-linker cassette and the PEG or PEG-linker cassette groups are linked together through the use of reactive groups, which are typically transformed by the linking process into a new organic functional group or unreactive species. The sugar reactive functional group(s), is located at any position on the sugar moiety. Reactive groups and classes of reactions useful in practicing the present invention are generally those that are well known in the art of bioconjugate chemistry. Currently favored classes of reactions available with reactive sugar moieties are those, which proceed under relatively mild conditions. These include, but are not limited to nucleophilic substitutions (*e.g.,* reactions of amines and alcohols with acyl halides, active esters), electrophilic substitutions (*e.g.,* enamine reactions) and additions to carbon-carbon and carbon-heteroatom multiple bonds (*e.g.,* Michael reaction, Diels-Alder addition). These and other useful reactions are discussed in, for example, March, ADVANCED ORGANIC CHEMISTRY, 3rd Ed., John Wiley & Sons, New York, 1985; Hermanson, BIOCONJUGATE TECHNIQUES, Academic Press, San Diego, 1996; and Feeney et al., MODIFICATION OF PROTEINS; Advances in Chemistry Series, Vol. 198, American Chemical Society, Washington, D.C., 1982.

Useful reactive functional groups pendent from a sugar nucleus or modifying group include, but are not limited to:
(a) carboxyl groups and various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters;
(b) hydroxyl groups, which can be converted to, *e.g.,* esters, ethers, aldehydes, *etc.*
(c) haloalkyl groups, wherein the halide can be later displaced with a nucleophilic group such as, for example, an amine, a carboxylate anion, thiol anion, carbanion, or an alkoxide ion, thereby resulting in the covalent attachment of a new group at the functional group of the halogen atom;
(d) dienophile groups, which are capable of participating in Diels-Alder reactions such as, for example, maleimido groups;
(e) aldehyde or ketone groups, such that subsequent derivatization is possible via formation of carbonyl derivatives such as, for example, imines, hydrazones, semicarbazones or oximes, or via such mechanisms as Grignard addition or alkyllithium addition;
(f) sulfonyl halide groups for subsequent reaction with amines, for example, to form sulfonamides;
(g) thiol groups, which can be, for example, converted to disulfides or reacted with acyl halides;
(h) amine or sulfhydryl groups, which can be, for example, acylated, alkylated or oxidized;
(i) alkenes, which can undergo, for example, cycloadditions, acylation, Michael addition, *etc*; and
(j) epoxides, which can react with, for example, amines and hydroxyl compounds.

The reactive functional groups can be chosen such that they do not participate in, or interfere with, the reactions necessary to assemble the reactive sugar nucleus or modifying group. Alternatively, a reactive functional group can be protected from participating in the reaction by the presence of a protecting group. Those of skill in the art understand how to protect a particular functional group such that it does not interfere with a chosen set of reaction conditions. For examples of useful protecting groups, *see,* for example, Greene et a/., PROTECTIVE GROUPS IN ORGANIC SYNTHESIS, John Wiley & Sons, New York, 1991.

In the discussion that follows, a number of specific examples of modified sugars that are useful in practicing the present invention are set forth. In the exemplary embodiments, a sialic acid derivative is utilized as the sugar nucleus to which the modifying group is attached. The focus of the discussion on sialic acid derivatives is for clarity of illustration only and should not be construed to limit the scope of the invention. Those of skill in the art will appreciate that a variety of other sugar moieties can be activated and derivatized in a manner analogous to that set forth using sialic acid as an example. For example, numerous methods are available for modifying galactose, glucose, N-acetylgalactosamine and fucose to name a few sugar substrates, which are readily modified by art recognized methods. *See,* for example, Elhalabi et al., Curr. Med. Chem. 6: 93 (1999); and Schafer et al., J. Org. Chem. 65: 24 (2000)).

In an exemplary embodiment, the modified sugar is based upon a 6-amino-N-acetyl-glycosyl moiety.

In the scheme above, the index n represents an integer from 1 to 2500. In an exemplary embodiment, this index is selected such that the polymer is about 10 kD, 15 kD or 20 kD in molecular weight. The symbol "A" represents an activating group, e.g., a halo, a component of an activated ester (e.g., a N-hydroxysuccinimide ester), a component of a carbonate (e.g., p-nitrophenyl carbonate) and the like. Those of skill in the art will appreciate that other PEG-amide nucleotide sugars are readily prepared by this and analogous methods.

The peptide is typically synthesized *de novo,* or recombinantly expressed in a prokaryotic cell (*e.g.,* bacterial cell, such as *E. coli*) or in a eukaryotic cell such as a mammalian, yeast, insect, fungal or plant cell. The peptide can be either a full-length protein or a fragment. Moreover, the peptide can be a wild type or mutated peptide. In an exemplary embodiment, the peptide includes a mutation that adds one or more N- or O-linked glycosylation sites to the peptide sequence.

The method of the invention also provides for modification of incompletely glycosylated peptides that are produced recombinantly. Many recombinantly produced glycoproteins are incompletely glycosylated, exposing carbohydrate residues that may have undesirable properties, e.g., immunogenicity, recognition by the RES. Employing a modified sugar in a method of the invention, the peptide can be simultaneously further glycosylated and derivatized with, e.g., a water-soluble polymer, therapeutic agent, or the like. The sugar moiety of the modified sugar can be the residue that would properly be conjugated to the acceptor in a fully glycosylated peptide, or another sugar moiety with desirable properties.

Those of skill will appreciate that the invention can be practiced using substantially any peptide or glycopeptide from any source. Exemplary peptides with which the invention can be practiced are set forth in WO 03/031464, and the references set forth therein.

Peptides modified by the methods of the invention can be synthetic or wild-type peptides or they can be mutated peptides, produced by methods known in the art, such as site-directed mutagenesis. Glycosylation of peptides is typically either N-linked or O-linked. An exemplary N-linkage is the attachment of the modified sugar to the side chain of an asparagine residue. The tripeptide sequences asparagine-X-serine and asparagine-X-threonine, where X is any amino acid except proline, are the recognition sequences for enzymatic attachment of a carbohydrate moiety to the asparagine side chain. Thus, the presence of either of these tripeptide sequences in a polypeptide creates a potential glycosylation site. O-linked glycosylation refers to the attachment of one sugar (*e.g.,* N-acetylgalactosamine, galactose, mannose, GlcNAc, glucose, fucose or xylose) to the hydroxy side chain of a hydroxyamino acid, preferably serine or threonine, although unusual or non-natural amino acids, e.g., 5-hydroxyproline or 5-hydroxylysine may also be used.

Moreover, in addition to peptides, the methods of the present invention can be practiced with other biological structures (*e.g.,* glycolipids, lipids, sphingoids, ceramides, whole cells, and the like, containing a glycosylation site).

Addition of glycosylation sites to a peptide or other structure is conveniently accomplished by altering the amino acid sequence such that it contains one or more glycosylation sites. The addition may also be made by the incorporation of one or more species presenting an -OH group, preferably serine or threonine residues, within the sequence of the peptide (for O-linked glycosylation sites). The addition may be made by mutation or by full chemical synthesis of the peptide. The peptide amino acid sequence is preferably altered through changes at the DNA level, particularly by mutating the DNA encoding the peptide at preselected bases such that codons are generated that will translate into the desired amino acids. The DNA mutation(s) are preferably made using methods known in the art.

In an exemplary embodiment, the glycosylation site is added by shuffling polynucleotides. Polynucleotides encoding a candidate peptide can be modulated with DNA shuffling protocols. DNA shuffling is a process of recursive recombination and mutation, performed by random fragmentation of a pool of related genes, followed by reassembly of the fragments by a polymerase chain reaction-like process. *See, e.g.,* Stemmer, Proc. Natl. Acad. Sci. USA 91:10747-10751 (1994); Stemmer, Nature 370:389-391 (1994); and U.S. Patent Nos. 5,605,793, 5,837,458, 5,830,721 and 5,811,238.

Exemplary peptides with which the present invention can be practiced, methods of adding or removing glycosylation sites, and adding or removing glycosyl structures or substructures are described in detail in WO03/031464 and related U.S. and PCT applications.

The present invention also takes advantage of adding to (or removing from) a peptide one or more selected glycosyl residues, after which a modified sugar is conjugated to at least one of the selected glycosyl residues of the peptide. The present embodiment is useful, for example, when it is desired to conjugate the modified sugar to a selected glycosyl residue that is either not present on a peptide or is not present in a desired amount. Thus, prior to coupling a modified sugar to a peptide, the selected glycosyl residue is conjugated to the peptide by enzymatic or chemical coupling. In another embodiment, the glycosylation pattern of a glycopeptide is altered prior to the conjugation of the modified sugar by the removal of a carbohydrate residue from the glycopeptide. *See,* for example WO 98/31826.

Addition or removal of any carbohydrate moieties present on the glycopeptide is accomplished either chemically or enzymatically. An exemplary chemical deglycosylation is brought about by exposure of the polypeptide variant to the compound trifluoromethanesulfonic acid, or an equivalent compound. This treatment results in the cleavage of most or all sugars except the linking sugar (N-acetylglucosamine or N-acetylgalactosamine), while leaving the peptide intact. Chemical deglycosylation is described by Hakimuddin et al., Arch. Biochem. Biophys. 259: 52 (1987) and by Edge et al., Anal. Biochem. 118: 131 (1981). Enzymatic cleavage of carbohydrate moieties on polypeptide variants can be achieved by the use of a variety of endo- and exo-glycosidases as described by Thotakura et al., Meth. Enzymol. 138: 350 (1987).

In an exemplary embodiment, the peptide is essentially completely desialylated with neuraminidase prior to performing glycoconjugation or remodeling steps on the peptide. Following the glycoconjugation or remodeling, the peptide is optionally re-sialylated using a sialyltransferase. In an exemplary embodiment, the re-sialylation occurs at essentially each (e.g., >80%, preferably greater than 85%, greater than 90%, preferably greater than 95% and more preferably greater than 96%, 97%, 98% or 99%) terminal saccharyl acceptor in a population of sialyl acceptors. In a preferred embodiment, the saccharide has a substantially uniform sialylation pattern (i.e., substantially uniform glycosylation pattern).

Chemical addition of glycosyl moieties is carried out by any art-recognized method. Enzymatic addition of sugar moieties is preferably achieved using a modification of the methods set forth herein, substituting native glycosyl units for the modified sugars used in the invention. Other methods of adding sugar moieties are disclosed in U.S. Patent No. 5,876,980, 6,030,815, 5,728,554, and 5,922,577.

Exemplary attachment points for selected glycosyl residue include, but are not limited to: (a) consensus sites for N-linked glycosylation, and sites for O-linked glycosylation; (b) terminal glycosyl moieties that are acceptors for a glycosyltransferase; (c) arginine, asparagine and histidine; (d) free carboxyl groups; (e) free sulfhydryl groups such as those of cysteine; (f) free hydroxyl groups such as those of serine, threonine, or hydroxyproline; (g) aromatic residues such as those of phenylalanine, tyrosine, or tryptophan; or (h) the amide group of glutamine. Exemplary methods of use in the present invention are described in WO 87/05330 published Sep. 11, 1987, and in Aplin and Wriston, CRC CRIT. REV. BIOCHEM., pp. 259-306 (1981).

Also disclosed is a method for linking two or more peptides through a linking group. The linking group is of any useful structure and may be selected from straight- and branched-chain structures. Preferably, each terminus of the linker, which is attached to a peptide, includes a modified sugar (i.e., a nascent intact glycosyl linking group).

In an exemplary method, two peptides are linked together via a linker moiety that includes a polymeric (e.g., PEG linker). The construct conforms to the general structure set forth in the cartoon above. As described herein, the construct of the invention includes two intact glycosyl linking groups (i.e., s + t = 1). The focus on a PEG linker that includes two glycosyl groups is for purposes of clarity and should not be interpreted as limiting the identity of linker arms of use in this embodiment of the invention.

Thus, a PEG moiety is functionalized at a first terminus with a first glycosyl unit and at a second terminus with a second glycosyl unit. The first and second glycosyl units are preferably substrates for different transferases, allowing orthogonal attachment of the first and second peptides to the first and second glycosyl units, respectively. In practice, the (glycosyl)¹-PEG-(glycosyl)² linker is contacted with the first peptide and a first transferase for which the first glycosyl unit is a substrate, thereby forming (peptide)¹-(glycosyl)¹-PEG-(glycosyl)². Transferase and/or unreacted peptide is then optionally removed from the reaction mixture. The second peptide and a second transferase for which the second glycosyl unit is a substrate are added to the (peptide)¹-(glycosyl)¹-PEG-(glycosyl)² conjugate, forming (peptide)¹-(glycosyl)¹-PEG-(glycosyl)²-(peptide)² ; at least one of the glycosyl residues is either directly or indirectly O-linked. Those of skill in the art will appreciate that the method outlined above is also applicable to forming conjugates between more than two peptides by, for example, the use of a branched PEG, dendrimer, poly(amino acid), polysaccharide or the like.

In an exemplary embodiment, the peptide that is modified by a method of the invention is a glycopeptide that is produced in mammalian cells (e.g., CHO cells) or in a transgenic animal and thus, contains N- and/or O-linked oligosaccharide chains, which are incompletely sialylated. The oligosaccharide chains of the glycopeptide lacking a sialic acid and containing a terminal galactose residue can be PEGylated, PPGylated or otherwise modified with a modified sialic acid.

In Scheme 1, the amino glycoside **1**, is treated with the active ester of a protected amino acid (*e.g.,* glycine) derivative, converting the sugar amine residue into the corresponding protected amino acid amide adduct. The adduct is treated with an aldolase to form α-hydroxy carboxylate **2**. Compound **2** is converted to the corresponding CMP derivative by the action of CMP-SA synthetase, followed by catalytic hydrogenation of the CMP derivative to produce compound **3**. The amine introduced via formation of the glycine adduct is utilized as a locus of PEG attachment by reacting compound **3** with an activated PEG or PPG derivative (*e.g.,* PEG-C(O)NHS, PEG-OC(O)O-p-nitrophenyl), producing species such as **4** or **5**, respectively. 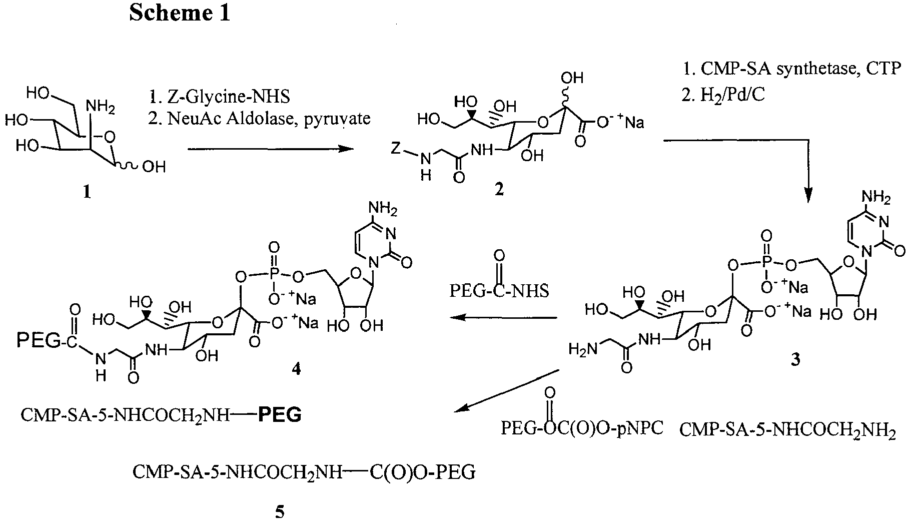 In an exemplary embodiment, a modified sugar can be attached to an O-glycan binding site on a peptide. The glycosyltransferases which can be used to produce this peptide conjugate include: for Ser56 (-Glc-(Xyl)n-Gal-SA-PEG - a galactosyltransferase and sialyltransferase; for Ser56 -Glc-(Xyl)n-Xyl-PEG - a xylosyltransferase; and for Ser60-Fuc-GlcNAc-(Gal)n-(SA)m-PEG - a GlcNAc transferase.

### III. A. Conjugation of Modified Sugars to Peptides

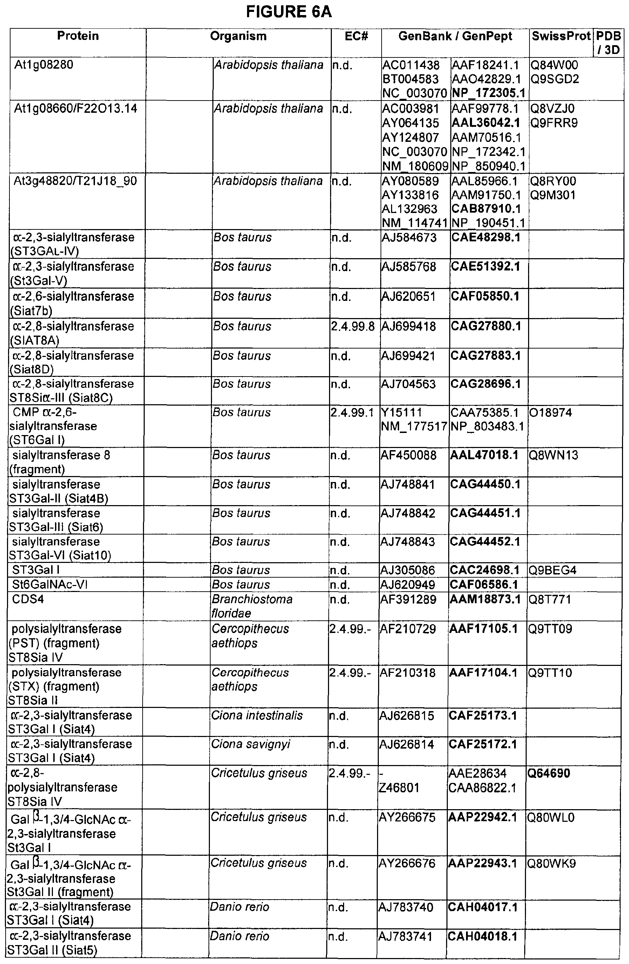

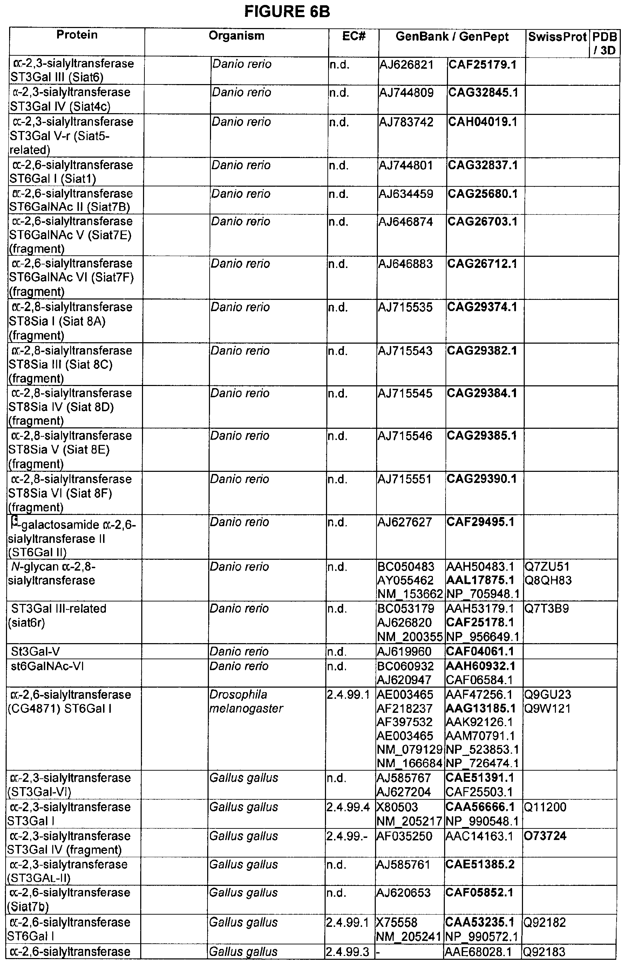

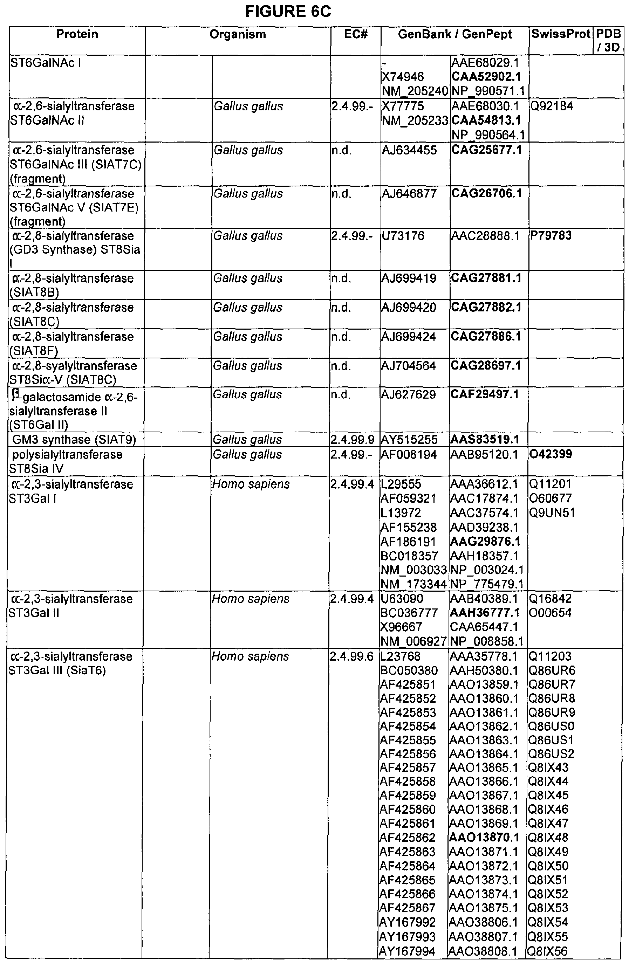

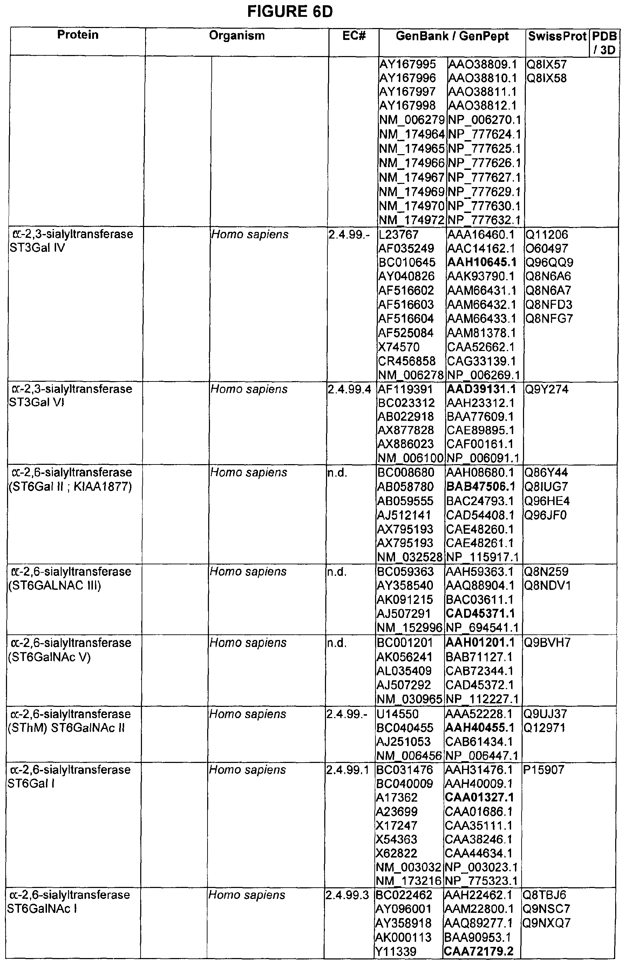

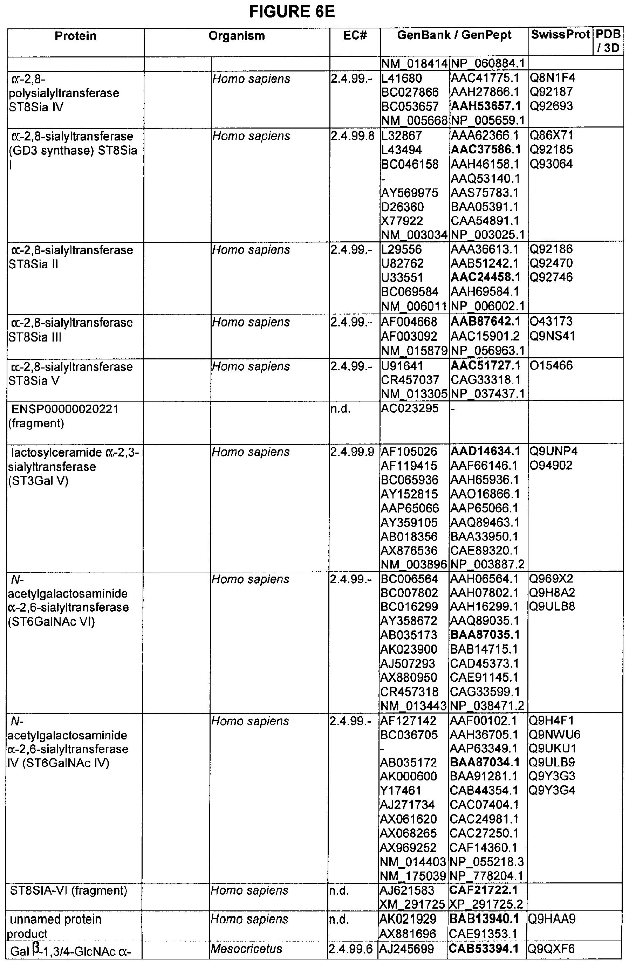

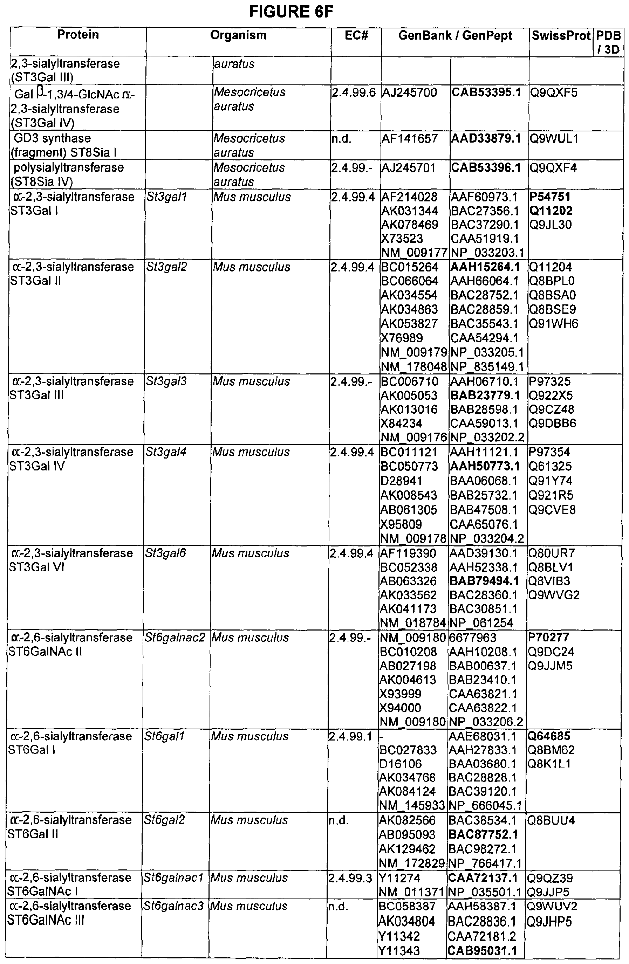

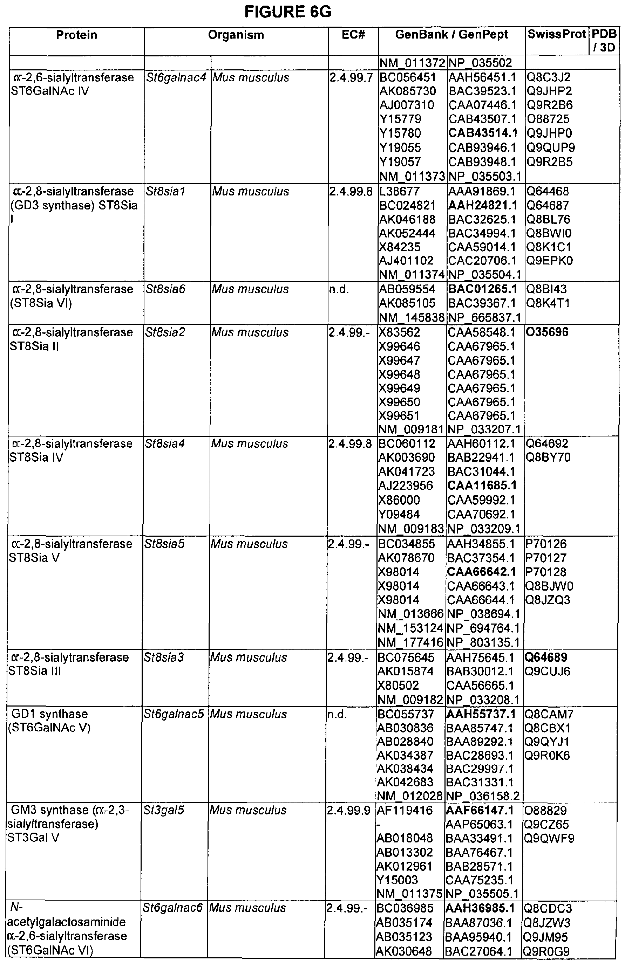

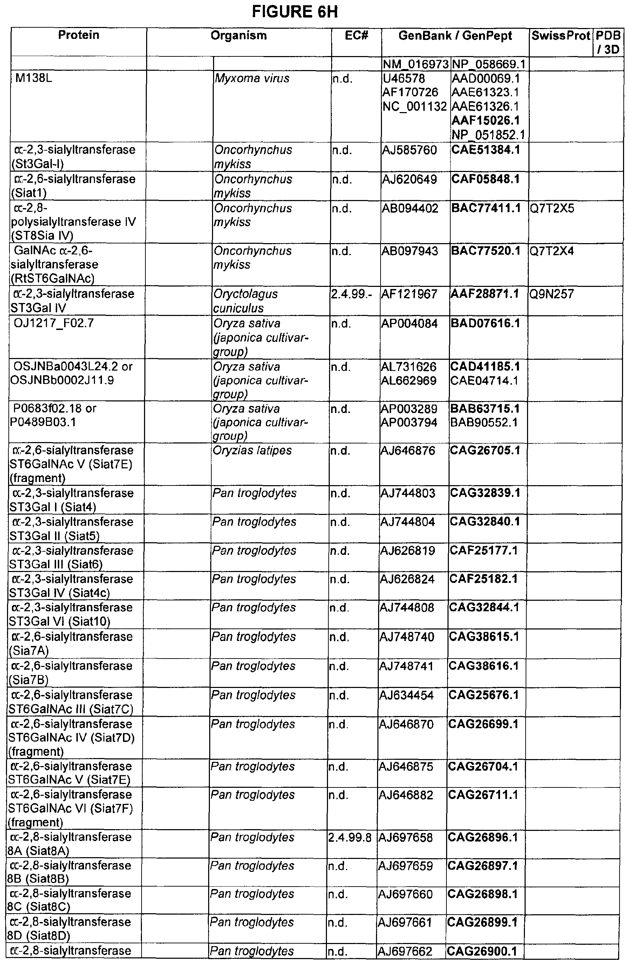

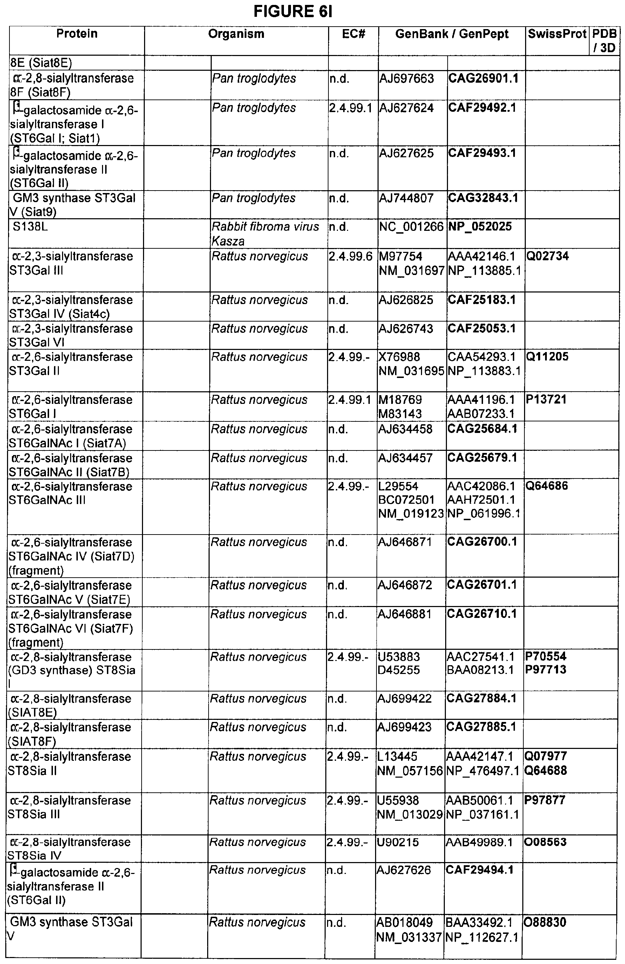

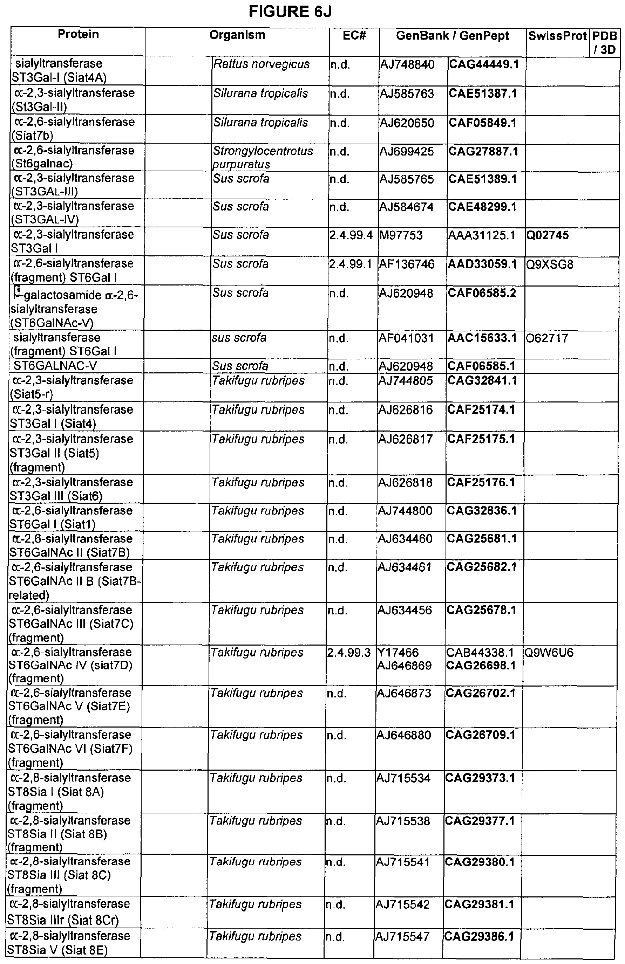

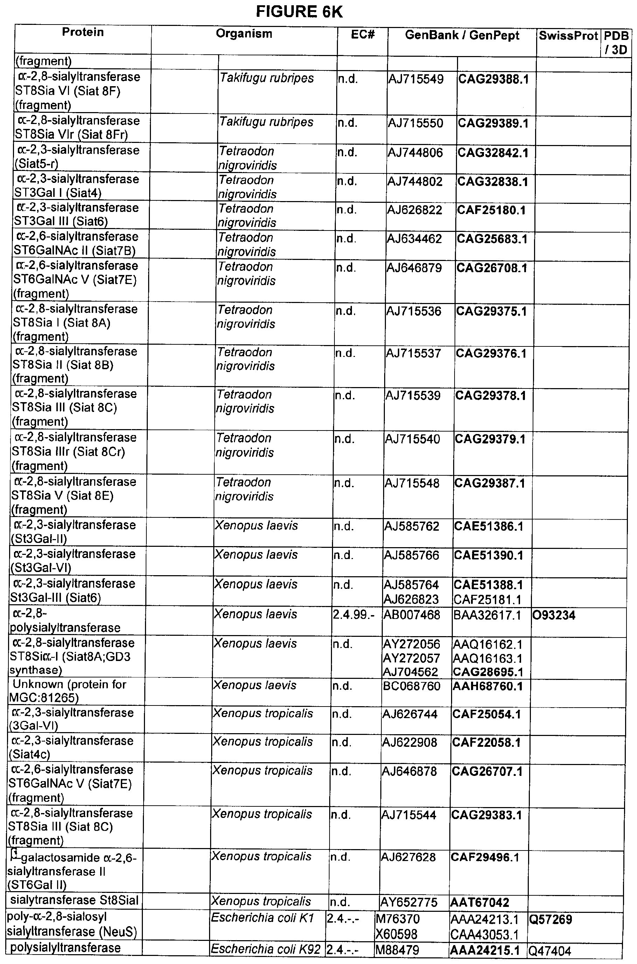

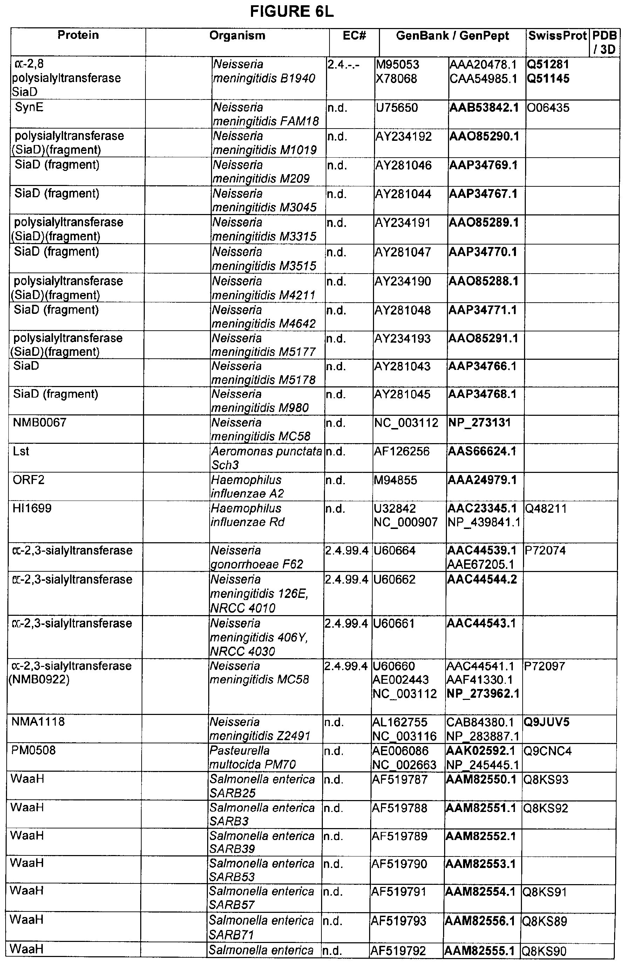

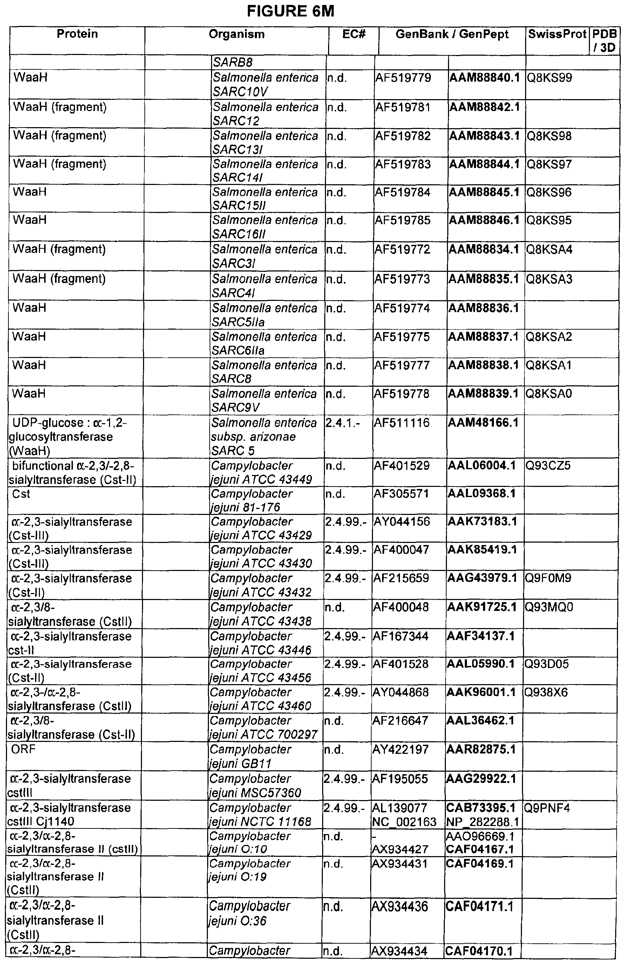

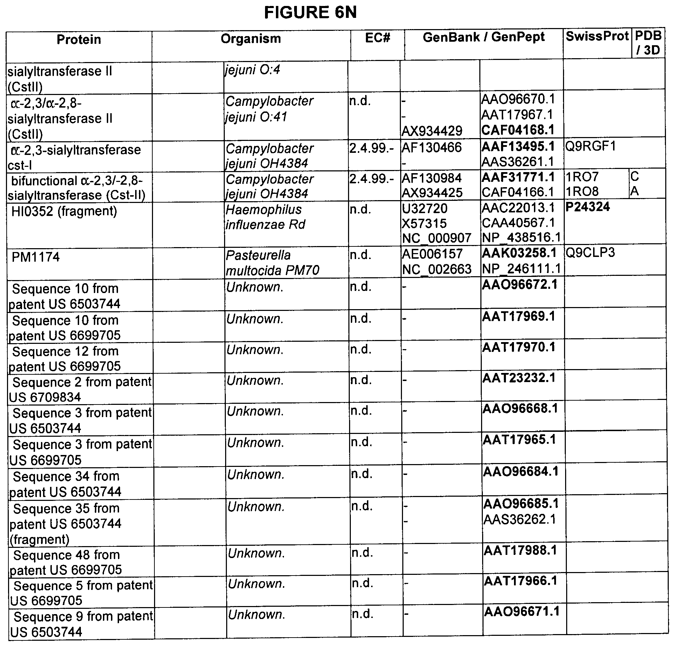

The PEG modified sugars are conjugated to a glycosylated or non-glycosylated peptide using an appropriate enzyme to mediate the conjugation. Preferably, the concentrations of the modified donor sugar(s), enzyme(s) and acceptor peptide(s) are selected such that glycosylation proceeds until the acceptor is consumed. The considerations discussed below, while set forth in the context of a sialyltransferase, are generally applicable to other glycosyltransferase reactions. A list of preferred sialyltransferases for use in the invention is provided in **FIG. 6****.**

A number of methods of using glycosyltransferases to synthesize desired oligosaccharide structures are known and are generally applicable to the instant invention. Exemplary methods are described, for instance, WO 96/32491, Ito et al., Pure Appl. Chem. 65: 753 (1993), U.S. Pat. Nos. 5,352,670, 5,374,541, 5,545,553, commonly owned U.S. Pat. Nos. 6,399,336, and 6,440,703, and commonly owned published PCT applications, WO 03/031464, WO 04/033651, WO 04/099231.

The present invention is practiced using a single glycosyltransferase or a combination of glycosyltransferases. For example, one can use a combination of a sialyltransferase and a galactosyltransferase. In those embodiments using more than one enzyme, the enzymes and substrates are preferably combined in an initial reaction mixture, or the enzymes and reagents for a second enzymatic reaction are added to the reaction medium once the first enzymatic reaction is complete or nearly complete. By conducting two enzymatic reactions in sequence in a single vessel, overall yields are improved over procedures in which an intermediate species is isolated. Moreover, cleanup and disposal of extra solvents and by-products is reduced.

In a preferred embodiment, each of the first and second enzyme is a glycosyltransferase. In another preferred embodiment, one enzyme is an endoglycosidase. In an additional preferred embodiment, more than two enzymes are used to assemble the modified glycoprotein of the invention. The enzymes are used to alter a saccharide structure on the peptide at any point either before or after the addition of the modified sugar to the peptide.

In another embodiment, the method makes use of one or more exo- or endoglycosidase. The glycosidase is typically a mutant, which is engineered to form glycosyl bonds rather than rupture them. The mutant glycanase typically includes a substitution of an amino acid residue for an active site acidic amino acid residue. For example, when the endoglycanase is endo-H, the substituted active site residues will typically be Asp at position 130, Glu at position 132 or a combination thereof. The amino acids are generally replaced with serine, alanine, asparagine, or glutamine.

The mutant enzyme catalyzes the reaction, usually by a synthesis step that is analogous to the reverse reaction of the endoglycanase hydrolysis step. In these embodiments, the glycosyl donor molecule (*e.g.,* a desired oligo- or mono-saccharide structure) contains a leaving group and the reaction proceeds with the addition of the donor molecule to a GlcNAc residue on the protein. For example, the leaving group can be a halogen, such as fluoride. In other embodiments, the leaving group is a Asn, or a Asn-peptide moiety. In further embodiments, the GlcNAc residue on the glycosyl donor molecule is modified. For example, the GlcNAc residue may comprise a 1,2 oxazoline moiety.

In a preferred embodiment, each of the enzymes utilized to produce a conjugate of the invention are present in a catalytic amount. The catalytic amount of a particular enzyme varies according to the concentration of that enzyme's substrate as well as to reaction conditions such as temperature, time and pH value. Means for determining the catalytic amount for a given enzyme under preselected substrate concentrations and reaction conditions are well known to those of skill in the art.

The temperature at which an above process is carried out can range from just above freezing to the temperature at which the most sensitive enzyme denatures. Preferred temperature ranges are about 0 °C to about 55°C, and more preferably about 20°C to about 37°C. In another exemplary embodiment, one or more components of the present method are conducted at an elevated temperature using a thermophilic enzyme.

The reaction mixture is maintained for a period of time sufficient for the acceptor to be glycosylated, thereby forming the desired conjugate. Some of the conjugate can often be detected after a few h, with recoverable amounts usually being obtained within 24 h or less. Those of skill in the art understand that the rate of reaction is dependent on a number of variable factors (*e.g,* enzyme concentration, donor concentration, acceptor concentration, temperature, solvent volume), which are optimized for a selected system.

The present invention also provides for the industrial-scale production of modified peptides. As used herein, an industrial scale generally produces at least one gram of finished, purified conjugate.

In the discussion that follows, the invention is exemplified by the conjugation of modified sialic acid moieties to a glycosylated peptide. The exemplary modified sialic acid is labeled with PEG. The focus of the following discussion on the use of PEG-modified sialic acid and glycosylated peptides is for clarity of illustration and is not intended to imply that the invention is limited to the conjugation of these two partners. One of skill understands that the discussion is generally applicable to the additions of modified glycosyl moieties other than sialic acid. Moreover, the discussion is equally applicable to the modification of a glycosyl unit with agents other than PEG including other PEG moieties, therapeutic moieties, and biomolecules.

An enzymatic approach can be used for the selective introduction of PEGylated or PPGylated carbohydrates onto a peptide or glycopeptide. The method utilizes modified sugars containing PEG, PPG, or a masked reactive functional group, and is combined with the appropriate glycosyltransferase or glycosynthase. By selecting the glycosyltransferase that will make the desired carbohydrate linkage and utilizing the modified sugar as the donor substrate, the PEG or PPG can be introduced directly onto the peptide backbone, onto existing sugar residues of a glycopeptide or onto sugar residues that have been added to a peptide.

In an exemplary embodiment, an acceptor for a sialyltransferase is present on the peptide to be modified either as a naturally occurring structure or it is placed there recombinantly, enzymatically or chemically. Suitable acceptors, include, for example, galactosyl acceptors such as Galβ1,4GlcNAc, Galβ1,4GalNAc, Galβ1,3GalNAc, lacto-N-tetraose, Galβ1,3GlcNAc, Galβ1,3Ara, Galβ1,6GlcNAc, Galβ1,4Glc (lactose), and other acceptors known to those of skill in the art *(see, e.g.,* Paulson et al., J. Biol. Chem. 253: 5617-5624 (1978)). Exemplary sialyltransferases are set forth herein.

In one embodiment, an acceptor for the sialyltransferase is present on the glycopeptide to be modified upon *in vivo* synthesis of the glycopeptide. Such glycopeptides can be sialylated using the claimed methods without prior modification of the glycosylation pattern of the glycopeptide. Alternatively, the methods of the invention can be used to sialylate a peptide that does not include a suitable acceptor; one first modifies the peptide to include an acceptor by methods known to those of skill in the art. In an exemplary embodiment, a GalNAc residue is added by the action of a GalNAc transferase.

In an exemplary embodiment, the galactosyl acceptor is assembled by attaching a galactose residue to an appropriate acceptor linked to the peptide, e.g., a GlcNAc. The method includes incubating the peptide to be modified with a reaction mixture that contains a suitable amount of a galactosyltransferase (*e.g.,* Galβ1,3 or Galβ1,4), and a suitable galactosyl donor (*e.g.,* UDP-galactose). The reaction is allowed to proceed substantially to completion or, alternatively, the reaction is terminated when a preselected amount of the galactose residue is added. Other methods of assembling a selected saccharide acceptor will be apparent to those of skill in the art.

In yet another embodiment, glycopeptide-linked oligosaccharides are first "trimmed," either in whole or in part, to expose either an acceptor for the sialyltransferase or a moiety to which one or more appropriate residues can be added to obtain a suitable acceptor. Enzymes such as glycosyltransferases and endoglycosidases (*see,* for example U.S. Patent No. 5,716,812) are useful for the attaching and trimming reactions. In another embodiment of this method, the sialic acid moieties of the peptide are essentially completely removed (e.g., at least 90, at least 95 or at least 99%), exposing an acceptor for a modified sialic acid.

In the discussion that follows, the method of the invention is exemplified by the use of modified sugars having a PEG moiety attached thereto. The focus of the discussion is for clarity of illustration. Those of skill will appreciate that the discussion is equally relevant to those embodiments in which the modified sugar bears a therapeutic moiety, biomolecule or the like.

In an exemplary embodiment of the invention in which a carbohydrate residue is "trimmed" prior to the addition of the modified sugar high mannose is trimmed back to the first generation biantennary structure. A modified sugar bearing a PEG moiety is conjugated to one or more of the sugar residues exposed by the "trimming back." In one example, a PEG moiety is added via a GlcNAc moiety conjugated to the PEG moiety. The modified GlcNAc is attached to one or both of the terminal mannose residues of the biantennary structure. Alternatively, an unmodified GlcNAc can be added to one or both of the termini of the branched species.

In another exemplary embodiment, a PEG moiety is added to one or both of the terminal mannose residues of the biantennary structure via a modified sugar having a galactose residue, which is conjugated to a GlcNAc residue added onto the terminal mannose residues. Alternatively, an unmodified Gal can be added to one or both terminal GlcNAc residues.

In yet a further example, a PEG moiety is added onto a Gal residue using a modified sialic acid such as those discussed above.

In another exemplary embodiment, a high mannose structure is "trimmed back" to the mannose from which the biantennary structure branches. In one example, a PEG moiety is added via a GlcNAc modified with the polymer. Alternatively, an unmodified GlcNAc is added to the mannose, followed by a Gal with an attached PEG moiety. In yet another embodiment, unmodified GlcNAc and Gal residues are sequentially added to the mannose, followed by a sialic acid moiety modified with a PEG moiety.

A high mannose structure can also be trimmed back to the elementary tri-mannosyl core.

In a further exemplary embodiment, high mannose is "trimmed back" to the GlcNAc to which the first mannose is attached. The GlcNAc is conjugated to a Gal residue bearing a PEG moiety. Alternatively, an unmodified Gal is added to the GlcNAc, followed by the addition of a sialic acid modified with a water-soluble sugar. In yet a further example, the terminal GlcNAc is conjugated with Gal and the GlcNAc is subsequently fucosylated with a modified fucose bearing a PEG moiety.

High mannose may also be trimmed back to the first GlcNAc attached to the Asn of the peptide. In one example, the GlcNAc of the GlcNAc-(Fuc)ₐ residue is conjugated wit ha GlcNAc bearing a water soluble polymer. In another example, the GlcNAc of the GlcNAc-(Fuc)ₐ residue is modified with Gal, which bears a water soluble polymer. In a still further embodiment, the GlcNAc is modified with Gal, followed by conjugation to the Gal of a sialic acid modified with a PEG moiety.

Other exemplary embodiments are set forth in commonly owned U.S. Patent application Publications: 20040132640; 20040063911; 20040137557; U.S. Patent application Nos: 10/369,979; 10/410,913; 10/360,770; 10/410,945 and PCT/US02/32263.

The Examples set forth above provide an illustration of the power of the methods set forth herein. Using the methods described herein, it is possible to "trim back" and build up a carbohydrate residue of substantially any desired structure. The modified sugar can be added to the termini of the carbohydrate moiety as set forth above, or it can be intermediate between the peptide core and the terminus of the carbohydrate.

In an exemplary embodiment, an existing sialic acid is removed from a glycopeptide using a sialidase, thereby unmasking all or most of the underlying galactosyl residues. Alternatively, a peptide or glycopeptide is labeled with galactose residues, or an oligosaccharide residue that terminates in a galactose unit. Following the exposure of or addition of the galactose residues, an appropriate sialyltransferase is used to add a modified sialic acid.

In another exemplary embodiment, an enzyme that transfers sialic acid onto sialic acid is utilized. This method can be practiced without treating a sialylated glycan with a sialidase to expose glycan residues beneath the sialic acid. An exemplary polymer-modified sialic acid is a sialic acid modified with poly(ethylene glycol). Other exemplary enzymes that add sialic acid and modified sialic acid moieties onto glycans that include a sialic acid residue or exchange an existing sialic acid residue on a glycan for these species include ST3Gal3, CST-II, ST8Sia-II, ST8Sia-III and ST8Sia-IV.

In yet a further approach, a masked reactive functionality is present on the sialic acid. The masked reactive group is preferably unaffected by the conditions used to attach the modified sialic acid to the Factor VII/Factor VIIa peptide. After the covalent attachment of the modified sialic acid to the peptide, the mask is removed and the peptide is conjugated with an agent such as PEG. The agent is conjugated to the peptide in a specific manner by its reaction with the unmasked reactive group on the modified sugar residue.

Any modified sugar can be used with its appropriate glycosyltransferase, depending on the terminal sugars of the oligosaccharide side chains of the glycopeptide. As discussed above, the terminal sugar of the glycopeptide required for introduction of the PEGylated structure can be introduced naturally during expression or it can be produced post expression using the appropriate glycosidase(s), glycosyltransferase(s) or mix of glycosidase(s) and glycosyltransferase(s).

In a further exemplary embodiment, UDP-galactose-PEG is reacted with β1,4-galactosyltransferase, thereby transferring the modified galactose to the appropriate terminal N-acetylglucosamine structure. The terminal GlcNAc residues on the glycopeptide may be produced during expression, as may occur in such expression systems as mammalian, insect, plant or fungus, but also can be produced by treating the glycopeptide with a sialidase and/or glycosidase and/or glycosyltransferase, as required.

In another exemplary embodiment, a GlcNAc transferase, such as GNT1-5, is utilized to transfer PEGylated-GlcNAc to a terminal mannose residue on a glycopeptide. In a still further exemplary embodiment, an the N- and/or O-linked glycan structures are enzymatically removed from a glycopeptide to expose an amino acid or a terminal glycosyl residue that is subsequently conjugated with the modified sugar. For example, an endoglycanase is used to remove the N-linked structures of a glycopeptide to expose a terminal GlcNAc as a GlcNAc-linked-Asn on the glycopeptide. UDP-Gal-PEG and the appropriate galactosyltransferase is used to introduce the PEG-galactose functionality onto the exposed GlcNAc.

In an alternative embodiment, the modified sugar is added directly to the peptide backbone using a glycosyltransferase known to transfer sugar residues to the peptide backbone. Exemplary glycosyltransferases useful in practicing the present invention include, but are not limited to, GalNAc transferases (GalNAc T1-14), GlcNAc transferases, fucosyltransferases, glucosyltransferases, xylosyltransferases, mannosyltransferases and the like. Use of this approach allows the direct addition of modified sugars onto peptides that lack any carbohydrates or, alternatively, onto existing glycopeptides. In both cases, the addition of the modified sugar occurs at specific positions on the peptide backbone as defined by the substrate specificity of the glycosyltransferase and not in a random manner as occurs during modification of a protein's peptide backbone using chemical methods. An array of agents can be introduced into proteins or glycopeptides that lack the glycosyltransferase substrate peptide sequence by engineering the appropriate amino acid sequence into the polypeptide chain.

In each of the exemplary embodiments set forth above, one or more additional chemical or enzymatic modification steps can be utilized following the conjugation of the modified sugar to the peptide. In an exemplary embodiment, an enzyme (*e.g.,* fucosyltransferase) is used to append a glycosyl unit (*e.g.,* fucose) onto the terminal modified sugar attached to the peptide. In another example, an enzymatic reaction is utilized to "cap" sites to which the modified sugar failed to conjugate. Alternatively, a chemical reaction is utilized to alter the structure of the conjugated modified sugar. For example, the conjugated modified sugar is reacted with agents that stabilize or destabilize its linkage with the peptide component to which the modified sugar is attached. In another example, a component of the modified sugar is deprotected following its conjugation to the peptide. One of skill will appreciate that there is an array of enzymatic and chemical procedures that are useful in the methods of the invention at a stage after the modified sugar is conjugated to the peptide. Further elaboration of the modified sugar-peptide conjugate is within the scope of the invention.

Enzymes and reaction conditions for preparing the conjugates of the present invention are discussed in detail in the parent of the instant application as well as co-owned published PCT patent applications WO 03/031464, WO 04/033651, WO 04/099231.

In a selected embodiment, a peptide, expressed in insect cells, is remodeled such that glycans on the remodeled glycopeptide include a GlcNAc-Gal glycosyl residue. The addition of GlcNAc and Gal can occur as separate reactions or as a single reaction in a single vessel. In this example, GlcNAc-transferase I and Gal-transferase I are used. The modified sialyl moiety is added using ST3Gal-III.

In another embodiment, the addition of GlcNAc, Gal and modified Sia can also occur in a single reaction vessel, using the enzymes set forth above. Each of the enzymatic remodeling and glycoPEGylation steps are carried out individually.

When the peptide is expressed in mammalian cells, different methods are of use. In one embodiment, the peptide is conjugated without need for remodeling prior to conjugation by contacting the peptide with a sialyltransferase that transfers the modified sialic acid directly onto a sialic acid on the peptide forming Sia-Sia-L-R¹, or exchanges a sialic acid on the peptide for the modified sialic acid, forming Sia-L-R¹. An exemplary enzyme of use in this method is CST-II. Other enzymes that add sialic acid to sialic acid are known to those of skill in the art and examples of such enzymes are set forth the figures appended hereto.

In yet another method of preparing the conjugates of the invention, the peptide expressed in a mammalian system is desialylated using a sialidase. The exposed Gal residue is sialylated with a modified sialic acid using a sialyltransferase specific for O-linked glycans, providing a peptide with an O-linked modified glycan. The desialylated, modified peptide is optionally partially or fully re-sialylated by using a sialyltransferase such as ST3GalIII.

In another aspect, also disclosed is a method of making a PEGylated peptide conjugate of the invention. The method includes: (a) contacting a peptide comprising a glycosyl group selected from: 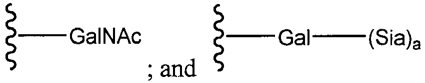 with a PEG-sialic acid donor having the formula which is a member selected from 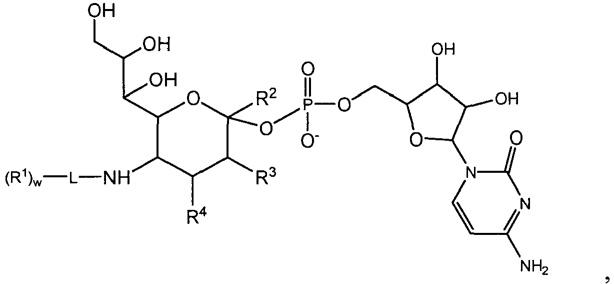 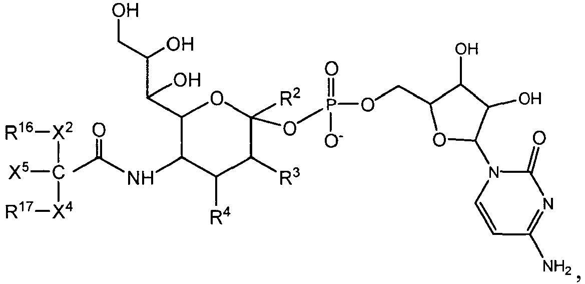 and 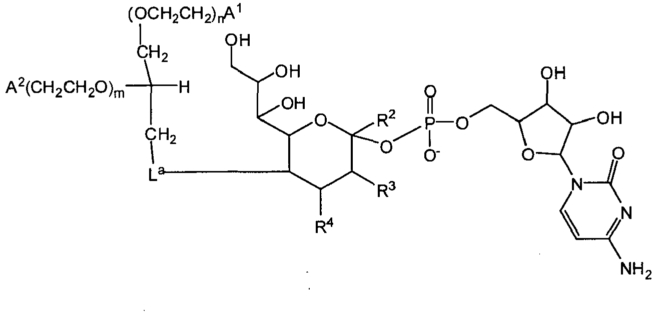 wherein the variables are as described above, and an enzyme that transfers PEG-sialic acid from said donor onto a member selected from the GalNAc, Gal and the Sia of said glycosyl group, under conditions appropriate for said transfer. An exemplary modified sialic acid donor is CMP-sialic acid modified, through a linker moiety, with a polymer, e.g., a straight chain or branched poly(ethylene glycol) moiety. As discussed herein, the peptide is optionally glycosylated with GalNAc and/or Gal and/or Sia ("Remodeled") prior to attaching the modified sugar. The remodeling steps can occur in sequence in the same vessel without purification of the glycosylated peptide between steps. Alternatively, following one or more remodeling step, the glycosylated peptide can be purified prior to submitting it to the next glycosylation or glycPEGylation step. In an exemplary embodiment, the method further comprises expressing the peptide in a host. In an exemplary embodiment, the host is a mammalian cell or an insect cell. In another exemplary embodiment, the mammalian cell is a member selected from a BHK cell and a CHO cell and the insect cell is a *Spodoptera frugiperda* cell.

As illustrated in the examples and discussed further below, placement of an acceptor moiety for the PEG-sugar is accomplished in any desired number of steps. For example, in one embodiment, the addition of GalNAc to the peptide can be followed by a second step in which the PEG-sugar is conjugated to the GalNAc in the same reaction vessel. Alternatively, these two steps can be carried out in a single vessel approximately simultaneously.

In an exemplary embodiment, the PEG-sialic acid donor has the formula: 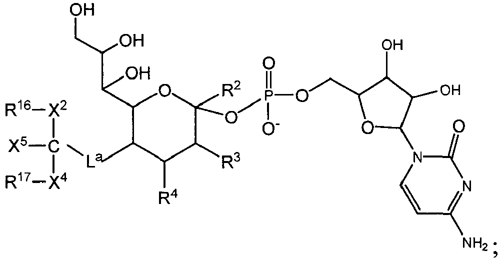 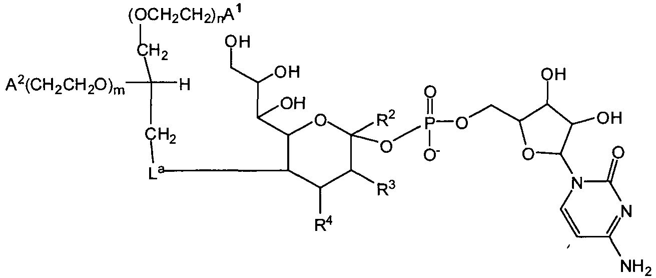 wherein the variables are as described above.

In a further exemplary embodiment, the peptide is expressed in an appropriate expression system prior to being glycopegylated or remodeled. Exemplary expression systems include Sf-9/baculovirus and Chinese Hamster Ovary (CHO) cells.

In an exemplary embodiment, the invention provides a method of making a peptide conjugate comprising a glycosyl linker comprising a modified sialyl residue having the formula: 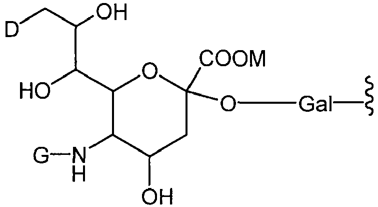 wherein D is a member selected from -OH and R¹-L-HN-; G is a member selected from R¹-L-and -C(O)(C₁-C₆)alkyl-R¹; R¹ is a moiety comprising a member selected from a straight-chain poly(ethylene glycol) residue and branched poly(ethylene glycol) residue; M is a member selected from H, a metal and a single negative charge; L is a linker which is a member selected from a bond, substituted or unsubstituted alkyl and substituted or unsubstituted heteroalkyl, such that when D is OH, G is R¹-L-, and when G is -C(O)(C₁-C₆)alkyl, D is R¹-L-NH-
said method comprising: (a) contacting a peptide comprising the glycosyl moiety: 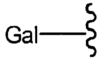 with a PEG-sialic acid donor moiety having the formula: 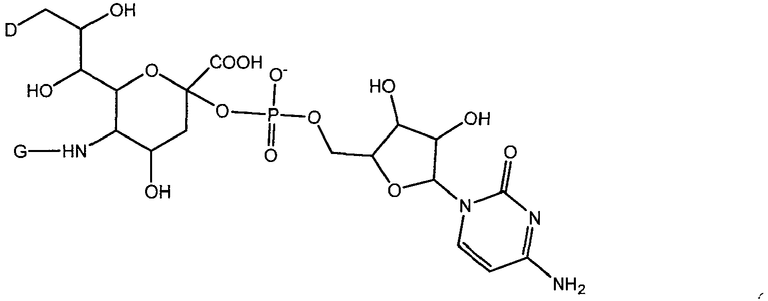 wherein the variables are as described above, and an enzyme that transfers said PEG-sialic acid onto the Gal of said glycosyl moiety, under conditions appropriate for said transfer.

In an exemplary embodiment, L-R¹ has the formula: 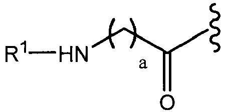 wherein a is an integer selected from 0 to 20.

In another exemplary embodiment, R¹ has a structure that is a member selected from: 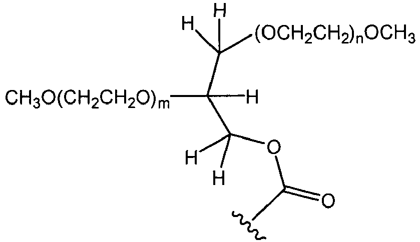 wherein m and n are integers independently selected from 1 to 2500; and q is an integer selected from 0 to 20.

Large scale or small scale amounts of peptide conjugate can be produced by the methods described herein. In an exemplary embodiment, the amount of peptide is a member selected from about 0.5 mg to about 100kg. In an exemplary embodiment, the amount of peptide is a member selected from about 0.1 kg to about 1 kg. In an exemplary embodiment, the amount of peptide is a member selected from about 0.5 kg to about 10kg. In an exemplary embodiment, the amount of peptide is a member selected from about 0.5 kg to about 3kg. In an exemplary embodiment, the amount of peptide is a member selected from about 0.1 kg to about 5kg. In an exemplary embodiment, the amount of peptide is a member selected from about 0.08 kg to about 0.2 kg. In an exemplary embodiment, the amount of peptide is a member selected from about 0.05 kg to about 0.4kg. In an exemplary embodiment, the amount of peptide is a member selected from about 0.1 kg to about 0.7kg. In an exemplary embodiment, the amount of peptide is a member selected from about 0.3 kg to about 1.75 kg. In an exemplary embodiment, the amount of peptide is a member selected from about 25 kg to about 65kg.

The concentration of peptide utilized in the reactions described herein is a member selected from about 0.5 to about 10 mg peptide/mL reaction mixture. In an exemplary embodiment, the peptide concentration is a member selected from about 0.5 to about 1 mg peptide/mL reaction mixture. In an exemplary embodiment, the peptide concentration is a member selected from about 0.8 to about 3 mg peptide/mL reaction mixture. In an exemplary embodiment, the peptide concentration is a member selected from about 2 to about 6 mg peptide/mL reaction mixture. In an exemplary embodiment, the peptide concentration is a member selected from about 4 to about 9 mg peptide/mL reaction mixture. In an exemplary embodiment, the peptide concentration is a member selected from about 1.2 to about 7.8 mg peptide/mL reaction mixture. In an exemplary embodiment, the peptide concentration is a member selected from about 6 to about 9.5 mg peptide/mL reaction mixture.

The concentration of PEGylated nucleotide sugar that can be utilized in the reactions described herein is a member selected from about 0.1 to about 1.0 mM. Factors which may increase or decrease the concentration include the size of the PEG, time of incubation, temperature, buffer components, as well as the type, and concentration, of glycosyltransferase used. In an exemplary embodiment, the PEGylated nucleotide sugar concentration is a member selected from about 0.1 to about 1.0 mM. In an exemplary embodiment, the PEGylated nucleotide concentration is a member selected from about 0.1 to about 0.5 mM. In an exemplary embodiment, the PEGylated nucleotide sugar concentration is a member selected from about 0.1 to about 0.3 mM. In an exemplary embodiment, the PEGylated nucleotide sugar concentration is a member selected from about 0.2 to about 0.7 mM. In an exemplary embodiment, the PEGylated nucleotide sugar concentration is a member selected from about 0.3 to about 0.5 mM. In an exemplary embodiment, the PEGylated nucleotide sugar concentration is a member selected from about 0.4 to about 1.0 mM. In an exemplary embodiment, the PEGylated nucleotide sugar concentration is a member selected from about 0.5 to about 0.7 mM. In an exemplary embodiment, the PEGylated nucleotide sugar concentration is a member selected from about 0.8 to about 0.95 mM. In an exemplary embodiment, the PEGylated nucleotide sugar concentration is a member selected from about 0.55 to about 1.0 mM.

The molar equivalents of the PEGylated nucleotide sugar that can be utilized in the reactions described herein are based on the theoretical number of PEGylated sugars that can be added to the protein. The theoretical number of PEGylated sugars is based on the theoretical number of sugar sites on the protein as well as the MW of the protein when compared to the MW and therefore moles of PEGylated nucleotide sugar. In an exemplary embodiment, the molar equivalents of PEGylated nucleotide sugar is an integer selected from 1 to 20. In an exemplary embodiment, the molar equivalents of PEGylated nucleotide sugar is an integer selected from 1 to 20. In an exemplary embodiment, the molar equivalents of PEGylated nucleotide sugar is an integer selected from 2 to 6. In an exemplary embodiment, the molar equivalents of PEGylated nucleotide sugar is an integer selected from 3 to 17. In an exemplary embodiment, the molar equivalents of PEGylated nucleotide sugar is an integer selected from 4 to 11. In an exemplary embodiment, the molar equivalents of PEGylated nucleotide sugar is an integer selected from 5 to 20. In an exemplary embodiment, the molar equivalents of PEGylated nucleotide sugar is an integer selected from 1 to 10. In an exemplary embodiment, the molar equivalents of PEGylated nucleotide sugar is an integer selected from 12 to 20. In an exemplary embodiment, the molar equivalents of PEGylated nucleotide sugar is an integer selected from 14 to 17. In an exemplary embodiment, the molar equivalents of PEGylated nucleotide sugar is an integer selected from 7 to 15. In an exemplary embodiment, the molar equivalents of PEGylated nucleotide sugar is an integer selected from 8 to 16.

### III. B. Simultaneous Desialylation and GlycoPEGylation

The present invention provides a "one-pot" method of glycopegylating. The one-pot method is distinct from other exemplary processes to make a peptide conjugate, which employ a sequential de-sialylation with sialidase, subsequent purification of the asialopeptide on an anion exchange column, then glycoPEGylation using CMP-sialic acid-PEG and a glycosyltransferase (such as ST3Gal3), exoglycosidase or an endoglycosidase. The peptide conjugate is then purified via anion exchange followed by size exclusion chromatography to produce the purified peptide conjugate.

The one-pot method is an improved method to manufacture a peptide conjugate. In this method, the de-sialylation and glycoPEGylation reactions are combined in a one-pot reaction which obviates the first anion exchange chromatography step used in the previously described process to purify the asialopeptide. This reduction in process steps produces several advantages. First, the number of process steps required to produce the peptide conjugate is reduced, which also reduces the operating complexity of the process. Second, the process time for the production of the peptide conjugates is reduced e.g., from 4 to 2 days. This reduces the raw material requirements and quality control costs associated with in-process controls. Third, the invention utilizes less sialidase, e.g., up to 20-fold less sialidase, e.g., 500 mU/L is required to produce the peptide conjugate relative to the process. This reduction in the use of sialidase significantly reduces the amount of contaminants, such as sialidase, in the reaction mixture.

In an exemplary embodiment, a peptide conjugate is prepared by the following method. In a first step, a peptide is combined with a sialidase, a modified sugar of the invention, and an enzyme capable of catalyzing the transfer of the glycosyl linking group from the modified sugar to the peptide, thus preparing the peptide conjugate. Any sialidase may be used in this method. Exemplary sialidases of use in the invention can be found in the CAZY database (*see* afmb.cnrs-mrs.fr/CAZY/index.html and www.cazy.org/CAZY). Exemplary sialidases can be purchased from any number of sources (QA-Bio, Calbiochem, Marukin, Prozyme, etc.). In an exemplary embodiment, the sialidase is a member selected from cytoplasmic sialidases, lysosomal sialidases, exo-α sialidases, and endosialidases. In another exemplary embodiment, the sialidase used is produced from bacteria such as *Clostridium perfringens* or *Streptococcus pneumoniae,* or from a virus such as an adenovirus. In an exemplary embodiment, the enzyme capable of catalyzing the transfer of the glycosyl linking group from the modified sugar to the peptide is a member selected from a glycosyltransferase, such as sialyltransferases and fucosyltransferases, as well as exoglycosidases and endoglycosidases. In an exemplary embodiment, the enzyme is a glycosyltransferase, which is ST3Gal3. In another exemplary embodiment, the enzyme used is produced from bacteria such as *Escherichia Coli* or a fungus such as *Aspergillus niger.* In another exemplary embodiment, the sialidase is added to the peptide before the glycosyltransferase for a specified time, allowing the sialidase reaction to proceed before initiating the GlycoPEGylation reaction with addition of the PEG-sialic acid reagent and the glycosyltransferase. Many of these examples are discussed herein. Finally, any modified sugar described herein can be utilized in this reaction.

In another exemplary embodiment, the method further comprises a 'capping' step. In this step, additional non-PEGylated sialic acid is added to the reaction mixture. In an exemplary embodiment, this sialic acid is added to the peptide or peptide conjugate thus preventing further addition of PEG-sialic acid. In another exemplary embodiment, this sialic acid impedes the function of the glycosyltransferase in the reaction mixture, effectively stopping the addition of glycosyl linking groups to the peptides or peptide conjugates. Most importantly, the sialic acid that is added to the reaction mixture caps the unglycoPEGylated glycans thereby providing a peptide conjugate that has improved pharmaceokinetics. In addition, this sialidase can be added directly the the glycoPEGylation reaction mixture when the extent of PEGylation to certain amounts is desired without prior purification.

In an exemplary embodiment, after the capping step, less than about 50% of the sialylation sites on the peptide or peptide conjugate does not comprise a sialyl moiety. In an exemplary embodiment, after the capping step, less than about 40% of the sialylation sites on the peptide or peptide conjugate does not comprise a sialyl moiety. In an exemplary embodiment, after the capping step, less than about 30% of the sialylation sites on the peptide or peptide conjugate does not comprise a sialyl moiety. In an exemplary embodiment, after the capping step, less than about 20% of the sialylation sites on the peptide or peptide conjugate does not comprise a sialyl moiety. In an exemplary embodiment, after the capping step, less than about 10% of the sialylation sites on the peptide or peptide conjugate does not comprise a sialyl moiety. In an exemplary embodiment, between about 20% and about 5% of the sialylation sites on the peptide or peptide conjugate does not comprise a sialyl moiety. In an exemplary embodiment, between about 25% and about 10% of the sialylation sites on the peptide or peptide conjugate does not comprise a sialyl moiety. In an exemplary embodiment, after the capping step, essentially all of the sialylation sites on the peptide or peptide conjugate comprise a sialyl moiety.

### III. C. Desialylation and Selective Modification of Peptides

In another exemplary embodiment, the present invention provides a method for desialylating a peptide. The method preferably provides a peptide that is at least about 40%, preferably 45%, preferably about 50%, preferably about 55%, preferably about 60%, preferably about 65%, preferably about 70%, preferably about 75%, preferably about 80%, preferably at least 85%, more preferably at least 90%, still more preferably, at least 92%, preferably at least 94%, even more preferably at least 96%, still more preferably at least 98%, and still more preferably 100% disialylated.

The method includes contacting the peptide with a sialidase, preferably for a time period. The preselected time period is sufficient to desialylate the peptide to the degree desired. In a preferred embodiment, the desialylated peptide is separated from the sialidase when the desired degree of desialylation is achieved. An exemplary desialylation reaction and purification cycle is set forth herein.

Those of skill are able to determine an appropriate preselected time period over which to conduct the desialylation reaction. In an exemplary embodiment, the period is less than 24 hours, preferably less than 8 hours, more preferably less than 6 hours, more preferably less than 4 hours, still more preferably less than 2 hours and even more preferably less than 1 hour.

In another exemplary embodiment, in the peptide conjugate preparation at the end of the desialylation reaction, at least 10% of the members of the population of peptides has only a single sialic acid attached thereto, preferably at least 20%, more preferably at least 30%, still more preferably at least 40%, even still more preferably at least 50% and more preferably at least 60%, and still more preferably completely desialylated.

In yet a further exemplary embodiment, in the preparation at the end of the desialylation reaction, at least 10% of the members of the population of peptides is fully desialylated, preferably at least 20%, more preferably at least 30%, even more preferably at least 40%, still more preferably at least 50% and even still more preferably at least 60%.

In still another exemplary embodiment, in the preparation at the end of the desialylation reaction, at least 10%, 20%, 30%, 40%, 50% or 60% of the members of the peptide population has only a single sialic acid, and at least 10%, 20%, 30%, 40%, 50% or 60% of the peptide is fully disialylated.

In a preferred embodiment, in the preparation at the end of the desialylation reaction, at least 50% of the population of peptides is fully disialylated and at least 40% of the members of the peptide population bears only a single sialic acid moiety.

Following desialylation, the peptide is optionally conjugated with a modified sugar. An exemplary modified sugar includes a saccharyl moiety bound to a branched or linear poly(ethylene glycol) moiety. The conjugation is catalyzed by an enzyme that transfers the modified sugar from a modified sugar donor onto an amino acid or glycosyl residue of the peptide. An exemplary modified sugar donor is a CMP-sialic acid that bears a branched or linear poly(ethylene glycol) moiety. An exemplary poly(ethylene glycol) moiety has a molecular weight of at least about 2 kD, more preferably at least about 5 kD, more preferably at least about 10 kD, preferably at least about 20 kD, more preferably at least about 30 kD, and more preferably at least about 40 kD.

In an exemplary embodiment, the enzyme utilized to transfer the modified sugar moiety from the modified sugar donor is a glycosyltransferase, e.g., sialyltransferase. An exemplary sialyltransferase of use in the methods of the invention is ST3Gal3.

An exemplary method of the invention results in a modified peptide bearing at least one, preferably at least two, preferably at least three modifying groups. In one embodiment, the peptide produced bears a single modifying group on the light chain of the peptide. In another embodiment, the method provides a modified peptide that bears a single modifying group on the heavy chain. In still another embodiment, the method provides a modified peptide with a single modifying group on the light chain and a single modifying group on the heavy chain.

In another aspect, the invention provides a method of preparing a modified peptide. The method includes contacting the peptide with a modified sugar donor bearing a modifying group and an enzyme capable of transferring a modified sugar moiety from the modified sugar donor onto an amino acid or glycosyl residue of the peptide.

In an exemplary embodiment, the method provides a population of modified peptides in which at least 40%, preferably at least 50%, preferably at least 60%, more preferably at least 70% and even more preferably at least 80% of the population members are mono-conjugated on the light chain of the peptide.

In an exemplary embodiment, the method provides a population of modified peptides in which at least 40%, preferably at least 50%, preferably at least 60%, more preferably at least 70% and even more preferably at least 80% of the population members are di-conjugated on the light chain of the peptide.

In an exemplary embodiment of this aspect, the method provides a population of modified peptides in which no more than 50%, preferably no more than 30%, preferably no more than 20%, more preferably no more than 10% of the population members are mono-conjugated on the heavy chain of the peptide.

In an exemplary embodiment of this aspect, the method provides a population of modified peptides in which no more than 50%, preferably no more than 30%, preferably no more than 20%, more preferably no more than 10% of the population members are di-conjugated on the heavy chain of the peptide.

The peptide can be subjected to the action of a sialidase prior to the contacting step, or the peptide can be used without prior desialylation. When the peptide is contacted with a sialidase it can be either essentially completely desialylated or only partially desialylated. In a preferred embodiment, the peptide is at least partially desialylated prior to the contacting step. The peptide may be essentially completely desialylated (essentially asialo) or only partially desialylated. In a preferred embodiment, the desialylated peptide is one of the desialylated embodiments described hereinabove.

### III. D. Additional aliquots of reagents added in the synthesis of Peptide Conjugates

In an exemplary embodiment of the synthesis of the peptide conjugates described herein, one or more additional aliquots of a reaction component/reagent is added to the reaction mixture after a selected period of time. In an exemplary embodiment, the peptide conjugate is a peptide conjugate. In another exemplary embodiment, the reaction component/reagent added is a modified sugar nucleotide. Introduction of a modified sugar nucleotide into the reaction will increase the likelihood of driving the GlycoPEGylation reaction to completion. In an exemplary embodiment, the nucleotide sugar is a CMP-SA-PEG described herein. In an exemplary embodiment, the reaction component/reagent added is a sialidase. In an exemplary embodiment, the reaction component/reagent added is a glycosyltransferase. In an exemplary embodiment, the reaction component/reagent added is magnesium. In an exemplary embodiment, the additional aliquot added represents about 10%, or 20%, or 30%, or 40%, or 50%, or 60%, or 70%, or 80% or 90% of the original amount in added at the start of the reaction. In an exemplary embodiment, the reaction component/reagent is added to the reaction about 3 hours, or 6 hours, or 8 hours, or 10 hours, or 12 hours, or 18 hours, or 24 hours, or 30 hours, or 36 hours after its start.

### III. E. Purification of Peptide Conjugates

The products produced by the above processes can be used without purification. However, it is usually preferred to recover the product and one or more of the intermediates, e.g., nucleotide sugars, branched and linear PEG species, modified sugars and modified nucleotide sugars. Standard, well-known techniques for recovery of glycosylated peptides such as thin or thick layer chromatography, column chromatography, ion exchange chromatography, or membrane filtration can be used. It is preferred to use membrane filtration, more preferably utilizing a reverse osmotic membrane, or one or more column chromatographic techniques for the recovery as is discussed hereinafter and in the literature cited herein. For instance, membrane filtration wherein the membranes have molecular weight cutoff of about 3000 to about 10,000 can be used to remove proteins such as glycosyl transferases. In certain instances, the molecular weight cutoff differences between the impurity and the product will be utilized in order to ensure product purification. For example, in order to purify product peptide-SA-PEG-40 kD from unreacted CMP-SA-PEG-40 kD, a filter must be chosen that will allow, for example, peptide-SA-PEG-40 kD to remain in the retentate while allowing CMP-SA-PEG-40 kD to flow into the filtrate. Nanofiltration or reverse osmosis can then be used to remove salts and/or purify the product saccharides (*see, e.g.,* WO 98/15581). Nanofilter membranes are a class of reverse osmosis membranes that pass monovalent salts but retain polyvalent salts and uncharged solutes larger than about 100 to about 2,000 Daltons, depending upon the membrane used. Thus, in a typical application, saccharides prepared by the methods of the present invention will be retained in the membrane and contaminating salts will pass through.

If the peptide is produced intracellularly, as a first step, the particulate debris, either host cells or lysed fragments, is removed. Following glycoPEGylation, the PEGylated peptide is purified by art-recognized methods , for example, by centrifugation or ultrafiltration; optionally, the protein may be concentrated with a commercially available protein concentration filter, followed by separating the polypeptide variant from other impurities by one or more steps selected from immunoaffinity chromatography, ion-exchange column fractionation (e.g., on diethylaminoethyl (DEAE) or matrices containing carboxymethyl or sulfopropyl groups), chromatography on Blue-Sepharose, CM Blue-Sepharose, MONO-Q, MONO-S, lentil lectin-Sepharose, WGA-Sepharose, Con A-Sepharose, Ether Toyopearl, Butyl Toyopearl, Phenyl Toyopearl, or protein A Sepharose, SDS-PAGE chromatography, silica chromatography, chromatofocusing, reverse phase HPLC (*e.g.,* silica gel with appended aliphatic groups), gel filtration using, *e.g.,* Sephadex molecular sieve or size-exclusion chromatography, chromatography on columns that selectively bind the polypeptide, and ethanol or ammonium sulfate precipitation. Purification can be used to separate one chain of the Factor VII/Factor VIIa peptide conjugate from the other, as further described later in this section.

Modified glycopeptides produced in culture are usually isolated by initial extraction from cells, enzymes, etc., followed by one or more concentration, salting-out, aqueous ion-exchange, or size-exclusion chromatography steps. Additionally, the modified glycoprotein may be purified by affinity chromatography. Finally, HPLC may be employed for final purification steps.

A protease inhibitor may be included in any of the foregoing steps to inhibit proteolysis and antibiotics or preservatives may be included to prevent the growth of adventitious contaminants. The protease inhibitors used in the foregoing steps may be low molecular weight inhibitors, including antipain, alpha-1-antitrypsin, anti-thrombin, leupeptin, amastatin, chymostatin, banzamidin, as well as other serine protease inhibitors (i.e. serpins). Generally, serine protease inhibitors should be used in concentrations ranging from 0.5 - 100 µM, although chymostatin in cell culture may be used in concentrations upward of 200 µM. Other serine protease inhibitors will include inhibitors specific to the chymotrypsin-like, the subtilisin-like, the alpha/beta hydrolase, or the signal peptidase clans of serine proteases. Besides serine proteases, other types of protease inhibitors may also be used, including cysteine protease inhibitors (1-10 µM) and aspartic protease inhibitors (1 - 5 µM), as well as non-specific protease inhibitors such as pepstatin (.1 - 5 µM). Protease inhibitors used in this invention may also include natural protease inhibitors, such as the hirustasin inhibitor isolated from leech. In some embodiments, protease inhibitors will comprise synthetic peptides or antibodies that are able to bind with specificity to the protease catalytic site to stabilize Factor VII/Factor VIIa without interfering with a glycoPEGylation reaction.

Within another embodiment, supernatants from systems which produce the modified glycopeptide of the invention are first concentrated using a commercially available protein concentration filter, for example, an Amicon or Millipore Pellicon ultrafiltration unit. Following the concentration step, the concentrate may be applied to a suitable purification matrix. For example, a suitable affinity matrix may comprise a ligand for the peptide, a lectin or antibody molecule bound to a suitable support. Alternatively, an anion-exchange resin may be employed, for example, a matrix or substrate having pendant DEAE groups. Suitable matrices include acrylamide, agarose, dextran, cellulose, or other types commonly employed in protein purification. Alternatively, a cation-exchange step may be employed. Suitable cation exchangers include various insoluble matrices comprising sulfopropyl or carboxymethyl groups. Sulfopropyl groups are particularly preferred.

Other methods of use in purification include size exclusion chromatography (SEC), hydroxyapatite chromatography, hydrophobic interaction chromatography and chromatography on Blue Sepharose. These and other useful methods are illustrated in co-assigned U.S. Provisional Patent No. (Attorney Docket No. 40853-01-5168-P1, filed May 6, 2005).

One or more RP-HPLC steps employing hydrophobic RP-HPLC media, e.g., silica gel having pendant methyl or other aliphatic groups, may be employed to further purify a polypeptide conjugate composition. Some or all of the foregoing purification steps, in various combinations, can also be employed to provide a homogeneous or essentially homogeneous modified glycoprotein.

The modified glycopeptide of the invention resulting from a large-scale fermentation may be purified by methods analogous to those disclosed by Urdal et al., J. Chromatog. 296: 171 (1984). This reference describes two sequential, RP-HPLC steps for purification of recombinant human IL-2 on a preparative HPLC column. Alternatively, techniques such as affinity chromatography may be utilized to purify the modified glycoprotein.

In an exemplary embodiment, the purification is accomplished by the methods set forth in commonly owned, co-assigned U.S. Provisional Patent No. 60/665,588, filed March 24,2005.

According to the present invention, pegylated peptides or peptide conjugate produced either via sequential de-sialylation or simultaneous sialylation can be purified or resolved by using magnesium chloride gradient.

### IV. Pharmaceutical Compositions

In another aspect, the invention provides a pharmaceutical composition in accordance with the appended claims. The pharmaceutical composition includes a pharmaceutically acceptable diluent and a covalent conjugate between a non-naturally-occurring, PEG moiety, and a glycosylated or non-glycosylated peptide. The polymer, therapeutic moiety or biomolecule is conjugated to the peptide via an intact glycosyl linking group interposed between and covalently linked to both the peptide and the polymer, therapeutic moiety or biomolecule.

Pharmaceutical compositions of the invention are suitable for use in a variety of drug delivery systems. Suitable formulations for use in the present invention are found in Remington's Pharmaceutical Sciences, Mace Publishing Company, Philadelphia, PA, 17th ed. (1985). For a brief review of methods for drug delivery, *see,* Langer, Science 249:1527-1533 (1990).

In an exemplary embodiment, the pharmaceutical formulation comprises a peptide conjugate and a pharmaceutically acceptable diluent which is a member selected from sodium chloride, calcium chloride dihydrate, glycylglycine, polysorbate 80, and mannitol. In another exemplary embodiment, the pharmaceutically acceptable diluent is sodium chloride and glycylglycine. In another exemplary embodiment, the pharmaceutically acceptable diluent is calcium chloride dihydrate and polysorbate 80. In another exemplary embodiment, the pharmaceutically acceptable diluent is mannitol.

The pharmaceutical compositions may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.,* polylactate polyglycolate) may also be employed as carriers for the pharmaceutical compositions of this invention. Suitable biodegradable microspheres are disclosed, for example, in U.S. Patent Nos. 4,897,268 and 5,075,109.

Commonly, the pharmaceutical compositions are administered parenterally, *e.g.,* intravenously. Thus, the invention provides compositions for parenteral administration that include the compound dissolved or suspended in an acceptable carrier, preferably an aqueous carrier, *e.g.,* water, buffered water, saline, PBS and the like. The compositions may contain pharmaceutically acceptable auxiliary substances as required to approximate physiological conditions, such as pH adjusting and buffering agents, tonicity adjusting agents, wetting agents, detergents and the like.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably from 5 to 9 and most preferably from 7 and 8.

In some embodiments the glycopeptides of the invention can be incorporated into liposomes formed from standard vesicle-forming lipids. A variety of methods are available for preparing liposomes, as described in, *e.g.,* Szoka et al., Ann. Rev. Biophys. Bioeng. 9: 467 (1980), U.S. Pat. Nos. 4,235,871, 4,501,728 and 4,837,028. The targeting of liposomes using a variety of targeting agents (*e.g.,* the sialyl galactosides of the invention) is well known in the art (*see, e.g.,* U.S. Patent Nos. 4,957,773 and 4,603,044).

Standard methods for coupling targeting agents to liposomes can be used. These methods generally involve incorporation into liposomes of lipid components, such as phosphatidylethanolamine, which can be activated for attachment of targeting agents, or derivatized lipophilic compounds, such as lipid-derivatized glycopeptides of the invention.

Targeting mechanisms generally require that the targeting agents be positioned on the surface of the liposome in such a manner that the target moieties are available for interaction with the target, for example, a cell surface receptor. The carbohydrates of the invention may be attached to a lipid molecule before the liposome is formed using methods known to those of skill in the art (*e.g.,* alkylation or acylation of a hydroxyl group present on the carbohydrate with a long chain alkyl halide or with a fatty acid, respectively). Alternatively, the liposome may be fashioned in such a way that a connector portion is first incorporated into the membrane at the time of forming the membrane. The connector portion must have a lipophilic portion, which is firmly embedded and anchored in the membrane. It must also have a reactive portion, which is chemically available on the aqueous surface of the liposome. The reactive portion is selected so that it will be chemically suitable to form a stable chemical bond with the targeting agent or carbohydrate, which is added later. In some cases it is possible to attach the target agent to the connector molecule directly, but in most instances it is more suitable to use a third molecule to act as a chemical bridge, thus linking the connector molecule which is in the membrane with the target agent or carbohydrate which is extended, three dimensionally, off of the vesicle surface.

The compounds prepared by the methods of the invention may also find use as diagnostic reagents. For example, labeled compounds can be used to locate areas of inflammation or tumor metastasis in a patient suspected of having an inflammation. For this use, the compounds can be labeled with ¹²⁵I, ¹⁴C, or tritium.

Preparative methods for species of use in preparing the compositions of the invention are generally set forth in various patent publications, e.g., US 20040137557; WO 04/083258; and WO 04/033651. The following examples are provided to illustrate the conjugates, and methods and of the present invention, but not to limit the claimed invention.

### EXAMPLES (not forming part of the invention)

### EXAMPLE 1

### Desialylation of Factor VIIa.

Factor VIIa which was expressed in serum-free media, Factor VIIa which was produced in serum containing media, plus three Factor VIIa mutants N145Q, N322Q, and analogue DVQ (V158D/E296V/M298Q).

In preparation for enzymatic desialylation, Factor VIIa was dialyzed into MES, 150 mM NaCl, 5 mM CaCl₂, 50mM MES, pH 6 overnight at 4°C in Snakeskin dialysis tubing with a MWCO of 10 kD. Desialylation of Factor VIIa (1 mg/mL) was performed with 10 U/L soluble sialidase from *Arthrobacter ureafaciens* (Calbiochem) at 32°C for 18 hours in the exchanged buffer.

### EXAMPLE 2

### Sialyl-PEGylation of Factor VIIa.

Sialyl-PEGylation ("GlycoPEGylation") was performed on asialo-Factor VIIa (1 mg/mL) with 100 U/L ST3Gal-III and 200 µM CMP-sialic acid-PEG (40 kD, 20 kD, 10 kD, 5 kD, and 2 kD) at 32°C in the desialylation buffer for 2-6 hours. After the proper reaction time had expired, the PEGylated sample was immediately purified to minimize further GlycoPEGylation.

To cap GlycoPEGylated Factor VII/Factor VIIa with samples capped with sialic acid, the sialidase was first removed from the asialo-Factor VIIa by anion-exchange chromatography as indicated below. Excess CMP-sialic acid (5 mM) was added and incubated at 32°C for 2 hours, capping GlycoPEGylated Factor VIIa with sialic acid. The sialyl-PEGylated forms of Factor VIIa were analyzed by non-reducing SDS-PAGE (Tris-glycine gels and/or NuPAGE gels) and a Colloidal Blue Staining Kit, as described by Invitrogen.

### EXAMPLE 3

### Purification of PEGylated Factor VIIa.

GlycoPEGylated samples of Factor VIIa were purified with a modified anion-exchange method. Samples were handled at 5°C. Immediately before loading the column, 1 g Chelex 100 (BioRad) per 10 mL Factor VIIa solution was added to the remodeled sample. After stirring for 10 min, the suspension was filtered on a cellulose acetate membrane (0.2 µm) with a vacuum system. The retained chelator resin on the filter was washed once with 1-2 mL water per 10 mL bulk. The conductivity of the filtrate was adjusted to 10 mS/cm at 5°C, and adjusted to pH 8.6, if necessary.

Anion exchange was performed at 8-10°C. A column containing Q Sepharose FF was prepared before loading by washing with 1 M NaOH (10 column volumes), water (5 column volumes), 2 M NaCl, 50 mM HOAc, pH 3 (10 column volumes), and equilibrating with 175 mM NaCl, 10 mM glycylglycine, pH 8.6 (10 column volumes). For each PEGylation reaction, 15-20 mg Factor VIIa was loaded on to an XK16 column (Amersham Biosciences) with 10 mL Q Sepharose FF (no more than 2 mg protein per mL resin) at a flow rate of 100 cm/h. For the 2 kD linear PEG, 20 mg Factor VIIa was loaded on to an XK26 column (Amersham Biosciences) with 40 mL Q Sepharose FF (0.5 mg protein per mg resin) at a flow rate of 100 cm/h.

After loading, the column was washed with 175 mM NaCl, 10 mM glycylglycine, pH 8.6 10 column volumes) and 50 mM NaCl, 10 mM glycylglycine, pH 8.6 (2 column volumes). Elution was performed with a step gradient of 15 mM CaCl₂ by using 50 mM NaCl, 10 mM glycylglycine, 15 mM CaCl₂, pH 8.6 (5 column volumes). The column was then washed with 1 M NaCl, 10 mM glycylglycine, pH 8.6 (5 column volumes). The effluent was monitored by absorbance at 280 nm. Fractions (5 mL) were collected during the flow-through and the two washes; 2.5 mL fractions were collected during the CaCl₂ and 1M salt elutions. Fractions containing Factor VIIa were analyzed by non-reducing SDS-PAGE (Tris-glycine gels and/or NUPAGE gels) and a Colloidal Blue Staining Kit. The appropriate fractions with Factor VIIa were pooled, and the pH was adjusted to 7.2 with 4 M HCl.

Factor VIIa-SA-PEG-10 kD was purified as described above, except for the following changes. EDTA (10 mM) was added to to the PEGylated Factor VIIa solution, the pH was adjusted to pH 6, and the conductivity was adjusted to 5mS/cm, at 5°C. About 20 mg of Factor VIIa-SA-PEG-10 kD was loaded on to an XK16 column (Amersham Biosciences) with 10 mL Poros 50 Micron HQ resin (no more than 2 mg protein per mL, resin) at a flow rate of 100 cm/h. After loading, the column was washed with 175 mM NaCl, 10 mM histidine pH 6 (10 column volumes) and 50 mM NaCl, 10 mM histidine, pH 6 (2 column volumes). Elution was performed with a step gradient of 20 mM CaCl₂ in 50 mM NaCl, 10 mM histidine, pH 6 (5 column volumes). The column was then washed with 1 M NaCl, 10 mM histidine, pH 6 (5 column volumes).

The anion-exchange eluate containing Factor VIIa-SA-PEG-10 kD (25mL) was concentrated to 5-7 mL by using an Amicon Ultra-15 10K centrifugal filter device, according to the manufacturer's directions (Millipore). Following concentration, size exclusion chromatography was performed. The sample (5-7 mL) was loaded onto a column containing Superdex 200 (HiLoad 16/60, prep grade; Amersham Biosciences) equilibrated in 50 mM NaCl, 10 mM glycylglycine, 15 mM CaCl₂, pH 7.2 for most of the PEGylated variants. Factor VIIa-SA-PEG-10 kD was separated from the unmodified, asialo-Factor VIIa at a flow rate of 1 mL/min, and the absorbance was monitored at 280 nm. Fractions (1 mL) containing Factor VIIa were collected and analyzed by non-reducing SDS-PAGE (Tris-glycine gels and/or NuPAGE gels) and a Colloidal Blue Staining Kit. Fractions containing the targeted PEGylated isoform and devoid of the unmodified, asialo-Factor VIIa were pooled and concentrated to 1 mg/mL using an Amicon Ultra-15 10K centrifugal filter device. Protein concentration was determined from absorbance readings at 280 nm using an extinction coefficient of 1.37 (mg/mL)⁻¹cm⁻¹.

### EXAMPLE 4

### Determination of PEGylated Isoforms by Reversed phase HPLC analysis.

PEGylated Factor VIIa was analyzed by HPLC on a reversed-phase column (Zorbax 300SB-C3, 5 µm particle size, 2.1 x 150 mm). The eluants were A) 0.1 TFA in water and B) 0.09 % TFA in acetonitrile. Detection was at 214 nm. The gradient, flow rate, and column temperature depended on the PEG length (40 kD, 20 kD, and 10 kD PEG: 35-65 %B in 30 min, 0.5 mL/min, 45°C; 10 kD PEG: 35-60 %B in 30 min, 0.5 mL/min, 45°C; 5 kD: 40-50 %B in 40 min, 0.5 mL/min, 45°C; 2 kD: 38-43 %B in 67 min, 0.6 mL/min, 55°C). The identity of each peak was assigned based on two or more of four different pieces of evidence: the known retention time of native Factor VIIa, the SDS-PAGE migration of the isolated peak, the MALDI-TOF mass spectrum of the isolated peak, and the orderly progression of the retention time of each peak with increasing number of attached PEG.

### EXAMPLE 5

### Determination of Site of PEG Attachment by Reversed-phase HPLC.

Factor VIIa and PEGylated Factor VIIa variants were reduced by mixing sample (10 µL at a concentration of 1 mg/mL) with reducing buffer (40 µL, 50 mM NaCl, 10 mM glycylglycine, 15 mM EDTA, 8 M urea, 20 mM DTT, pH 8.6) for 15 min at room temperature. Water (50 µL) was added and the sample cooled to 4°C until injected on the HPLC ( < 12 hrs). The HPLC column, eluants, and detection were as described above for non-reduced samples. The flow rate was 0.5 mL/min and the gradient was 30-55 %B in 90 min, followed by a brief wash cycle up to 90 %B. The identity of each peak was assigned as described in Example 4.

### EXAMPLE 6

### Factor VIIa Clotting Assay.

PEGylated samples and standards were tested in duplicate, and were diluted in 100mM NaCl, 5mM CaCl₂. 0.1% BSA (wt/vol), 50mM Tris, pH 7.4. The standard and samples were assayed over a range from 0.1 to 10 ng/mL. Equal volumes of diluted standards and samples were mixed with Factor VIIa deficient plasma (Diagnostica Stago), and stored on ice for no greater than 4 hours before they were assayed.

Clotting times were measured with a STart4 coagulometer (Diagnostica Stago). The coagulometer measured the time elapsed until an *in vitro* clot was formed, as indicated by the stopping of the gentle back-and-forth movement of a magnetic ball in a sample cuvette.

Into each cuvette, one magnetic ball was deposited, plus 100 µL Factor VIIa sample/deficient plasma and 100 µL of a diluted rat brain cephalin solution (stored on ice for no greater than 4 hours). Each reagent was added with 5 seconds between each well, and the final mixture was incubated for 300 seconds at 37°C. Diluted rat brain cephalin (RBC) solution was made from 2 mL RBC stock solution (1 vial RBC stock, from Haemachem, plus 10 mL 150mM NaCl) and 4 mL 100mM NaCl, 5mM CaCl₂, 0.1% BSA (wt/vol), 50mM Tris, pH 7.4.

At 300 seconds, the assay was started by the addition of 100 µL of a preheated (37°C) solution of soluble tissue factor (2µg/mL; amino acids 1-209) in 100mM NaCl. 12.5mM CaCl₂, 0.1% BSA (wt/vol), 50mM Tris, pH 7.4. Again, this next solution was added with a 5 second interval between samples.

The clotting times from the diluted standards were used to generate a standard curve (log clot time versus log Factor VIIa concentration). The resulting linear regression from the curve was used to determine the relative clotting activities of PEGylated variants. PEGylated Factor VIIa variants were compared against an aliquotted stock of Factor VIIa.

### EXAMPLE 7

### GlycoPEGylation of Recombinant Factor VIIa produced in BHK cells

This example sets forth the PEGylation of recombinant Factor VIIa made in BHK cells.

*Preparation of Asialo-Factor VIIa.* Recombinant Factor VIIa was produced in BHK cells (baby hamster kidney cells). Factor VIIa (14.2 mg) was dissolved at 1 mg/mL in buffer solution (pH 7.4, 0.05 M Tris, 0.15 M NaCl, 0.001 M CaCl₂, 0.05% NaN₃) and was incubated with 300 mU/mL sialidase (*Vibrio cholera*)*-agarose* conjugate for 3 days at 32 °C. To monitor the reaction a small aliquot of the reaction was diluted with the appropriate buffer and an IEF gel performed according to Invitrogen procedures (Figure 157). The mixture was centrifuged at 3,500 rpm and the supernatant was collected. The resin was washed three times (3×2 mL) with the above buffer solution (pH 7.4, 0.05 M Tris, 0.15 M NaCl, 0.05% NaN₃) and the combined washes were concentrated in a Centricon-Plus-20. The remaining solution was buffer exchanged with 0.05 M Tris (pH 7.4), 0.15 M NaCl, 0.05% NaN₃ to a final volume of 14.4 mL.

*Preparation of Factor VIIa-SA-PEG-1kD and Factor VIIa-SA-PEG-10 kD.* The desialylation of Factor VIIa solution was split into two equal 7.2 mL samples. To each sample was added either CMP-SA-PEG-1 kD (7.4 mg) or CMP-SA-PEG-10 kD (7.4 mg). ST3Gal3 (1.58U) was added to both tubes and the reaction mixtures were incubated at 32°C for 96 hrs. The reaction was monitored by SDS-PAGE gel using reagents and conditions described by Invitrogen. When the reaction was complete, the reaction mixture was purified using a Toso Haas TSK-Gel-3000 preparative column using PBS buffer (pH 7.1) and collecting fractions based on UV absorption. The combined fractions containing the product were concentrated at 4°C in Centricon-Plus-20 centrifugal filters (Millipore, Bedford, MA) and the concentrated solution reformulated to yield 1.97 mg (bicinchoninic acid protein assay, BCA assay, Sigma-Aldrich, St. Louis MO) of Factor VIIa-SA-PEG. The product of the reaction was analyzed using SDS-PAGE and IEF analysis according to the procedures and reagents supplied by Invitrogen. Samples were dialyzed against water and analyzed by MALDI-TOF.

### EXAMPLE 8

### Factor VIIa-SA-PEG-10kD: One Pot Method

Factor VIIa (5 mg diluted in the product formulation buffer to a final concentration of 1 mg/mL), CMP-SA-PEG-10 kD (10mM 60 µL) and *A. niger* enzyme ST3Gal3 (33 U/L) and 10 mM histidine, 50 mM NaCl, 20 mM CaCl₂ were combined in a reaction vessel along with either 10 U/L, 1 U/L, 0.5 U/L or 0.1 U/L of sialidase (CalBiochem). The ingredients were mixed and incubated at 32°C. Reaction progress was measured by analyzing aliquots at 30 minute intervals for the first four hours. An aliquot was then removed at the 20 hour timepoint and subjected to SDS-PAGE. Extent of PEGylation was determined by removing 1 mL at 1.5, 2.5 and 3.5 hour timepoint and purifying the sample on a Poros 50HQ column.

For the reaction conditions containing 10 U/L of sialidase, no appreciable amount of Factor VIIa-SA-PEG product was formed. For the reaction conditions containing 1 U/L of sialidase, about 17.6 % of the Factor VIIa in the reaction mixture was either mono or diPEGylated after 1.5 hours. This increased to 29% after 2.5 hours, and 40.3% after 3.5 hours. For the reaction conditions containing 0.5 U/L of sialidase, about 44.5 % of the Factor VIIa in the reaction mixture was either mono or diPEGylated after 3 hours, and 0.8% was triPEGylated or greater. After 20 hours, 69.4% was either mono or diPEGylated, and 18.3% was triPEGylated or greater.

For the reaction conditions containing 0.1 U/L of sialidase, about 29.6% of the Factor VIIa in the reaction mixture was either mono or diPEGylated after 3 hours. After 20 hours, 71.3% was either mono or diPEGylated, and 15.1% was triPEGylated or greater.

### EXAMPLE 9

### Preparation of Cysteine-PEG₂ (2)

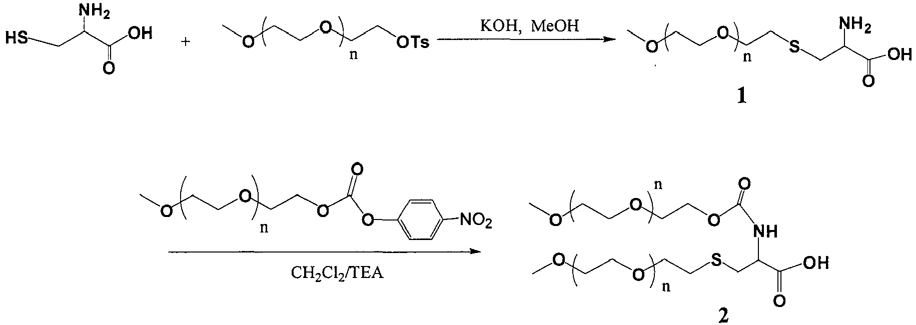

### a. Synthesis of Compound 1

Potassium hydroxide (84.2 mg, 1.5 mmol, as a powder) was added to a solution of L-cysteine (93.7mg, 0.75 mmol) in anhydrous methanol (20 L) under argon. The mixture was stirred at room temperature for 30 min, and then mPEG-O-tosylate of molecular mass 20 kilodalton (Ts; 1.0 g, 0.05 mmol) was added in several portions over 2 hours. The mixture was stirred at room temperature for 5 days, and concentrated by rotary evaporation. The residue was diluted with water (30 mL), and stirred at room temperature for 2 hours to destroy any excess 20 kilodalton mPEG-O-tosylate. The solution was then neutralized with acetic acid, the pH adjusted to pH 5.0 and loaded onto a reverse phase chromatography (C-18 silica) column. The column was eluted with a gradient of methanol/water (the product elutes at about 70% methanol), product elution monitored by evaporative light scattering, and the appropriate fractions collected and diluted with water (500 mL). This solution was chromatographed (ion exchange, XK 50 Q, BIG Beads, 300 mL, hydroxide form; gradient of water to water/acetic acid-0.75N) and the pH of the appropriate fractions lowered to 6.0 with acetic acid. This solution was then captured on a reversed phase column (C-18 silica) and eluted with a gradient of methanol/water as described above. The product fractions were pooled, concentrated, redissolved in water and freeze-dried to afford 453 mg (44%) of a white solid (**1**).

Structural data for the compound were as follows: ¹H-NMR (500 MHz; D₂O) δ 2.83 (t, 2H, O-C-CH₂-S), 3.05 (q, 1H, S-CHH-CHN), 3.18 (q, 1H, (q, 1H, S-CHH-CHN), 3.38 (s, 3H, CH₃O), 3.7 (t, OCH₂CH₂O), 3.95 (q, 1H, CHN). The purity of the product was confirmed by SDS PAGE.

### b. Synthesis of Cysteine-PEG₂ (2)

Triethylamine (∼0.5 mL) was added dropwise to a solution of compound 1 (440 mg, 22 µmol) dissolved in anhydrous CH₂Cl₂ (30 mL) until the solution was basic. A solution of 20 kilodalton mPEG-O-p-nitrophenyl carbonate (660 mg, 33 µmol) and N-hydroxysuccinimide (3.6 mg, 30.8 µmol) in CH₂Cl₂ (20 mL) was added in several portions over 1 hour at room temperature. The reaction mixture was stirred at room temperature for 24 hours. The solvent was then removed by rotary evaporation, the residue was dissolved in water (100 mL), and the pH adjusted to 9.5 with 1.0 N NaOH. The basic solution was stirred at room temperature for 2 hours and was then neutralized with acetic acid to a pH 7.0. The solution was then loaded onto a reversed phase chromatography (C-18 silica) column. The column was eluted with a gradient of methanol/water (the product elutes at about 70% methanol), product elution monitored by evaporative light scattering, and the appropriate fractions collected and diluted with water (500 mL). This solution was chromatographed (ion exchange, XK 50 Q, BIG Beads, 300 mL, hydroxide form; gradient of water to water/acetic acid-0.75N) and the pH of the appropriate fractions lowered to 6.0 with acetic acid. This solution was then captured on a reversed phase column (C-18 silica) and eluted with a gradient of methanol/water as described above. The product fractions were pooled, concentrated, redissolved in water and freeze-dried to afford 575 mg (70 %) of a white solid **(2).**

Structural data for the compound were as follows: ¹H-NMR (500 MHz; D₂O) δ 2.83 (t, 2H, O-C-CH₂-S), 2.95 (t, 2H, O-C-CH₂-S), 3.12 (q, 1H, S-CHH-CHN), 3.39 (s, 3H CH₃O), 3.71 (t, OCH₂CH₂O). The purity of the product was confirmed by SDS PAGE.

### EXAMPLE 10

### Factor VIIa-SA-PEG-40kD

*GlycoPEGylation of Factor VIIa (One Pot with Capping).* GlycoPEGylation of Factor VIIa was accomplished in a one-pot reaction where desialation and PEGylation occur simultaneously, followed by capping with sialic acid. The reaction was performed in a jacketed glass vessel controlled at 32°C by a recirculating waterbath. First, the concentrated 0.2µm-filtered Factor VIIa was introduced into the vessel and heated to 32°C by mixing with a stir bar for 20 minutes. A solution of sialidase was made from dry powder in 10mM histidine/50mM NaCl/20mM CaCl₂, pH 6.0 at a concentration of 4,000 U/L. Once the Factor VIIa reached 32°C, the sialidase was added to the Factor VIIa, and the reaction was mixed for approximately 5 minutes to ensure a uniform solution after time which the mixing was stopped. The desialation was allowed to proceed for 1.0 h at 32°C. During the desialation reaction, the CMP-SA-PEG-40 kD was dissolved into 10mM histidine/50mM NaCl/20mM CaCl₂, pH 6.0 buffer, and the concentration of was determined by UV absorbance at 271nm. After the CMP-SA-PEG-40 kD was dissolved, the CMP-SA-PEG-40 kD was added to the reaction, as well as the ST3Gal3, and the reaction was mixed for approximately 15 minutes with a stir bar to ensure a uniform solution. An additional volume of 85mL of buffer was added to make the reaction 1.0 L. The reaction was allowed to proceed without stirring for 24 hours before CMP-SA was added to a concentration of 4.3 mM to quench the reaction and cap the remaining terminal galactose residues with sialic acid. The quenching was allowed to proceed with mixing for 30 minutes at 32°C. The total volume of the reaction was 1.0 L before quenching. Timepoint samples (1 mL) were taken at 0, 4.5, 7.5, and 24 h, quenched with CMP-SA, and analyzed by RP-HPLC and SDS-PAGE.

*Purification of Factor VIIa-SA-PEG-40kD.* After capping, the solution was diluted with 2.0 L of 10mM histidine, pH 6.0 that had been stored overnight at 4 °C and the sample was filtered through a 0.2µm Millipak 60 filter. The resulting load volume was 3.1 L. The AEX2 chromatography was performed at 20-25°C (ambient room temperature) on an Akta Pilot system. After loading, a 10 column volumes wash with equilibration buffer was performed, and the product was eluted from the column using a 10 column volume gradient of MgCl₂ which resulted in resolution of PEGylated-Factor VIIa species from unPEGylated Factor VIIa. The loading for this column was intentionally kept low, targeting < 2 mg Factor VIIa/mL resin. SDS-PAGE gels were run in addition to RP-HPLC analysis of selected fractions and pools of fractions in order to make the pool of bulk product. Pooled fractions were pH adjusted to 6.0 with 1M NaOH and stored in the cold room at 2-8°C overnight.

*Final Concentration*/*Diafiltration, aseptic filtration and aliquoting.* The pooled fractions were filtered through a Millipak 20 0.2µm filter and stored overnight at 2-8°C. To perform the concentration/diafiltration, a Millipore 0.1m² 30 kD regenerated cellulose membrane was used in a system fitted with a peristaltic pump and silicone tubing. The system was assembled and flushed with water, then sanitized with 0.1M NaOH for at least 1 hour, and then stored in 0.1M NaOH until equilibration with 10 mM histidine/ 5 mM CaCl₂/ 100 mM NaCl pH 6.0 diafiltration buffer immediately before use. The product was concentrated to approximately 400 mL and then diafiltered at constant volume with approximately 5 diavolumes of buffer. The product was then concentrated to approximately 300mL and recovered after a low pressure recirculation for 5 minutes, and the membranes were rinsed with 200 mL of diafiltration buffer by a recirculation for 5 minutes. The wash was recovered with product, and another 50mL of buffer was recirculated for another 5 minutes for a final wash. The resulting bulk was approximately 510 mL, and that was filtered through a 1L vacuum filter fitted with a 0.2µm PES membrane (Millipore). The aseptically-filtered bulk was then aliquoted into 25mL aliquots in 50mL sterile falcon tubes and frozen at -80°C.

**Analysis of the PEGylation reaction by HPLC (Example 10)**

| | Conjugation Reaction Time | | | | Purification |
|---|---|---|---|---|---|
| | 0 hrs | 4.5 hrs | 7.5 hrs | 24 hrs | After Chromatography |
| % Unpegylated | 94.7 | 76.1 | 66.6 | 51.0 | 0.6 |
| % Monopegylated | 0.9 | 17.9 | 26.1 | 39.1 | 85.6 |
| % Dipegylated | 0.1 | 0.9 | 1.9 | 5.1 | 5.1 |
| % Tripegylated | 0.0 | 0.0 | 0.0 | 0.2 | 0.2 |

After 24 hours, the bulk product PEG-state distribution was: 0.7% unpegylated, 85.3% mono-pegylated, 11.5% di-pegylated, and 0.3% tri-pegylated. Column chromatography is the main step in the process that generates the product distribution, largely through removing unpegylated material from mono- and di-pegylated species.

It is understood that the examples and embodiments described herein are for illustrative purposes only and that various modifications or changes in light thereof will be suggested to persons skilled in the art and are to be included within the scope of the appended claims.

## Claims

1. A peptide conjugate comprising:
a) a peptide which is Factor IX and which is covalently attached to a moiety which is a member selected from: 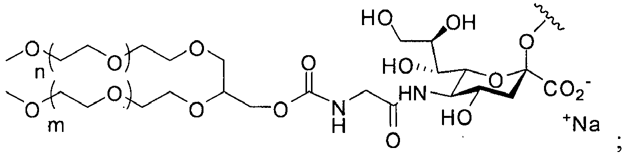 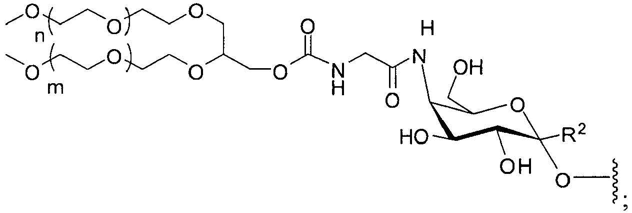 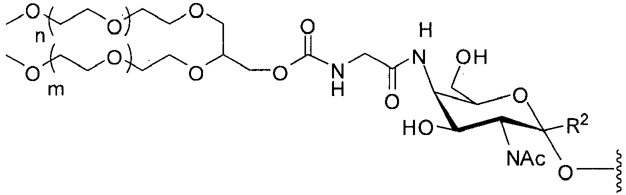 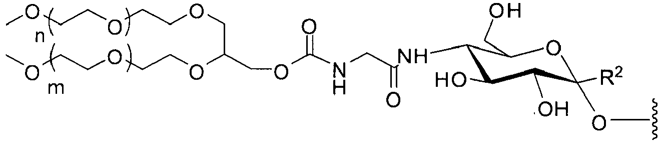 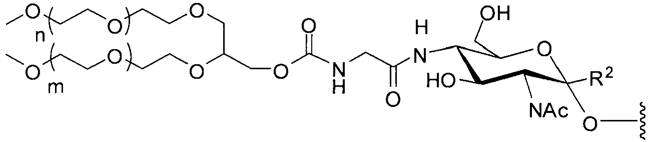 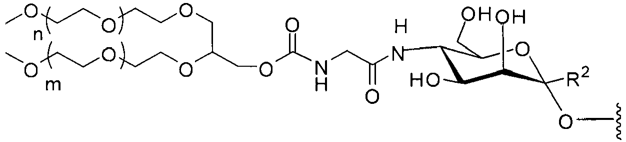 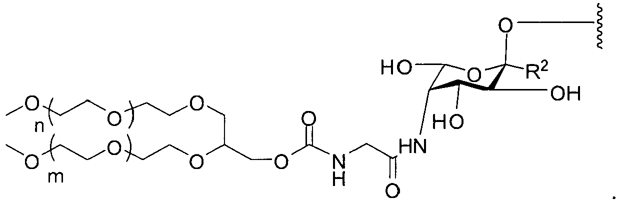 wherein the indices m and n are integers independently selected from 100 to 4000, wherein R² is a member selected from H, CH₂OR⁷, COOR⁷, and OR⁷, wherein R⁷ is a member selected from H, substituted or unsubstituted alkyl, and substituted or unsubstituted heteroalkyl, or wherein said moiety is: 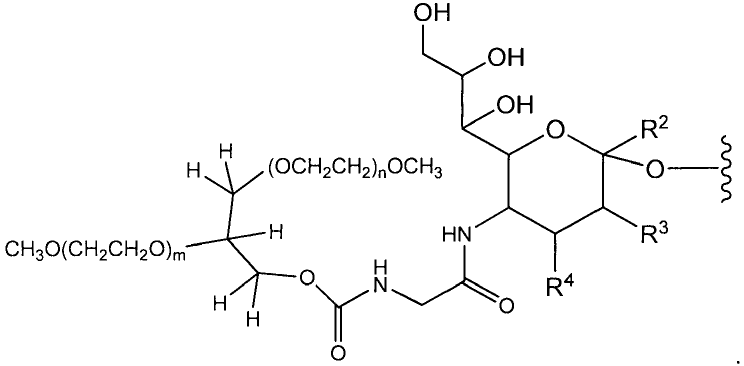 wherein m, n and R² are as defined above, and wherein R³ and R⁴ are members independently selected from H, substituted or unsubstituted alkyl, OR⁸ and NHC(O)R⁹, and wherein R⁸ and R⁹ are independently selected from H, substituted or unsubstituted alkyl, substituted or unsubstituted heteroalkyl, sialic acid and polysialic acid.

2. A pharmaceutical formulation comprising a peptide conjugate according to claim 1 and a pharmaceutically acceptable diluent.

## Patentansprüche

1. Peptidkonjugat, umfassend:
a) Peptid, das Faktor IX ist und das kovalent an einen Rest gebunden ist, der ein Mitglied ist, ausgewählt aus: 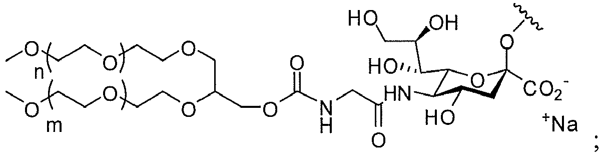 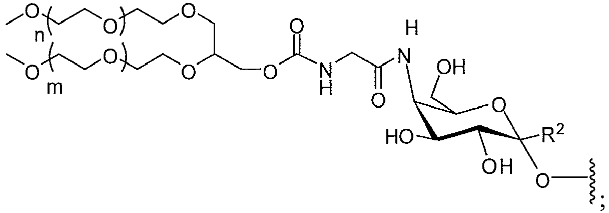 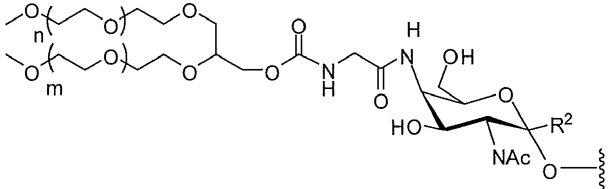 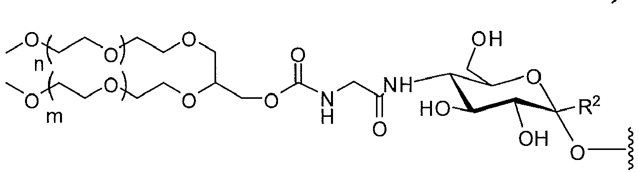 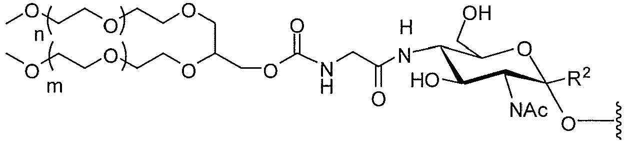 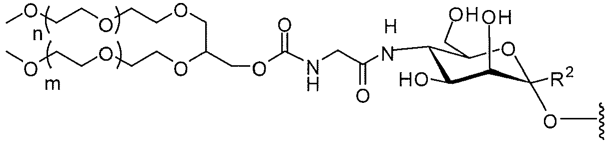 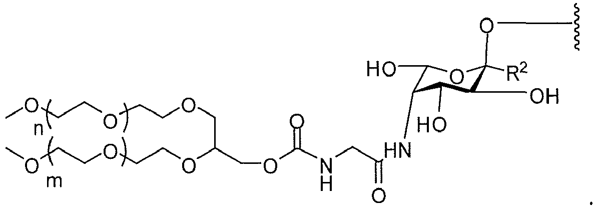 wobei die Indizes m und n ganze Zahlen sind, unabhängig ausgewählt aus 100 bis 4000, wobei R² ein Mitglied ist, ausgewählt aus H, CH₂OR⁷, COOR⁷, und OR⁷, wobei R⁷ ein Mitglied ist, ausgewählt aus H, substituiertem oder unsubstituiertem Alkyl, und substituiertem oder unsubstituiertem Heteroalkyl, oder wobei der Rest ist: 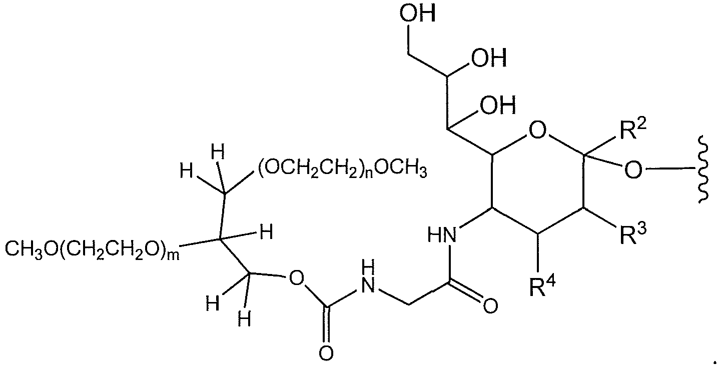 wobei m, n und R² wie oben definiert sind, und wobei R³ und R⁴ unabhängig ausgewählte Mitglieder sind aus H, substituiertem oder unsubstituiertem Alkyl, OR⁸ und NHC(O)R⁹, und wobei R⁸ und R⁹ unabhängig ausgewählt sind aus H, substituiertem oder unsubstituiertem Alkyl, substituiertem oder unsubstituiertem Heteroalkyl, Sialinsäure und Polysialinsäure.

2. Pharmazeutische Formulierung, umfassend ein Peptidkonjugat nach Anspruch 1 und ein pharmazeutisch akzeptables Verdünnungsmittel.

## Revendications

1. Conjugué peptidique comprenant :
a) un peptide qui est le Facteur IX et qui est lié par covalence à un groupement qui est un élément choisi parmi : 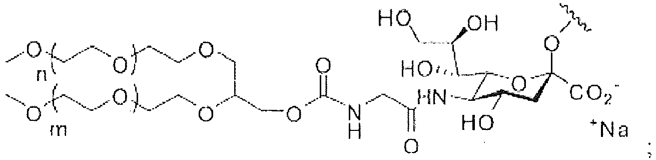 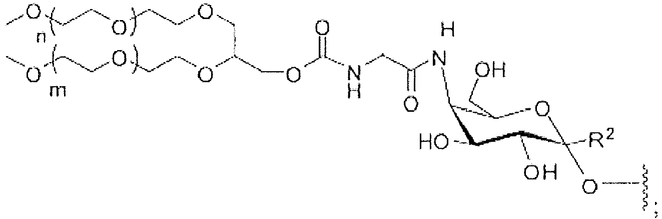 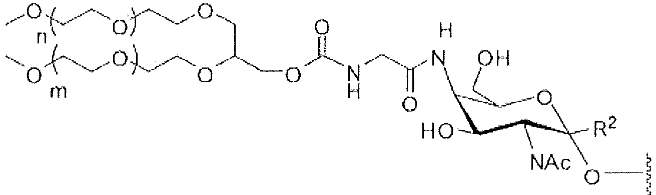 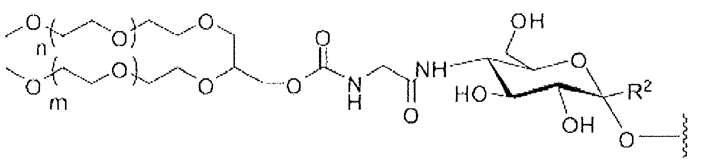 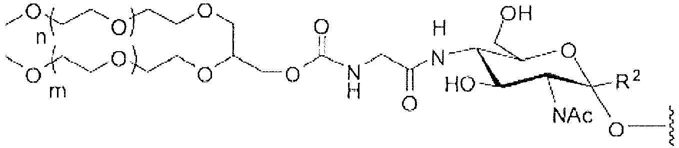 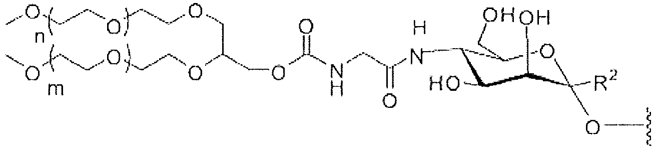 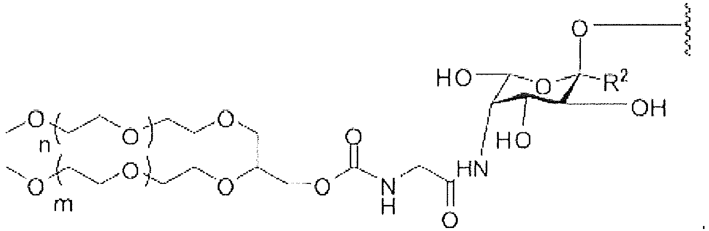 dans lequel les indices m et n sont des nombres entiers indépendamment choisis parmi 100 à 4 000, dans lequel R² est un élément choisi parmi un H, un CH₂OR⁷, un COOR⁷ et un OR⁷, dans lequel R⁷ est un élément choisi parmi un H, un alkyle substitué ou non substitué et un hétéroalkyle substitué ou non substitué, ou dans lequel ledit groupement est : 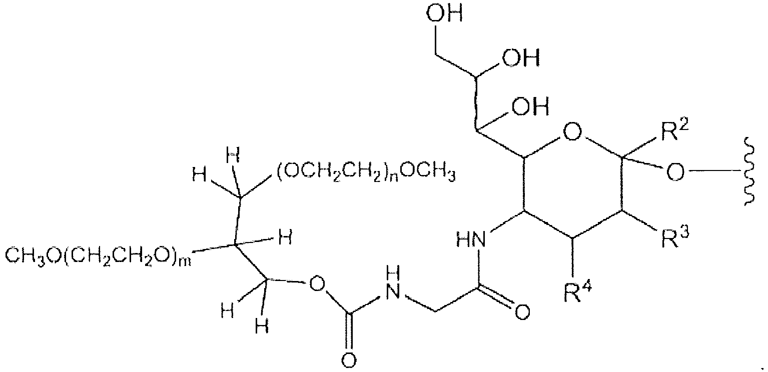 dans lequel m, n et R² sont tels que définis ci-dessus, et dans lequel R³ et R⁴ sont des éléments indépendamment choisis parmi un H, un alkyle substitué ou non substitué, un OR⁸ et un NHC(O)R⁹, et dans lequel R⁸ et R⁹ sont indépendamment choisis parmi un H, un alkyle substitué ou non substitué, un hétéroalkyle substitué ou non substitué, un acide sialique et un acide polysialique.

2. Formulation pharmaceutique comprenant un conjugué peptidique selon la revendication 1 et un diluant pharmaceutiquement acceptable.
